# EUROPEAN PATENT APPLICATION

(11) **EP 1 201 661 A1**
(43) Date of publication of application: **02.05.2002**
(21) Application number: 00944389.6
(22) Date of filing: 10.07.2000
(51) Int. Cl.: C07D 275/03

(54) **HETEROAROMATIC RING COMPOUNDS**

(30) Priority: 15.07.1999 JP 20144799; 03.03.2000 JP 2000058217
(71) Applicant: Sumitomo Pharmaceuticals Company, Limited, Osaka 541-8510 (JP)
(72) Inventor: NAKATSUKA, Masashi, Mishima-gun, Osaka 618-0001 (JP); NAKATANI, Shogo, Nishinomiya-shi, Hyogo 662-0831 (JP); OKADA, Shin-ichiro, Takatsuki-shi, Osaka 569-1118 (JP); TSUBOI, Katsunori, Nishinomiya-shi, Hyogo 662-0831 (JP); NISHIKAKU, Fumio, Itami-shi, Hyogo 664-0897 (JP)
(74) Representative: Cresswell, Thomas Anthony
(86) International application number: JP0004616
(87) International publication number: WO0105774

(57) **Abstract**

Heteroaromatic ring compounds or pharmaceutically acceptable salts thereof, which have excellent characteristics and have strong curative effects to immuno-imbalance and choronic inflammation. Representative is the compound of the formula:

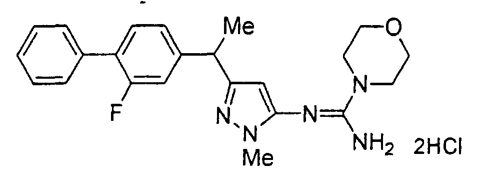

## Description

### Technical Field

The present invention relates to heteroaromatic ring compounds which have excellent characteristics and are useful as therapeutic drugs for autoimmune disease, inflammatory disease, etc.

### Background

Acidic nonsteroidal anti-inflammatory drugs or steroidal drugs have been used as therapeutic drugs for inflammatory diseases but are limited in their side effects. In addition, treatments using such drugs, despite their ability to ameliorate symptoms cannot remove the fundamental cause of the diseases. With the progress of elucidation of the pathophysiology of autoimmune diseases such as rheumatoid arthritis accompanied by serious inflammation, it has been suggested that an immune system disorders are deeply concerned in the onset of inflammation, its progression and maintenance of a chronic state. For these reasons, drugs capable of modifying the diseases by acting on the immune system, such as gold compounds and D-penicillamine have been noted as drugs for causal treatment. They, however, are not always satisfactory because of their side effects and deficiency in lasting efficacy. In these circumstances, WO 98/47880 reports isoxazole derivatives having a guanizino group, which are useful as a therapeutic or prophylactic drug for autoimmune diseases, inflammatory diseases, etc. and have excellent immunomodulating and anti-choronic-inflammatory effects and have little side effect. However, compounds with more excellent pharmacological activities and characteristics have been desired.

### Description of the Invention

The present invention is intended to provide compounds which have excellent characteristics and are useful as therapeutic drugs for autoimmune disease, inflammatory disease, etc.

The present invention relates to the following inventions:
**1.** A heteroaromatic ring compound represented by the following formula, or a pharmaceutically acceptable salt thereof: 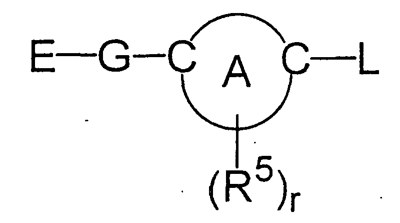 wherein E is a group of the formula: 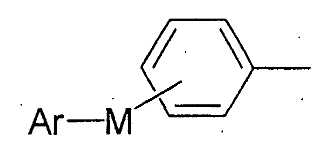 wherein Ar is benzene, furan, thiophene or pyridine; and M is single bond, -O-, -S-, -SO-, -SO₂-, -CQ-, -CH(OR¹¹)-, -C(OR¹¹)₂-, -C(=NOR¹¹)-, -C(=NR¹²)-, -C(=NNR¹³R¹⁴)-, -CO- or -CS-,
   wherein -CQ- is 1,3-dioxane ring or 1,3-dioxolane ring; R¹¹ is hydrogen atom or a lower alkyl group; R¹² is hydrogen atom or a lower alkyl group; R¹³ and R¹⁴ are independently hydrogen atom, a lower alkyl group, or a substituted or unsubstituted aryl group;
   or E is a group of the formula: 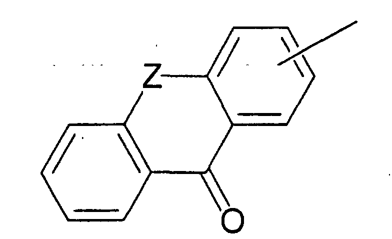 wherein Z is single bond, -O-, -S-, -SO- or -SO₂-;
   or E is a group of the formula: 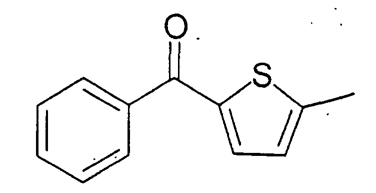 wherein said E may be substituted by one to four members optionally selected from the group consisting of halogen atoms, lower alkyl groups, nitro group, formyl group, acetyl group, cyano group, -OR¹¹, -CO₂R²⁹ and -CONR³⁰R³¹,
   wherein R¹¹ is as defined above; R²⁹ is a lower alkyl group; R³⁰ and R³¹ are independently hydrogen atom or a lower alkyl group;
   G is -C(R⁶R⁷)- or -C(=CR⁶R⁷)- and is connected with the carbon atom of the ring A,
   wherein R⁶ and R⁷ are independently hydrogen atom, a lower alkyl group or a lower alkoxy group; or R⁶ and R⁷ may be taken together with the carbon atom attached thereto to form a substituted or unsubstituted hydrocarbon ring, a substituted or unsubstituted 1,3-dioxane ring, or a substituted or unsubstituted 1,3-dioxolane ring;
   A is pyrrole, furan, thiophene, isothiazole, 1,3,4-oxadiazole, 1,3,4-thiadiazole, 1,2,4-thiadiazole, pyrazole, 1,2,4-triazole, pyridine, pyrazine, pyrimidine, pyridazine or 1,3,5-triazine;
   R⁵ is a substituent connected with a carbon atom or a nitrogen atom of the ring A, and r is an integer of 0 to 3;
   when R⁵ is a substitutent connected with a carbon atom of pyrrole, furan, thiophene, pyrazole, isothiazole, pyridine, pyrazine, pyrimidine, pyridazine or 1,3,5-triazine, R⁵ is a halogen atom, hydroxy group, nitro group, cyano group, carboxy group, a substituted or unsubstituted amino group, a substituted or unsubstituted hydroxyamino group, a substituted or unsubstituted carbamoyl group, a substituted or unsubstituted sulfamoyl group, -R⁸, -OR⁸, -CO₂R⁹, -SR¹⁰, -SO-R¹⁰, -SO₂-R¹⁰, -C(O)SR¹⁰, -C(S)OR¹⁰ or -CS₂R¹⁰,
   wherein R⁸ is a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, a substituted or unsubstituted aryl group, a substituted or unsubstituted aralkyl group, a substituted or unsubstituted heterocyclic group or an acyl group;
   R⁹ is a substituted or unsubstituted alkyl group, a substituted or unsubstituted alkenyl group, a substituted or unsubstituted alkynyl group, a substituted or unsubstituted aryl group, a substituted or unsubstituted aralkyl group, or a substituted or unsubstituted heterocyclic group; and
   R¹⁰ is a substituted or unsubstituted alkyl group, a substituted or unsubstituted aryl group, or a substituted or unsubstituted aralkyl group;
   when R⁵ is a substitutent connected with a nitrogen atom of pyrrole, pyrazole or 1,2,4-triazole, R⁵ is nitro group, cyano group, a substituted or unsubstituted carbamoyl group, a substituted or unsubstituted sulfamoyl group, a protective group for NH group, -R⁸, -OR⁸ or -CO₂R⁹,
   wherein R⁸ and R⁹ are as defined above;
   L is a group of the following formula, which is connected with a carbon atom of the ring A: 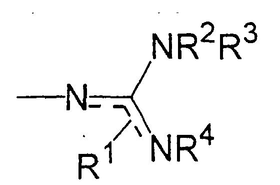 wherein one of the two broken lines is a double bond together with the solid line, while the other is a single bond together with the other solid line;
   R¹ is bonded to the nitrogen atom bonded through the single bond represented by the broken line and the solid line; and
   R¹, R², R³ and R⁴ are independently hydrogen atom, hydroxy group, nitro group, cyano group, a substituted or unsubstituted amino group, a substituted or unsubstituted hydroxyamino group, a substituted or unsubstituted carbamoyl group, a substituted or unsubstituted sulfamoyl group, a protecting group for NH group, -R⁸, -OR⁸, -CO₂R⁹, -SR¹⁰, -SO-R¹⁰, -SO₂-R¹⁰, -C(O)SR¹⁰, -C(S)OR¹⁰ or -CS₂R¹⁰, wherein R⁸, R⁹ and R¹⁰ are as defined above; or
   any two of R¹, R², R³ and R⁴ may be taken together with one nitrogen atom or with two nitrogen atoms and one carbon atom to form a substituted or unsubstituted nitrogen-containing aliphatic heterocyclic ring ; or
   any three of R¹, R², R³ and R⁴ may be taken together with two nitrogen atoms and one carbon atom to form a substituted or unsubstituted bicyclic nitrogen-containing aliphatic heterocyclic ring; or
   the formula: -NR²R³ may be a group of the formula:

   -N=C(NR⁴³R⁴⁴)NH₂ or -NH-C(NR⁴³R⁴⁴)=NH,

   wherein R⁴³ and R⁴⁴ are as defined in (1) or (2),
   (1) each is independently hydrogen atom, an acyl group, a substituted or unsubstituted alkyl group, or a protecting group for NH group,
   (2) when taken together, they form with the nitrogen atom a substituted or unsbustituted 5- to 7-membered nitrogen-containing aliphatic heterocyclic group.
**2.** A heteroaromatic ring compound or a pharmaceutically acceptable salt thereof according to **1.**, wherein E is a group of the formula: 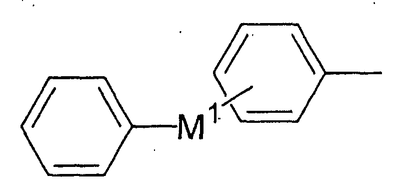 wherein M¹ is single bond, -CQ-, -CH(OR¹¹)-, -C(OR¹¹)₂-, -C(=NOR¹¹)-, -C(=NR¹²)-, -C(=NNR¹³R¹⁴)-, -CO- or -CS-, wherein Q, R¹¹, R¹², R¹³ and R¹⁴ are as defined above;
   wherein said E may be substituted by one to four members optionally selected from the group consisting of halogen atoms, lower alkyl groups, nitro group, formyl group, acetyl group, cyano group, -OR¹¹, -CO₂R²⁹ and -CONR³⁰R³¹, wherein R¹¹, R²⁹, R³⁰ and R³¹ are as defined above.
**3.** A heteroaromatic ring compound or a pharmaceutically acceptable salt thereof according to **1.** or **2.**, wherein E is a group of the formula: 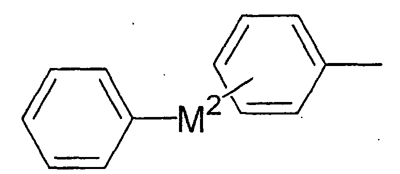 wherein M² is single bond or -CO-;
   wherein said E may be substituted by one or two members optionally selected from the group consisting of halogen atoms, lower alkyl groups, acetyl group, cyano group and -OR¹¹, wherein R¹¹ is as defined above.
**4.** A heteroaromatic ring compound or a pharmaceutically acceptable salt thereof according to any one of **1.** to **3.**, wherein E is biphenyl-4-yl, 2-fluorobiphenyl-4-yl, 2'-fluorobiphenyl-4-yl, 3-benzoylphenyl or 4-benzoylphenyl.
**5.** A heteroaromatic ring compound or a pharmaceutically acceptable salt thereof according to any one of **1.** to **4.**, wherein G is -C(R²³R²⁴)- wherein R²³ and R²⁴ are independently hydrogen atom, a lower alkyl group or a lower alkoxy group; or R²³ and R²⁴ may be taken together with the carbon atom attached thereto to form a hydrocarbon ring of 3 to 6 carbon atoms, 1,3-dioxane ring, or 1,3-dioxolane ring.
**6.** A heteroaromatic ring compound or a pharmaceutically acceptable salt thereof according to any one of **1.** to **5.**, wherein G is -C(R⁴⁹R⁵⁰)- wherein R⁴⁹ and R⁵⁰ are independently hydrogen atom or methyl group; or R⁴⁹ and R⁵⁰ may be taken together with the carbon atom to form cyclopropane.
**7.** A heteroaromatic ring compound or a pharmaceutically acceptable salt thereof according to any one of **1.** to **6.**, wherein A is pyrrole, furan, thiophene, isothiazole, 1,3,4-oxadiazole, 1,3,4-thiadiazole, 1,2,4-thiadiazole, pyrazole, 1,2,4-triazole, pyridine, pyrazine, pyrimidine, pyridazine or 1,3,5-triazine.
8. A heteroaromatic ring compound or a pharmaceutically acceptable salt thereof according to any one of **1.** to **7.**, wherein A is a group of the formulae: 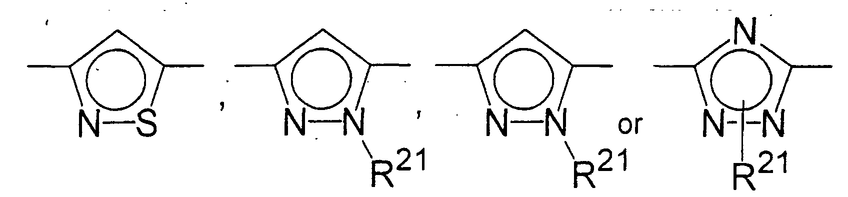 wherein R²¹ is a substituent connected with a nitrogen atom and is hydrogen atom, a lower alkyl group or acetyl group.
**9.** A heteroaromatic ring compound or a pharmaceutically acceptable salt thereof according to any one of **1**. to **8.**, wherein L is a group selected from the groups of the formulae [1] to [6]: 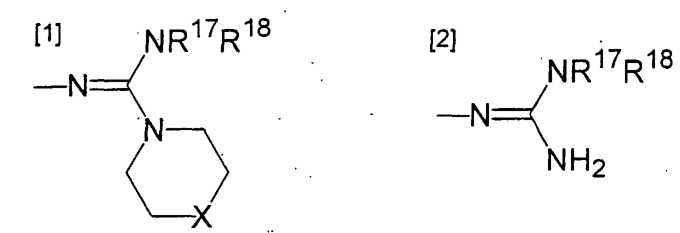 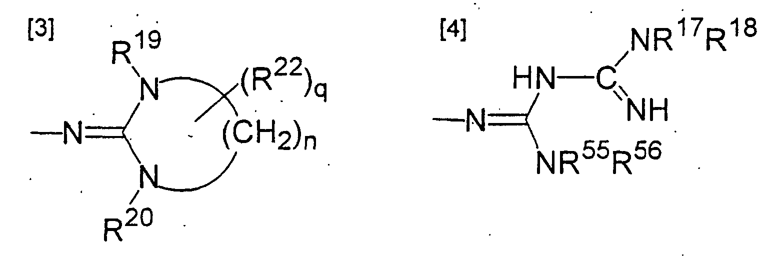 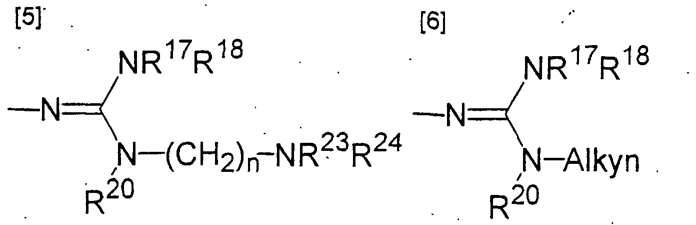 wherein,
   in the formulae [1] and [2], X is -CH₂-, -O-, -S- or -SO₂-; R¹⁷ and R¹⁸ are independently hydrogen atom, a substituted or unsubstituted alkyl group or cyano group; or R¹⁷ and R¹⁸ may be taken together with the nitrogen atom to form a substituted or unsubstituted 5- to 7-membered nitrogen-containing aliphatic heterocyclic ring;
   in the formula [3], R¹⁹ and R²⁰ are independently hydrogen atom or a substituted or unsubstituted alkyl group; R²² is a substituted or unsubstituted alkyl group, a substituted or unsubstituted alkoxy group, a substituted or unsubstituted amino group or hydroxy group; q is an integer of 0 to 4; n is an integer of 2 to 4; or R²² and R¹⁹ may be taken together with two nitrogen atoms and two carbon atoms to form a substituted or unsubstituted 8- to 11-membered bicyclic nitrogen-containing aliphatic heterocyclic ring;
   in the formula [4], R¹⁷ and R¹⁸ are as defined above; R⁵⁵ and R⁵⁶ are independently hydrogen atom or a substituted or unsubstituted alkyl group; or R⁵⁵ and R⁵⁶ may be taken together with the nitrogen atom to form a substituted or unsubstituted 5- to 7-membered nitrogen-containing aliphatic heterocyclic ring;
   in the formula [5], R¹⁷, R¹⁸, R²⁰ and n are as defined above; R²³ and R²⁴ are independently hydrogen atom or a substituted, unsubstituted alkyl group, an acyl group, a substituted or unsubstituted carbamoyl group or -SO₂-R¹⁰ wherein R¹⁰ is as defined above; or R²³ and R²⁴ may be taken together with the nitrogen atom to form a substituted or unsubstituted 5- to 7-membered nitrogen-containing aliphatic heterocyclic ring;
   in the formula [6] R¹⁷, R¹⁸ and R²⁰ are as defined above; Alkyn is an. alkynyl group.
**10.** A heteroaromatic ring compound or a pharmaceutically acceptable salt thereof according to any one of **1**. to **9**, wherein L is a group selected from the groups of the formulae [1] or [3]: $\frac{}{}$ 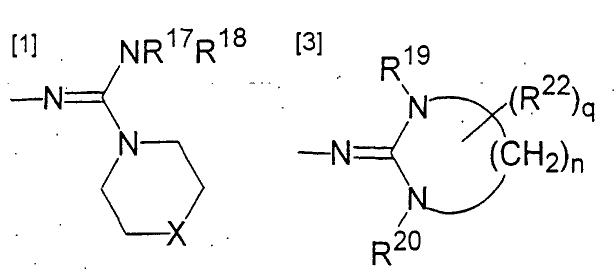 wherein, in the formulae [1] and [3], X, R¹⁷, R¹⁸, R¹⁹, R²⁰, R²², q and n are as defined above.
**11.** A heteroaromatic ring compound represented by the following formula or a pharmaceutically acceptable salt thereof: 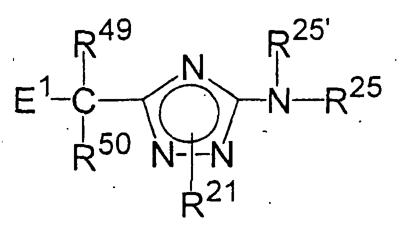 wherein E¹ is a group of the formula: 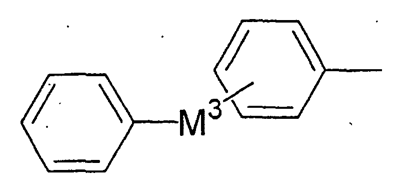 wherein M³ is single bond, -CQ or -CO-, wherein Q is as defined above;
   wherein said E¹ may be substituted by one or two halogen atom(s) or lower alkyl group(s); R²¹, R⁴⁹ and R⁵⁰ are as defined above; R²⁵ and R^{25'} are independently hydrogen atom or a lower alkyl group.
**12.** A pharmaceutical composition comprising as an active ingredient a heteroaromatic ring compound or a pharmaceutically acceptable salt thereof according to any one of **1.** to **11..**
**13.** A pharmaceutical composition according to **12**., which is a drug for the treatment or prophylaxis of autoimmune diseases or inflammatory diseases.
**14.** A pharmaceutical composition according to **12**., which is an anti-rheumatic drug or an anti-inflammatory drug.

Names of the substituents used in this description are familiar to one skilled in the art. However, examples for the substituents are shown in details as follows.

The aryl group includes, for example, aryl groups of 6 to 14 carbon atoms. Specific examples thereof are phenyl, 1-naphthyl, 2-naphthyl, azulenyl, phenanthryl, anthryl, etc.

The heterocyclic group includes, for example, 5- to 14-membered monocyclic to tricyclic aromatic or aliphatic heterocyclic groups containing 1 to 6 nitrogen atoms, oxygen atoms and/or sulfur atoms.

The aromatic heterocyclic group includes, for example, furyl, thienyl, benzothienyl, isobenzofuranyl, pyrrolyl, benzofuryl, imidazolyl, pyrazolyl, isoxazolyl, isothiazolyl, thiazolyl, oxazolyl, benzimidazolyl, benzthiazolyl, benzoxazolyl, pyridyl, pyrazinyl, pyrimidinyl, pyridazinyl, triazinyl, quinolyl, isoquinolyl, quinazolinyl, quinoxalinyl, carbazolyl, etc.

The aliphatic heterocyclic group includes, for example, tetrahydrofuryl, pyrrolidinyl, pyrazolidinyl, imidazolidinyl, 4,5-dihydro-1H-imidazolyl, piperidinyl, morpholinyl, thiomorpholinyl, 1-oxothiomorpholinyl, 1,1-dioxothiomorpholinyl, piperazinyl, thiazolidinyl, hexahydropyrimidinyl, 1,4,5,6-tetrahydropyrimidinyl, 3,6-dihydro-2H-1,3,5-oxadiazinyl, 4,5-dihydro-1,1-dioxo-1,2,4-thiadiazolyl, 5,6-dihydro-4H-1,1-dioxo-1,2,4-thiadiazinyl, 2,3-dihydro-1,1,3-trioxo-4H-1,2,4,6-thiatriazinyl, azepanyl, chromenyl, 2,3-dihydro-1,4-benzodioxinyl, 4,5,6,7-tetrahydro-1H-diazepinyl, etc.

As the substituent of each of the substituted aryl group and the substituted heterocyclic group, there may be exemplified any substituents in the following groups a) to g), and each of the substituted aryl group and the substituted heterocyclic group may optionally have one or more of these substituents.
a): Halogen atoms, nitro group, cyano group, azide group, mercapto group, substituted or unsubstituted amino groups, substituted or unsubstituted hydroxyamino groups, substituted or unsubstituted lower alkoxyamino groups, hydroxy group, oxo group, acyl groups, acyloxy groups, carboxy group, substituted or unsubstituted carbamoyl groups, substituted or unsubstituted carbamoyloxy groups and substituted or unsubstituted sulfamoyl groups.
b): -R¹⁵, -OR¹⁵, -CO₂R¹⁵, -SO₃R¹⁵, -SR¹⁵, -OCH₂R¹⁵, -SCH₂R¹⁵ and -C(=NOH)R¹⁵ wherein R¹⁵ is a phenyl group or a monocyclic heterocyclic group, wherein said phenyl group and monocyclic heterocyclic group may be substituted by at least one member optionally selected from the group consisting of, for example, halogen atoms, lower alkyl groups, lower haloalkyl groups, cyano group, nitro group, azide group, hydroxy group, lower alkoxy groups, lower haloalkoxy groups, substituted or unsubstituted amino groups, substituted or unsubstituted carbamoyl groups, carboxy group, lower alkylcarbonyl groups, lower alkoxycarbonyl groups, lower alkylthio groups, lower alkylsulfinyl groups, lower alkylsulfonyl groups, etc.
c): Alkyl groups, alkoxy groups, alkoxy carbonyl groups, alkoxy(thiocarbonyl) groups, alkylthio groups, (alkylthio)thiocarbonyl groups, (alkylthio)carbonyl groups, alkylcarbonyl groups, alkylthioyl groups, alkylsulfinyl groups, alkylsulfonyl groups, alkylcarbonyloxy groups, alkylthioyloxy groups and alkylsulfonyloxy groups, each of which groups may be substituted by at least one member optionally selected from the group consisting of, for example, halogen atoms; nitro group; cyano group; mercapto group; oxo group; thioxo group; substituted or unsubstituted amino groups; hydroxy group; acyl groups; acyloxy groups; carboxy group; substituted or unsubstituted carbamoyl groups; substituted or unsubstituted carbamoyloxy groups; substituted or unsubstituted sulfamoyl groups; -R¹⁵; -OR¹⁵; -SR¹⁵; -OCH₂R¹⁵; -SCH₂R¹⁵
   wherein R¹⁵ is as defined above;
   lower cycloalkyl groups, wherein said lower cycloalkyl groups may be substituted by at least one member optionally selected from the group consisting of, for example, halogen atoms, lower alkyl groups, lower haloalkyl groups, substituted or unsubstituted amino groups, hydroxy group, lower alkoxy groups, lower haloalkoxy groups, etc.;
   lower alkoxy groups; lower alkoxycarbonyl groups; and lower alkylthio groups, wherein said lower alkoxy groups, lower alkoxycarbonyl groups and lower alkylthio groups may be substituted by at least one member optionally selected from the group consisting of, for example, halogen atoms, lower cycloalkyl groups, monocyclic heterocyclic groups, phenyl group, cyano group, nitro group, hydroxy group, lower alkoxy groups, lower haloalkoxy groups, substituted or unsubstituted amino groups, substituted or unsubstituted carbamoyl groups, carboxy group, lower alkylcarbonyl groups, lower alkoxycarbonyl groups, lower alkylthio groups, lower alkylsulfinyl groups and lower alkylsulfonyl groups, etc.
d): Alkenyl groups, which may be substituted by at least one member optionally selected from the group consisting of, for example, halogen atoms, nitro group, cyano group, mercapto group, oxo group, thioxo group, substituted or unsubstituted amino groups, hydroxy group, lower alkoxy groups, lower haloalkoxy groups, lower alkoxycarbonyl groups, lower alkylthio groups, acyl groups, acyloxy groups, carboxy group, substituted or unsubstituted carbamoyl groups, -R¹⁵, -OR¹⁵, -SR¹⁵, -OCH₂R¹⁵ and -SCH₂R¹⁵ wherein R¹⁵ is as defined above, etc.
e): Alkynyl groups, which may be substituted by at least one member optionally selected from the group consisting of, for example, halogen atoms, nitro group, cyano group , mercapto group, oxo group, thioxo group, substituted or unsubstituted amino groups, hydroxy group, lower alkoxy groups, lower haloalkoxy groups, lower alkoxycarbonyl groups, lower alkylthio groups, acyl groups, acyloxy groups, carboxy group, substituted or unsubstituted carbamoyl groups, -R¹⁵, -OR¹⁵, -SR¹⁵, -OCH₂R¹⁵ and -SCH₂R¹⁵ wherein R¹⁵ is as defined above, etc.
f): Alkenyloxy groups, alkenyloxycarbonyl groups, alkenylcarbonyl groups, alkenylcarbonyloxy groups, alkynyloxy groups and alkynyloxycarbonyl groups, each of which groups may be substituted by at least one member optionally selected from the group consisting of, for example, halogen atoms, oxo group, substituted or unsubstituted amino groups, hydroxy group, lower alkoxy groups, lower haloalkoxy groups, acyl groups, acyloxy groups, lower alkylthio groups, carboxy group, substituted or unsubstltuted carbamoyl groups, lower alkoxycarbonyl groups, phenyl group, etc.
g): Lower cycloalkyl groups, lower cycloalkyloxy groups, lower cycloalkylcarbonyl groups, lower cycloalkylcarbonyloxy groups, lower cycloalkyloxycarbonyl groups, lower cycloalkenyl groups, lower cycloalkenyloxy groups, lower cycloalkenylcarbonyl groups, lower cycloalkenylcarbonyloxy groups and lower cycloalkenyloxycarbonyl groups, each of which groups may be substituted by at least one member optionally selected from the group consisting of, for example, halogen atoms, nitro group, cyano group, mercapto group, oxo group, thioxo group, lower alkyl groups, lower haloalkyl groups, substituted or unsubstituted amino groups, hydroxy group, lower alkoxy groups, lower haloalkoxy groups, acyl groups, acyloxy groups, lower alkylthio groups, carboxy groups, substituted or unsubstituted carbamoyl groups, lower alkoxycarbonyl groups, etc.

Specific examples of the substituent of the substituted aryl group and substituted heterocyclic group are methyl, 2-methyl-1-propyl, hexyl, 2-methyl-2-propyl, 2-propyl, phenyl, trifluoromethyl, 2,2,2-trifluoroethyl, 1,1,1,2,2,2-pentafluoroethyl, 6,6,6-trifluorohexyl, hydroxymethyl, hydroxyethyl, methoxymethyl, hexyloxymethyl, cyclopropylmethoxymethyl, acetoxymethyl, N,N-dimethylcarbamoyloxymethyl, methanesulfonyloxymethyl, N,N-dimethylsulfamoyloxymethyl, 2-(1-pyrrolidinyl)ethoxymethyl, 2-methoxyethyl, carboxymethyl, methoxycarbonylmethyl, carbamoylmethyl, amidinomethyl, methylthiomethyl, cyanomethyl, aminomethyl, aminoethyl, N,N-dimethylaminoethyl, methanesulfonylaminoethyl, sulfamoylethyl, morpholinoethyl, N-methanesulfonylaminoethyl, N-acetylaminomethyl, ethenyl, 2-propenyl, ethynyl, 2-propynyl, 2-methoxycarbonylethenyl, fluoro, chloro, bromo, nitro, cyano, hydroxy, amino, N,N-dimethylamino, mercapto, sulfo, carboxy, amidino, methoxy, cyclopropylmethoxy, 2-(1-pyrrolidinyl)ethoxy, methoxycarbonylmethoxy, 2-acetoxyethoxy, 2-hydroxyethoxy, 2-methoxyethoxy, 4,4,5,5,5-pentafluoropentoxy, 2-methanesulfinylethoxy, phenoxy, benzyloxy, 4-methoxybenzyloxy, methoxycarbonyloxy, 1-pyrrolidinyl, 3-hydroxy-1-pyrrolidinyl, acetylamino, N-acetyl-N-methylamino, N-methanesulfonylamino, N-methanesulfonyl-N-methylamino, methoxycarbonyl, 2-methyl-2-propoxycarbonyl, 2,2,2-trifluoroethoxycarbonyl, carbamoyl, N,N-dimethylcarbamoyl, 2-thiazolidinyl, 2-oxazolidinyl, 5-tetrazolyl, methanesulfinyl, sulfamoyl, N,N-dimethylsulfamoyl, acetyl, benzoyl, pivaloyl, trifluoroacetyl, formyl, ethylenedioxymethyl, imino, methoxyimino, etc.

The alkyl group includes, for example, linear or branched alkyl groups of 1 to 10 carbon atoms. Specific examples thereof are methyl, ethyl, propyl, 1-methylethyl, butyl, 1-methylpropyl, 2-methyl-1-propyl, 1,1-dimethyiethyl, pentyl, 1,1-dimethylpropyl, 2,2-dimethylpropyl, 1-methylbutyl, 3-methylbutyl, hexyl, 2-methylpentyl, 3,3-dimethylbutyl, heptyl, 1-ethylpentyl, 5-methylhexyl, octyl, 1,5-dimethylhexyl, 2-ethylhexyl, nonyl, decyl, etc.

The lower alkyl group includes alkyl groups of 1 to 6 carbon atoms.

As the substituent of the substituted alkyl group and the substituted lower alkyl group, there may be exemplified any substituents in the following groups a) to d), and the substituted alkyl group and the substituted lower alkyl group may optionally have one or more of these substituents.
a): Halogen atoms, nitro group, cyano group, mercapto group, oxo group, thioxo group, substituted or unsubstituted amino groups, substituted or unsubstituted hydroxyamino groups, substituted or unsubstituted lower alkoxyamino groups, hydroxy group, acyl groups, acyloxy groups, carboxy group, substituted or unsubstituted carbamoyl groups, substituted or unsubstituted carbamoyloxy groups, and substituted or unsubstituted sulfamoyl groups.
b): Lower cycloalkyl groups, lower cycloalkyloxy groups, lower cycloalkylcarbonyl groups, lower cycloalkylcarbonyloxy groups, lower cycloalkyloxycarbonyl groups, lower cycloalkenyl groups, lower cycloalkenyloxy groups, lower cycloalkenylcarbonyl groups, lower cycloalkenylcarbonyloxy groups and lower cycloalkenyloxycarbonyl groups, each of which groups may be substituted by at least one member optionally selected from the group consisting of, for example, halogen atoms, nitro group, cyano group, mercapto group, oxo group, thioxo group, lower alkyl groups, lower haloalkyl groups, substituted or unsubstituted amino groups, hydroxy group, lower alkoxy groups, lower haloalkoxy groups, acyl groups, acyloxy groups, lower alkylthio groups, carboxy groups, substituted or unsubstituted carbamoyl groups, lower alkoxycarbonyl groups, etc.
c): Alkoxy groups, alkoxycarbonyl groups, alkoxy(thiocarbonyl) groups, alkylthio groups, (alkylthio)thiocarbonyl groups, (alkylthio)carbonyl groups, alkylcarbonyl groups, alkylthioyl groups, alkylsulfinyl groups, alkylsulfonyl groups, alkylcarbonyloxy groups, alkylthioyloxy groups and alkylsulfonyloxy groups,
   each of which groups may be substituted by at least one member optionally selected from the group consisting of, for example, halogen atoms; nitro group; cyano group; mercapto group; oxo group; thioxo group; substituted or unsubstituted amino groups; hydroxy group: acyl groups; acyloxy groups; carboxy group: substituted or unsubstituted carbamoyl groups; substituted or unsubstituted carbamoyloxy groups; substituted or unsubstituted sulfamoyl groups; -R¹⁵; -OR¹⁵; -SR¹⁵; -OCH₂R¹⁵; -SCH₂R¹⁵
   wherein R¹⁵ is as defined above;
   lower cycloalkyl groups, wherein said lower cycloalkyl groups may be substituted by at least one member optionally selected from the group consisting of, for example, halogen atoms, lower alkyl groups, lower haloalkyl groups, substituted or unsubstituted amino groups, hydroxy group, lower alkoxy groups, lower haloalkoxy groups, etc.;
   lower alkoxy groups; lower alkoxycarbonyl groups; and lower alkylthio groups, wherein said lower alkoxy groups, lower alkoxycarbonyl group and lower alkylthio groups may be substituted by at least one member optionally selected from the group consisting of, for example, halogen atoms, lower cycloalkyl groups, monocyclic heterocyclic groups, phenyl group, cyano group, nitro group, hydroxy group, lower alkoxy groups, lower haloalkoxy groups, substituted or unsubstituted amino groups, substituted or unsubstituted carbamoyl groups, carboxy group, lower alkylcarbonyl groups, lower alkoxycarbonyl groups, lower alkylthio groups, lower alkylsulfinyl groups, lower alkylsulfonyl groups, etc.
d): -R¹⁵, -OR¹⁵, -SR¹⁵, -OCH₂R¹⁵ and -SCH₂R¹⁵ wherein R¹⁵ is as defined above.

Specific examples of the substituted alkyl group and the substituted lower alkyl group are trifluoromethyl, 2-nitroethyl, 2-cyanopropyl, 4-mercaptobutyl, 3-oxobutyl, 2-morpholinoethyl, 2-piperidinoethyl, 2-hydroxyethyl, 3-methoxypropyl, ethoxycarbonylmethyl, cyclopropylmethyl, cyclohexylmethyl, 6-cyclohexylhexyl, 3-cyclohexenylbutyl, 2-phenylbutyl, benzyl, 2-naphthylmethyl, phenethyl, 2-pyridylmethyl, 3-pyridylmethyl, 4-pyridylmethyl, 2-quinolylmethyl, 3-quinolylmethyl, 3-thienylpropyl, hydroxymethyl, hydroxyethyl, aminomethyl, aminoethyl, N,N-dimethylaminoethyl, carboxymethyl, ethoxycarbonylmethyl, sulfamoylethy, carbamoylmethyl, etc.

The lower haloalkyl group represents a lower alkyl group substituted by 1 to 5 halogen atoms.

The alkoxy group represents an oxy group having an alkyl group bonded thereto. Specific examples thereof are methoxy, ethoxy, propoxy, 2-propoxy, butoxy, 1,1-dimethylethoxy, pentoxy, hexoxy, etc. As the substituent of the substituted alkoxy group, there may be exemplified the same substituents as those exemplified above as the substituent of the substituted alkyl group. Specific examples of the substituted alkoxy group are cyclopropylmethoxy, trifluoromethoxy, 2-pyrrolidinoethoxy, benzyloxy, 2-pyridylmethoxy, etc.

The lower alkoxy group includes alkoxy group of 1 to 6 carbon atoms. The haloalkoxy group represents an alkoxy group substituted by 1 to 5 halogen atoms.

The alkoxycarbonyl group represents a carbonyl group having an alkoxy group bonded thereto. Specific examples thereof are methoxycarbonyl, ethoxycarbonyl, propoxycarbonyl, 2-propoxycarbonyl, etc. As the substituent of the substituted alkoxycarbonyl group, there may be exemplified the same substituents as those exemplified above as the substituent of the substituted alkyl group.

The alkenyl group includes linear or branched alkenyl groups of 2 to 10 carbon atoms having 1 to 3 double bonds. Specific examples thereof are ethenyl, 1-propenyl, 2-propenyl, 1-methylethenyl, 1-butenyl, 2-butenyl, 3-butenyl, 2-methyl-2-propenyl, 1-pentenyl, 2-pentenyl, 4-pentenyl, 3-methyl-2-butenyl, 1-hexenyl, 2-hexenyl, 1-heptenyl, 2-heptenyl, 1-octenyl, 2-octenyl, 1,3-octadienyl, 2-nonenyl, 1,3-nonadienyl, 2-decenyl, etc. Preferable examples of the alkenyl group are, for example, ethenyl, 1-propenyl and 1-butenyl. The lower alkenyl group includes alkenyl groups of 2 to 6 carbon atoms.

The substituent of the substituted alkenyl group includes, for example, halogen atoms, nitro group, cyano group, mercapto group, oxo group, thioxo group, substituted or unsubstituted amino groups, hydroxy group, lower alkoxy groups, lower haloalkoxy groups, lower alkoxycarbonyl groups, lower alkylthio groups, acyl groups, acyloxy groups, carboxy group, substituted or unsubstituted carbamoyl groups, -R¹⁵, -OR¹⁵, -SR¹⁵,-OCH₂R¹⁵, -SCH₂R¹⁵ wherein R¹⁵ is as defined above etc.

The alkenyloxy group represents an oxy group having an alkenyl group bonded thereto.

The alkynyl group includes linear or branched alkynyl groups of 2 to 10 carbon atoms having 1 to 3 triple bonds. Specific examples thereof are ethynyl, 1-propynyl, 2-propynyl, 1-butynyl, 2-butynyl, 3-butynyl, 1-pentynyl, 2-pentynyl, 4-pentynyl, 1-octynyl, 6-methyl-1-heptynyl, 2-decynyl, etc. Preferable examples of the alkynyl group are, for example, 1-propynyl, 1-butynyl, etc. The lower alkynyl group includes alkynyl groups of 2 to 6 carbon atoms.

The substituent of the substituted alkynyl group includes, for example, halogen atoms, nitro group, cyano group, mercapto group, oxo group, thioxo group, substituted or unsubstituted amino groups, hydroxy group, lower alkoxy groups, lower haloalkoxy groups, acyl groups, acyloxy groups, lower alkylthio groups, carboxy group, substituted or unsubstituted carbamoyl groups, lower alkoxycarbonyl groups, -R¹⁵, -OR¹⁵, -SR¹⁵, -OCH₂R¹⁵, -SCH₂R¹⁵ wherein R¹⁵ is as defined above, etc.

The alkynyloxy group represents an oxy group having an alkynyl group bonded thereto.

The cycloalkyl group includes, for example, cycloalkyl groups of 3 to 10 carbon atoms. Specific examples thereof are cyclopropyl, cyclobutyl, cyclopentyl, cyclohexyl, cycloheptyl, etc. The lower cycloalkyl group includes cycloalkyl groups of 3 to 6 carbon atoms. The cycloalkyloxy group represents an oxy group having a cycloalkyl group bonded thereto.

The cycloalkenyl group includes, for example, cycloalkenyl groups of 3 to 10 carbon atoms. Specific examples thereof are cyclohexenyl, etc. The lower cycloalkenyl group includes cycloalkenyl groups of 3 to 6 carbon atoms. The cycloalkenyloxy group refers to an oxy group having a cycloalkenyl group bonded thereto.

The substituent of the substituted cycloalkyl group and the substituted cycloalkenyl group includes, for example, halogen atoms, nitro group, cyano group, mercapto group, oxo group, thioxo group, lower alkyl groups, lower haloalkyl groups, substituted or unsubstltuted amino groups, hydroxy group, lower alkoxy groups, lower haloalkoxy groups, acyl groups, acyloxy groups, lower alkylthio groups, carboxy group, substituted or unsubstituted carbamoyl groups, lower alkoxycarbonyl groups, etc.

The aralkyl group includes a lower alkyl group substituted by aryl group(s) as defined above. The lower alkyl group includes, for example, linear or branched alkyl groups of 1 to 6 carbon atoms. Specific examples thereof are methyl, ethyl, propyl, 1-methylethyl, butyl, 1-methylpropyl, 2-methyl-1-propyl, 1,1-dimethyiethyl, pentyl, 1,1-dimethylpropyl, 2,2-dimethylpropyl, 1-methylbutyl, 3-methylbutyl, hexyl, 2-methylpentyl, 3,3-dimethylbutyl, etc. As the substituent of the substituted aralkyl group, there may be exemplified the same substituents as those exemplified above as the substituent of the substituted aryl group.

The acyl group represents -CO-R¹⁶ or -CS-R¹⁶, wherein R¹⁶ is hydrogen atom, a substituted or unsubstituted alkyl group, a substituted or unsubstituted aryl group, a substituted or unsubstituted aralkyl group or a substituted or unsubstituted heterocyclic group. Specific examples of the acyl group are formyl, acetyl, aminoacetyl, (1-morpholino)acetyl, propanoyl, 2-propanoyl, pivaloyl, valeryl, trifluoroacetyl, benzoyl, naphthoyl, nicotinoyl, natural or unnatural amino acid residues, etc. The acyloxy group represents an oxy group having an acyl group bonded thereto.

The substituent of the substituted carbamoyl group includes, for example, alkyl groups which may be substituted by an aryl group or a heterocyclic group, substituted or unsubstituted aryl groups, aralkyl groups, heterocyclic groups, etc. The substituted carbamoyl group may have a plurality of the same or different substituents independently introduced thereinto. Specific examples of the substituted carbamoyl group are ethylcarbamoyl, dimethylcarbamoyl, phenylcarbamoyl, 2-pyridylcarbamoyl, benzylcarbamoyl, (3-pyridylmethyl)carbamoyl, etc.

The substituent of the substituted sulfamoyl group includes, for example, alkyl groups, aryl groups, aralkyl groups, heterocyclic groups, etc. The substituted sulfamoyl group may have a plurality of the same or different substituents independently introduced thereinto. Specific examples of the substituted sulfamoyl group are ethylsulfamoyl, dimethylsulfamoyl, phenylsulfamoyl, 2-pyridylsulfamoyl, etc.

The substituent of the substituted amino group includes, for example, acyl groups, alkyl groups, sulfo groups, amidino groups, etc. The substituted amino group may have a plurality of the same or different substituents independently introduced thereinto. Specific examples of the substituted amino group are acetamide, propionamide, butylamide, 2-butylamide, methylamino, 2-methyl-1-propylamino, 2-hydroxyethylamino, 2-aminoethylamino, dimethylamino, diethylamino, methylcarbamate, ureido, methanesulfonylamino, guanidino, etc.

The substituent of the substituted hydroxyamino group may be on either the nitrogen atom or the oxygen atom. As the substituent, there may be exemplified the same substituents as those exemplified above as the substituent of the substituted amino group.

The halogen atom includes, for example, fluorine atom, chlorine atom, bromine atom, iodine atom, etc.

The alkylene group includes, for example, linear or branched alkylene groups of 1 to 10 carbon atoms. Specific examples thereof are methylene, ethylene, trimethylene, tetramethylene, pentamethylene, hexamethylene, heptamethylene, octamethylene, nonamethylene, decamethylene, methylmethylene, ethylmethylene, dimethylmethylene, 1,1-dimethylethylene, 1,2-dimethylethylene, 1-methyltrimethylene, 2-methyltrimethylene, 1,1-dimethyltrimethylene, 1,2-dimethyltrimethylene, 1,3-dimethyltrimethylene, 2,2-dimethyltri-methylene, 1-ethyltrimethylene, 2-ethyltrimethylene, 1,1-diethyltrimethylene, 1,2-diethyltrimethylene, 1,3-diethyltrimethylene, 2,2-diethyltrimethylene, etc.

The lower alkylene group includes, for example, linear or branched alkylene groups of 1 to 6 carbon atoms.

As the protecting group for NH group, various conventional protecting groups may be used (T.W. Greene and P.G.M. Wuts, "Protective Groups in Organic Synthesis" 2nd Edition, John Willey and Sons, inc., pp. 315-405 (1991)). Examples thereof are carbamate type protecting groups such as methoxycarbonyl, ethoxycarbonyl, *tert*-butoxycarbonyl, benzyloxycarbonyl and the like, amide type protecting groups such as acetyl, benzoyl and the like, benzyl, nitro, *p*-toluenesulfonyl, benzenesulfonyl, methanesulfonyl, etc.

In the substituted or unsubstituted nitrogen-containing aliphatic heterocyclic ring which any two of R¹, R², R³ and R⁴ form when taken together with the nitrogen atom(s), the nitrogen-containing aliphatic heterocyclic ring includes, for example, 5- to 12-membered monocyclic or bicyclic saturated or unsaturated nitrogen-containing heterocyclic rings containing 1 to 6 nitrogen atoms, oxygen atoms and/or sulfur atoms which contain at least one nitrogen atom. Specific examples thereof are pyrrolidine, imidazolidine, thiazolidine, 4,5-dihydro-1H-imidazole, piperidine, piperidin-4-one, piperazine, morpholine, thiomorpholine, thiomorpholine-1-oxide, thiomorpholine-1,1-dioxide, 1,4,5,6-tetrahydropyrimidine, hexahydropyrimidine, hexahydropyrimidine-4-one, 3,6-dihydro-2H-1,3,5-oxadiazine, 4,5-dihydro-1,1-dioxo-1,2,4-thiadiazole, 5,6-dihydro-4H-1,1-dioxo-1,2,4-thiadiazine, 2,3-dihydro-1,1,3-trioxo-4H-1,2,4,6-thiatriazine, azepane, 4,5,6,7-tetrahydro-1H-diazepine, etc.

In the substituted or unsubstituted bicyclic nitrogen-containing aliphatic heterocyclic ring which any three of R¹, R², R³ and R⁴ form when taken together with two nitrogen atoms and one carbon atom, the bicyclic nitrogen-containing aliphatic heterocyclic ring includes, for example, 7- to 12-membered bicyclic saturated or unsaturated nitrogen-containing heterocyclic rings containing 2 to 6 nitrogen atoms, oxygen atoms and/or sulfur atoms which contain at least two nitrogen atoms. Specific examples thereof are hexahydro-1H-pyrrolo[1,2-c]imidazole, octahydroimidazo[1,5-a]pyridine, octahydro- 1H-imidazo[1,5-a]azepine, octahydropyrrolo[1,2-c]pyrimidine, octahydro-1H-pyrido[1,2-c]pyrimidine, decahydropyrimido [1,6-a] azepine, octahydro-1H-pyrrolo[1,2-c][1,3]diazepine, decahydropyrido[1,2-c] [1,3]diazepine, 5,6,7,7a-tetrahydro-1H-pyrrolo[1,2-c]imidazole, 1,5,6,7,8,8a-hexahydroimidazo[1,5-a]pyridine, 5,6,7,8,9,9a-hexahydro-1H-imidazo[1,5-a]azepine, 3,4,4a,5,6,7-hexahydropyrrolo[1,2-c]pyrimidine, 4,4a,5,6,7,8-hexahydro-3H-pyrido[1,2-c]pyrimidine, 3,4,4a,5,6,7,8,9-octahydropyrimido[1,6-a]azepine, 4,5,5a,6,7,8-hexahydro-3H-pyrrolo[1,2-c][1,3]diazepine, 3,4,5,5a,6,7,8,9-octahydropyrido[1,2-c][1,3]diazepine, etc.

As the substituent of the substituted nitrogen-containing aliphatic heterocyclic ring and the substituted bicyclic nitrogen-containing aliphatic heterocyclic ring, there may be exemplified the same substituents as those exemplified above as the substituent of the substituted heterocyclic group.

The 5- to 7-membered nitrogen-containing aliphatic heterocyclic ring includes, for example, pyrrolidine, imidazolidine, pyrazolidine, piperidine, piperazine, thiazolidine, morpholine, thiomorpholine, thiomorpholine-1-oxide, thiomorpholine-1,1-dioxide, 1,2,4-thiadiazolidine-1,1-dioxide, etc.

As the substituent of the substituted 5- to 7-membered nitrogen-containing aliphatic heterocyclic ring, there may be exemplified the same substituents as those exemplified above as the substituent of the substituted heterocyclic group.

The 8- to 11-membered bicyclic nitrogen-containing aliphatic heterocyclic ring includes, for example, hexahydro-1H-pyrrolo[1,2-c]imidazole, octahydroimidazo[1,5-a]pyridine, octahydro-1H-imidazo[1,5-a]azepine, octahydropyrrolo[1,2-c]pyrimidine, octahydro-1H-pyrido[1,2-c]pyrimidine, decahydropyrimido[1,6-a]azepine, octahydro-1H-pyrrolo[1,2-c][1,3]diazepine, decahydropyrido[1,2-c][1,3]diazepine, 5,6,7,7a-tetrahydro-1H-pyrrolo[1,2-c]imidazole, 1,5,6,7,8,8a-hexahydroimidazo[1,5-a]pyridine, 5,6,7,8,9,9a-hexahydro-1H-imidazo[1,5-a]azepine, 3,4,4a,5,6,7-hexahydropyrrolo[1,2-c]pyrimidine, 4,4a,5,6,7,8-hexahydro-3H-pyrido[1,2-c]pyrimidine, 3,4,4a,5,6,7,8,9-octahydropyrimido[1,6-a]azepine, 4,5,5a,6,7,8-hexahydro-3H-pyrrolo[1,2-c][1,3]diazepine, 3,4,5,5a,6,7,8,9-octahydropyrido[1,2-c][1,3]diazepine, etc.

As the substituent of the substituted 8- to 11-membered bicyclic nitrogen-containing aliphatic heterocyclic ring, there may be exemplified the same substituents as those exemplified above as the substituent of the substituted heterocyclic group.

The substituted or unsubstituted hydrocarbon ring which R⁶ and R⁷ form when taken together with the carbon atom, includes, for example, substituted or unsubstituted cycloalkane rings of 3 to 8 carbon atoms or substituted or unsubstituted cycloalkene rings of 3 to 8 carbon atoms. Specific examples of the cycloalkane rings or cycloalkene rings are cyclopropane, cyclobutane, cyclopentane, cyclohexane, cycloheptane, cyclopropene, cyclobutene, cyclopentene, cyclohexene, cycloheptene, etc. As the substituent of the substituted hydrocarbon ring, there may be exemplified the same substituents as those exemplified above as the substituent of the substituted cycloalkyl group.

The present invention includes all stereo isomers, optical isomers, tautomers and the like of the heteroaromatic ring compounds described in the claims. The present invention also includes solvates (e.g. hydrates and the like) and all crystal forms of heteroaromatic ring compounds or pharmaceutically acceptable salts thereof described in the claims.

The pharmaceutically acceptable salt of the heteroaromatic ring compound of the present invention includes acid addition salts and base addition salts. The acid addition salts include, for example, salts with inorganic acids, such as hydrochloride, hydrobromide, sulfate, hydroiodide, nitrate, phosphate, etc.; and salts with organic acids, such as citrate, oxalate, acetate, formate, propionate, benzoate, trifluoroacetate, fumarate, maleate, tartrate, aspartate, glutamate, methanesulfonate, benzenesulfonate, camphorsulfonate, etc. The base addition salts include salts with inorganic bases, such as sodium salt, potassium salt, calcium salt, magnesium salt, ammonium salt, etc.; and salts with organic bases such as triethylammonium salt, triethanol ammonium salt, pyridinium salt, diisopropylammonium salt, etc.

The heteroaromatic ring compound of the present invention having at least one asymmetric center in the molecule may be produced by using the corresponding starting compound having the asymmetric center, or introducing the asymmetric center thereinto at one of production steps. For example, when producing the optical isomer of the heteroaromatic ring compound, the isomer may be produced by using the corresponding optically active starting material, or making an optical resolution at one of production steps.

When used as a medicine, the heteroaromatic ring compound or pharmaceutically acceptable salt thereof of the present invention may be administered orally or parenterally (for example, intravenously, subcutaneously, intramuscularly, locally, rectally, percutaneously, or through nose). Pharmaceutical forms for the oral administration include, for example, tablets, capsules, pills, granules, powders, solutions, syrups, suspensions, etc. Pharmaceutical forms for the parenteral administration include, for example, aqueous or oily preparations for injection, ointments, creams, lotions, aerosols, suppositories, patches, etc. These preparations are prepared by conventional techniques and may contain conventional acceptable carriers, excipients, binders, stabilizers, etc. When said heteroaromatic ring compound or salt thereof is used in the form of an injection, there may be added a buffer, a solubilizer, a tonicity agent and the like which are acceptable.

Although dose and frequency of administrations of the heteroaromatic ring compound or a pharmaceutically acceptable salt thereof of the present invention are varied depending on symptom, age, body weight and administration route, the heteroaromatic ring compound or a pharmaceutically acceptable salt thereof may be administered to an adult usually in a dose of approximately 1 - 2,000 mg, preferably 10 - 500 mg, in terms of the compound of the present invention as active ingredient, per day in one portion or several portions.

The compound of the present invention may be produced, for example, by the processes described below. 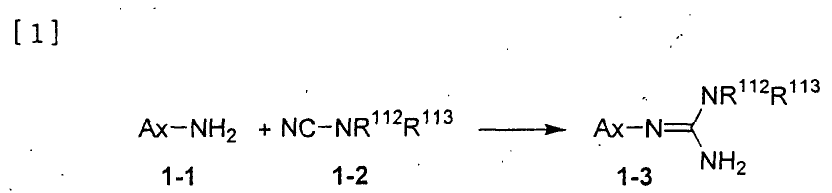 wherein R¹¹² and R¹¹³ are independently hydrogen atom, hydroxy group, nitro group, cyano group, a substituted or unsubstituted amino group, a substituted or unsubstituted hydroxyamino group, a substituted or unsubstituted carbamoyl group, a substituted or unsubstituted sulfamoyl group, a protective group for NH group, -R⁸, -OR⁸, -CO₂R⁹, -SR¹⁰, -SO-R¹⁰, -SO₂-R¹⁰, -(CO)SR¹⁰, -(CS)OR¹⁰ or -CS₂R¹⁰, wherein R⁸, R⁹ and R¹⁰ are as defined above; or R¹¹² and R¹¹³ may be taken together with the nitrogen atom to form a substituted or unsubstituted nitrogen-containing aliphatic heterocyclic ring; or -NR¹¹²R¹¹³ may be -N=C(NR⁴³R⁴⁴)NH₂ or -NHC(NR⁴³R⁴⁴)=NH, wherein R⁴³ and R⁴⁴ are as defined above;
Ax is E-G-A(R⁵)ᵣ-, wherein A, E, G, R⁵ and r are as defined above.

A compound of the formula 1-3 of the present invention may be produced by reacting a compound of the formula 1-1 with a cyanamide derivative (1-2) in the presence of a base or an acid in an inert solvent or without solvents at a reaction temperature of 15 to 130 °C. The amount of the cyanamide derivative may be 1 to 20 equivalents, preferably 1.0 to 1.2 equivalents, per equivalent of the compound of the formula 1-1.

The base includes lithium hydride, sodium hydride, potassium hydride, sodium carbonate, potassium carbonate, lithium amide, sodium amide, potassium amide, butyl lithium, *sec*-butyl lithium, *tert*-butyl lithium, lithium diisopropylamide, butyl magnesium chloride, *sec*-butyl magnesium chloride, *tert*-butyl magnesium chloride, sodium methoxide, potassium methoxide, magnesium methoxide, sodium ethoxide, potassium ethoxide, magnesium ethoxide, lithium *tert*-butoxide, sodium *tert*-butoxide, potassium *tert*-butoxide, etc. Preferable examples thereof are lithium hydride, sodium hydride, potassium carbonate, lithium amide, sodium amide, lithium *tert*-butoxide, potassium *tert*-butoxide ,etc. When an alkoxide is used as a base, the alkoxide can be prepared by treating a suitable base with an alcohol such as *tert*-butanol and the like *in situ.* In this case, preferable bases are lithium hydride, sodium hydride, lithium amide, sodium amide, potassium amide, etc. When an amide such as lithium amide, sodium amide and the like is used, preferable reaction temperature is 50 to 80 °C, and an inert gas such as nitrogen gas, argon gas and the like is preferably blown in the reaction mixture during the reaction.

The acid includes hydrochloric acid, sulfuric acid, nitric acid, phosphoric acid, Lewis acid, etc. Preferable examples thereof are hydrochloric acid, sulfuric acid, phosphoric acid, aluminum chloride, titanium trichloride, titanium tetrachloride, tin dichloride, boron trifluoride etherate, etc. The amount of the base or the acid may be, for example, 1 to 3 equivalents, preferably 1 to 2 equivalents; per equivalent of the compound of the formula 1-1.

Preferable solvents are, for example, dimethylformamide, dimethylacetamide, tetrahydrofuran, toluene, acetonitrile, *tert*-butanol, methylene chloride, chloroform, 1,2-dichloroethane, chlorobenzene, etc.

When R¹¹² or R¹¹³ is a protecting group for NH group in the compound of the formula 1-3, deprotection may be carried out if desired. This deprotection may be carried out according to a conventional method (for example, "Protective Groups in Organic Synthesis", 2nd Edition, T. W. Greene and P. G. M. Wuts, John Willey and Sons Inc., New York, p 315-362 (1991)). As the protecting group for NH group, various conventional protecting groups may be used. Preferable examples thereof are carbamate type protecting groups such as methoxycarbonyl, ethoxycarbonyl, *tert*-butoxycarbonyl, benzyloxycarbonyl and the like; amide type protecting groups such as N-acetyl, N-benzoyl and the like; benzyl; nitro; *p*-toluenesulfonyl; methanesulfonyl; and the like.

If necessary, a substituent or a protecting group may be introduced to an NH group in a compound of the formula 1-3 according to a conventional amino- modification method (for example, R. C. Larock, "Comprehensive Organic Transformations", VCH Publishers Inc New York (1989), p 397-398, 401-402; "Protective Groups in Organic Synthesis", 2nd Edition, T. W. Greene and P. G. M. Wuts, John Willey and Sons Inc., New York, p 315-362 (1991)). 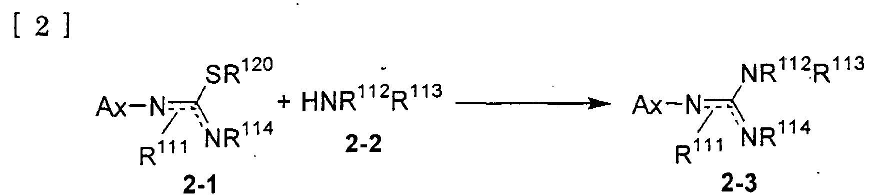 wherein R¹²⁰ is a lower alkyl or an aralkyl;
R¹¹¹ and R¹¹⁴ are independently hydrogen atom, hydroxy group, nitro group, cyano group, a substituted or unsubstituted amino group, a substituted or unsubstituted hydroxyamino group, a substituted or unsubstituted carbamoyl group, a substituted or unsubstituted sulfamoyl group, a protective group for NH group, -R⁸, -OR⁸, -CO₂R⁹, -SR¹⁰, -SO-R¹⁰, -SO₂-R¹⁰, -(CO)SR¹⁰, -(CS)OR¹⁰ or -CS₂R¹⁰, wherein R⁸, R⁹ and R¹⁰ are as defined above; or R¹¹¹ and R¹¹⁴ may be taken together with the nitrogen atom to form a substituted or unsubstituted nitrogen-containing aliphatic heterocyclic ring;
Ax, R¹¹², R¹¹³ and two broken lines are as defined above.

A compound of the formula 2-3 of the present invention may be produced by reacting a compound of the formula 2-1 with an amine derivative (2-2), if necessary, in the presence of a reaction support agent, if necessary, in an inert solvent at a reaction temperature of 0 to 140 °C for 1 hour to 3 days. The reaction support agent includes, for example, silver nitrate, silver carbonate, mercury chloride, ammonium chloride, ammonium acetate, sodium acetate, acetic acid, oxalic acid, sodium hydroxide, sodium carbonate, sodium bicarbonate, 1,8-diazabicyclo[5.4.0]undec-7-ene, triethylamine, pyridine or a mixture thereof and the like. When a reaction support agent is liquid, the agent may be used as a solvent. Preferable examples thereof are triethylamine, triethylamine-silver nitrate, ammonium chloride and ammonium acetate, and it is also preferable not to use any reaction support agents. The solvent includes, for example, water, acetonitrile; an alcohol such as methanol, ethanol, isopropanol and the like; an amide such as N,N-dimethylformamide and the like; an ether such as diethyl ether, *tert*-butyl methyl ether, tetrahydrofuran, dioxane and the like; an aromatic hydrocarbon such as benzene, toluene, chlorobenzene and the like; a chlorinated hydrocarbon such as methylene chloride, chloroform, carbon tetrachloride, 1,2-dichloroethane and the like; and a mixture thereof, etc. Preferable examples thereof are acetonitrile, acetonitrile-tetrahydorofuran, an alcohol and the like. 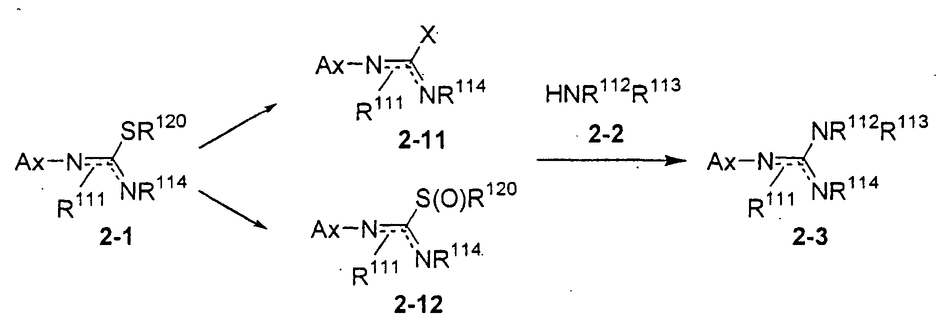 wherein X is chlorine atom, bromine atom or iodine atom; Ax, R¹¹¹, R¹¹³, R¹¹³, R¹¹⁴, R¹²⁰ and two broken lines are as defined above.

A compound of the formula 2-3 may be also produced by reacting a compound of the formula 2-11 or the formula 2-12 with an amine derivative (2-2) according to the method described above. The compound of the formula 2-11 can be produced from the compound of the formula 2-1 according to a conventional method. For example, the compound may be produced by using chlorine, sulfuryl chloride, bromine or iodine in a chlorinated hydrocarbon solvent such as methylene chloride, chloroform, carbon tetrachloride, 1,2-dichloroethane and the like. The compound of the formula 2-12 may be produced by using a suitable oxidizing agent, preferably *m*-chloroperbenzoic acid. 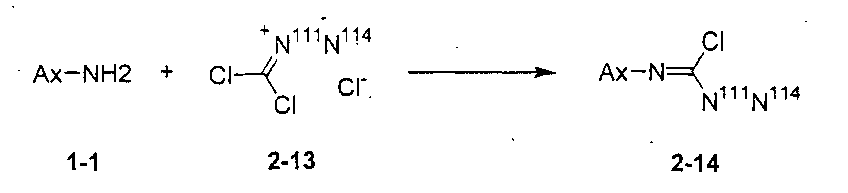 wherein Ax, R¹¹¹ and R¹¹⁴ are as defined above.

A compound of the formula 2-14, which corresponds to a compound of the formula 2-11, may be also produced by reacting a compound of the formula 1-1 with a phosgene iminium salt (2-13) according to a conventional method (for example, Angew. Chem. Internat. Edit. 12, 806(1973)).

When R¹¹¹, R¹¹², R¹¹³ or R¹¹⁴ in a compound of the formula 2-3 is a protecting group for NH group, deprotection thereof may be carried out in the same manner as above, if desired.

When a compound of the formula 2-3 has an NH group, a substituent or a protecting group may be introduced to the NH group in the same manner as the above conventional method, if desired. 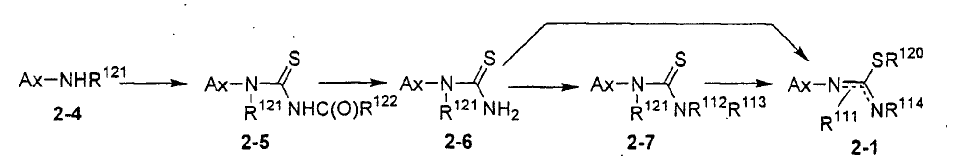 wherein R¹²² is a lower alkyl or an aryl;
R¹²¹ is hydrogen atom, hydroxy group, nitro group, cyano group, a substituted or unsubstituted amino group, a substituted or unsubstituted hydroxyamino group, a substituted or unsubstituted carbamoyl group, a substituted or unsubstituted sulfamoyl group, a protective group for NH group, -R⁸, -OR⁸, -CO₂R⁹, -SR¹⁰, -SO-R¹⁰, -SO₂-R¹⁰, -(CO)SR¹⁰, -(CS)OR¹⁰ or -CS₂R¹⁰, wherein R⁸, R⁹ and R¹⁰ are as defined above;
Ax, R¹¹¹, R¹¹², R¹¹³, R¹¹⁴, R¹²⁰ and two broken lines are as defined above.

A compound of the formula 2-1 may be produced according to the method described below. A heteroaromatic ring compound having an amino group (2-4) can be reacted with an acylisothiocyanate represented by the formula: R¹²²C(O)N=C=S according to a conventional method (for example, JP-A-63-152368) to produce an acylthioureide compound (2-5), which can be hydrolyzed to form a thioureide compound (2-6). If desired, a substituent or a protecting group may be introduced to the compound of the formula 2-6 in the same manner as the conventional amino-modification method described above to produce the compound of the formula 2-7. Subsequently, the compound of the formula 2-1 can be produced by reacting the compound of the formula 2-6 or the formula 2-7 with X-R¹²⁰, wherein X is a halogen atom, according to a conventional S-alkylation method (for example, WO 98/47880).

A compound of the formula 2-1 may also be produced according to the method described below. 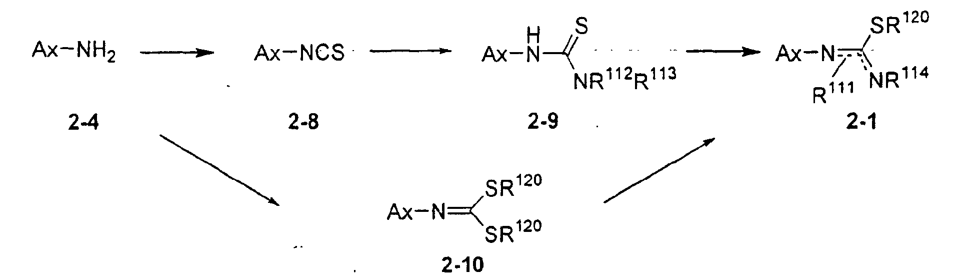 wherein Ax, R¹¹¹, R¹¹², R¹¹³, R¹¹⁴, R¹²⁰ and two broken lines are as defined above.

A compound of the formula 2-1 may also be produced according to the conventional method described below. A compound represented by the formula: Ax-NH₂ can be reacted with thiophosgene in the presence of a suitable base to produce an isocyanate represented by the formula:$\text{Ax-N=C=S.}$ The compound of the formula 2-1 can be produced by reaction with a desired amine, followed by S-alkylation. The compound can also be produced through the compound of the formula 2-10. The compound of the formula 2-10 can be produced by reacting carbon disulfide with X-R¹²⁰, wherein X is a halogen atom, in the presence of a base. And the compound of the formula 2-1 can be produced by reacting the compound of the formula 2-10 with an amine represented by the formula: NHR¹¹²R¹¹³. The base includes sodium hydroxide, potassium hydroxide, triethylamine, pyridine , 1,8-diazabicyclo[5.4.0]undec-7-ene, sodium hydride, potassium hydride, lithium hydride and the like. The solvent includes, for example, water, acetonitrile; an alcohol such as methanol, ethanol, isopropanol and the like; an amide such as N,N-dimethylformamide and the like; an ether such as diethyl ether, *tert*-butyl methyl ether, tetrahydrofuran ,dioxane and the like; an aromatic hydrocarbon such as benzene, toluene, chlorobenzene and the like; a chlorinated hydrocarbon such as methylene chloride, chloroform, carbon tetrachloride, 1,2-dichloroethane and the like; and a mixture thereof, etc.

When a compound of the formula 2-1 has an NH group, a substituent or a protecting group may be introduced to the NH group in the same manner as the conventional amino-modification method described above, if desired. 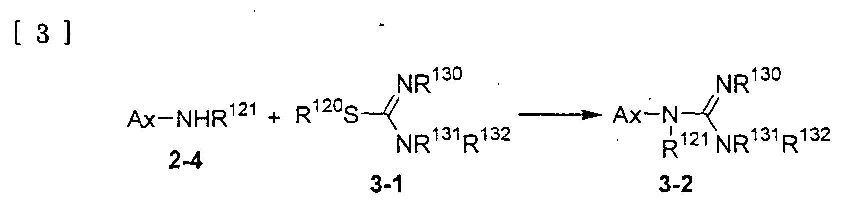 wherein R¹³⁰, R¹³¹ and R¹³² are independently hydrogen atom, hydroxy group, nitro group, cyano group, a substituted or unsubstituted amino group, a substituted or unsubstituted hydroxyamino group, a substituted or unsubstituted carbamoyl group, a substituted or unsubstituted sulfamoyl group, a protective group for NH group, -R⁸, -OR⁸, -CO₂R⁹, -SR¹⁰, -SO-R¹⁰, -SO₂-R¹⁰, -(CO)SR¹⁰, -(CS)OR¹⁰ or -CS₂R¹⁰, wherein R⁸, R⁹ and R¹⁰ are as defined above; or any two of R¹³⁰, R¹³¹ and R¹³² may be taken together with the nitrogen atom to form a substituted or unsubstituted nitrogen-containing aliphatic heterocyclic ring; or -NR¹³¹R¹³² may be -N=C(NR⁴³R⁴⁴)NH₂ or -NHC(NR⁴³R⁴⁴)=NH, wherein R⁴³ and R⁴⁴ are as defined above;
Ax, R¹²⁰ and R¹²¹ are as defined above.

A compound of the formula 3-2 of the present invention may be produced by reacting a compound of the formula 2-4 with a compound of the formula 3-1, if necessary, in the presence of a reaction support agent, if necessary, in an inert solvent at a reaction temperature of 0 to 140 °C. The reaction support agent includes, for example, silver nitrate, silver carbonate, mercury chloride, ammonium chloride, ammonium acetate, sodium acetate, acetic acid, oxalic acid, sodium hydroxide, sodium carbonate, sodium bicarbonate, 1,8-diazabicyclo[5.4.0]undec-7-ene, triethylamine, pyridine or a mixture thereof and the like. When a reaction support agent is liquid, the agent may be used as a solvent. Preferable examples thereof are triethylamine, triethylamine-silver nitrate, ammonium chloride, ammonium acetate and pyridine and it is also preferable not to use any reaction support agents. The solvent includes, for example, water; acetonitrile; an alcohol such as methanol, ethanol, isopropanol and the like; an amide such as N,N-dimethylformamide and the like; an ether such as diethyl ether, *tert*-butyl methyl ether, tetrahydrofuran, 1,4-dioxane and the like; an aromatic hydrocarbon such as benzene, toluene, chlorobenzene and the like; a chlorinated hydrocarbon such as methylene chloride, chloroform, carbon tetrachloride, 1,2-dichloroethane and the like; and a mixture thereof, etc. Preferable examples thereof are acetonitrile, acetonitrile-tetrahydrofuran, N,N-dimethylformamide, an alcohol and the like.

When R¹²¹, R¹³⁰, R¹³¹ or R¹³² in a compound of the formula 3-2 is a protecting group for NH group, deprotection thereof may be carried out in the same manner as above, if desired.

When a compound of the formula 3-2 has an NH group, a substituent or a protecting group may be introduced to the NH group in the same manner as the conventional amino-modification method described above, if desired. 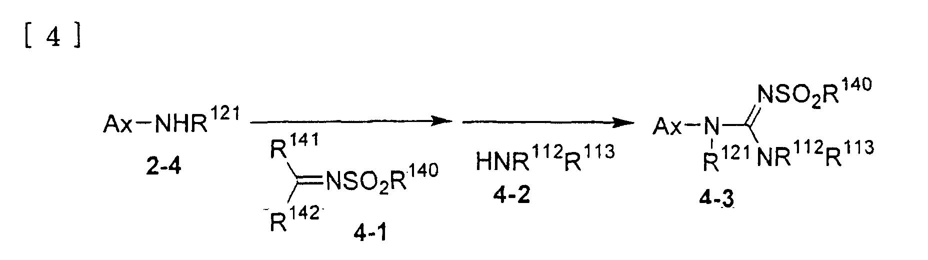 wherein R¹⁴⁰ is a substituted or unsubstituted alkyl group, a substituted or unsubstituted akenyl group, a substituted or unsubstituted alkynyl group, a substituted or unsubstituted cycloalkyl group, a substituted or unsubstituted cycloalkenyl group, a substituted or unsubstituted aryl group, or a substituted or unsubstituted aromatic heterocyclic group;
R¹⁴¹ and R¹⁴² are independently a halogen atom or methylthio group; and
Ax, R¹¹², R¹¹³ and R¹²¹ are as defined above.

A compound of the formula 4-3 of the present invention may be produced by reacting a compound of the formula 2-4 with a methylenesulfonamide derivative of the formula 4-1, which is known or can be produced according to a conventional method (for example, Chem. Ber., 99, 2900 (1966)), in an inert solvent at a reaction temperature of -20 to 80 °C, followed by reacting an amine of the formula 4-2. The solvent includes, for example, acetonitrile; an ether such as diethyl ether, *tert*-butyl methyl ether, tetrahydrofuran, 1,4-dioxane and the like; an aromatic hydrocarbon such as benzene, toluene, chlorobenzene and the like; a chlorinated hydrocarbon such as methylene chloride, chloroform, carbon tetrachloride, 1,2-dichloroethane and the like; and a mixture thereof, etc. Preferable examples thereof are acetonitrile, diethyl ether, tetrahydorofuran, 1,4-dioxane, benzene, toluene, methylene chloride, carbon tetrachloride and the like. Preferable examples of the compound of the formula 4-1 are compounds wherein both of R¹⁴¹ and R¹⁴² are chlorine atoms.

When R¹¹², R¹¹³ or R¹²¹ in the compound of the formula 4-3 is a protecting group for NH group, deprotection thereof may be carried out according to the method described above, if desired. Removal of the group represented by the formula: -SO₂R¹⁴⁰ may also be carried out according to a conventional method (for example, "Protective Groups in Organic Synthesis", 2nd Edition, T. W. Greene and P. G. M. Wuts, John Willey and Sons Inc., New York, p 379-385 (1991)).

When a compound of the formula 4-3 has an NH group, a substituent or a protecting group may be introduced to the NH group in the same manner as the conventional amino-modification method described above, if desired. 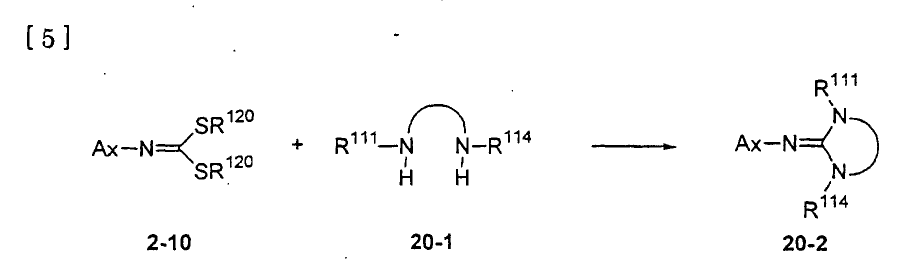 wherein Ax, R¹¹¹, R¹¹⁴ and R¹²⁰ are as defined above.

A compound of the formula 20-2 of the present invention may be produced by reacting a compound of the formula 2-10 with a diamine derivative (20-1), which is known or can be produced according to a conventional method, if necessary, in the presence of a reaction support agent, if necessary, in an inert solvent at a reaction temperature of 0 to 140 °C for 1 hour to 3 days. The reaction support agent includes, for example, silver nitrate, silver carbonate, mercury chloride, ammonium chloride, ammonium acetate, sodium acetate, acetic acid, oxalic acid, sodium hydroxide, sodium carbonate, sodium bicarbonate, 1,8-diazabicyclo[5.4.0]undec-7-ene, triethylamine, pyridine or a mixture thereof and the like. When a reaction support agent is liquid, the agent may be used as a solvent. The solvent includes, for example, water; acetonitrile; an alcohol such as methanol, ethanol, isopropanol and the like; an amide such as N,N-dimethylformamide and the like; an ether such as diethyl ether, *tert*-butyl methyl ether, tetrahydrofuran, dioxane and the like; an aromatic hydrocarbon such as benzene, toluene, chlorobenzene and the like; a chlorinated hydrocarbon such as methylene chloride, chloroform, carbon tetrachloride, 1,2-dichloroethane and the like; and a mixture thereof, etc. Preferable examples thereof are acetonitrile, acetonitrile-tetrahydrofuran, an alcohol and the like.

When R¹¹¹ or R¹¹⁴ in a compound of the formula 20-2 is a protecting group for NH group, deprotection thereof may be carried out in the same manner as above, if desired.

When a compound of the formula 20-2 has an NH group, a substituent or a protecting group may be introduced to the NH group in the same manner as the conventional amino-modification method described above, if desired.

The compounds of the formula 1-1 or the formula 2-4 which are starting compounds in the method described above, are known or can be produced according to a conventional method. For example, the compounds can be produced by the method described below.

### (1) Method of producing a 3-aminoisothiazole

An aminoisothiazole (5-3) may be produced by the methods described below. 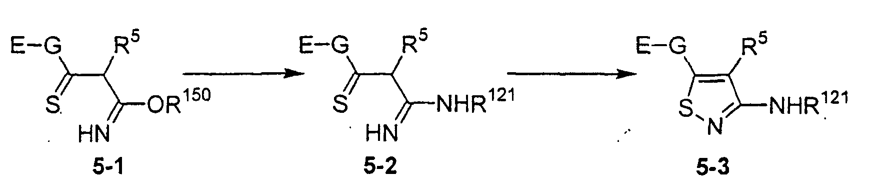 wherein R¹⁵⁰ is a lower alkyl group; E, G, R⁵ and R¹²¹ are as defined above.

A desired 3-aminoisothiazole (5-3) may be produced by reacting a compound of the formula 5-1 with a compound represented by the formula: H₂NR¹²¹ according to a conventional method (Chem. Ber., 96, 944 (1963)). 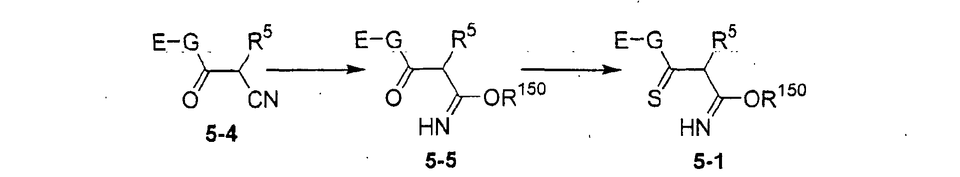 wherein E, G, R⁵ and R¹⁵⁰ are as defined above.

A compound of the formula 5-1 described above may be produced by reacting a ketonitrile compound (5-4), which is known or can be produced according to a conventional method (for example, JP-A-63-152368), with an acid in an alcohol represented by the formula: R¹⁵⁰-OH according to a conventional method (for example, Org. Syn. Col. Vol. II, 284(1943)) to produce an imidic acid ester (5-5), followed by treating the ester (5-5) with hydrogen sulfide according to a conventional method (for example, Chem. Ber., 96, 944(1963)). 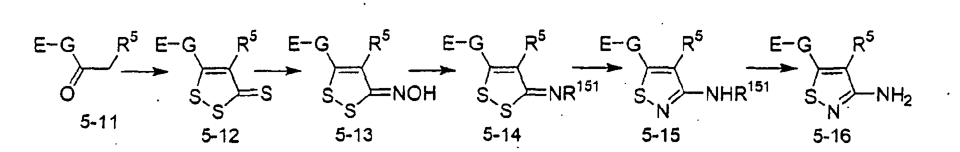 wherein R¹⁵¹ is an acyl group; E, G and R⁵ are as defined above.

As an alternative method, a keto compound of the formula 5-11 may be converted to a 3H-1,2-dithiole-3-thione derivative (5-12) according to a conventional method (for example, Tetrahedron Lett., 34, 7231 (1993)). Then a desired 3-aminoisothiazole (5-15) may be produced from the derivative according to a conventional method (for example, Liebigs Ann. Chem., 1977, 20). If desired, the 3-aminoisothiazole (5-15) may be converted to a compound of the formula 5-16 according to a conventional de-acylation method (for example, T. W. Greene and P. G. M.
Wuts, "Protective Groups in Organic Synthesis", 2nd Ed., John Wiley and Sons, inc., p.349-356 (1991)).

### (2) Method of producing a 5-aminoisothiazole

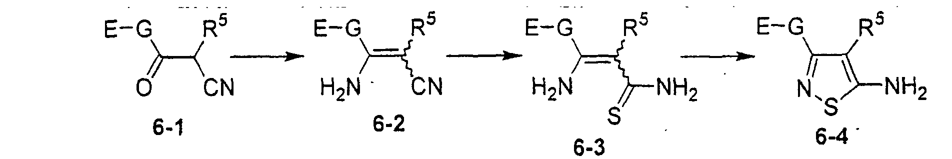

wherein E, G and R⁵ are as defined above.

A 5-aminoisothiazole (6-4) may be produced from a ketonitrile compound (6-1), which is known or can be produced according to a conventional method (for example, JP-A-63-152368), through a thioamide (6-3) according to a conventional method (for example, JP-A-60-13749). Isothiazole formation methods are not restricted to the method described above, and an isothiazole may be produced according to a conventional method (for example, A. R. Katritzky et al., "Comprehensive Heterocyclic Chemistry" Vol.6, p.166-173 (1984)).

### (3) Method of producing a 3-amino-1,2,4-thiadiazole

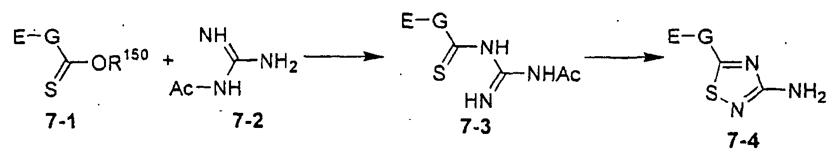

wherein Ac is acetyl; E, G and R¹⁵⁰ are as defined above.

A 3-amino-1,2,4-thiadiazole (7-4) may be produced by reacting a thioic O-acid ester (7-1), which is known or can be produced according to a conventional method, with an acetylguanidine (7-2), followed by bromination and ring-closure reaction to the obtained compound of the formula 7-3 according to a conventional method (for example, Liebigs Ann. Chem., 1975, 1961). Methods using an unsubstituted guanidine in place of an acetylguanidine (7-2) (for example, Chem. Ber. 89, 1033 (1956)) are also applicable.

### (4) Method of producing a 5-amino-1,2,4-thiadiazole

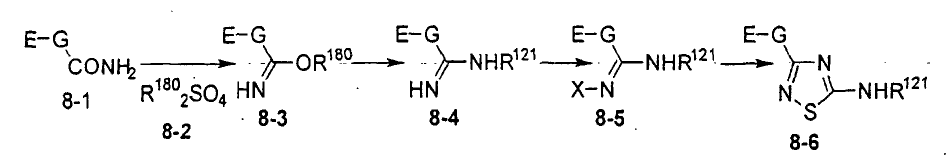

wherein R¹⁸⁰ is a lower alkyl group; X is a halogen atom; E, G and R¹²¹ are as defined above.

A 5-amino-1,2,4-thiadiazole (8-6) may be produced by the method described below. A compound of the formula 8-3 can be produced by reacting an amide compound (8-1) in a dialkyl sulfate (8-2) at a reaction temperature of 15 to 200 °C, followed by neutralization. Preferable examples of the dialkyl sulfate are dimethyl sulfate and the like. An amidine (8-4) can be produced by reacting the compound of the formula 8-3 with ammonium chloride or an amine represented by the formula: H₂N-R¹²¹ in an inert solvent at a reaction temperature of 0 to 100 °C. Preferable examples of the solvent are methanol, ethanol and the like. The desired 5-amino-1,2,4-thiadiazole (8-6) can be produced by reacting the amidine (8-4) according to a conventional method (for example, Bull. Chem. Soc. Jpn., 46, 1765(1973); Chem. Ber., 93, 397(1960); Chem. Ber., 87, 57(1954)) through a compound of the formula 8-5. 1,2,4-Thiadiazole formation methods are not restricted to the method described above, and a 1,2,4-thiadiazole may be produced according to conventional producing methods (for example, A. R. Katritzky et al., "Comprehensive Heterocyclic Chemistry" Vol.6, p.492-508 (1984)).

### (5) Method of producing a 2-amino-1,3,4-thiadiazole

A 2-amino-1,3,4-thiadiazole may be produced by the processes described below. 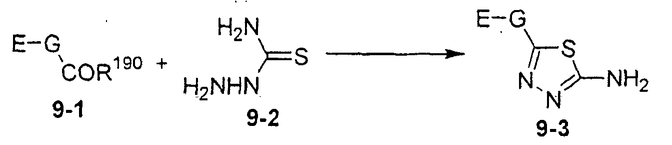 wherein R¹⁹⁰ is hydroxy group or a halogen atom; E and G are as defined above.

A desired 2-amino-1,3,4-thiadiazole (9-3) may be produced by reacting a compound of the formula 9-1, which is known or can be produced according to a conventional method, with a thiosemicarbazide (9-2) according to a conventional method (for example, JP-A-58-135873; J. Pharm. Soc. Jpn., 72, 1536(1952)). 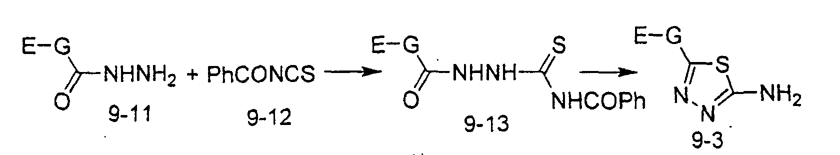 wherein Ph is phenyl; E and G are as defined above.

As an alternative method, a compound of the formula 9-11, which is known or can be produced according to a conventional method, may be converted to a compound of the formula 9-13 by reaction with benzoyl isothiocyanate (9-12) in an inert solvent at a reaction temperature of 0 to 80 °C. Preferable solvents are chloroform, methylene chloride, 1,2-dichloroethane and the like. The desired 2-amino-1,3,4-thiadizaole (9-3) may be produced by treating the compound (9-13) with sulfuric acid at a reaction temperature of 50 to 100 °C. 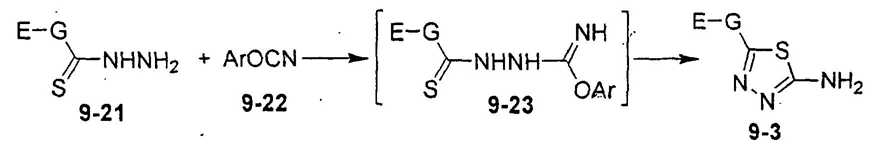 wherein Ar is an aryl group; E and G are as defined above.

As a further alternative method, the desired 2-amino-1,3,4-thiadizaole (9-3) may be produced from a compound of the formula 9-21 or the formula 9-22, which is known or can be produced according to a conventional method, according to a conventional method (for example, Chem. Ber., 98, 1359 (1965)). 1,3,4-Thiadiazole formation methods are not restricted to the method described above, and a 1,3,4-thiadiazole may be produced according to conventional producing methods (for example, A. R. Katritzky et al., "Comprehensive Heterocyclic Chemistry" Vol.6, p. 568-575 (1984)).

### (6) Method of producing a 3-aminopyrazole

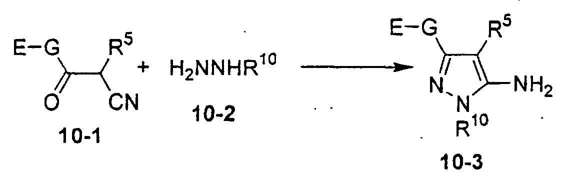

wherein E, G, R⁵ and R¹⁰ are as defined above.

A 2-aminopyrazole derivative (10-3) may be produced by reacting a cyanoketone (12-1), which is known or can be produced according to a conventional method (for example, JP-A-63-152368; Tetrahedron, 53, 1729 (1997)), with a hydrazine derivative (10-2) according to a conventional method (for example, Tetrahedron, 52, 7893 (1996)) in an inert solvent at a reaction temperature of 15 to 120 °C. Preferable solvents are methanol, ethanol, 1,4-dioxane, acetic acid and the like. Pyrazole formation methods are not restricted to the method described above, and a pyrazole may be produced according to conventional producing methods (for example, A. R. Katritzky et al., "Comprehensive Heterocyclic Chemistry" Vol.6, p. 273-291 (1984)).

### (7) Method of producing a 2-amino-1,3,4-oxadiazole

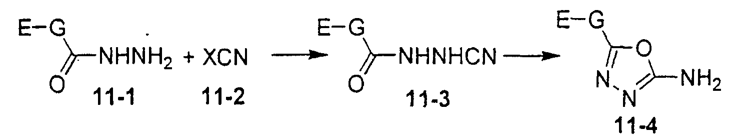

wherein E, G and X are as defined above.

A 2-amino-1,3,4-oxadiazole (11-4) may be produced by reacting a compound of the formula 11-1, which is known or can be produced according to a conventional method, with a cyanogen halide (11-2) according to a conventional method (for example, DD 52668 [Chem. Abs., 68, 68996p(1968)]), followed by ring-closure reaction. Preferable example of the compound of the formula 11-2 is cyanogen bromide.
1,3,4-Thiadiazole formation methods are not restricted to the method described above, and a 1,3,4-thiadiazole may be produced according to conventional producing methods (for example, A. R. Katritzky et al., "Comprehensive Heterocyclic Chemistry" Vol.6, p. 440-445 (1984)).

### (8) Method of producing a 3-amino-1,2,4-triazole

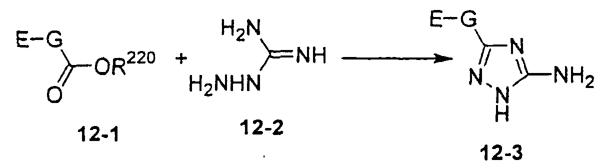

wherein R²²⁰ is a lower alkyl group; E and G are as defined above.

A 3-amino-1,2,4-triazole derivative (12-3) may be produced by reacting an ester (12-1) with an aminoguanidine (12-2) in the presence of a base in an inert solvent at a reaction temperature of 15 to 110 °C according to a conventional method (for example, J. Med. Chem., 41, 2985 (1998)). Preferable bases are sodium methoxide, sodium ethoxide, sodium hydroxide, potassium hydroxide and the like. Preferable solvents are alcohols such as methanol, ethanol, etc.; an ether such as tetrahydrofuran, 1,4-dioxane, etc.; water; and a mixture thereof. 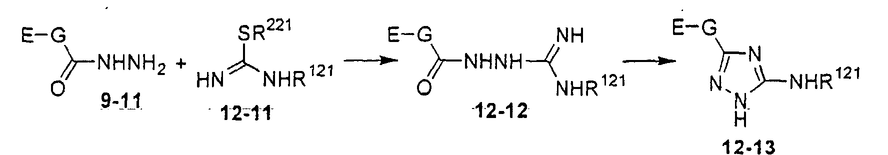 wherein R²²¹ is a lower alkyl group; E, G and R¹²¹ are as defined above.

As an alternative method, a triazole (12-13) may be produced by reacting an acylhydrazine (9-11) with an S-alkylisothiourea derivative (12-11) according to a conventional method (for example, J. Med. Chem., 28, 1628 (1985)), followed by ring-closure reaction by heating. 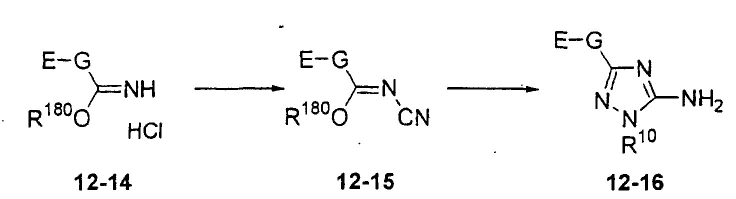 wherein E, G, R¹⁰ and R¹⁸⁰ are as defined above.

As a further alternative method, triazoles may be produced according to a conventional method (for example, J. Org. Chem. 39, 1522 (1974)). An N-cyanoimidate can be produced by reacting an imidate hydrochloride with a cyanamide in an inert solvent at a reaction temperature of room temperature to 50 °C. Preferable examples of the solvent are methanol, ethanol and the like. The desired 3-amino-1,2,4-triazole derivative can be produced by reacting the N-cyanoimidate with a substituted hydrazine in an inert solvent at a reaction temperature of 0 °C to the boiling point of the solvent. Preferable of the solvent are methanol, ethanol and the like. 1,2,4-Triazole formation methods are not restricted to the method described above, and a 1,2,4-triazole may be produced according to conventional producing methods (for example, A. R. Katritzky et al., "Comprehensive Heterocyclic Chemistry" Vol.6, p. 761-784 (1984)).

A substituent or a protecting group may be introduced to a NH group in the amino-heteroaromatic ring compound produced above in the same manner as the conventional amino-modification method described above, if desired.

### (9) Method of producing a 2-aminopyridine, 2-aminoamidine or 2-aminopyrazine

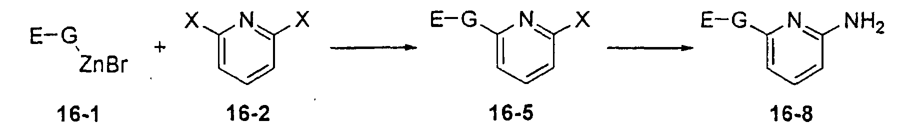

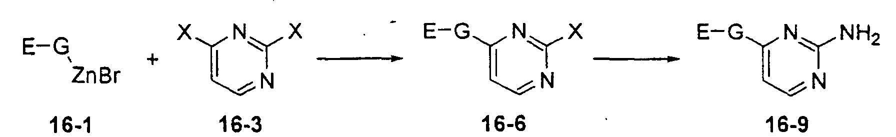

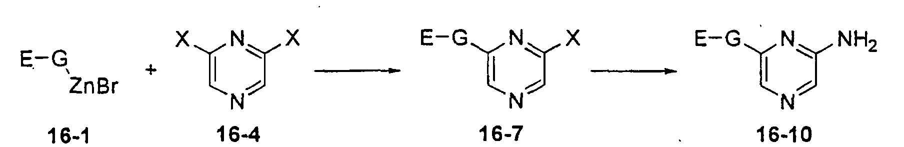

wherein X is chlorine atom or bromine atom; E and G are as defined above.

A 2-aminopyridine derivative, 2-aminoamidine derivative or 2-aminopyrazine derivative may be produced by coupling reaction of a compound of the formula 16-1 with a 2,6-dihalopyridine (16-2), 2,6-dihalopyrimidine (16-3) or 2,6-dihalopyradine (16-4) using a palladium catalyst (for example, tetrakis(triphenylphosphine)palladium) according to a conventional method (for example, WO 94/26715), followed by reaction with ammonia.

The compound of the present invention may be also produced, for example, by the methods described below, depending on the type of heteroaromatic rings.

### [6] Method of producing a 3-guanidinoisothiazole

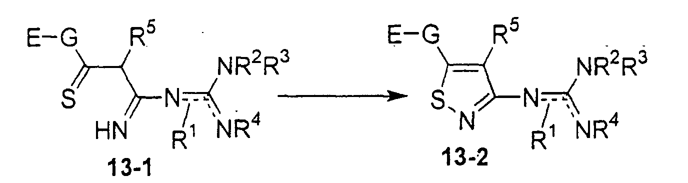

wherein E, G, R¹, R², R³, R⁴, R⁵ and two broken lines are as defined above.

A 3-guanidinoisothiazole (13-2) of the present invention may be produced according to the method described below. The 3-guanidinoisothiazole can be produced by reacting a compound of the formula 13-1 with iodine or bromine in the presence of a base in an inert solvent at a reaction temperature of -20 to 80 °C. The solvent includes, for example, an alcohol such as methanol, ethanol, isopropanol and the like; an ester such as methyl acetate, ethyl acetate and the like; and a mixture thereof, etc. Preferable examples thereof are alcohols. The base includes sodium hydroxide, sodium carbonate, sodium bicarbonate, potassium hydroxide, potassium carbonate, 1,8-diazebicyclo[5.4.0]undec-7-ene, triethylamine, pyridine, a mixture thereof and the like. Preferable example thereof is pyridine. 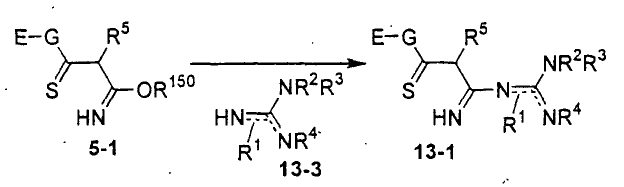 wherein E, G, R¹, R², R³, R⁴, R⁵, R¹⁵⁰ and two broken lines are as defined above.

A compound of the formula 13-1 described above may be produced by reacting a compound of the formula 5-1 produced by the method described above with a guanidine (13-3) in an inert solvent at a reaction temperature of 0 to 110 °C. The solvent includes, for example, water; acetonitrile; an alcohol such as methanol, ethanol, isopropanol and the like; an amide such as N,N-dimethylformamide; an ether such as diethyl ether, *tert*-butyl methyl ether, tetrahhydrofuran, 1,4-dioxane and the like; an aromatic hydrocarbon such as benzene, toluene, chlorobenzene and the like; a chlorinated hydrocarbon such as methylene chloride, chloroform, carbon tetrachloride, 1,2-dichloroethane and the like; and a mixture thereof, etc. Preferable examples thereof are acetonitrile and alcohols.

### [7] Method of producing a 5-guanidino-1,2,4-thiadiazole

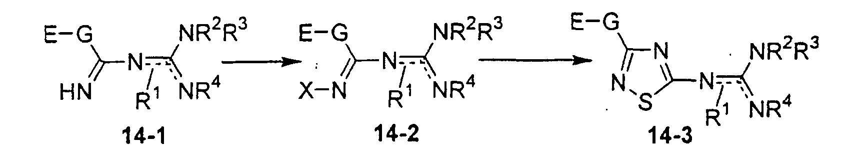

wherein E, G, X, R¹, R², R³, R⁴ and two broken lines are as defined above.

A 5-guanidino-1,2,4-thiadiazole (14-3) of the present invention may be produced by the methods described below. A compound of the formula 14-2 can be produced by reacting a compound of the formula 14-1 with a halogenation agent in an inert solvent at a reaction temperature of 0 to 30 °C. Preferable solvents are water, water-diethyl ether and the like. Preferable halogenation agents are sodium hypochlorite and sodium hypobromite. A desired 5-guanidino-1,2,4-thiadiazole (14-3) can be produced by reacting the compound of the formula 14-2 with a thiocyanate in an inert solvent at a reaction temperature of 0 to 30 °C. Preferable solvents are alcohols such as methanol, ethanol and the like. Preferable thiocyanate is potassium thiocyanate. 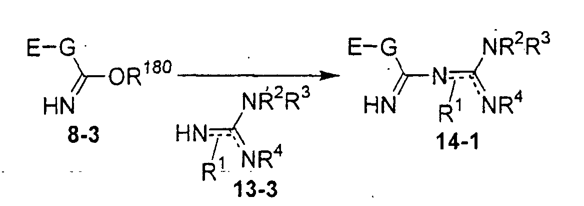 wherein E, G, R¹, R², R³, R⁴, R¹⁸⁰ and two broken lines are as defined above.

A compound of the formula 14-1 described above may be produced by the methods described below. The desired amidine (14-1) can be produced by reacting the compound of the formula 8-3 produced by the method described above with a guanidine derivative of the formula 13-3 in an inert solvent at a reaction temperature of 0 to 100 °C. Preferable solvents are alcohols such as methanol, ethanol and the like.

When a guanidino-heteroaromatic ring compound produced by the method described above has an NH group, a substituent or a protecting group may be introduced to the NH group in the same manner as the conventional amino-modification method described above, if desired.

When a compound described above having the partial structure: E has carbonyl group in the partial structure: E, a protecting group may be introduced to the carbonyl group, if desired. For example, the protecting groups described in "Protective Groups in Organic Synthesis", 2nd Edition, T. W. Greene and P. G. M. Wuts, John Willey and Sons Inc., New York, p 175-223 (1991) can be used. Preferable examples thereof are non-cyclic acetals such as dimethyl acetal and the like; cyclic acetals such as 1,3-dioxane, 1,3-dioxolane and the like; thioacetals such as 1,3-dithiane and the like; oxime etc. Deprotection thereof can also be carried out, for example, in the same manner as the methods described in the same literature. 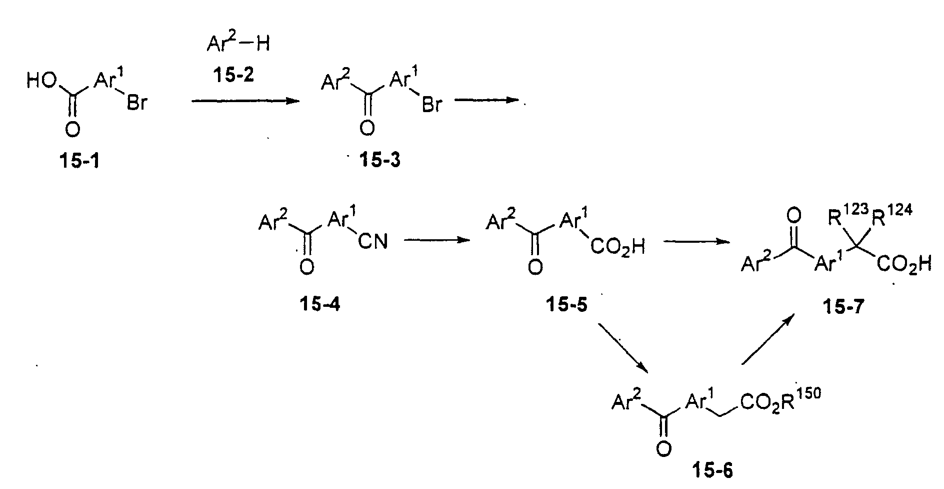 wherein Ar¹ is a benzene ring which may be substituted by one to four members optionally selected from the group consisting of fluorine atom, chlorine atom, iodine atom, nitro group, formyl group, acetyl group, cyano group, -R¹⁷, -OR¹¹, -CO₂R²⁹ and -CONR³⁰R³¹, wherein R¹⁷, R¹¹, R²⁹, R³⁰ and R³¹ are as defined above;
Ar² is a benzene ring which may be substituted by one to four members optionally selected from the group consisting of fluorine atom, chlorine atom, iodine atom, -R¹⁷ and -OR¹¹, wherein R¹⁷ and R¹¹ are as defined above;
The ortho position of the carboxylic acid of the formula 1-1 may be bonded to the compound of the formula 1-2 through single bond, -O-, -S-, -SO- or -SO₂-;
R¹²³ and R¹²⁴ are independently hydrogen atom or a lower alkyl group; or
R¹²³ and R¹²⁴ may be taken together with the carbon atom attached thereto to form a substituted or unsubstituted hydrocarbon ring;
R¹⁵⁰ is an alkyl group.

An ester of the formula 15-6 or a carboxylic acid of the formula 15-7, which is a starting compound of the present invention, may be produced by the methods described below. A benzene derivative having a carboxy group and bromine atom (15-1) can be reacted according to a conventional acid-chloride formation method to produce an acid chloride, which can be reacted with a benzene derivative of the formula 15-2 according to a conventional Friedel-Crafts reaction (for example, R. C. Larock, "Comprehensive Organic Transformations", VCH Publishers, Inc., p. 703-708 (1989)) to produce a benzophenone derivative (15-3). Then, the benzophenone derivative can be converted according to a conventional cyano-substitution method (for example, Synthetic Communications, 1997, 27, 1199) to produce a compound of the formula 15-4, which can be subjected to hydration to produce a carboxylic acid (15-5). Subsequently, an acid chloride can be produced from the carboxylic acid (15-5) according to a conventional acid-chloride formation method, and next the acid chloride can be treated with diazomethane or trimethylsilyldiazomethane according to a conventional Arndt-Eistert synthetic method (for example, R. C. Larock, "Comprehensive Organic Transformations", VCH Publishers, Inc., p.933 (1989)) to produce a carboxylic acid (15-7; R¹²³ and R¹²⁴ are hydrogen atoms) or an ester (15-6). Alternatively, the compound of the formula 15-7 can be produced by introducing alkyl group(s) to the ester (15-6) using a compound of the formula: X-R¹²³, X-R¹²⁴ or X-R¹²³-R¹²⁴-X, wherein X is chlorine atom, bromine atom or iodine atom; R¹²³ and R¹²⁴ are as defined above, according to a conventional C-alkylation method.

Specific examples of compounds included in the present invention are the compounds described below. These compounds, however, are for exemplification, and the present invention is not limited to them. In the specific examples, the meanings of the abbreviations used are as follows:
Me: methyl
Et: ethyl
Ph: phenyl

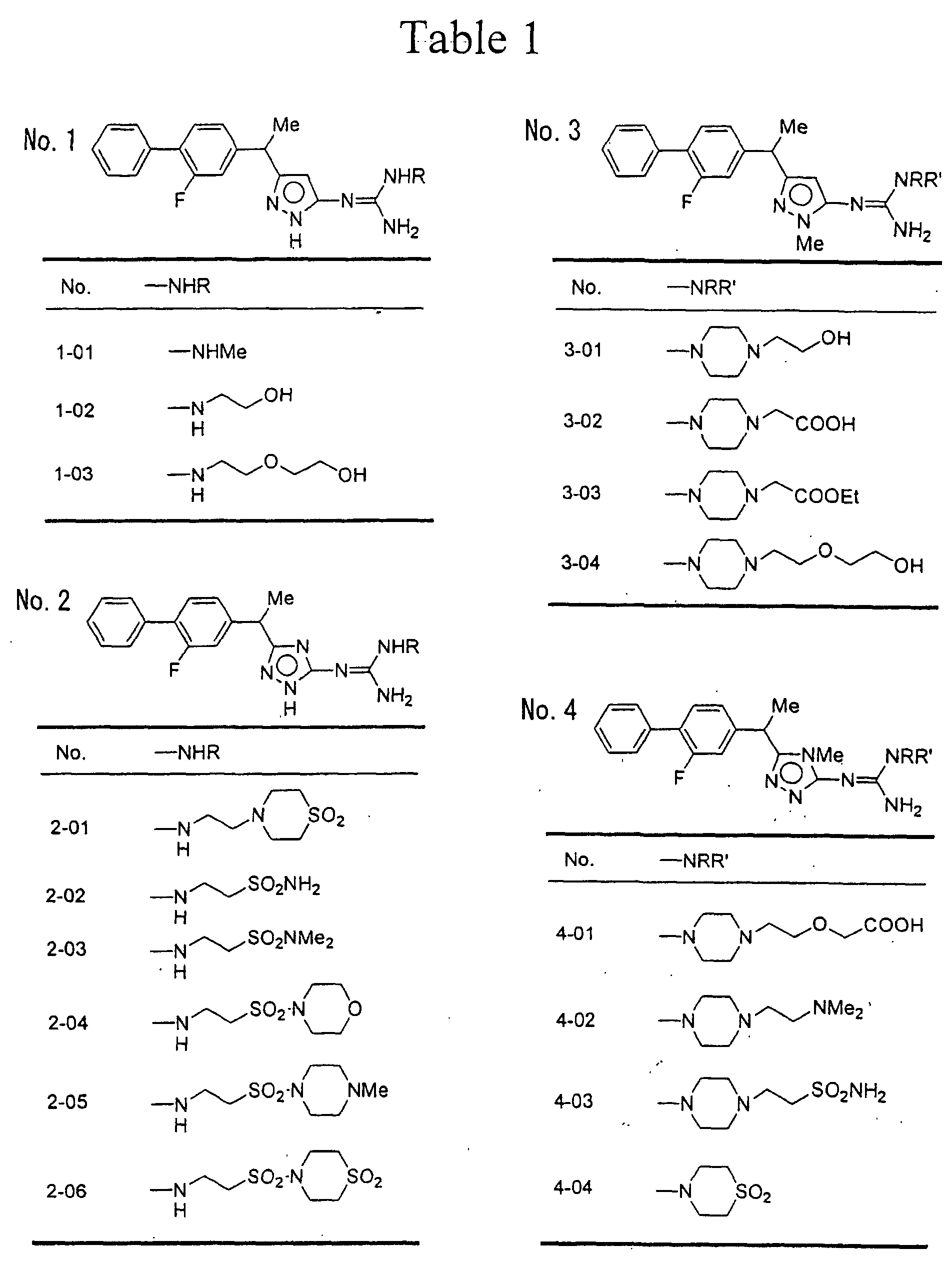

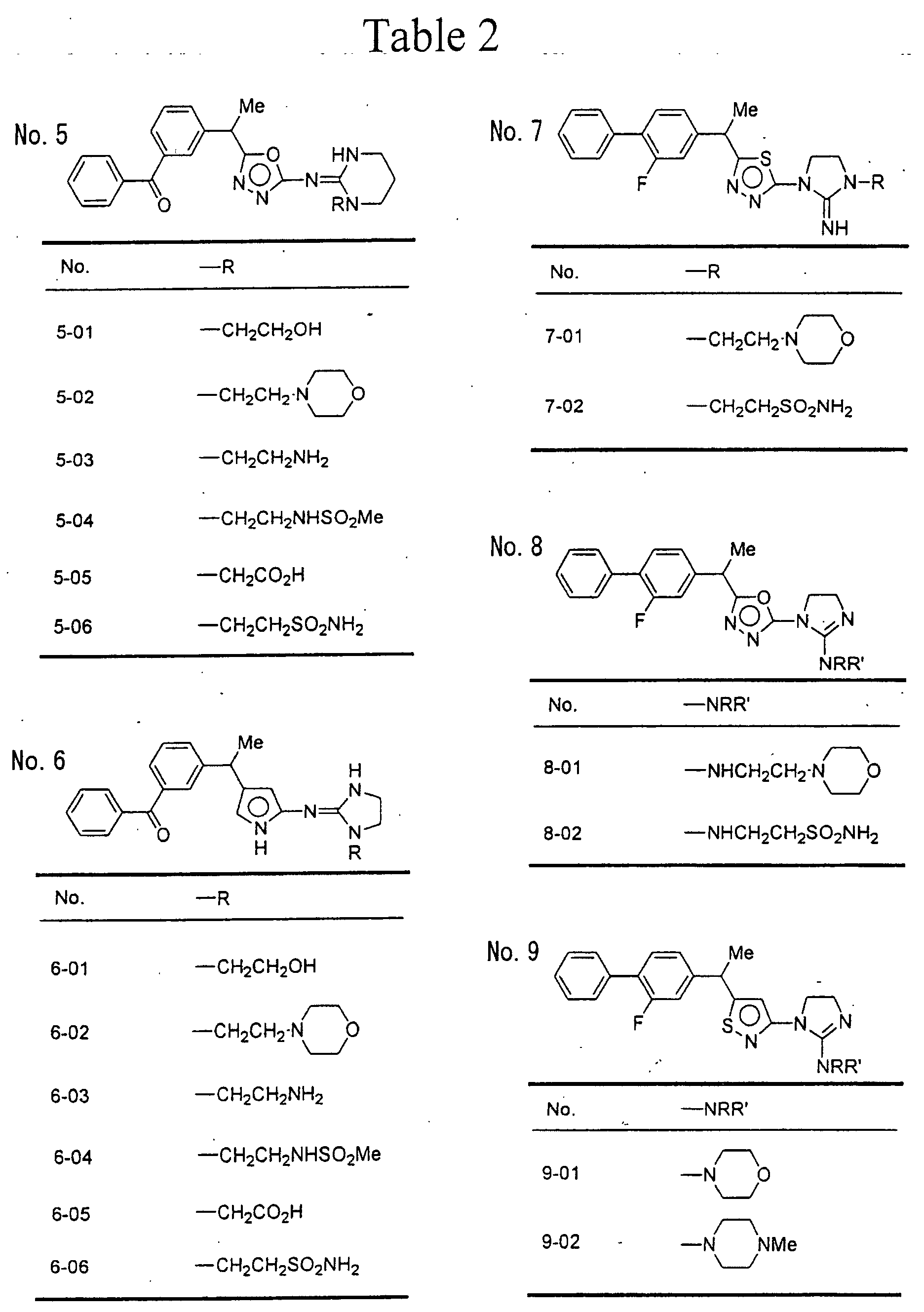

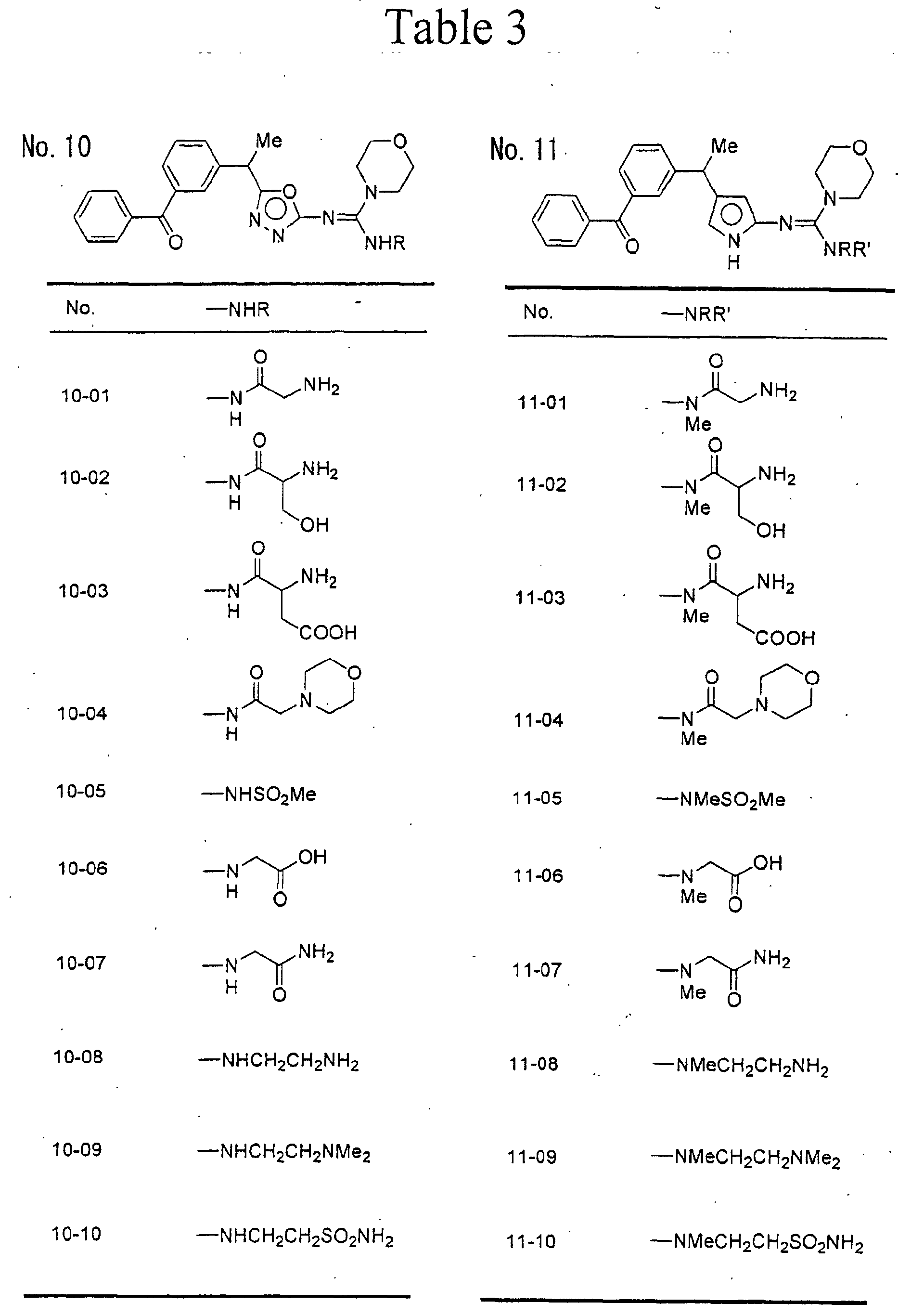

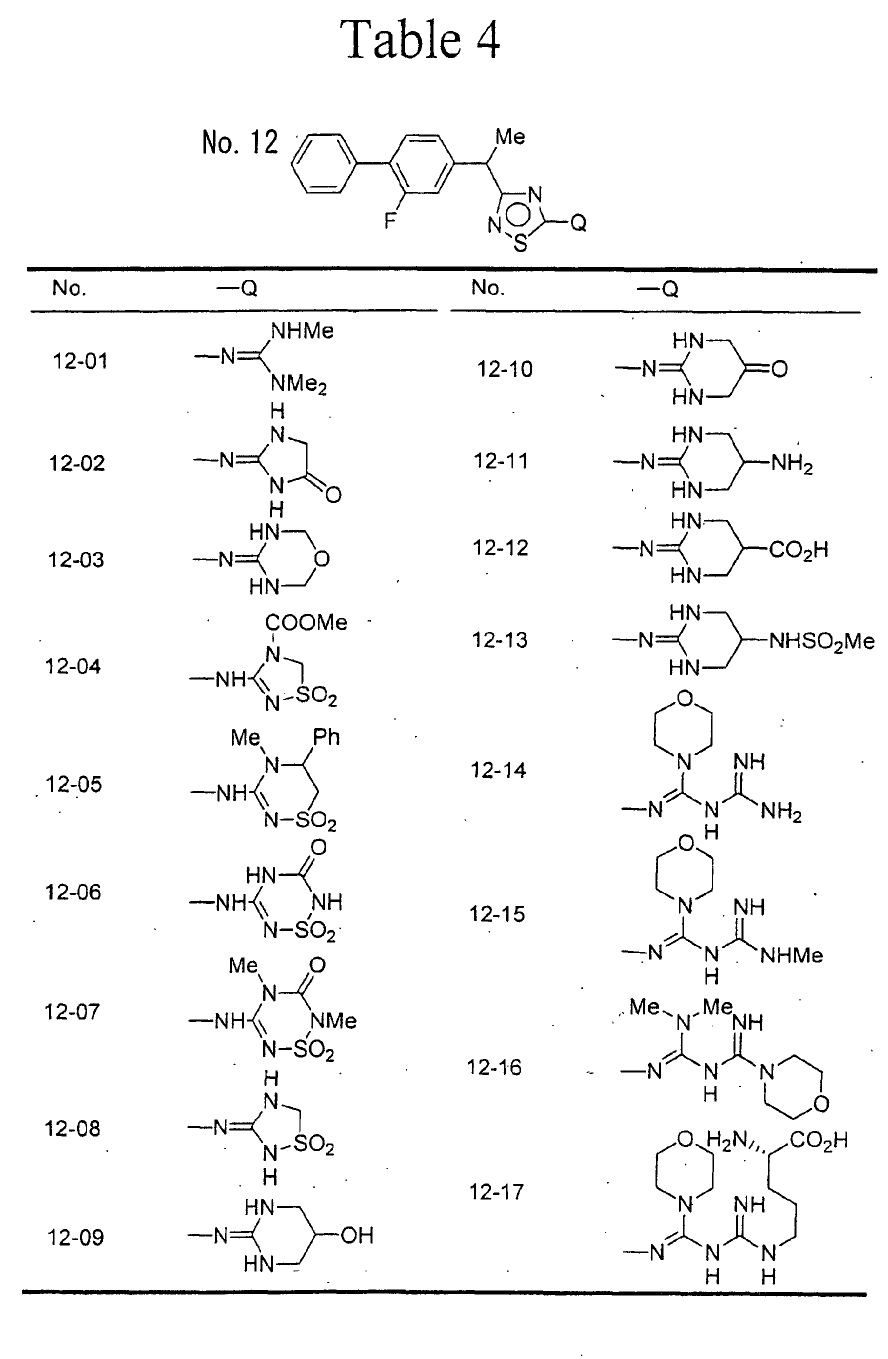

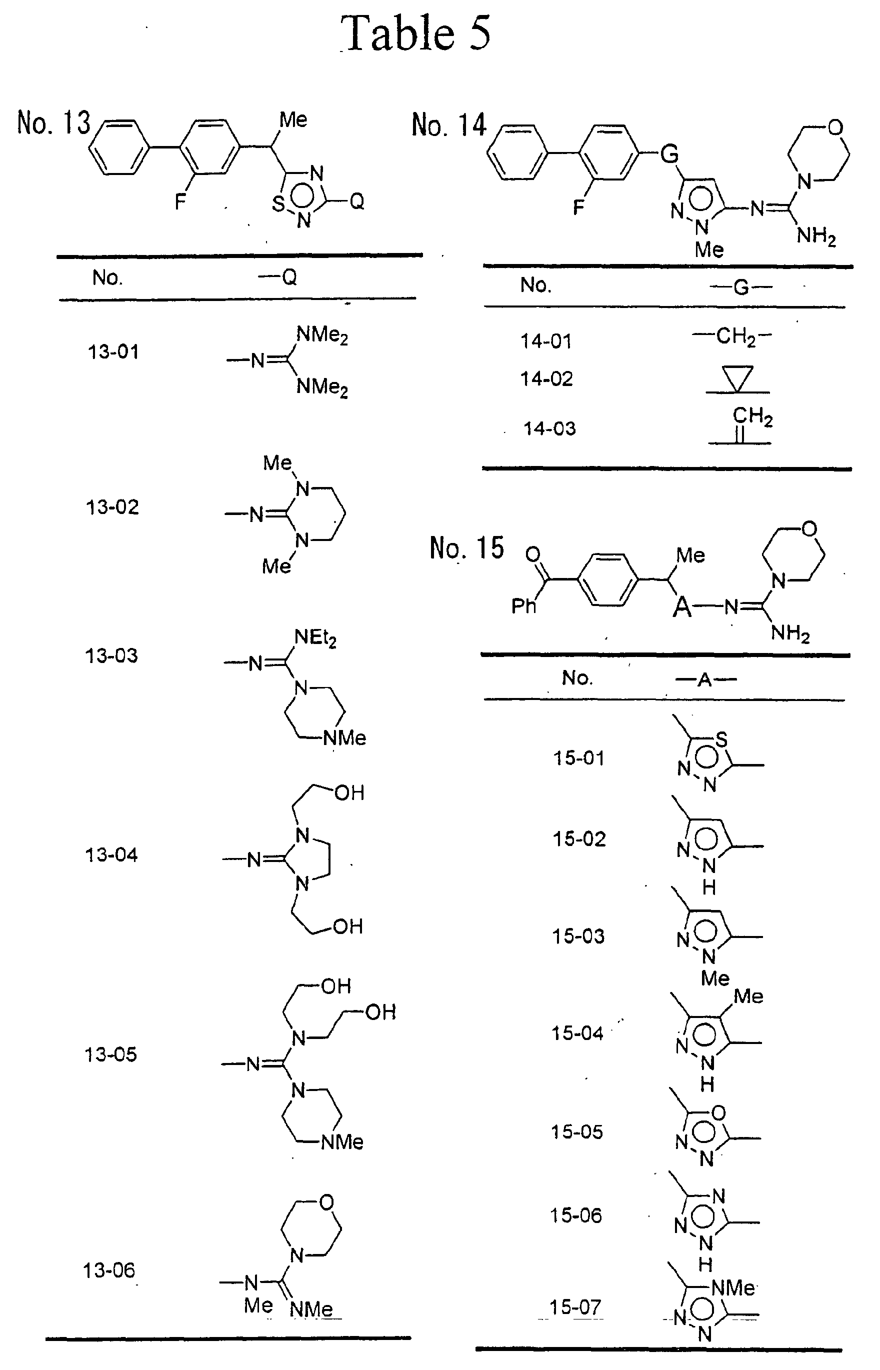

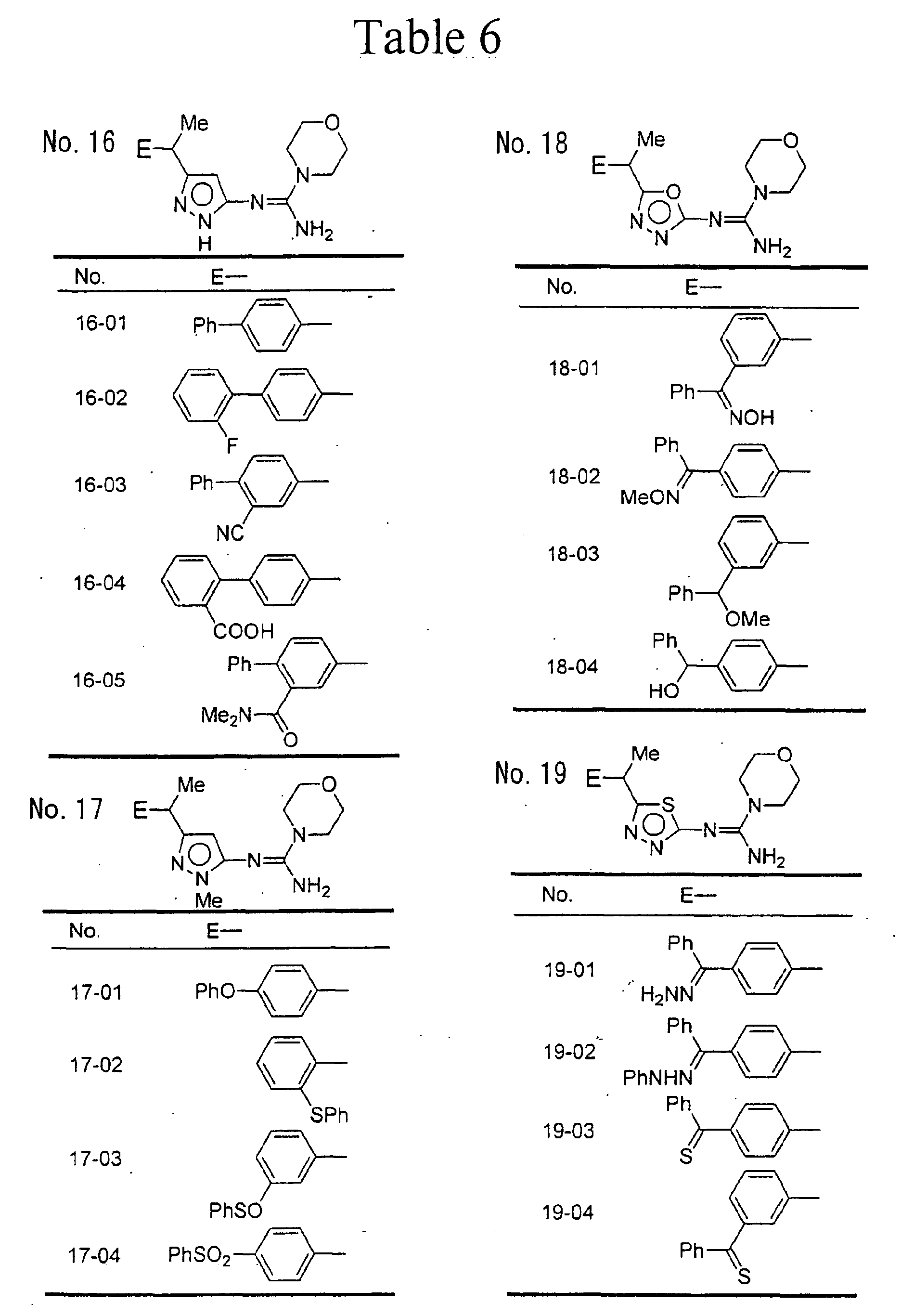

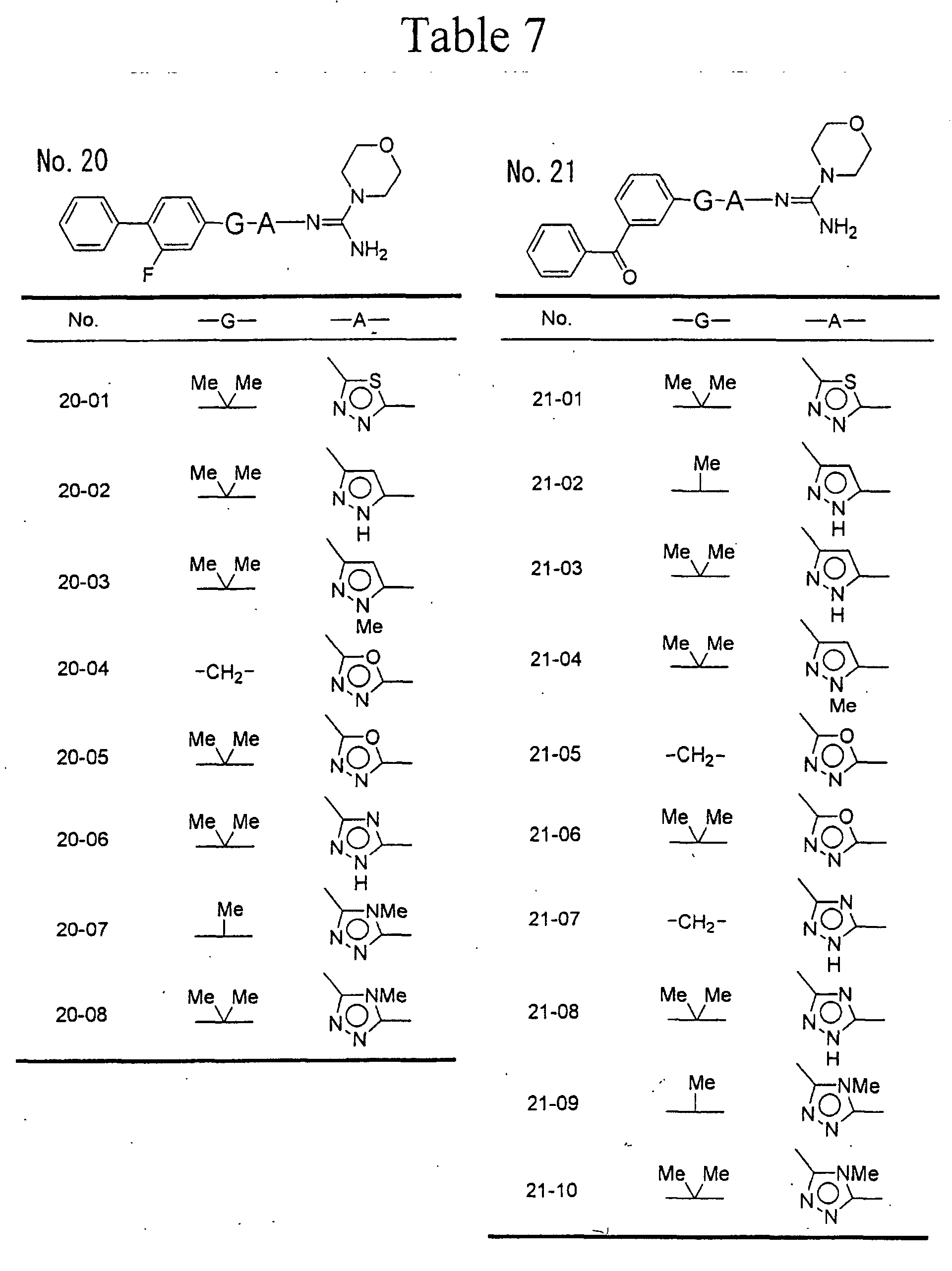

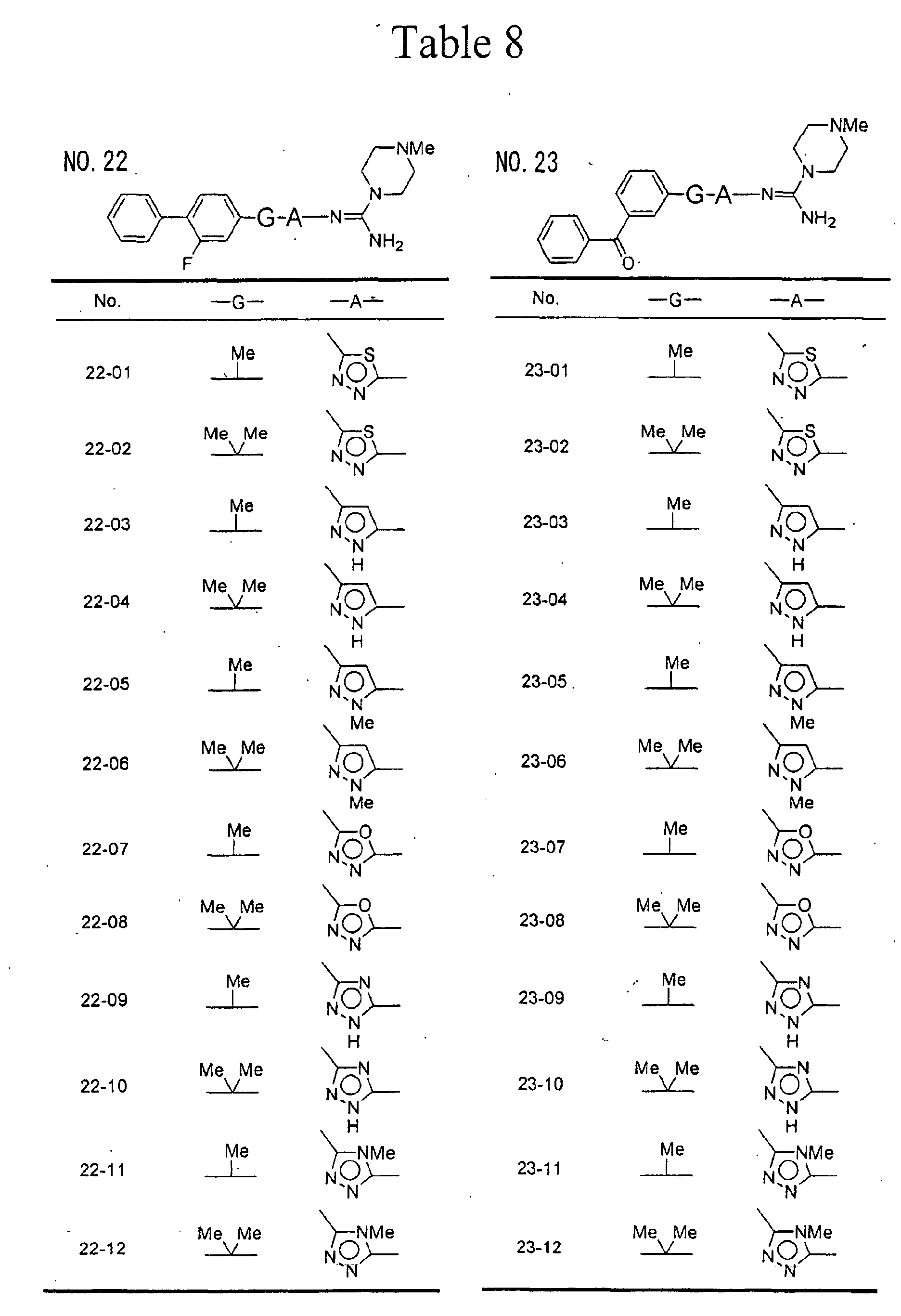

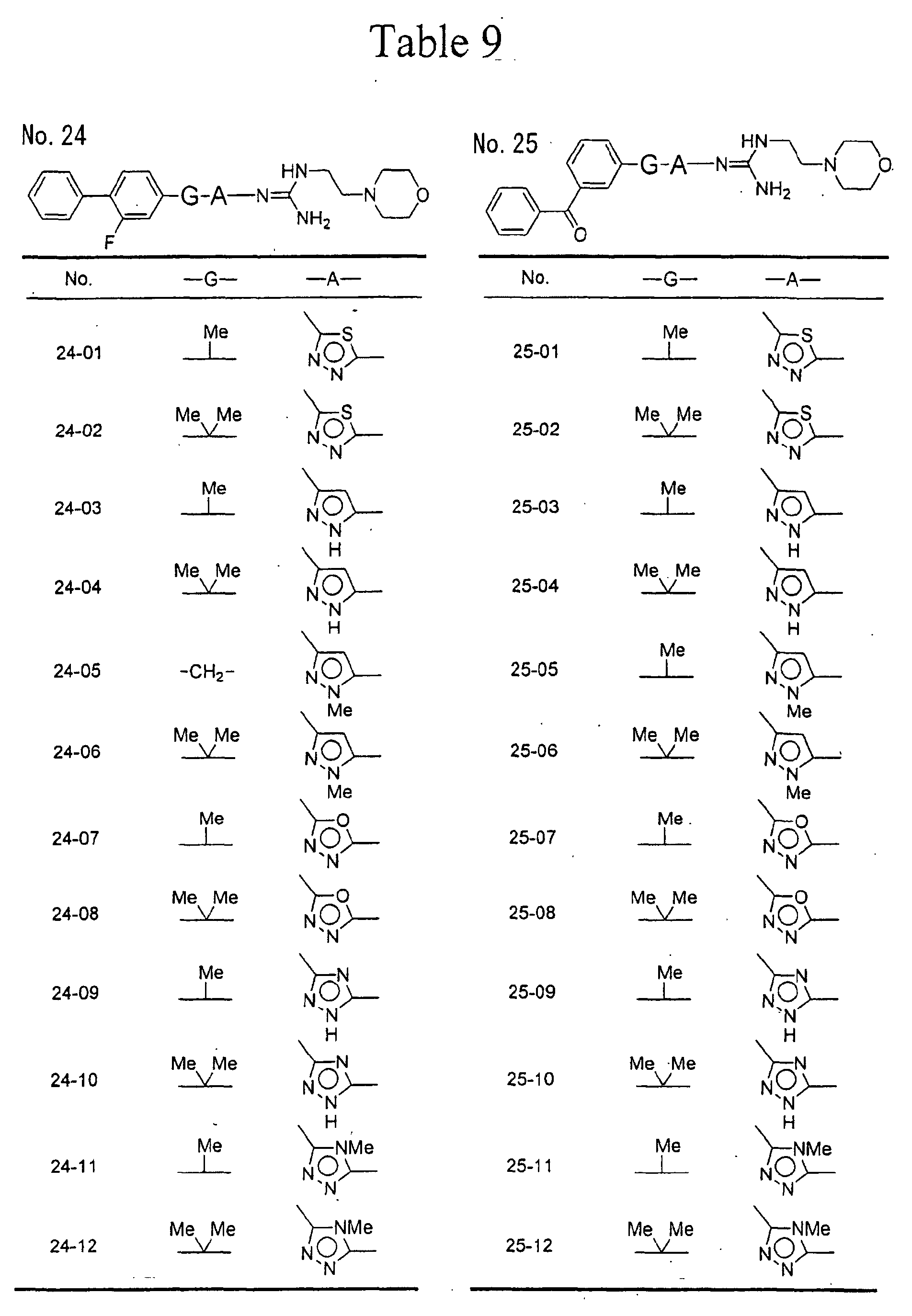

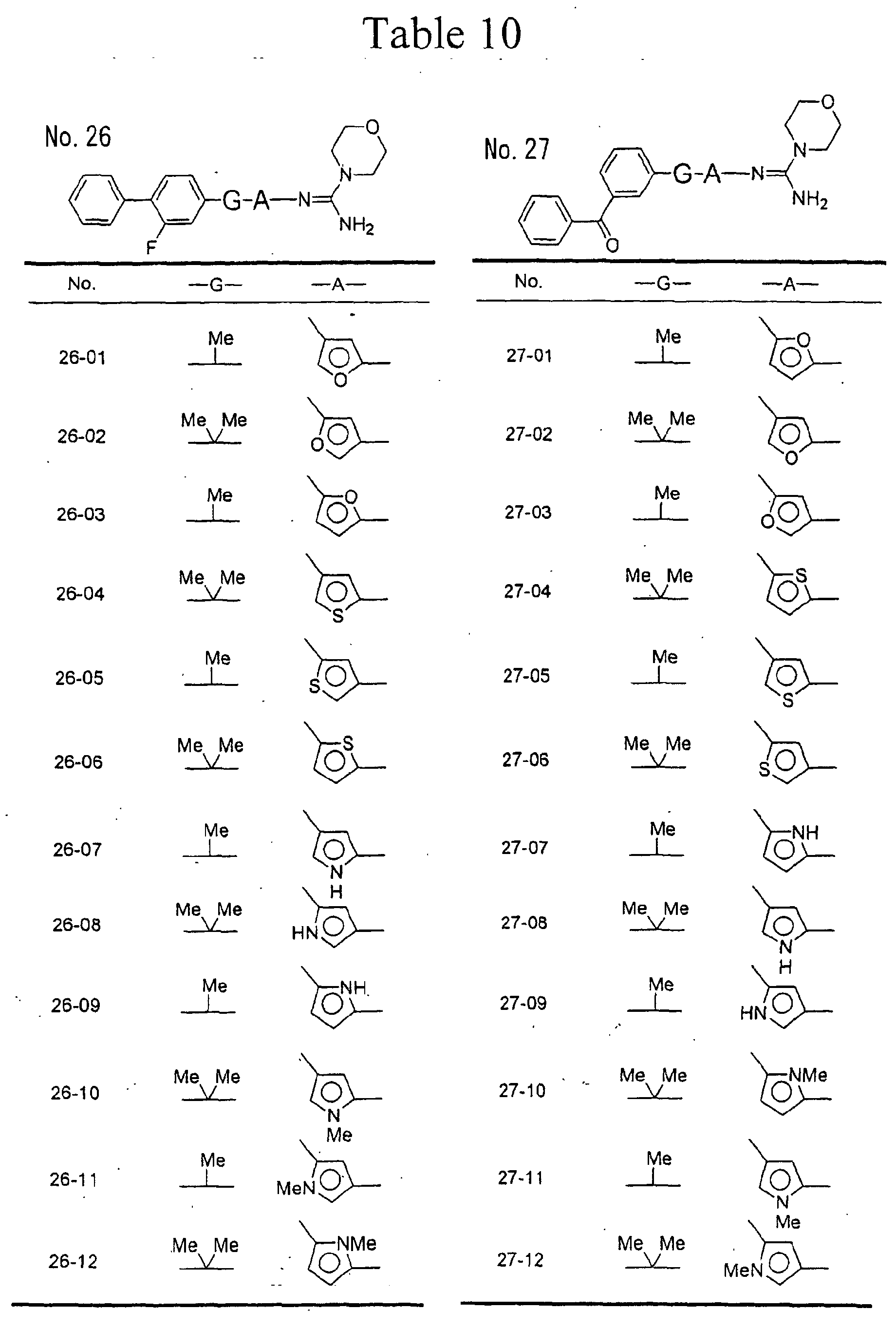

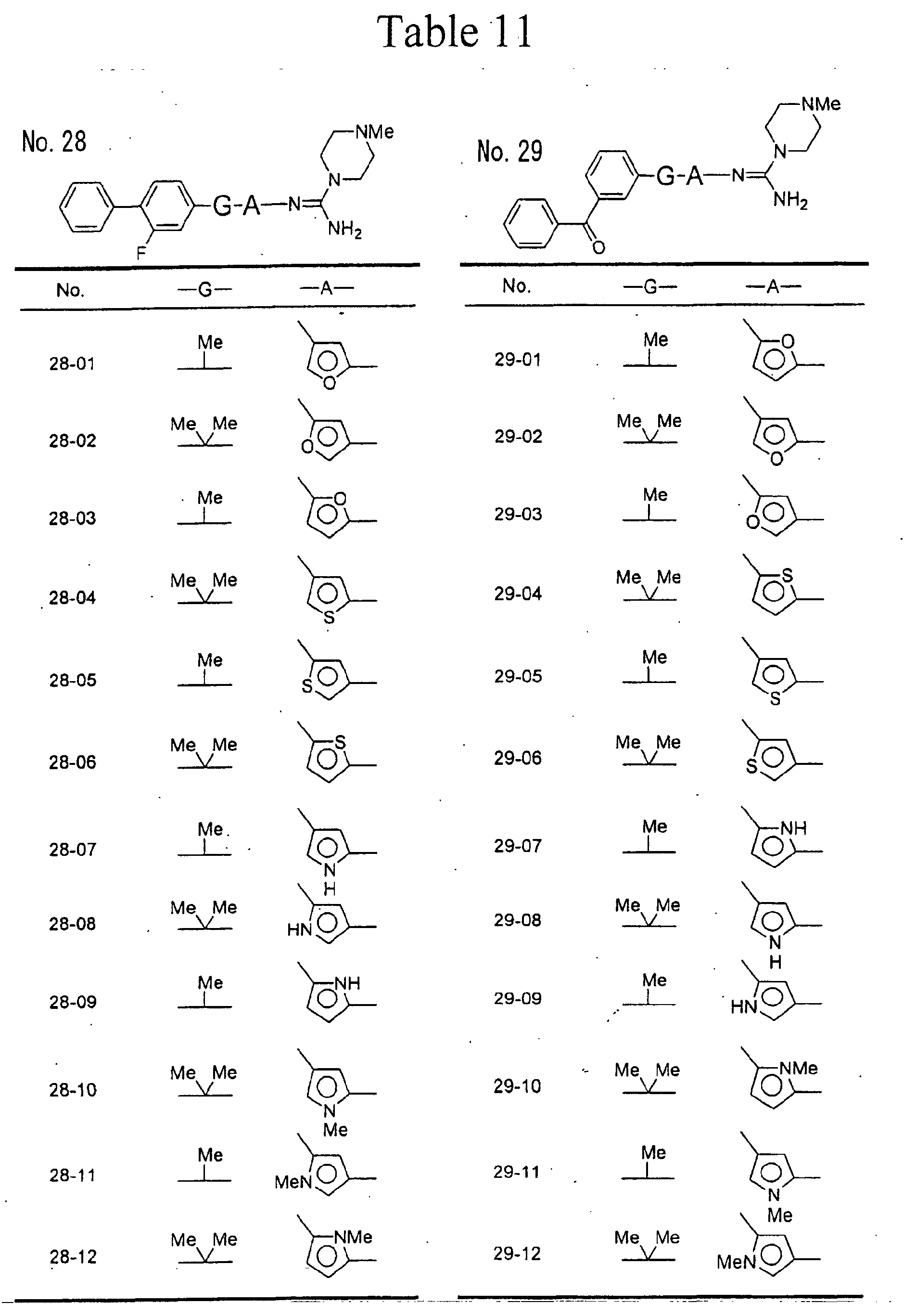

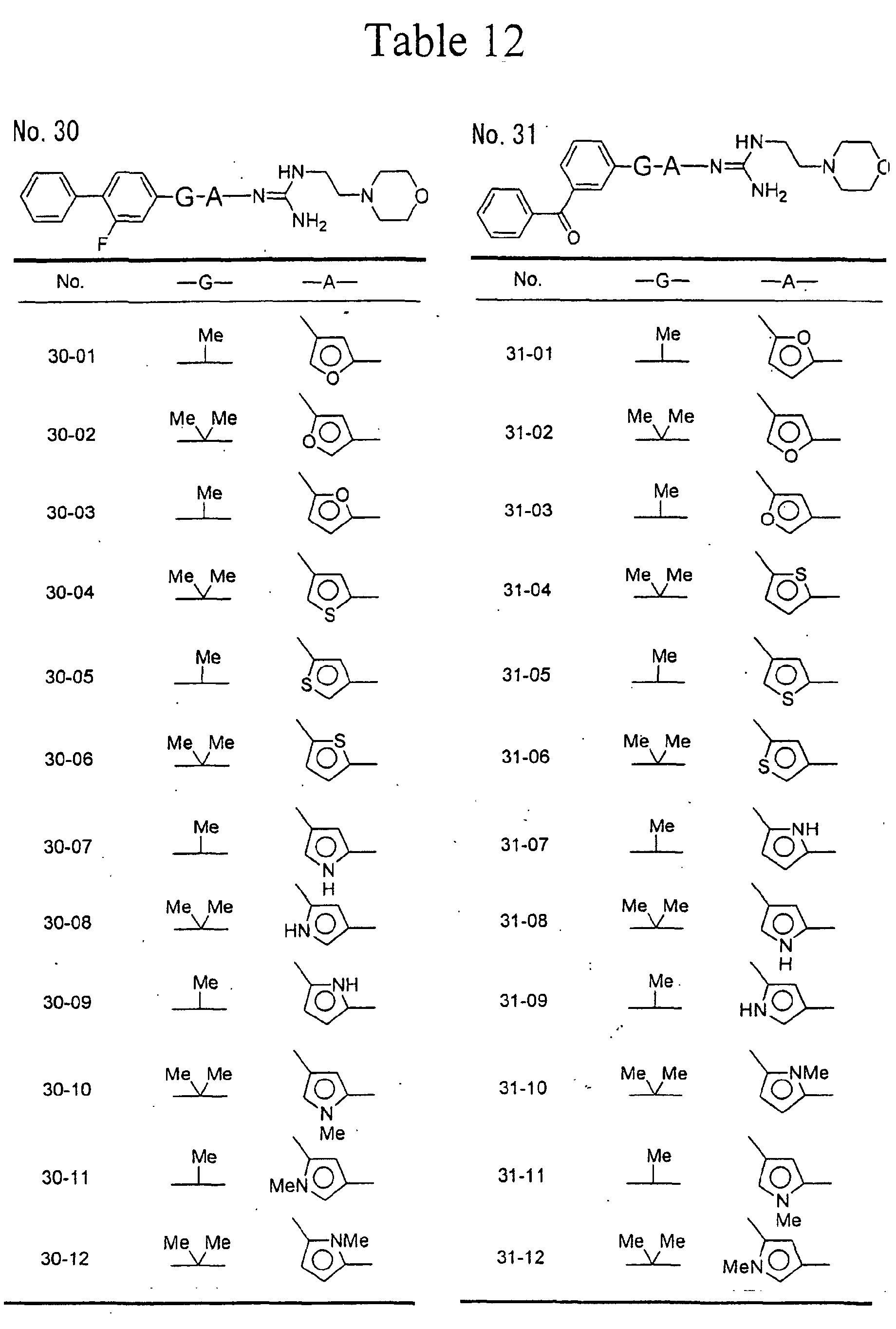

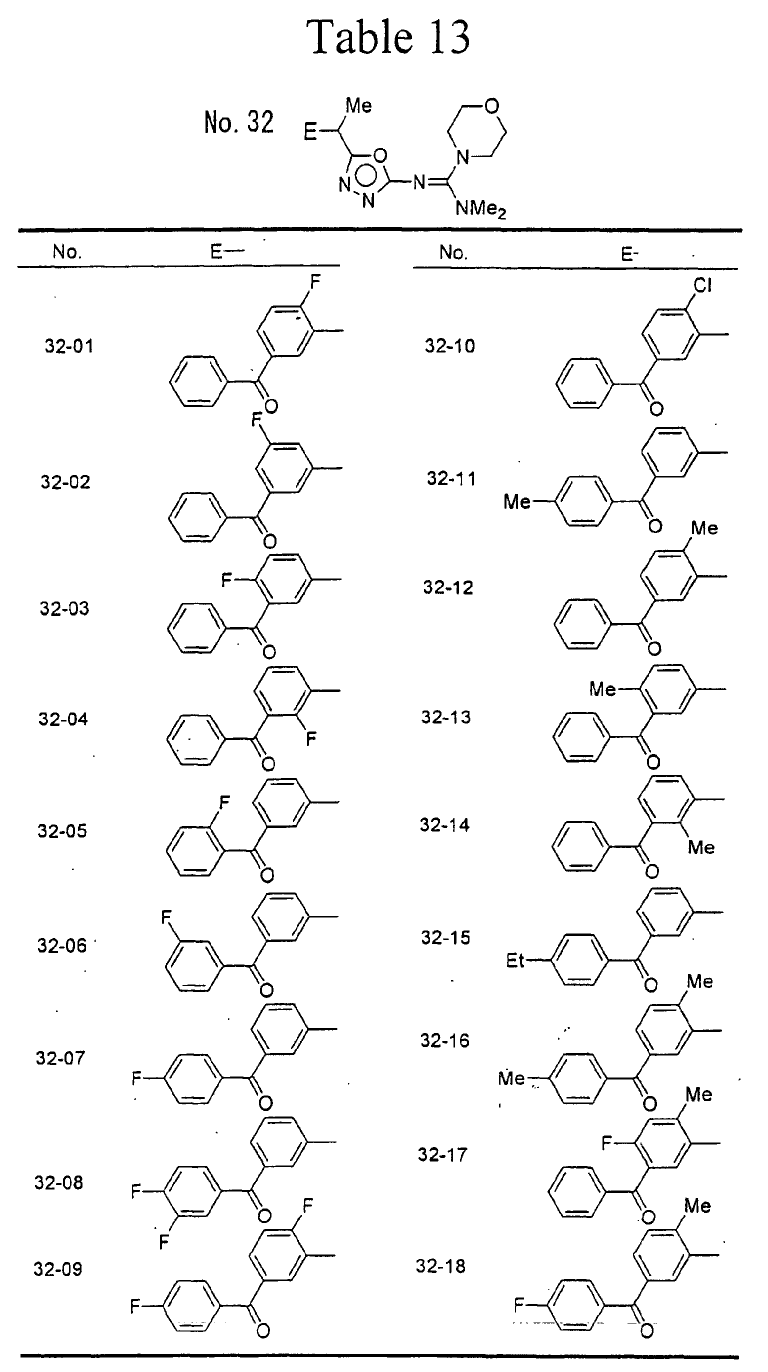

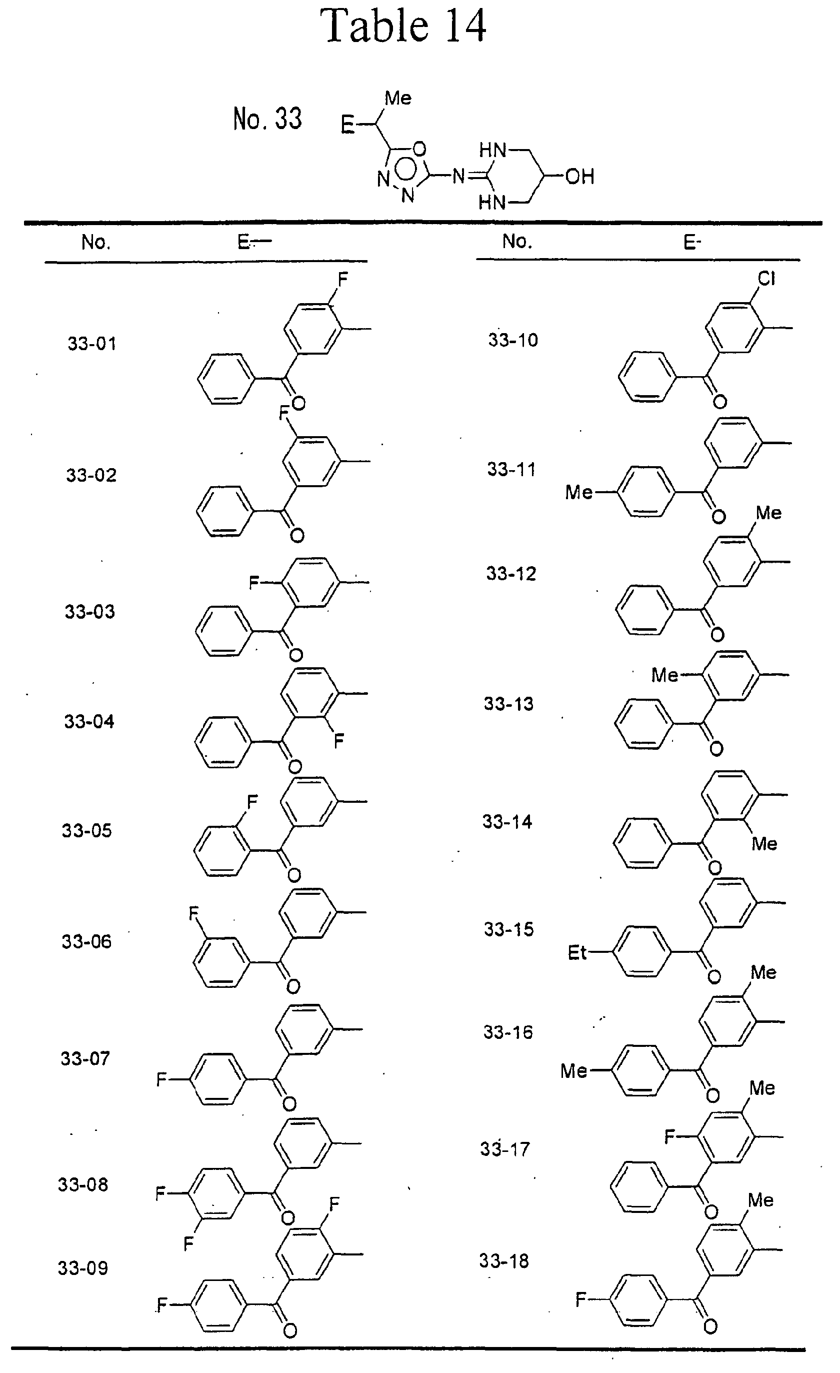

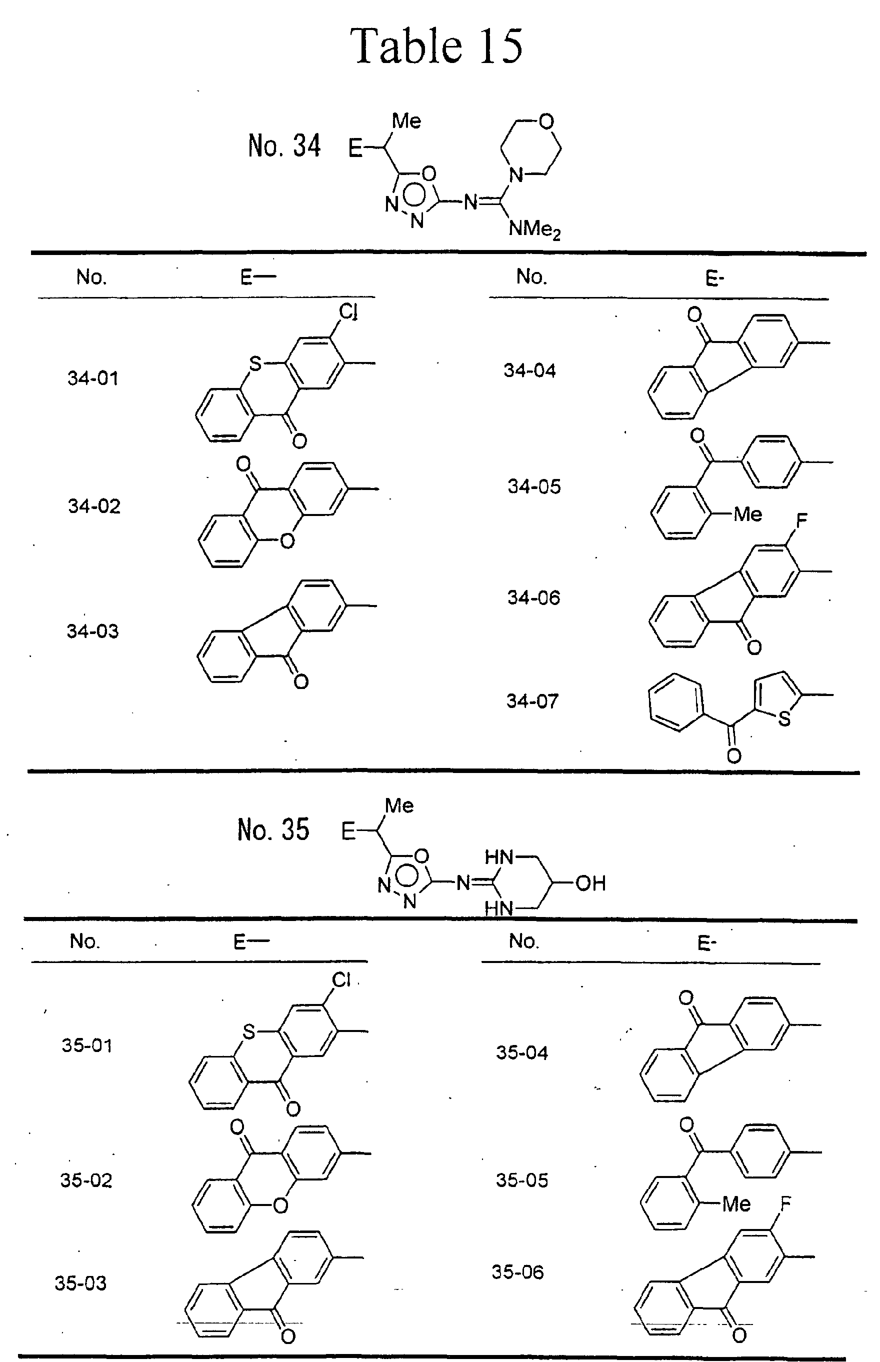

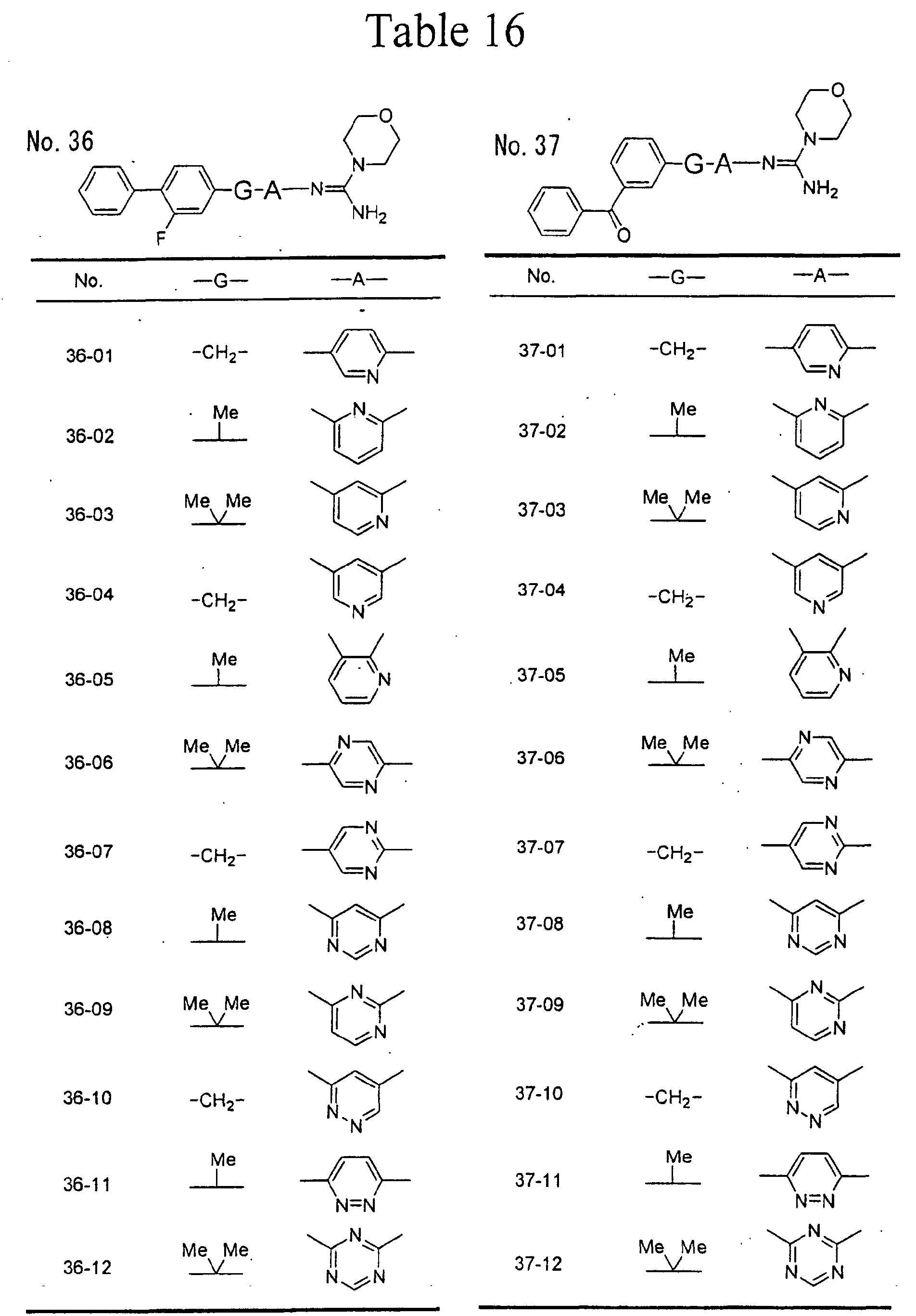

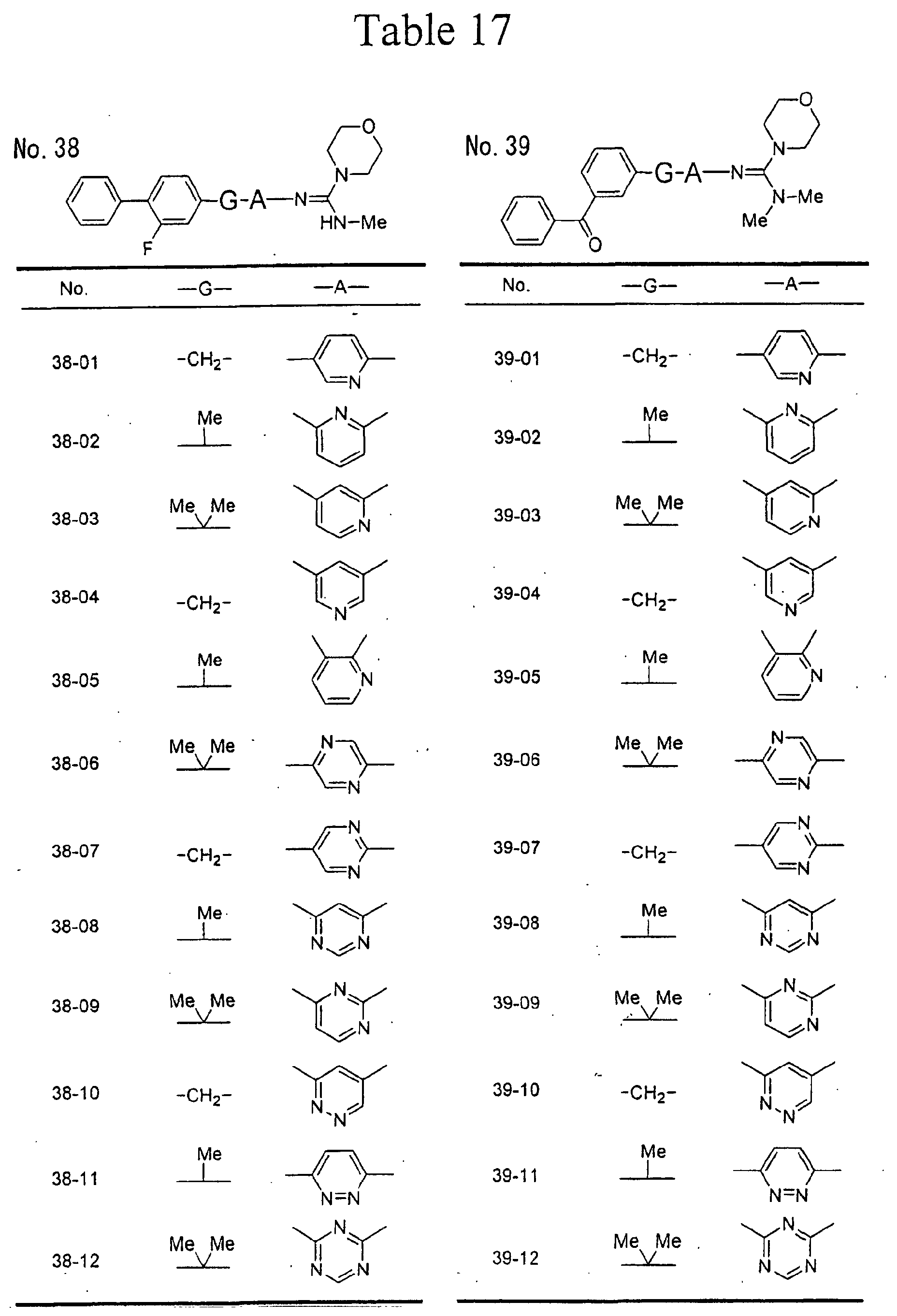

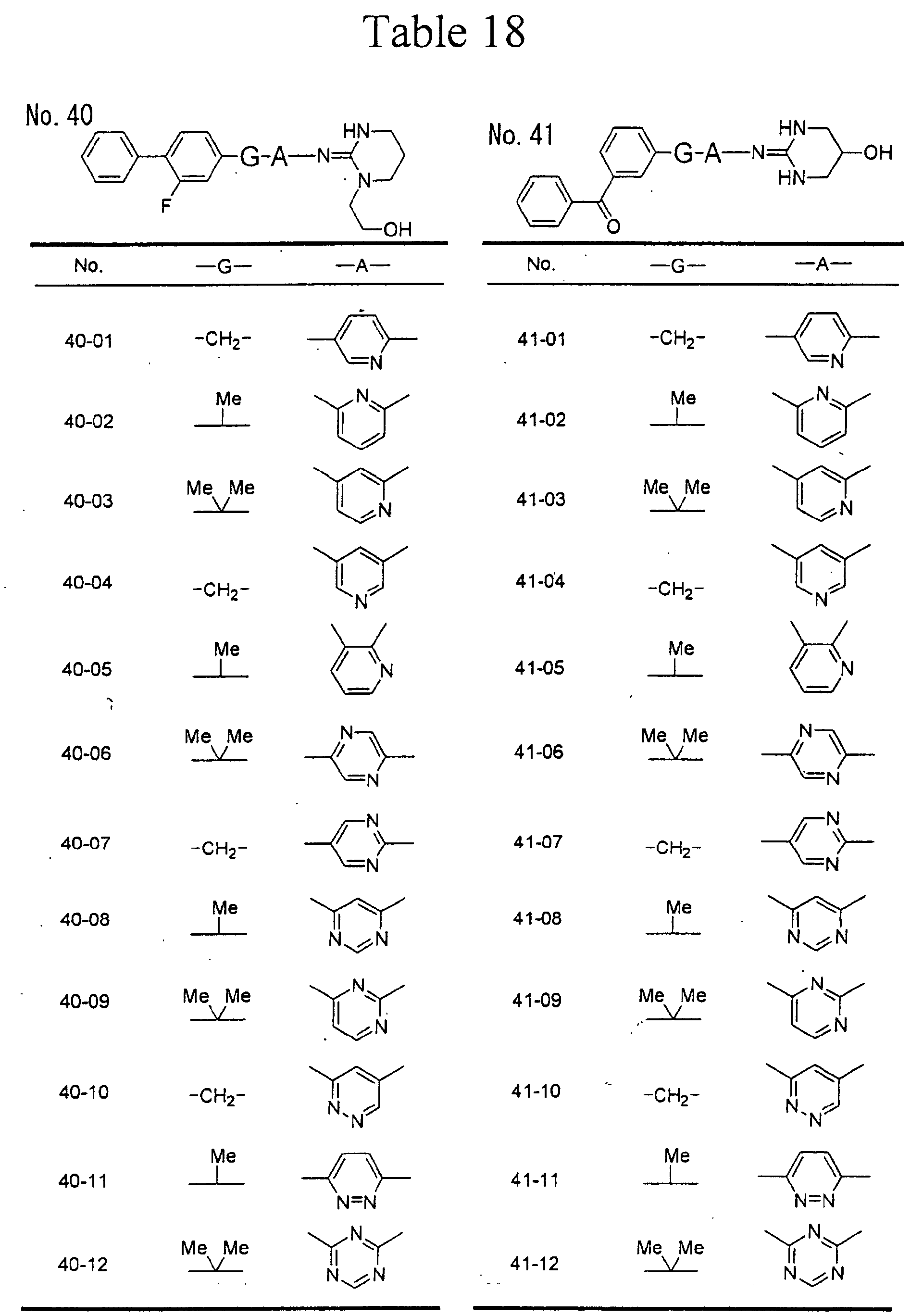

### Examples

The present invention is explained below with examples and reference examples but is, of course, not limited by them.

In the examples and the like, the meanings of the abbreviations used are as follows:
- THF: : tetrahydrofuran
- DMSO: dimethylsulfoxide
- DMF: : dimethylformamide
- IPA: : isopropylalcohol
- TFA: : trifluoroacetic acid
- Et: : ethyl
- Me: : methyl
- Bu-t :: *tert*-butyl
- Bz: : benzoyl
- Boc :: *tert*-butoxycarbonyl

### Example 1

### N'-{3-[1-(3-Benzoylphenyl)ethyl]-5-isothiazolyl}-N-methyl-4-morpholine carboximidamide hydrochloride

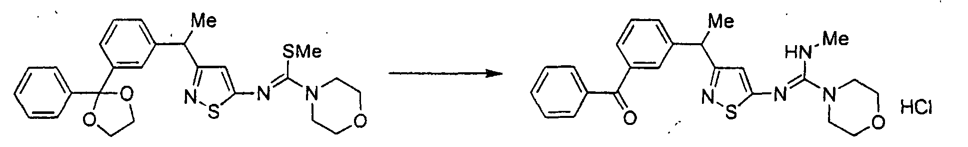

The desired compound was obtained from the compound obtained in Reference Example 4 and methylamine by the same procedure as in Example 7 and Example 6.
¹H-NMR(300MHz, CDCl₃) δ ppm: 1.70(d,3H,J=6.6Hz), 3.15(br.s,3H), 3.48(br.s,4H), 3.70(br.s,4H), 4.29(q,1H,J=6.6Hz), 6.74(s,1H),
7.38-7.52(m, 4H), 7.56-7.63(m,2H), 7.7(br.s,1H), 7.75-7.80(m,2H),
9.52(br.s,1H), 11.55(br.s,1H).

### Example 2

### Phenyl-(3-{1-[5-(tetrahydro-2(1H)-pyrimidinylideneamino)-3-isothiazolyl] ethyl}phenyl)methanone hydrochloride

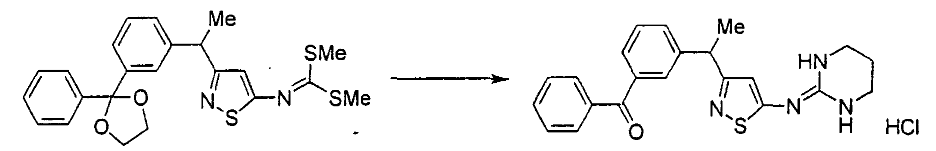

The desired compound was obtained from the compound obtained in Reference Example 3 by the same procedure as in Example 15 and Example 6.
¹H-NMR(300MHz, CDCl₃) δ ppm: 1.67(d,3H,J=7.2Hz), 1.98(br.s,2H), 3.45(br.s,4H), 4.27(q,1H,J=7.2Hz), 6.84(s,1H), 7.36-7.41(m,1H),
7.46-7.65(m,6H), 7.75-7.77(m,2H), 7.91(br.s,2H), 11.44(br.s,1H).

### Example 3

### N'-{3-[1-(3-Benzoylphenyl)ethyl]-5-isothiazolyl}-N,N-dimethyl-4-morpholinecarboximidamide hydrochloride

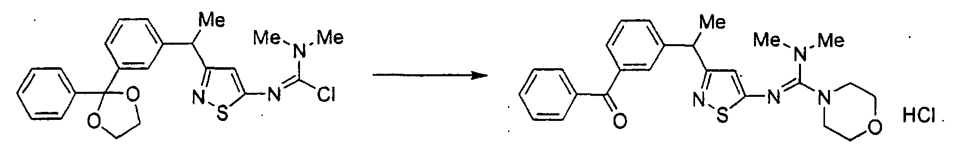

The compound (4.00 g) obtained in Reference Example 5 was dissolved in morpholine (40 mL), and the solution was stirred at 100 °C for 30 min. Morpholine was removed by an evaporator, after which chloroform was added to the resulting residue. The mixture was washed with water, dried and concentrated by an evaporator. The resulting residue was purified with a silica gel column chromatography (chloroform; chloroform/methanol = 96/4) to obtain a guanidine (3.78 g). The guanidine was treated with TFA to remove the acetal-protecting group, followed by treatment with 4N-HCl/dioxane in ethanol to obtain the desired compound (2.95 g).
¹H-NMR(300MHz, CDCl₃) δ ppm: 1.72(d,3H,J=7.2Hz), 3.07(br.s,6H), 3.54(br.s,4H), 3.87(br.s,4H), 4.37(q,1H,J=7.2Hz), 6.91(s,1H),
7.38-7.54(m,4H), 7.56-7.64(m,2H), 7.71(br.s,1H), 7.76-7.81(m,2H).

### Example 4

### N'-{5-[1-(2-Fluoro[1,1'-biphenyl]-4-yl)ethyl]-1,3,4-oxadiazol-2-yl}-4-morpholinecarboximidamide

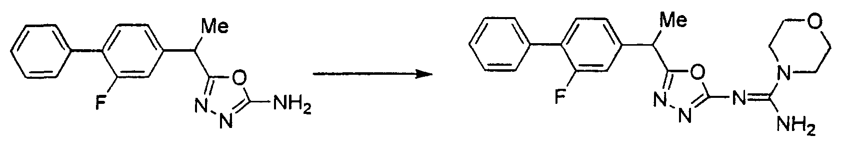

Under a nitrogen atmosphere, a mixture of lithium amide (199 mg), toluene (5 mL) and *tert*-butanol (2.5 g) was kept at 80 °C for 30 min, and nitrogen gas was passed through the reaction vessel at 80 °C for 30 min. The mixture was cooled to room temperature, and a mixture of the compound (1.23 g) obtained in Reference Example 6, toluene (5 mL), cyanomorpholine (486 mg) and *tert*-butanol (1 g) was added dropwise thereto. The mixture was stirred at 80 °C for 3 hours. 5% Brine was added thereto, and the mixture was extracted with chloroform and the resulting organic layer was concentrated under reduced pressure. The starting material (369 mg) was recovered by crystallization in chloroform from the mixture. The mother liquid was concentrated under reduced pressure, followed by crystallization in ethanol/water to obtain the desired compound (839 mg).
¹H-NMR(300MHz, CDCl₃) δ ppm: 1.74(d, 3H, J=7.2Hz), 3.51-3.54(m, 4H), 3.72-3.75(m, 4H), 4.25(q, 1H, J=7.2Hz), 6.97(br-s, 2H), 7.10-7.18(m, 2H), 7.33-7.46(m, 4H), 7.50-7.53(m, 2H)
IR(KBr)[cm⁻¹]: 3364, 3204, 2978, 2863, 1652, 1583, 1537, 1499, 1485, 1451, 1422, 1377, 1292, 1271, 1227, 1161, 1121

### Example 5

### N'-(5-{1-[3-(2-Phenyl-1,3-dioxolan-2-yl)phenyl]ethyl}-1,3,4-oxadiazol-2-yl)-4-morpholinecarboximidamide

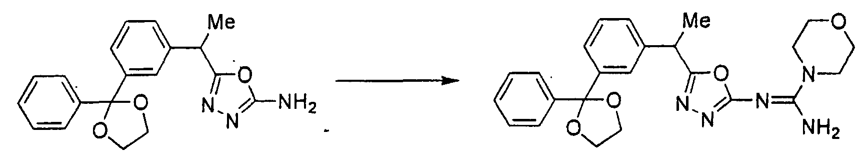

Under a nitrogen atmosphere, a mixture of *tert*-butanol (1.48 g) and toluene (5 mL) was added dropwise to a mixture of lithium amide (459 mg) and toluene (15 mL) at 80 °C, and nitrogen gas was passed through the reaction vessel at 80 °C for 30 min. To this mixture was added dropwise a toluene solution (15 mL) of the compound (3.37 g) obtained in Reference Example 7 and cyanomorpholine (1.12 g), which taking 40 min, and stirred at 80 °C for 5 hours. Water was added thereto, and the mixture was extracted with chloroform. The resulting organic layer was washed with water, dried and concentrated under reduced pressure The resulting residue was purified with a silica gel column chromatography (chloroform/methanol = 50/1) to obtain the desired compound (2.69 g, white amorphous form).
¹H-NMR(270MHz, CDCl₃) δ ppm: 1.68(d, 3H, J=7.3Hz), 3.49-3.52(m, 4H), 3.71-3.74(m, 4H), 4.04(s, 4H), 4.20(q, 1H, J=7.3Hz), 6.95(br-s, 2H), 7.23-7.36(m, 6H), 7.48-7.51(m, 3H)

### Example 6

### N'-{5-[1-(3-Benzoylphenyl)ethyl]-1,3,4-oxadiazol-2-yl}-4-morpholine carboximidamide hydrochloride

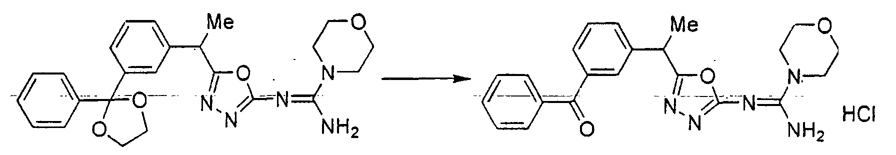

Under a nitrogen atmosphere, the compound (2.53 g) obtained in Example 5 was dissolved in TFA (10 mL), and water (2 mL) was added thereto and stirred for 1 day. The mixture was concentrated under reduced pressure. Toluene was added to the residue, followed by concentration, which operation was repeated twice. The resulting residue was purified with a silica gel column chromatography (chloroform/methanol = 100/1) to give a crude compound (1.89 g), which was treated with 1N-HCl/ether to obtain the desired compound (1.48 g).
¹H-NMR(300MHz, CD₃OD) δ ppm: 1.71(d, 3H, J=7.2Hz), 3.59-3.62(m, 4H), 3.68(m, 4H), 4.39(q, 1H, J=7.2Hz), 7.51-7.56(m, 3H), 7.63-7.71(m, 3H), 7.76-7,79(m, 3H)

### Example 7

### N-Methyl-N'-(5-{1-[3-(2-phenyl-1,3-dioxolan-2-yl)phenyl]ethyl}-1,3,4-oxadiazol-2-yl)-4-morpholinecarboximidamide

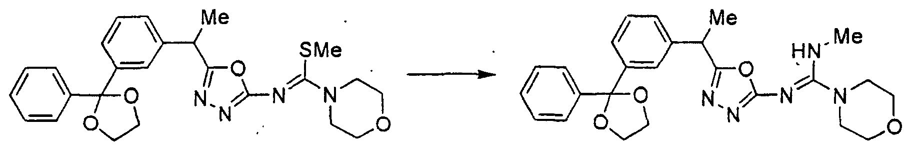

Under a nitrogen atmosphere, the compound (2.94 g) obtained in Reference Example 9 was dissolved in THF (12 mL), and 40% aqueous methylamine solution (7.11 g) was added thereto and stirred at 80 °C for 5 hours. The mixture was concentrated under reduced pressure, and toluene was added thereto. The mixture was concentrated to obtain the desired compound (2.83 g, yellow liquid).
¹H-NMR(270MHz, CDCl₃) δ ppm: 1.68(d, 3H, J=7.2Hz), 2.96(d, 3H, J=5.0Hz), 3.33-3.37(m, 4H), 3.73-3.76(m, 4H), 4.05(s, 4H), 4.21(q, 1H, J=7.2Hz), 7.16-7.37(m, 6H), 7.48-7.51(m, 3H), 8.39(br-d, 1H, J=5.0Hz)

### Example 8

### N'-{5-[1-(3-Benzoylphenyl)ethyl]-1,3,4-oxadiazol-2-yl}-N-methyl-4-morpholinecarboximidamide hydrochloride

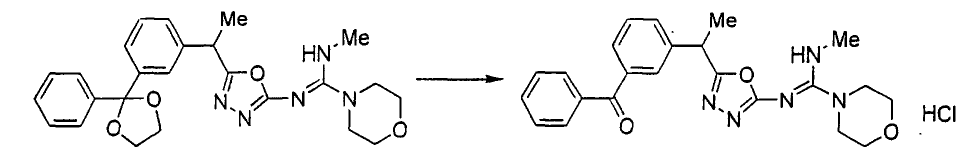

The desired compound was obtained from the compound obtained in Example 7 by the same procedure as in Example 6.
¹H-NMR(300MHz, CD₃OD) δ ppm: 1.70(d, 3H, J=7.2Hz), 2.78(s, 3H), 3.40-3.36(m, 4H), 3.54-3.60(m, 4H), 4.37(q, 1H, J=7.2Hz), 7.51-7.58(m, 3H), 7.62-7.68(m, 3H), 7.76-7.79(m, 3H)

### Example 9

### N,N-Dimethyl-N'-(5-{1-[3-(2-phenyl-1,3-dioxolan-2-yl)phenyl]ethyl}-1,3,4-oxadiazol-2-yl)-4-morpholinecarboximidamide

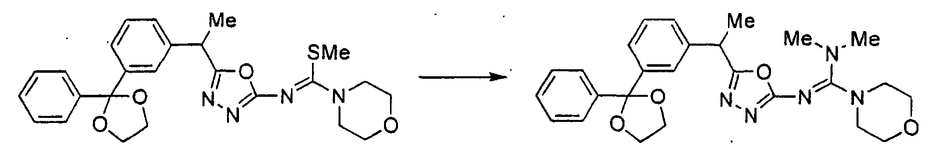

The desired compound was obtained from the compound obtained in Reference Example 9 and dimethylamine by the same procedure as in Example 7.
¹H-NMR(270MHz, CDCl₃) δ ppm: 1.68(d, 3H, J=6.9Hz), 2.76(s, 6H), 3.23-3.26(m, 4H), 3.65-3.68(m, 4H), 4.03(s, 4H), 4.17(q, 1H, J=6.9Hz), 7.24-7.36(m, 6H), 7.46-7.50(m, 3H)

### Example 10

### N'-{5-[1-(3-Benzoylphenyl)ethyl]-1,3,4-oxadiazol-2-yl}-N,N-dimethyl-4-morpholinecarboximidamide hydrochloride

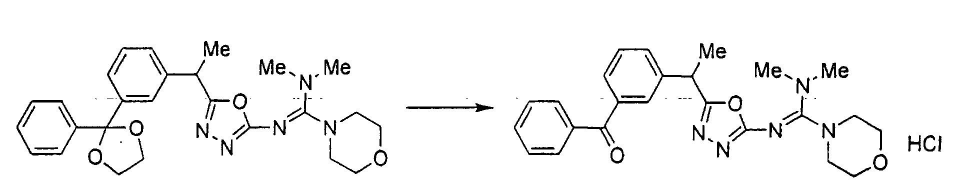

The desired compound was obtained from the compound obtained in Example 9 by the same procedure as in Example 6.
¹H-NMR(300MHz, CD₃OD) δ ppm: 1.75(d, 3H, J=7.2Hz), 2.96(s, 6H), 3.29-3.34(m, 4H), 3.56-3.63(m, 4H), 4.45(q, 1H, J=7.2Hz), 7.51-7.60(m, 3H), 7.64-7.72(m, 3H), 7.77-7.81(m, 3H)

### Example 11

### N-(2-Hydroxyethyl)-N'-(5-{1-[3-(2-phenyl-1,3-dioxolan-2-yl)phenyl]ethyl}-1,3,4-oxadiazol-2-yl)-4-morpholinecarboximidamide

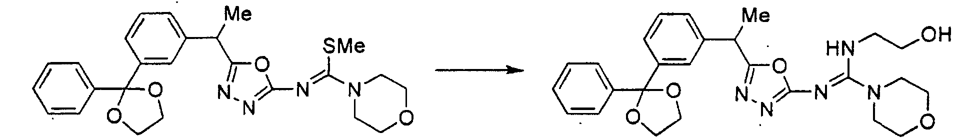

The desired compound was obtained from the compound obtained in Reference Example 9 and ethanolamine by the same procedure as in Example 7.
¹H-NMR(270MHz, CDCl₃) δ ppm: 1.69(d, 3H, J=7.2Hz), 3.31-3.36(m, 2H), 3.43-3.46(m, 4H), 3.71-3.75(m, 6H), 4.05(s, 4H), 4.20(q, 1H, J=7.2Hz), 6.47-7.47(m, 1H), 7.22-7.38(m, 6H), 7.47-7.52(m, 3H)

### Example 12

### N'-{5-[1-(3-Benzoylphenyl)ethyl]-1,3,4-oxadiazol-2-yl}-N-(2-hydroxyethyl)-4-morpholinecarboximidamide hydrochloride

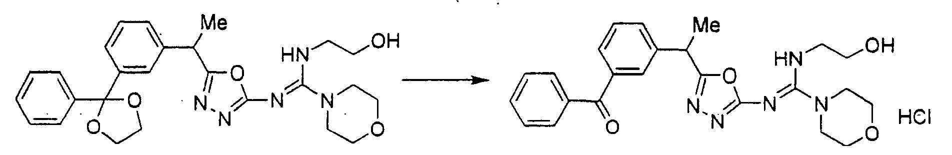

The desired compound was obtained from the compound obtained in Example 11 by the same procedure as in Example 6.
¹H-NMR(300MHz, CD₃OD) δ 1.70(d, 3H, J=7.2Hz), 3.25-3.44(m, 6H), 3.51-3.63(m, 6H), 4.36(q, 1H, J=7.2Hz), 7.51-7.58(m, 3H), 7.62-7.71(m, 3H), 7.77-7.79(m, 3H)

### Example 13

### N-(2-Imidazolidinylidene)-5-{1-[3-(2-phenyl-1,3-dioxolan-2-yl)phenyl] ethyl}-1,3,4-oxadiazol-2-amine

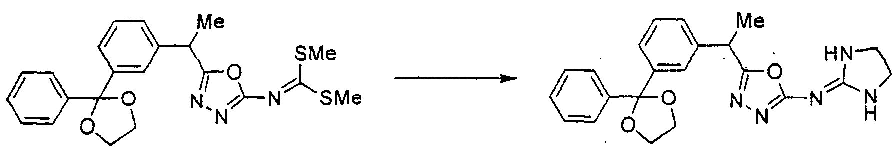

The compound (2.97 g) obtained in Reference Example 8 was dissolved in THF (300 mL), and a THF solution (5 mL) of ethylenediamine (404 mg) was added thereto and stirred at room temperature for 13 hours. The mixture was concentrated under reduced pressure. The resulting residue was purified with a silica gel column chromatography (chloroform/methanol = 1/0 - 100/1 - 50/1) to obtain the desired compound (2.46 g, 90%, white amorphous form).
¹H-NMR(300MHz, CDCl₃) δ ppm: 1.67(d, 3H, J=7.2Hz), 4.04(br-s, 4H), 4.20(q, 1H, J=7.2Hz), 7.22-7.36(m, 6H), 7.47-7.50(m, 3H)

### Example 14

### (3-{1-[5-(2-Imidazolidinylideneamino)-1,3,4-oxadiazol-2-yl]ethyl}phenyl) (phenyl)methanone hydrochloride

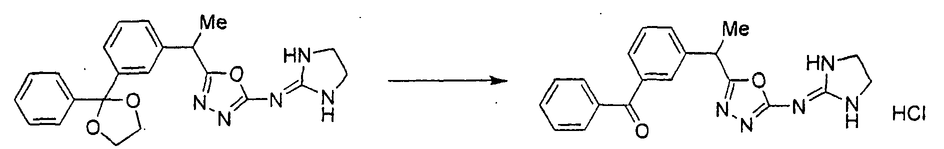

The desired compound was obtained from the compound obtained in Example 13 by the same procedure as in Example 6.
¹H-NMR(300MHz, d₆-DMSO) δ ppm: 1.61(d, 3H, J=7.1Hz), 3.59(s, 4H), 4.47(q, 1H, J=7.1Hz), 7.52-7.74(m, 9H), 8.04(br-s, 2H)

### Example 15

### 5-{1-[3-(2-Phenyl-1,3-dioxolan-2-yl)phenyl]ethyl}-N-tetrahydro-2(1H)-pyrimidinylidene-1,3,4-oxadiazol-2-amine

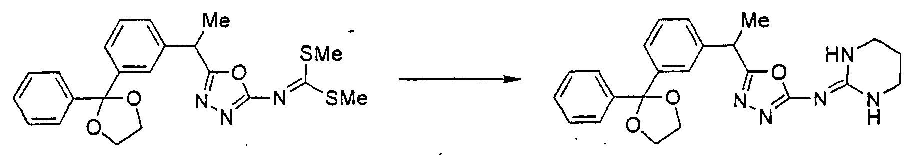

The desired compound was obtained from the compound obtained in Reference Example 8 and 1,3-diaminopropane by the same procedure as in Example 13.
¹H-NMR(300MHz, CDCl₃) δ ppm: 1.66(d, 3H, J=7.2Hz), 1.73-1.81(m, 2H), 3.22(br-s, 4H), 4.03(s, 4H), 4.16(q, 1H, J=7.2Hz), 7.17-7.34(m, 6H), 7.47-7.50(m, 3H)

### Example 16

### Phenyl(3-{1-[5-(tetrahydro-2(1H)-pyrimidinylideneamino)-1,3,4-oxadiazol-2-yl]ethyl}phenyl)methanone hydrochloride

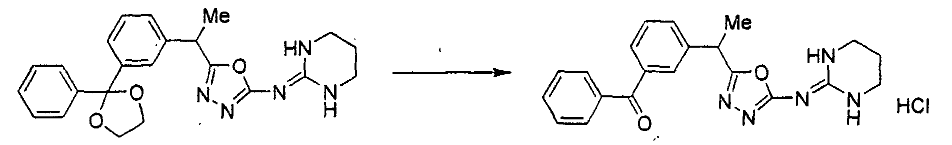

The desired compound was obtained from the compound obtained in Example 15 by the same procedure as in Example 6.
¹H-NMR(300MHz, d₆-DMSO) δ ppm: 1.61(d, 3H, J=7.2Hz), 1.77-1.87(m, 2H), 3.28-3.36(m, 4H), 4.50(q, 1H, J=7.2Hz), 7.54-7.74(m, 9H), 8,48(br-s, 2H)

### Example 17

### 2-[(5-{1-[3-(2-Phenyl-1,3-dioxolan-2-yl)phenyl]ethyl}-1,3,4-oxadiazol-2-yl) imino]hexahydro-5-pyrimidinol

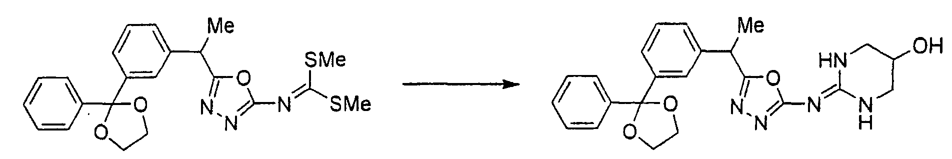

The desired compound was obtained from the compound obtained in Reference Example 8 and 1,3-diamino-2-propanol by the same procedure as in Example 13.
¹H-NMR(300MHz, d₆-DMSO) δ ppm: 1.52(d, 3H, J=7.2Hz), 3.08-3.18(m, 2H), 3.29-3.38(m, 2H), 3.96(br-s, 5H), 4.23(q, 1H, J=7.2Hz), 5.19(d, 1H, J=3.7Hz), 7.15-7.18(m, 1H), 7.25-7.36(m, 5H), 7.40-7.43(m, 3H), 7.72(br-s, 2H)

### Example 18

### [3-(1-{5-[(5-Hydroxytetrahydro-2(1H)-pyrimidinylidene)amino]-1,3,4-oxadiazol-2-yl}ethyl)phenyl](phenyl)methanone hydrochloride

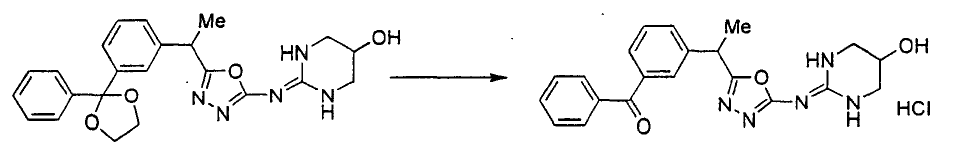

The desired compound was obtained from the compound obtained in Example 17 by the same procedure as in Example 6.
¹H-NMR(300MHz, d₆-DMSO) δ ppm: 1.61(d, 3H, J=7.3Hz), 3.19-3.38(m, 4H), 4.01-4.07(m, 1H), 4.50(q, 1H, J=7.3Hz), 7.54-7.74(m, 9H), 8.41(br-s, 2H)

### Example 19

### N-Methyl-N'-(5-{1-[3-(2-phenyl-1,3-dioxolan-2-yl)phenyl]ethyl}-1,3,4-oxadiazol-2-yl)-4-thiomorpholinecarboximidamide

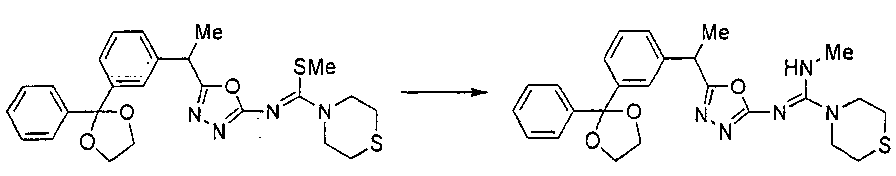

The desired compound was obtained from the compound obtained in Reference Example 10 and methylamine by the same procedure as in Example 7.
¹H-NMR(300MHz, CDCl₃) δ ppm: 1.68(d, 3H, J=7.2Hz), 2.68-2.72(m,-4H), 2.93(d; 3H, J=5.0Hz), 3.59-3.63(m, 4H), 4.05(s, 4H), 4.21(q, 1H, J=7.2Hz), 7.21-7.37(m, 6H), 7.48-7.51(m, 3H), 8.33-8.41(m, 1H)

### Example 20

### N'-{5-[1-(3-Benzoylphenyl)ethyl]-1,3,4-oxadiazol-2-yl}-N-methyl-4-thiomorpholinecarboximidamide hydrochloride

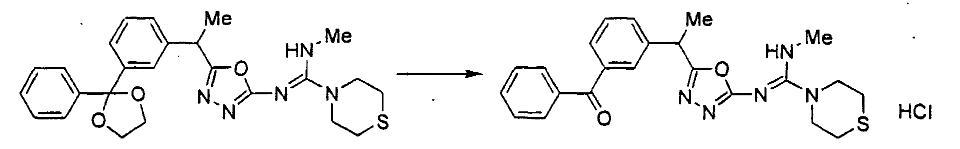

The desired compound was obtained from the compound obtained in Example 19 by the same procedure as in Example 6.
¹H-NMR(400MHz, d₆-DMSO) δ ppm: 1.55(s, 3H, J=7.1Hz), 2.58-2.59(m, 4H), 2.63(d, 3H, J=4.4Hz), 3.68-3.70(q, 1H, J=7.1Hz), 7.50-7.55(m, 3H), 7.60-7.70(m, 6H), 8.80(br, 1H)

### Example 21

### N,N-Dimethyl-N'-(5-{1-[3-(2-phenyl-1,3-dioxolan-2-yl)phenyl]ethyl}-1,3,4-oxadiazol-2-yl)-4-thiomorpholinecarboximidamide

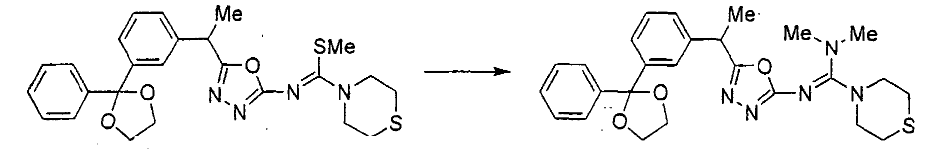

The desired compound was obtained from the compound obtained in Reference Example 10 and dimethylamine by the same procedure as in Example 7.
¹H-NMR(300MHz, CDCl₃) δ ppm: 1.69(d, 3H, J=7.2Hz), 2.59(m, 4H), 2.76(s, 6H), 3.45-3.48(m, 4H), 4.03(s, 4H), 4.20(q, 1H, J=7.2Hz), 7.22-7.37(m, 6H), 7.47-7.50(m, 3H)

### Example 22

### N'-{5-[1-(3-Benzoylphenyl)ethyl]-1,3,4-oxadiazol-2-yl}-N,N-dimethyl-4-thiomorpholinecarboximidamide hydrochloride

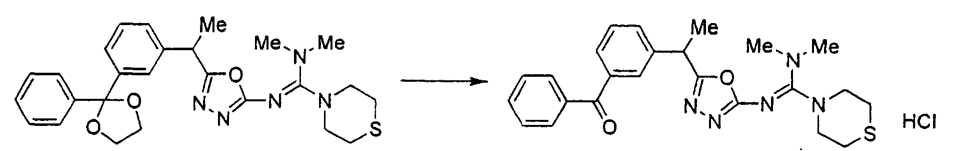

The desired compound was obtained from the compound obtained in Example 21 by the same procedure as in Example 6.
¹H-NMR(400MHz, d₆-DMSO) δ ppm: 1.63(d, 3H, J=7.1Hz), 2.61-2.63(m, 4H), 2.87(s, 6H), 3.42(br, 4H), 4.53(q, 1H, J=7.1Hz), 7.54-7.59(m, 3H), 7.64-7.74(m, 6H)

### Example 23

### N-(2-Hydroxyethyl)-N'-(5-{1-[3-(2-phenyl-1,3-dioxolan-2-yl)phenyl]ethyl}-1,3,4-oxadiazol-2-yl)-4-thiomorpholinecarboximidamide

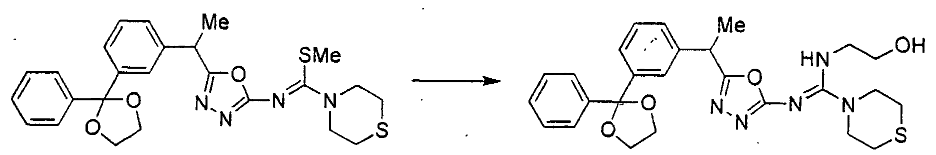

The desired compound was obtained from the compound obtained in Reference Example 10 and ethanolamine by the same procedure as in Example 7.
¹H-NMR(300MHz, CDCl₃) δ ppm: 1.69(d, 3H, J=7.2Hz), 2.67-2.70(m, 4H), 3.28-3.31(m, 4H), 3.68-3.76(m, 4H+2H), 4.05(s, 4H), 4.20(q, 1H, J=7.2Hz), 6.82-6.88(m, 1H), 7.21-7.37(m, 6H), 7.49-7.52(m, 3H)

### Example 24

### N'-{5-[1-(3-Benzoylphenyl)ethyl]-1,3,4-oxadiazol-2-yl}-N-(2-hydroxvethyl)-4-thiomorpholinecarboximidamide hydrochloride

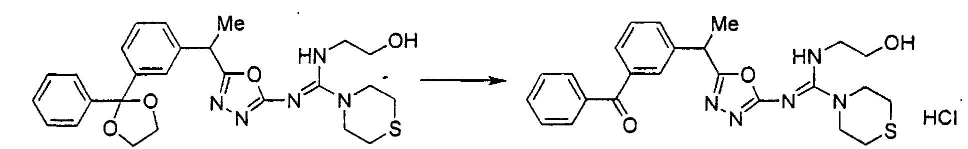

The desired compound was obtained from the compound obtained in Example 23 by the same procedure as in Example 6.
¹H-NMR(400MHz, d₆-DMSO) δ ppm: 1.59(d, 3H, J=7.1Hz), 2.61-2.62(m, 4H), 3.12-3.18(m, 2H), 3.43(t, 2H, J=5.5Hz), 3.70-3.71(m, 4H), 4.46(q, 1H, 7.1Hz), 7.54-7.58(m, 3H), 7.64-7.74(m, 6H), 8.70(br, 1H)

### Example 25

### N'-{5-[1-(3-Benzoylphenyl)ethyl]-1,3,4-oxadiazol-2-yl}-N-methyl-4-thiomorpholinecarboximidamide 1,1-dioxide hydrochloride

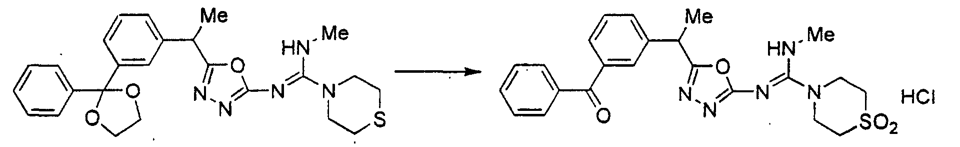

The desired compound was obtained from the compound obtained in Example 19 by the same procedure as in Example 27.
¹H-NMR(400MHz, d₆-DMSO) δ ppm: 1.60(d, 3H, J=7.2Hz), 2.69(d, 3H, J=4.4Hz), 3.32(br, 4H), 3.91(br, 4H), 4.48(q, 1H, J=7.1Hz), 7.56-7.59(m, 3H), 7.65-7.74(m, 6H), 9.09(br, 1H)

### Example 26

### N'-{5-[1-(3-Benzoylphenyl)ethyl]-1,3,4-oxadiazol-2-yl}-N,N-dimethyl-4-thiomorpholinecarboximidamide 1,1-dioxide hydrochloride

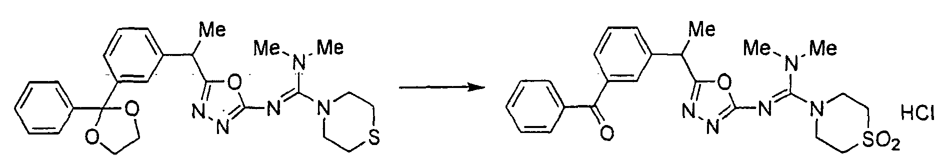

The desired compound was obtained from the compound obtained in Example 21 by the same procedure as in Example 27.
¹H-NMR(400MHz, d₆-DMSO) δ ppm: 1,62(d, 3H, J=7.2H), 2.87(s, 6H), 3.27(br, 4H), 3.65(br, 4H), 4.51(q, 1H, J=7.1Hz), 7.54-7.58(m, 3H), 7.63(m, 6H)

### Example 27

### N'-{5-[1-(3-Benzoylphenyl)ethyl]-1,3,4-oxadiazol-2-yl}-N-(2-hydroxyethyl)-4-thiomorpholinecarboximidamide 1,1-dioxide hydrochloride

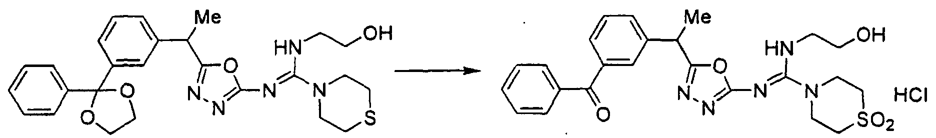

Under a nitrogen atmosphere, the compound (2.08 g) obtained in Example 23 was dissolved in methanol (35 mL), and an aqueous solution (15 mL) of OXONE (3.26 g) was added dropwise thereto, which taking 5 min and stirred at room temperature for 3.5 hours. Water was added thereto and the mixture was extracted with chloroform, washed with 5% brine, dried and concentrated under reduced pressure. The resulting residue was dissolved in TFA (10 mL) and water (0.5 mL) was added thereto at 0 °C and stirred at room temperature for 30 min. The mixture was concentrated under reduced pressure, and chloroform was added thereto, followed by neutralization with an aqueous saturated sodium bicarbonate. The organic layer was washed with 5% brine, dried, and concentrated under reduced pressure. The residue was purified with a silica gel column chromatography (chloroform/methanol = 1/0 - 100/1 - 50/1) to give a crude compound (2.01 g, 99%, white amorphous form), which was treated with 1N-HCl/ether to obtain the desired compound (1.99 g, 92%, white crystals).
¹H-NMR(400MHz, d₆-DMSO) δ ppm: 1.60(d, 3H, J=7.2Hz), 3.12-3.23(m, 2H), 3.29(br, 4H), 3.45(t, 2H, J=5.5Hz), 3.88(br, 4H), 4.47(q, 1H, J=7.1Hz), 7.55-7.58(m, 3H), 7.64-7.75(m, 6H), 8.90(br, 1H)

### Example 28

### N'-[5-(5-Benzoyl-2-methylbenzyl)-1,3,4-oxadiazol-2-yl]-4-morpholine carboximidamide

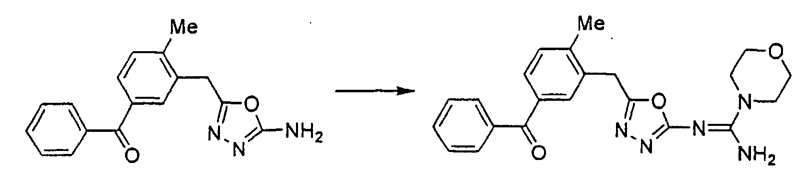

Under a nitrogen atmosphere, a toluene solution (2 mL) of *tert*-butanol (51 mg) was added dropwise to a toluene solution (2 mL) of lithium amide (16 mg) at 80 °C. To this mixture was added dropwise a toluene suspension (3 mL) of the compound (0.10 g) obtained in Reference Example 16 and cyanomorpholine (38 mg), and stirred for 3 hours. After cooling to room temperature, the mixture was added to water (20 mL), and the mixture was extracted with ethyl acetate (20 mL). The organic layer was washed with a saturated brine, dried over sodium sulfate and concentrated under reduced pressure The resulting residue was purified with a silica gel column chromatography (chloroform/methanol = 20/1) to obtain the desired compound (78 mg).
¹H-NMR(300MHz, CDCl₃) δ ppm: 2.46 (s, 3H), 3.52 (t, 4H, J=5.0 Hz), 3.73 (t, 4H, J=5.0 Hz), 4.11 (s, 2H), 6.96 (brs, 2H), 7.28 (d, 1H, J=7.0 Hz), 7.44-7.63 (m, 4H), 7.74-7.79 (m, 3H).

### Example 29

### N-[Di(4-morpholinyl)methylene]-5-[1-(2-fluoro[1,1'-biphenyl]-4-yl)ethyl]-1,3,4-thiadiazol-2-amine hydrochloride

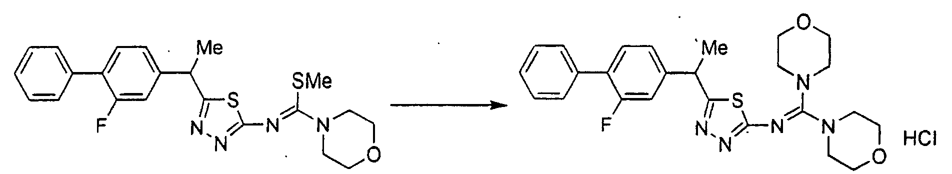

The desired compound was obtained from the compound obtained in Reference Example 19 and morpholine by the same procedure as in Example 42.
¹H-NMR(300MHz, CDCl₃) δ ppm: 1.81(d, 3H, J=7.2Hz), 3.55(br, 8H), 3.88(br, 8H), 4.46(q, 1H, J=7.2Hz), 7.06-7.18(m, 2H), 7.34-7.56(m, 6H)

### Example 30

### N'-{5-[1-(2-Fluoro[1,1'-biphenyl]-4-yl)ethyl]-1,3,4-thiadiazol-2-yl}-N,N-dimethyl-4-morpholinecarboximidamide hydrochloride

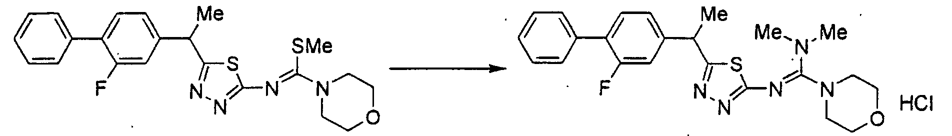

The desired compound was obtained from the compound obtained in Reference Example 19 and dimethylamine by the same procedure as in Example 42.
¹H-NMR(300MHz, CDCl₃) δ ppm: 1.80(d, 3H, J=7.2Hz), 2.99(s, 6H), 3.38-3.48(m, 4H), 3.77-3.84(m, 4H), 4.47(q, 1H, J=7.2Hz), 7.08-7.19(m, 2H), 7.33-7.56(m, 6H)

### Example 31

### N'-{5-[1-(2-Fluoro[1,1'-biphenyl]-4-yl)ethyl]-1,3,4-thiadiazol-2-yl}-N-[2-(4-morpholinyl)ethyl]-4-morpholinecarboximidamide

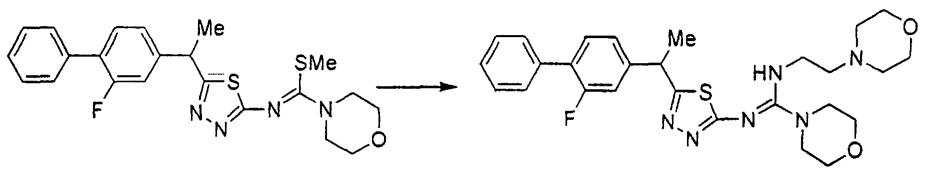

The desired compound was obtained from the compound obtained in Reference Example 19 and 4-(2-aminoethyl)morpholine by the same procedure as in Example 39.
¹H-NMR(300MHz, CDCl₃) δ ppm: 1.78(d, 3H, J=7.2Hz), 2.41-2.48(m, 4H), 2.54(t, 2H, J=6.3Hz), 3.30(brq, 2H, J=6.3Hz), 3.33-3.40(m, 4H), 3.63-3.76(m, 8H), 4.46(q, 1H, J=7.2Hz), 7.08-7.20(m, 2H), 7.35-7.54(m, 6H), 8.74(br, 1H)

### Example 32

### N-{5-[1-(2-Fluoro[1,1'-biphenyl]-4-yl)ethyl]-1,3,4-thiadiazol-2-yl}-N-tetrahvdro-2(1H)-pyrimidinylideneamine

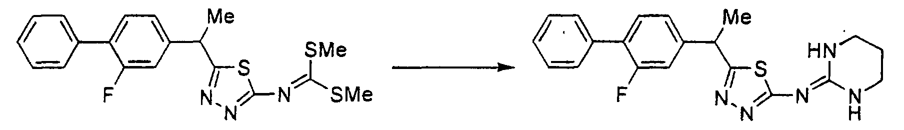

The desired compound was obtained from the compound obtained in Reference Example 18 and 1,3-diaminopropane by the same procedure as in Example 44.
¹H-NMR(300MHz, CDCl₃) δ ppm: 1.76(d, 3H, J=7.2Hz), 1.95(m, 1H), 3.37-3.44(m, 4H), 4.39(q, 1H, J=7.2Hz), 7.08-7.18(m, 2H), 7.32-7.55(m, 6H)

### Example 33

### N-(1,3-Dimethyl-2-imidazolidinvlidene)-N-{5-[1-(2-fluoro[1,1'-biphenyl]-4-yl)ethyl]-1,3,4-thiadiazol-2-yl}amine

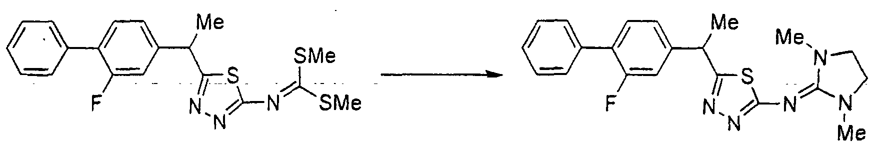

The desired compound was obtained from the compound obtained in Reference Example 18 and N,N'-dimethylethylenediamine by the same procedure as in Example 44.
¹H-NMR(300MHz, CDCl₃) δ ppm: 1.77(d, 3H, J=7.2Hz), 2.83(s, 6H), 3.46(s, 4H), 4.45(q, 1H, J=7.2Hz), 7.10-7.21(m, 2H), 7.32-7.57(m, 6H)

### Example 34

### 5-[1-(2-Fluoro[1,1'-biphenyl]-4-yl)ethyl]-N-(2-imidazolidinylidene)-1,3,4-thiadiazol-2-amine

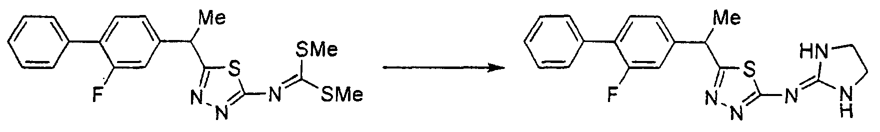

The desired compound was obtained from the compound obtained in Reference Example 18 and ethylenediamine by the same procedure as in Example 44.
¹H-NMR(300MHz, CDCl₃) δ ppm: 1:77(d, 3H, J=7.2Hz), 3.69(br, 4H), 4.43(q, 1H, J=7.2Hz), 7.08-7.18(m, 2H), 7.32-7.55(m, 6H)

### Example 35

### 2-({5-[1-(2-Fluoro[1,1'-biphenyl]-4-yl)ethyl]-1,3,4-thiadiazol-2-yl}imino) hexahydro-5-pyrimidinol

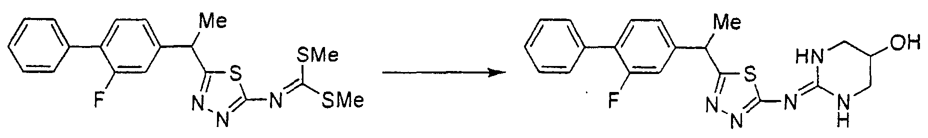

The desired compound was obtained from the compound obtained in Reference Example 18 and 1,3-diamino-2-hydroxypropane by the same procedure as in Example 44.
¹H-NMR (300MHz, d₆-DMSO) δ ppm:1.63(d, 3H, J=7.2Hz), 3.07-3.17(m, 2H), 3.28-3.37(m, 2H), 3.94(m, 1H), 4.46(q, 1H, J=7.2Hz), 5.18(d, 1H, J=3.5Hz), 7.18-7.26(m, 2H), 7.35-7.55(m, 6H), 8.06(br, 2H)

### Example 36

### N'-{5-[1-(2-Fluoro[1,1'-biphenyl]-4-yl)ethyl]-1,3,4-thiadiazol-2-yl}-4-morpholinecarboximidamide hydrochloride

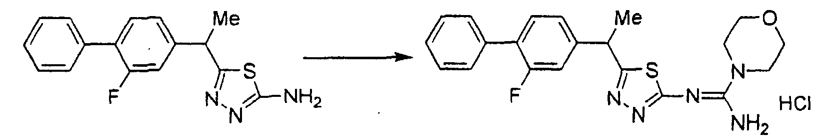

A THF solution (50 mL) of the compound (2 g) obtained in Reference Example 17 was added to a THF suspension (10 mL) of 60% NaH (0.54 g) at 0 °C. Fifteen minutes later, cyanomorpholine (0.98 mL) was added thereto and stirred for 1 hour. After warmed to room temperature, the mixture was stirred overnight. Water was added thereto and the mixture was extracted with ethyl acetate. The organic layer was washed with water and dried. The solvent was evaporated under reduced pressure. The residue was purified with a silica gel column chromatography to give a crude compound (1.7 g), which was dissolverd in dioxane (15 mL) and treated with 4N-HCl/dioxane to obtain the desired compound (1.1 g).
Melting points: 199 - 202 °C
¹H-NMR(300MHz, CDCl₃) δ ppm: 1.79(d, 3H, J=7.2Hz), 3.78-3.89(m, 8H), 4.51(q, 1H, J=7.2Hz), 7.04-7.16(m, 2H), 7.33-7.53(m, 6H)
IR(KBr)[cm⁻¹]: 3440, 3215, 3096, 2856, 1652, 1620, 1540, 1471, 1120, 698
Elementary analysis(%) : Calculated: C, 51.31; H, 5.17; N, 15.63; Cl, 7.91, Found: C, 56.30; H, 5.21, N, 15.72; Cl, 8.11

### Example 37

### N'-{5-[1-(3-Benzoylphenyl)ethyl]-1,3,4-thiadiazol-2-yl}-4-morpholine carboximidamide

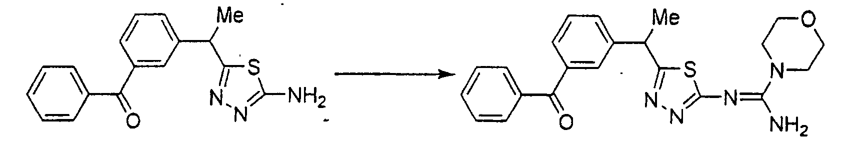

A THF solution (15 mL) of the compound (2 g) obtained in Reference Example 22 was added to a THF suspension (20 mL) of 60% NaH (0.54 g) at 0 °C. Thirty minutes later, cyanomorpholine (0.99 mL) was added thereto and stirred for 30 min. After warmed to room temperature, the mixture was stirred for 2 days. Water was added thereto and the mixture was extracted with ethyl acetate. The organic layer was washed with water and dried. The solvent was evaporated under reduced pressure. The residue was purified with a silica gel column chromatography to give a crude compound (2.0 g), which was recystallized in IPA to obtain the desired compound (1.5 g).
¹H-NMR(300MHz, CDCl₃) δ ppm: 1.78(d, 3H, J=7.2Hz), 3.47-3.55(m, 4H), 3.70-3.78(m, 4H), 4.47(q, 1H, J=7.2Hz), 7.39-7.69(m, 6H), 7.76-7.82(m, 3H)

### Example 38

### N-Cyano-N'-{3-[1-(2-fluoro[1,1'-biphenyl]-4-yl)ethyl]-1,2,4-thiadiazol-5-yl}-4-morpholinecarboximidamide

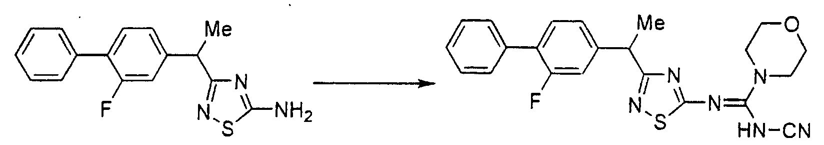

The compound (2 g) obtained in Reference Example 25 was dissolved in THF (10 mL), and 60% NaH in oil (536 mg) was added thereto at room temperature. Then the compound (2.48 g) obtained in Reference Example 56 was added thereto and stirred for 1 hour. The reaction mixture was poured into an aqueous saturated ammonium chloride solution, and extracted with ethyl acetate, dried over magnesium sulfate and concentrated by an evaporater. The residue was purified with a silica gel flash chromatography (hexane/ethyl acetate = 2/1) to obtain the desired compound (1.87 g, colorless solid).
¹H-NMR(300MHz, CDCl₃) δ ppm: 1.71(d, 3H, J=7.1Hz), 3.68-3.78(m, 8H), 6.11(q, 1H, J=7.1Hz), 6.64(br, 1H), 7.11-7.24(m, 2H), 7:32-7.53(m, 6H)

### Example 39

### N'-{3-[1-(2-Fluoro[1,1'-biphenyl]-4-yl)ethyl]-1,2,4-thiadiazol-5-yl}-N-[(4-morpholinyl)methyl]-4-morpholinecarboximidamide

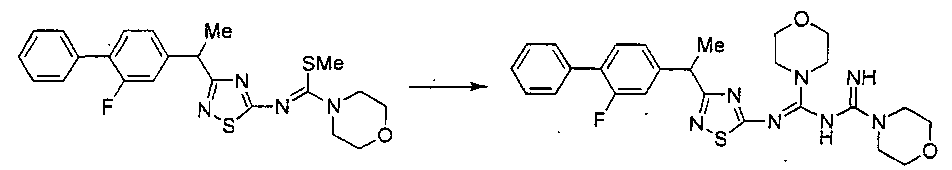

The compound (2.35 g) obtained in Reference Example 27 was dissolved in methylene chloride (25 mL), and sulfuryl chloride (1.29 mL) was added thereto and stirred for 30 min. The solvent and excess sulfuryl chloride were removed under reduced pressure by an evaporater. The residue was dissolved again in methylene chloride (25 mL). Morpholinoformamidine (5.77 g) was added thereto at room temperature and stirred for 13 hours. The reaction mixture was added to a saturated brine, extracted with ethyl acetate, dried over magnesium sulfate and concentrated by an evaporater The residue was purified with a silica gel flash chromatography (chloroform/methanol = 99/1 - 33/1) to obtain the desired compound (1.70 g, colorless solid).
¹H-NMR(300MHz, CDCl₃) δ ppm: 1.72(d, 3H, J=7.1Hz), 3.54-3.76(m, 16H), 4.33(q, 1H J=7.0Hz), 4.69(br, 2H), 7.13-7.51(m, 8H)

### Example 40

### N'-{3-[1-(2-Fluoro[1,1'-biphenyl]-4-yl)ethyl]-1,2,4-thiadiazol-5-yl}-N,N-dimethyl-4-morpholinecarboximidamide

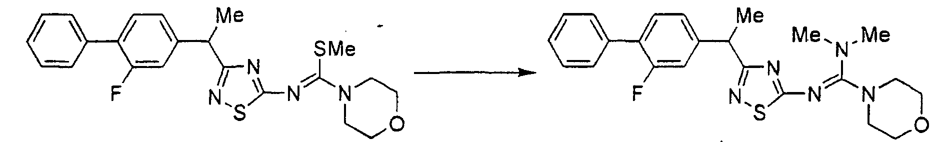

The desired compound was obtained from the compound obtained in Reference Example 27 and dimethylamine by the same procedure as in Example 39.
¹H-NMR(300MHz, CDCl₃) δ ppm: 1.74(d, 3H, J=7.1Hz), 2.80(s, 6H), 3.21-3.25(m, 4H), 3.65-3.68(m, 4H), 4.31(q, 1H, 7.1Hz), 7.16-7.22(m, 2H), 7.31-7.47(m, 4H), 7.51-7.54(m, 2H)

### Example 41

### {3-[1-(5-{[Di(4-morpholinyl)methylene]amino}-1,2,4-thiadiazol-3-yl)ethyl] phenyl}(phenyl)methanone

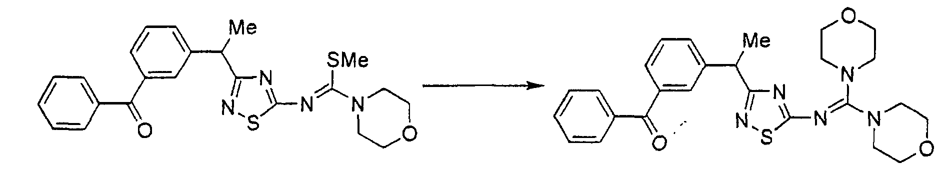

The desired compound was obtained from the compound obtained in Reference Example 31 and morpholine by the same procedure as in Example 39.
¹H-MMR(300MHz, CDCl₃) δ ppm: 1.75(d, 3H 7.1Hz), 3.18-3.20(m, 8H), 3.60-3.63(m, 8H), 4.36(q, 1H, J=7.1Hz), 7.37-7.63(m, 6H), 7.78-7.86(m, 3H)

### Example 42

### N'-{3-[1-(3-Benzoylphenyl)ethyl]-1,2,4-thiadiazol-5-yl}-N,N-dimethyl-4-morpholinecarboximidamide hydrochoride

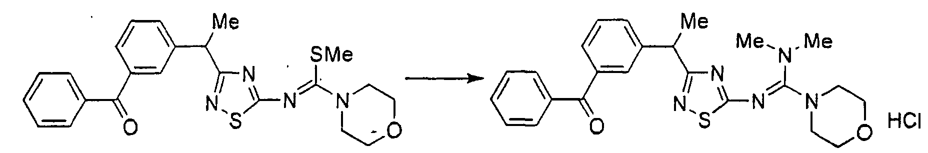

The compound (2.71 g) obtained in Reference Example 31 was dissolved in methylene chloride (12 mL), and sulfuryl chloride (1.44 mL) was added thereto at 0 °C and stirred at room temperature for 30 min. The solvent and excess sulfuryl chloride were removed under reduced pressure by an evaporater. The residue was dissolved again in methylene chloride (12 mL). 40% Aqueous dimethylamine solution (7.52 mL) was added thereto at room temperature and stirred for 1 hour. The reaction mixture was added to a saturated brine, extracted with ethyl acetate, dried over magnesium sulfate and concentrated by an evaporater. The residue was purified with a silica gel flash chromatography (ethyl acetate) to give a crude compound (2.02 g, yellow amorphous form), which was treated with 1N-HCl/ether to obtain the desired compound (1.80 g, colorless solid).
¹H-NMR(300MHz, d₆-DMSO) δ ppm: 1.66(d, 3H, J=7.1Hz), 2.90(s, 6H), 3.34-3.36(m, 4H), 3.62-3.63(m, 4H), 4.46(q, 1H, J=7.0Hz), 7.48-7.71(m, 9H)

### Example 43

### N'-{3-[1-(3-Benzoylphenyl)ethyl]-1,2,4-thiadiazol-5-yl}-N-methyl-4-morpholinecarboximidamide hydrochloride

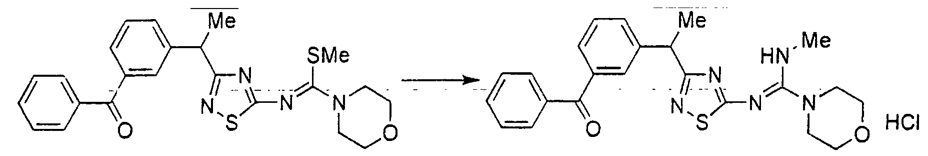

The desired compound was obtained from the compound obtained in Reference Example 31 and methylamine by the same procedure as in Example 42.
¹H-NMR(300MHz, d₆-DMSO) δ ppm: 1.62(d, 3H, J=7.0Hz), 2.78(d, 3H, J=4.4Hz), 3.38-3.40(m, 4H), 3.57-3.59(m, 4H), 4.41(q, 1H, J=7.1Hz), 7.49-7.72(m, 9H), 9.01(br, 1H)

### Example 44

### (3-{1-[5-(2-Imidazolidinylideneamino)-1,2,4-thiadiazol-3-yl]ethyl}phenyl) (phenyl)methanone hydrochloride

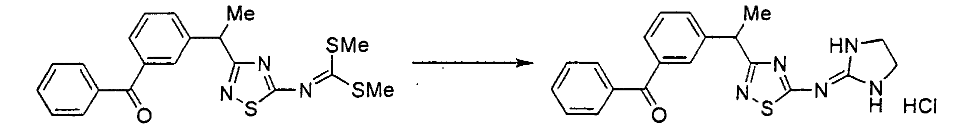

The compound (2.00 g) obtained in Reference Example 30 was dissolved in ethanol (50 mL), and ethylenediamine (0.65 mL) was added thereto at room temperature and stirred for 2 hours. The reaction mixture was concentrated by an evaporater. The residue was added to a saturated brine, extracted with ethyl acetate, dried over magnesium sulfate and concentrated by an evaporater. The residue was purified with a silica gel flash chromatography (ethyl acetate) to give a crude compound (1.32 g, colorless solid), which was treated with 4N-HCl/dioxane to obtain the desired compound (1.47 g, light yellow amorphous form).
¹H-NMR(300MHz, d₆-DMSO) δ ppm: 1.67(d, 3H, J=7.3Hz), 3.72(s, 4H), 4.49(q, 1H, J=7.1Hz), 7.47-7.74(m, 9H), 8.98(br, 2H)

### Example 45

### [3-(1-{5-[(5-Hydroxytetrahydro-2(1H)-pyrimidinylidene)amino]-1,2,4-thiadiazol-3-yl}ethyl)phenyl](phenyl)methanone hydrochloride

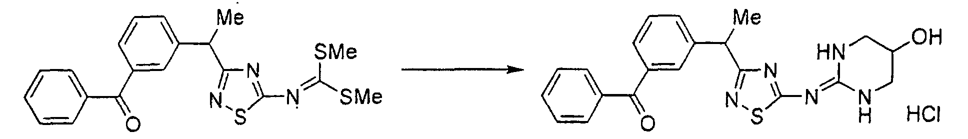

The desired compound was obtained from the compound obtained in Reference Example 30 and 1,3-diamino-2-hydroxypropane by the same procedure as in Example 44.
¹H-NMR(300MHz, d₆-DMSO) δ ppm: 1.66(d, 3H, J=7.1Hz), 3.29-3.90(m, 4H), 4.12(br, 1H), 4.53(q, 1H, J=7.0Hz), 7.48-7.73(m, 9H), 9.10(br, 2H)

### Example 46

### Phenyl(3-{1-[5-(tetrahydro-2(1H)-pyrimidinylideneamino)-1,2,4-thiadiazol-3-yl]ethyl}phenyl)methanone hydrochloride

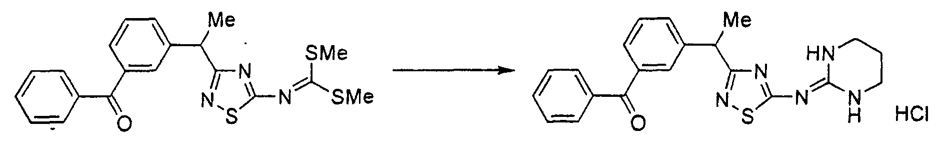

The desired compound was obtained from the compound obtained in Reference Example 30 and 1,3-diaminopropane by the same procedure as in Example 44.
¹H-NMR(300MHz, d₆-DMSO) δ ppm: 1.65(d, 3H, J=7.0Hz), 1.87(br, 2H), 3.39(br, 4H), 4.52(q, 1H, J=7.1Hz), 7.47-7.74(m, 9H), 9.17(br, 2H)

### Example 47

### [3-(1-{5-[(1-(2-Hydroxyethyl)tetrahydro-2(1H)-pyrimidinylidene)amino]-1,2,4-thiadiazol-3-yl}ethyl)phenyl](phenyl)methanone hydrochloride

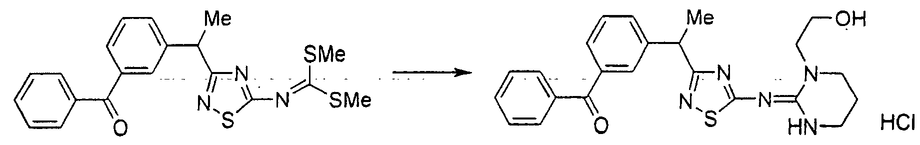

The desired compound was obtained from the compound obtained in Reference Example 30 and 2-hydroxyethylaminopropylamine by the same procedure as in Example 44.
¹H-NMR(300MHz, d₆-DMSO) δ ppm: 1.64(d, 3H, J=7.1Hz), 1.92(br, 2H), 3.38-3.72(m, 8H), 4.49 (q, 1H, J=7.1Hz), 7.48-7.74(m, 9H), 9.68(br, 1H)

### Example 48

### N'-{3-[1-(2-Fluoro[1,1'-biphenyl]-4-yl)ethyl]-1H-pyrazol-5-yl}-4-morpholine carboximidamide

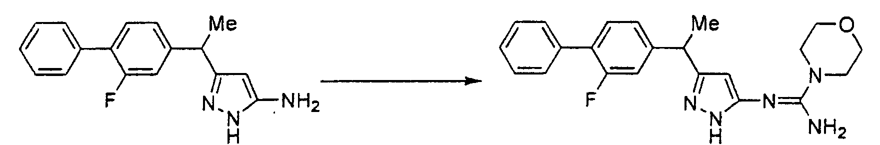

The desired compound was obtained from the compound obtained in Reference Example 32 by the same procedure as in Example 4.
Melting poins: 175 - 176 °C
¹H-NMR(300MHz, CDCl₃) δ ppm: 1.69(d, 3H, J=7.3Hz), 3.69-3,81(8H, m), 4.23(q, 1H, J=7.3Hz), 5.78(s, 1H), 5.79(br-s, 1H), 7.06-7.17(m, 2H), 7.32-7.54(m, 6H)
IR(KBr)[cm⁻¹]:3306, 3067, 2857, 1653, 1602, 1546, 1483, 1443, 1415, 1371, 1266, 1112, 1069, 990, 971, 915, 890, 832, 769, 742, 698

### Example 49

### N'-{3-[1-(2-Fluoro[1,1'-biphenyl]-4-yl)ethyl]-1-methyl-1H-pyrazol-5-yl}-4-morpholinecarboximidamide dihydrochloride

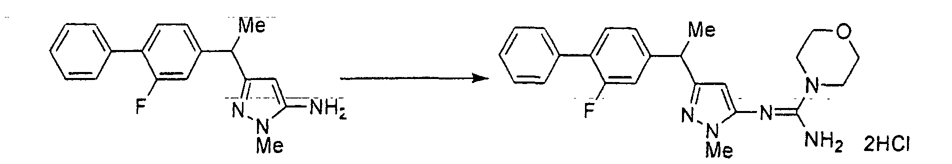

The desired compound was obtained from the compound obtained in Reference Example 33 by the same procedure as in Example 36.
Melting point: 175 - 179 °C
¹H-NMR(300MHz, d₆-DMSO) δ ppm: 1.56(d, 3H, J=7.2Hz), 3.53-3.60(m, 4H), 3.63(s, 3H), 3.64-3.72(m, 4H), 4.14(q, 1H, J=7.2Hz), 6.13(s, 1H), 7.18-7.25(m, 2H), 7.34-7.53(m, 6H), 8.00(br-s, 2H), 9.98(br-s, 1H)
IR(KBr)[cm⁻¹]: 3416, 3084, 2973, 1668, 1618, 1534, 1419, 1115, 699

### Example 50

### N'-{3-[1-(3-Benzoylphenyl)ethyl]-1-methyl-1H-pyrazol-5-yl}-4-morpholine carboximidamide dihydrochloride

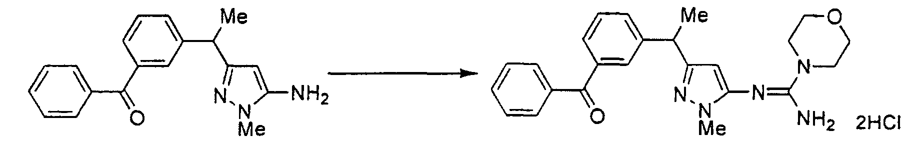

The desired compound was obtained from the compound obtained in Reference Example 35 by the same procedure as in Example 48.
¹H-NMR(300MHz, d₆-DMSO) δ ppm: 1.56(d, 3H, J=7.2Hz), 3.51-3.57(m, 4H), 3.62(s, 3H), 3.64-3.70(m, 4H), 4.18(q, 1H, J=7.2Hz), 6.07(s, 1H), 7.44-7.74(m, 9H), 7.97(br-s, 2H), 9.86(br-s, 1H)

### Example 51

### N'-{3-[1-(2-Fluoro[1,1'-biphenyl]-4-yl)ethyl]-1H-1,2,4-triazol-5-yl}-4-morpholinecarboximidamide hydrochloride

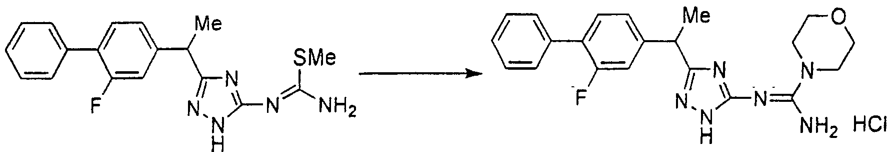

The desired compound was obtained from the compound obtained in Reference Example 39 by the same procedure as in Example 52, followed by treatment with 1N HCl/ether.
¹H-NMR(300MHz, d₆-DMSO) δ ppm: 1.66(d, 3H, J=7.2Hz), 3.50-3.70(m, 8H), 4.44(m, 1H), 7.20-7.55(m, 8H), 8.78(br, 2H), 10.92(br, 1H), 14.26(br, 1H)

### Example 52

### N'-{3-[1-(2-Fluoro[1,1'-biphenyl]-4-yl)ethyl]-1-methyl-1H-1,2,4-triazol-5-yl}-4-morpholinecarboximidamide

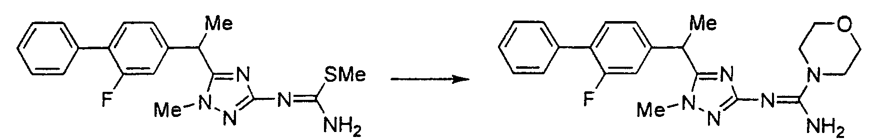

The compound (1.6 g) obtained in Reference Example 40 was dissolved in DMF (30 mL), and morpholine (3.8 mL) was added thereto and stirred at 120 °C for 20 hours. The reaction mixture was poured to water and extracted with ethyl acetate. The organic layer was washed with water, dried and concentrated. The residue was purified with a silica gel column chromatography (chloroform/methanol = 20/1) to obtain the desired compound (0.9 g).
¹H-NMR(300MHz, CDCl₃) δ ppm: 1.74(d, 3H, 7.2Hz), 3.52-3.57(m, 4H), 3.56(s, 3H), 3.72-3.77(m, 4H), 4.15(q, 1H, J=7.2Hz), 6.53(br, 2H), 7.00-7.08(m, 2H), 7.32-7.54(m, 6H)

### Example 53

### N'-{3-[1-(3-Benzoylphenyl)ethyl]-1-methyl-1H-1,2,4-triazol-5-yl}-4- morpholinecarboximidamide hydrochloride

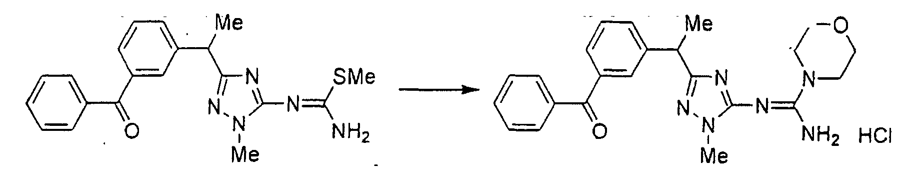

The desired compound was obtained from the compound obtained in Reference Example 46 by the-same procedure as in Example 66.
¹H-NMR(300MHz, CDCl₃) δ ppm: 1.74(br, 3H), 3.74(br, 11H), 4.53(br 1H), 7.34-7.86(m, 9H)

### Example 54

### N"-{3-[1-(3-Benzoylphenyl)ethyl]-1-methyl-1H-1,2,4-triazol-5-yl}-N-[2-(4-morpholinyl)ethyl]guanidine hydrochloride

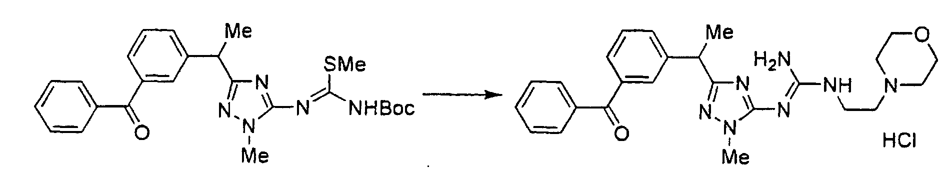

The compound (1.33 g) obtained in Reference Example 47 was dissolved in acetonitrile (15 mL), and triethylamine (0.46 mL) and aminoethylmorpholine (0.84 mL) were added thereto. The mixture was cooled to 0 °C and an acetonitrile solution (5 mL) of silver nitrate (0.82 g) was added dropwise thereto. Five minutes later, the mixture was warmed to room temperature and stirred for 6 hours. The reaction mixture was filtered on a Celite bed and concentrated. Ethyl acetate and water were added to the residue. The organic layer was separated and the aqueous layer was extracted. The organic layers were washed with an aqueous saturated ammonium chloride solution and water, dried, and concentrated to give a crude compound (1.4 g). The compound was dissolved in methylene chloride (10 mL) and TFA (3 mL) was added dropwise thereto at room temperature and was kept overnight. The reaction mixture was concentrated. Chloroform and an aqueous saturated sodium bicarbonate solution was added thereto. The organic layer was separated and the aqueous layer was extracted. The organic layers were dried and concentrated. The residue was purified with a silica gel column chromatography (chloroform/methanol = 20/1 - 10/1) to give a crude compound (1.12 g), which was treated with 1N-HCl/ether to obtain the desired compound (1.13 g).
¹H-NMR(300MHz, d₆-DMSO) δ ppm: 1.59(d, 3H, J=7.2Hz), 3.00-4.00(m, 15H), 4.28(brq, 1H, J=7.2Hz), 7.46-7.75(m, 9H)

### Example 55

### [3-(1-{5-[(5-Hydroxytetrahydro-2(1H)-pyrimidinylidene)amino]-1-methyl-1H-1,2,4-triazol-3-yl}ethyl)phenyl](phenyl)methanone hydrochloride

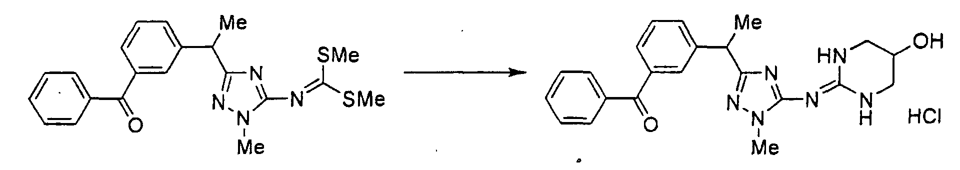

The desired compound was obtained from the compound obtained in Reference Example 48 and 1,3-diamino-2-hydroxypropane by the same procedure as in Example 44.
¹H-NMR(300MHz, CDCl₃) δ ppm: 1.64(d, 3H, J=7.3Hz), 3.46-3.68(m, 4H), 3.89(s, 3H), 4.16(q, 1H, J=7.3Hz), 4.36(m, 1H), 7.34-7.50(m, 4H), 7.54-7.62(m, 2H), 7.74-7.84(m, 3H)

### Example 56

### (3-{1-[1-Methyl-5-(tetrahydro-2(1H)-pyrimidinylideneamino)-1H-1,2,4-triazol-3-yl]ethyl}phenyl)(phenyl)methanone hydrochloride

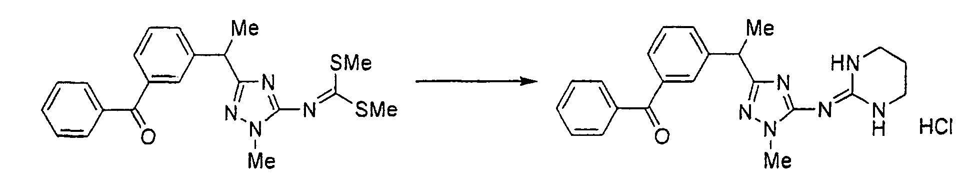

The desired compound was obtained from the compound obtained in Reference Example 48 and 1,3-diaminopropane by the same procedure as in Example 44.
¹H-NMR(300MHz, CDCl₃) δ ppm: 1.65(d, 3H, J=7.2Hz), 2.02(m, 2H), 3.44-3.60(m, 4H), 3.94(s, 3H), 4.16(q, 1H, J=7.2Hz), 7.38(t, 1H, J=7.6Hz), 7.43-7.62(m, 5H), 7.74-7.87(m, 3H), 9.19(br, 1H), 9.77(br, 1H)

### Example 57

### [3-(1-{5-[((2E)-1-(2-Hydroxyethyl)tetrahydro-2(1H)-pyrimidinylidene) amino]-1-methyl-1H-1,2,4-triazol-3-yl}ethyl)phenyl](phenyl)methanone hydrochloride

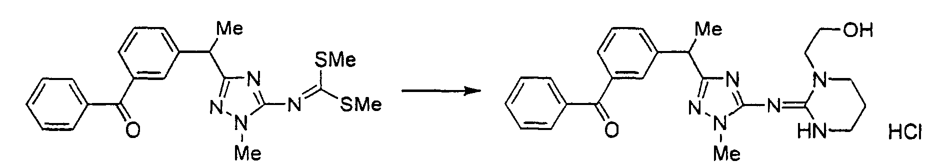

The desired compound was obtained from the compound obtained in Reference Example 48 and 2-(2-aminoethylamino)ethanol by the same procedure as in Example 44.
¹H-NMR(300MHz, CDCl₃) δ ppm: 1.63(d, 3H, J=7.2Hz), 2.07(m, 2H), 3.45-3.55(m, 4H), 3.68-3.80(m, 7H), 3.83-3.90(m, 4H), 4.23(q, 1H, J=7.2Hz), 7.32-7.58(m, 6H), 7.70-7.80(m, 3H), 9.31(br, 1H)

### Example 58

### N'-{3-[1-(3-Benzoylphenyl)ethyl]-1-methyl-1H-1,2,4-triazol-5-yl}-N-[2-(dimethylamino)ethyl]-4-morpholinecarboximidamide hydrochloride

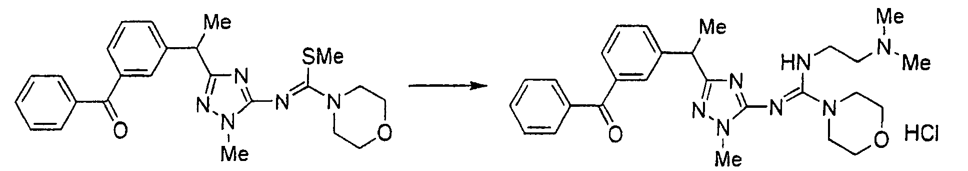

The desired compound was obtained from the compound obtained in Reference Example 49 and 2-dimethylaminoethylamine by the same procedure as in Example 42.
¹H-NMR(300MHz, CDCl₃) δ ppm: 1.75(d, 3H, J=7.3Hz), 2.95(brs, 6H), 3.20-3.60(m, 13H), 3.72-3.94(m, 2H), 4.27(q, 1H, J=7.3Hz), 7.43-7.65(m, 5H), 7.73-7.80(m, 4H), 9.04(br, 1H), 10.73(br, 1H), 14.18(br, 1H)

### Example 59

### 2-{[(E)-({3-[1-(3-Benzoylphenyl)ethyl]-1-methyl-1H-1,2,4-triazol-5-yl} imino)(4-morpholinyl)methyl]amino}acetamide hydrochloride

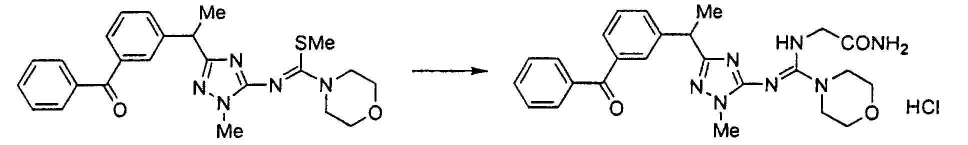

The desired compound was obtained from the compound obtained in Reference Example 49, glycineamide hydrochloride and triethylamine by the same procedure as in Example 42.
¹H-NMR(300MHz, CDCl₃) δ ppm: 1.71(d, 3H, J=7.3Hz), 3.39-3.67(m, 11H), 3.90-4.12(m, 2H), 4.29(q, 1H, J=7.3Hz), 6.21(br, 1H), 7.40-7.80(m, 9H), 8.02(br, 1H), 8.18(br, 1H)

### Example 60

### tert-Butyl

### [[(E)-({3-[1-(3-Benzoylphenyl)ethyl]-1-methyl-1H-1,2,4-triazol-5-yl}imino) (4-morpholinyl)methyl](methyl)amino]acetate

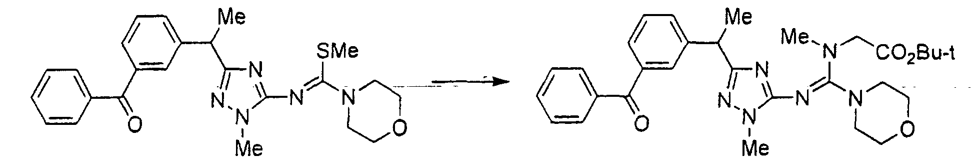

The desired compound was obtained from the compound obtained in Reference Example 49, glycine *tert*-butyl hydrochloride and triethylamine by the same procedure as in Example 39.
¹H-NMR(300MHz, CDCl₃) δ ppm: 1.42(s, 9H), 1.67(d, 3H, J=7.3Hz), 2.89(s, 3H), 3.03-3.13(m, 4H), 3.53-3.60(m, 4H), 3.56(s, 3H), 3.65(s, 2H), 4.15(q, 1H, J=7.3Hz), 7.33-7.48(m, 3H), 7.51-7.62(m, 3H), 7.74-7.80(m, 2H), 7.84(m, 1H)

### Example 61

### [[(E)-({3-[1-(3-Benzoylphenyl)ethyl]-1-methyl-1H-1,2,4-triazol-5-yl}imino) (4-morpholinyl)methyl](methyl)amino]acetic acid

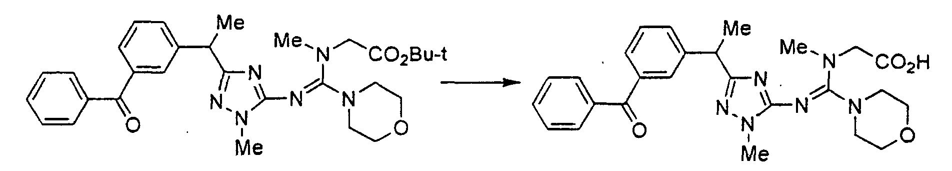

The compound (1.42 g) obtained in Example 60 was dissolved in methylene chloride (5 mL) and TFA (5 mL) and stirred overnight. The mixture was diluted with chloroform, cooled to 0 °C and neutralized with an aqueous saturated sodium bicarbonate solution. The organic layer was separated and the aqueous layer was extracted. The organic layers were dried, and concentrated. The residue was purified with a silica gel column chromatography (chloroform/methanol = 20/1 - 10/1) to obtain the desired compound (0.90 g).
¹H-NMR(300MHz, CDCl₃) δ ppm: 1.75(d, 3H, J=7.3Hz), 2.58(s, 3H), 3.42(br, 4H), 3.66(s, 3H), 3.70-3.77(m, 4H), 3.84(br, 2H), 4.30(q, 1H, J=7.3Hz), 7.41-7.53(m, 3H), 7.56-7.66(m, 2H), 7.73-7.82(m, 4H)

### Example 62

### N'-{3-[1-(3-Benzoylphenyl)ethyl]-1-methyl-1H-1,2,4-triazol-5-yl}-N,N-dimethyl-4-morpholinecarboximidamide hydrochloride

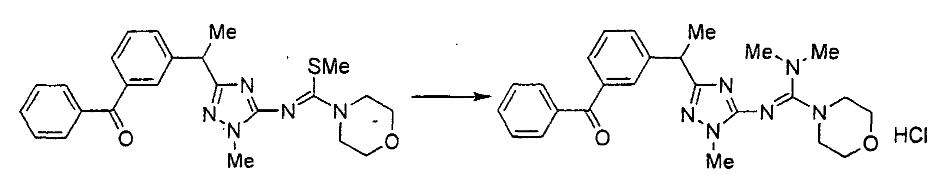

The desired compound was obtained from the compound obtained in Reference Example 49 by the same procedure as in Example 42.
¹H-NMR(300MHz, CDCl₃) δ ppm: 1.77(d, 3H, J=7.2Hz), 2.95(s, 3H), 3.33-3.40(m, 4H), 3.62-3.69(m, 7H), 4.53(q, 1H, J=7.2Hz), 7.44-7.68(m, 5H), 7.78-7.89(m, 4H)

### Example 63

### N'-{3'-[1-(3-Benzoylpheyl)ethyl]-1-methyl-1H-1,2,4-triazol-5-yl}-N-methyl-4-morpholinecarboximidamide hydrochloride

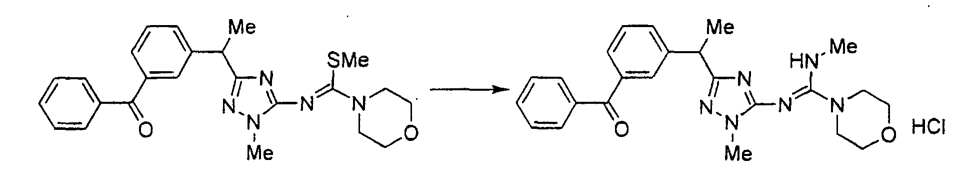

The desired compound was obtained from the compound obtained in Reference Example 49 and methylamine by the same procedure as in Example 42.
¹H-NMR(300MHz, CDCl₃) δ ppm: 1.73(d, 3H, J=7.2Hz), 2.69(br, 3H), 3.54-3.72(m, 11H), 4.38(q, 1H, J=7.2Hz), 7.40-7.52(m, 3H), 7.55-7.66(m, 2H), 7.70-7.82(m, 4H)

### Example 64

### N'-{3-[1-(3-Benzoylphenyl)ethyl]-1-methyl-1H-1,2,4-triazol-5-yl}-N-(2-hydroxyethyl)-4-morpholinecarboximidamide hydrochloride

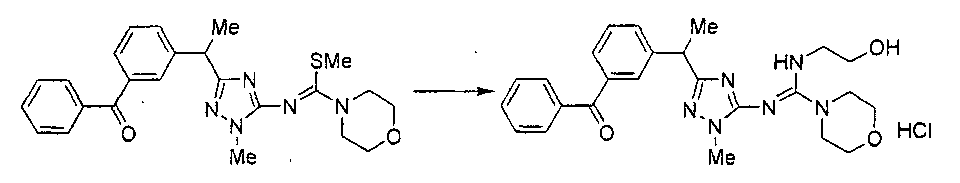

The desired compound was obtained from the compound obtained in Reference Example 49 and ethanolamine by the same procedure as in Example 42.
¹H-NMR(300MHz, CDCl₃) δ ppm: 1.73(d, 3H, J=7.0Hz), 3.22-3.73(m, 15H), 4.34(q, 1H, J=7.0Hz), 7.42-7.52(m, 3H), 7.56-7.66(m, 2H), 7.70-7.83(m, 4H)

### Example 65

### N'-{5-[1-(3-Benzoylphenyl)ethyl]-1-methyl-1H-1,2,4-triazol-3-yl}-4-morpholinecarboximidamide hydrochloride

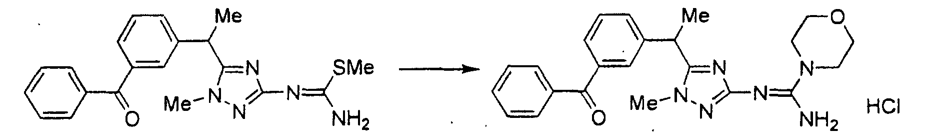

The desired compound was obtained from the compound obtained in Reference Example 52 and morpholine by the same procedure as in Example 51.
¹H-NMR(300MHz, CDCl₃) δ ppm: 1.66(d, 3H, J=7.0Hz), 3.55(s, 3H), 3.74-3.90(m, 8H), 4.21(q, 1H, J=7.0Hz), 7.30-7.78(m, 9H), 9.28(br, 2H)

### Example 66

### N'-{3-[1-(3-Benzolyphenyl)ethyl]-1H-1,2,4-triazol-5-yl}-4-morpholine carboximidamide hydrochloride

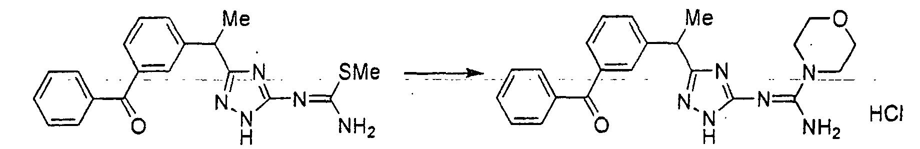

The compound (1.2 g) obtained in Reference Example 51 was dissolved in 2-propanol (20 mL), and morpholine (0.2 mL) and morpholine hydrochloride (4.1 g) were added thereto and stirred under reflux for 8 hours. Water and ethyl acetate were added to the reaction mixture, and the organic layer was separated and the aqueous layer was extracted. The organic layers were washed with water, dried, and concentrated. The residue was purified with a silica gel column chromatography (ethyl acetate/hexane = 2/1 - chloroform/methanol = 10/1) to give a crude compound (1.3 g), which was treated with 1N HCl/ether to obtain the desired compound (1.3 g).
¹H-NMR(300MHz, d₆-DMSO) δ ppm: 1.66(d, 3H, J=7.2Hz), 3.52-3.70(m, 8H), 4.49(q, 1H, J=7.2Hz), 7.49-7.75(m, 9H), 8.74(br, 2H)

### Example 67

### Phenyl(3-{1-[1-(phenylsulfonyl)-5-(tetrahydro-2(1H)-pyrimidinylidene amino)-1H-1,2,4-triazol-3-yl]ethyl}phenyl)methanone

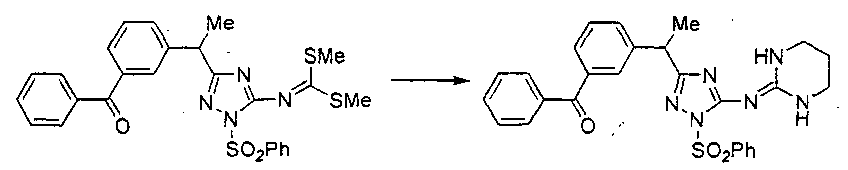

The desired compound was obtained from the compound obtained in Reference Example 54 and 1,3-diaminopropane by the same procedure as in Example 13.
¹H-NMR(300MHz, CDCl₃) δ ppm: 1.61(d, 3H, J=7.2Hz), 1.89(m, 2H), 3.31-3.38(m, 4H), 4.13(q, 1H, J=7.2Hz), 7.31-7.62(m, 9H), 7.75-7.80(m, 2H), 7.84(m, 1H), 8.07-8.12(m, 2H)

### Example 68

### Phenyl(3-{1-[5-(tetrahydro-2(1H)-pyrimidinylideneamino)-1H-1,2,4-triazol-3-yl]ethyl}phenyl)methanone hydrochloride

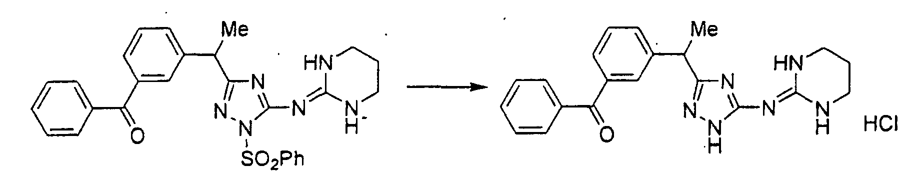

The compound (0.95 g) obtained in Example 67 was dissolved in THF (10 mL) and methanol (5 mL), and 4N aqueous sodium hydroxide solution (2.5 mL) was added dropwise at room temperature. Thirty minutes later, water and chloroform were added thereto. The organic layer was separated and the aqueous layer was extracted. The organic layers were dried, and concentrated. The residue was purified with a silica gel column chromatography (chloroform/methanol = 15/1 - 5/1) to give a crude compound (0.48 g), which was treated with 1N HCl/ether to obtain the desired compound (0.42 g).
¹H-NMR(300MHz, d₆-DMSO) δ ppm: 1.65(d, 3H, J=7.3Hz), 1.86(m, 2H), 3.34-3.44(m, 4H), 4.46(q, 1H, J=7.3Hz), 7.48-7.74(m, 9H), 8.60(br, 2H), 11.16(br, 1H)

### Example 69

### N'-[3-[1-(3-Benzoylphenyl)ethyl]-1-(phenylsulfonyl)-1H-1,2,4-triazol-5-yl]-N,N-dimethyl-4-morpholinecarboximidamide

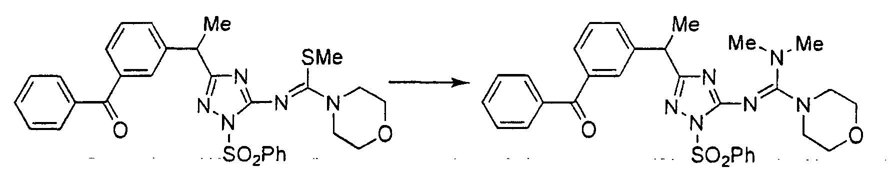

The desired compound was obtained from the compound obtained in Reference Example 55 and dimethylamine by the same procedure as in Example 42.
¹H-NMR(300MHz, CDCl₃) δ ppm: 1.63(d, 3H, J=7.3Hz), 2.75(s, 6H), 3.10-3.16(m, 4H), 3.58-3.64(m, 4H), 4.16(q, 1H, J=7.3Hz), 7.31-7.64(m, 9H), 7.75-7.83(m, 3H), 8.01-8.07(m, 2H)

### Example 70

### N'-{3-[1-(3-Benzoylphenyl)ethyl]-1H-1,2,4-triazol-5-yl}-N,N-dimethyl-4-morpholinecarboximidamide hydrochloride

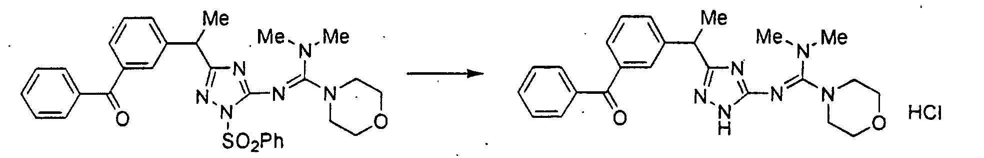

The desired compound was obtained from the compound obtained in Example 69 by the same procedure as in Example 68.
¹H-NMR(300MHz, CDCl₃) δ ppm: 1.80(d, 3H, J=7.2Hz), 3.07(br, 6H), 3.40-3.80(m, 8H), 4.69(brq, 1H, J=7.2Hz), 7.37-7.62(m, 5H), 7.72-7.80(m, 3H), 7.96(m, 1H)

### Example 71

### N'-[3-[1-(3-Benzoylphenyl)ethyl]-1-(phenylsulfonyl)-1H-1,2,4-triazol-5--yl]-N-methyl-4-morpholinecarboximidamide

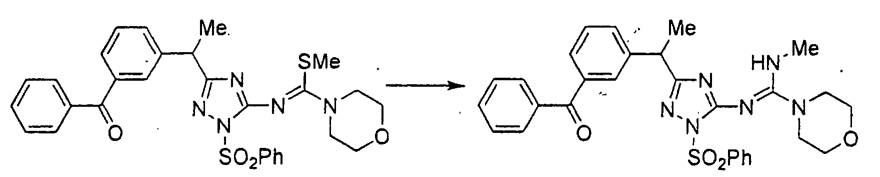

The desired compound was obtained from the compound obtained in Reference Example 55 by the same procedure as in Example 42.
¹H-NMR(300MHz, CDCl₃) δ ppm: 1.65(d, 3H, J=7.3Hz), 2.86(d, 3H, J=5.1Hz), 3.40-3.47(m, 4H), 3.70-3.75(m, 4H), 4.21(q, 1H, J=7.3Hz), 7.33-7.64(m, 9H), 7.75-7.81(m, 2H), 7.88(m, 1H), 8.02-8.07(m, 2H)

### Example 72

### N'-{3-[1-(3-Benzoylphenyl)ethyl]-1H-1,2,4-triazol-5-yl}-N-methyl-4-morpholinecarboximidamide hydrochloride

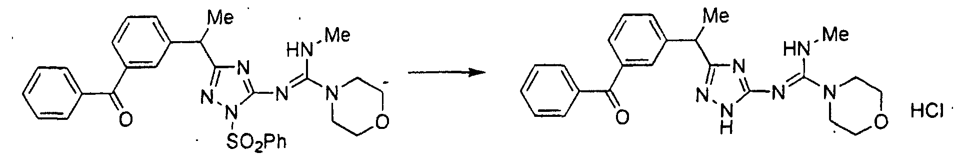

The desired compound was obtained from the compound obtained in Reference Example 71 by the same procedure as in Example 68.
¹H-NMR(300MHz, CDCl₃) δ ppm: 1.83(d, 3H, J=7.0Hz), 3:03(br, 3H), 3.60-3.84(m, 8H), 4.60(m, 1H), 7.38-7.90(m, 9H), 9.40(br, 1H)

### Example 73

### N'-{5-[1-(3-Benzoylphenyl)ethyl]-1,2,4-thiadiazol-3-yl}-N,N-dimethyl-4-morpholinecarboximidamide hydrochloride

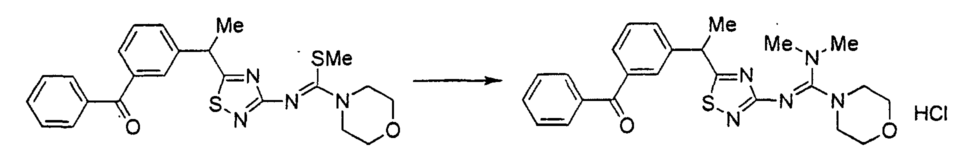

The desired compound was obtained from the compound obtained in Reference Example 62 and dimethylamine by the same procedure as in Example 42.
¹H-NMR(300MHz, d₆-DMSO) δ ppm: 12.41(br,1H), 7.48-7.81(m,9H), 4.52-4.59(m,1H), 3.83(brs,4H), 3.65-3.76(m,4H), 3.11(s,6H), 1.84(d,J=7.1Hz,3H)

### Example 74

### N'-{5-[1-(3-Benzoylphenyl)ethyl]-1,2,4-thidiazol-3-yl}-N-[2-(dimethyl amino)ethyl)-N-methyl-4-morpholinecarboximidamide dihydrochloride

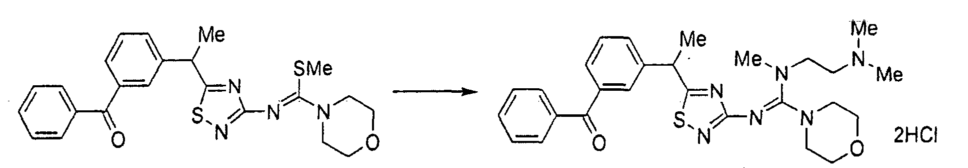

The desired compound was obtained from the compound obtained in Reference Example 62 and N,N,N'-trimethylethylenediamine by the same procedure as in Example 42.
¹H-NMR(300MHz, d₆-DMSO) δ ppm: 12.09(br,1H), 7.48-7.82(m,9H), 4.53-4.63(m,1H), 4.22(br,2H), 3.65-3.85(m,10H), 3.39(br,3H), 2.92(brs,6H), 1.85(d,J=7.1Hz,3H)

### Example 75

### N'-{3-[1-(3-Benzoylphenyl)ethyl]-1H-1,2,4-triazol-5-yl}-N-ethyl-4-morpholinecarboximidamide hydrochloride

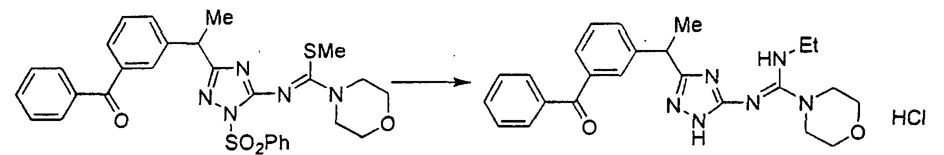

The compound (2.00 g) obtained in Reference Example 55 was dissolved in dioxane (7.0 mL), and 70% aqueous ethylamine solution (1.40 mL) was added thereto and stirred under reflux for 1 hour. After the starting material disappeared by TLC analysis, 2N aqueous sodium hydroxide solution (3.5 mL) was added thereto and stirred under reflux for 1 hour. The reaction mixture was poured into water and extracted with ethyl acetate. The organic layers were dried over magnesium sulfate, and concentrated by an evaporater. The residue was purified with a silica gel column chromatography (ethyl acetate) to give a crude compound (1.62 g, colorless amorphous form), which was treated with 1M HCl/ether to obtain the desired compound (1.49 g).
¹H-NMR(400MHz, d₆-DMSO) δ ppm: 1.06(t, 3H, J=7.1Hz), 1.63(d, 3H, J=7.2Hz), 3.10-3.16(m, 2H), 3.33(br s, 4H), 3.55(br s, 4H), 4.23(br q, 1H, J=7.0Hz), 7.50-7.72(m, 9H), 8.76(br, 1H)

### Example 76

### N'-{3-[1-(3-Benzoylphenyl)ethyl]-1H-1,2,4-triazol-5-yl}-N-propyl-4-morpholinecarboximidamide trifluoroacetic acid salt

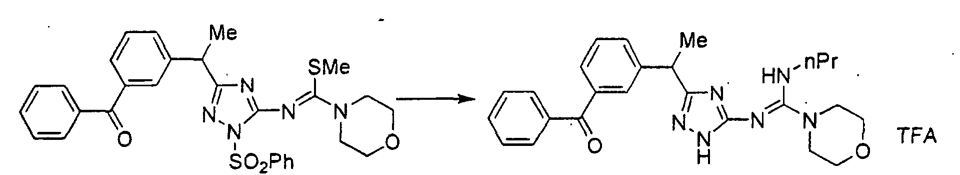

The compound (288 mg) obtained in Reference Example 55 was dissolved in dioxane (2 mL), and n-propylamine (0.082 mL) was added thereto and stirred at room temperature for 4 hours. After the starting material disappeared by TLC analysis, 2N aqueous sodium hydroxide solution (1 mL) was added thereto and stirred under reflux for 1 hour. The reaction mixture was poured into water and extracted with ethyl acetate. The organic layers were dried over magnesium sulfate, and concentrated by an evaporater to give a crude compound (244 mg). A part of the crude compound (80 mg) was purified with a high performance liquid chromatography (column: YMC CombiPrep ODS-A 75φ x 30mm; solvent: 0.05% trifluoroacetic acid/water : 0.035% trifluoroacetic acid/acetonitril = 95/5 - 5/95) to obtain the desired compound (43 mg, oil).
HPLC r.t. 13.48 min (column: Waters Puresil C18, eluent: 10mM-AcONH₄/H₂O(pH 4):CH₃CN=80:20 - 20:80 (30min))
LC/MS (m/e) 447.4
¹H-NMR(300MHz, CDCl₃) δ ppm: 0.92(t, 3H, J=7.4Hz), 1.64(sext, 2H, J=7.2Hz), 1.75(d, 3H, J=7.3Hz), 3.20-3.30(m, 2H), 3.55-3.60(m, 4H), 3.75-3.80(m, 4H), 4.40(q, 1H, J=7.0Hz), 7.40-7.65(m, 6H), 7.76-7.79(m, 3H), 9.21(br s, 1H)

### Example 77 - Example 296

Further to the compounds of the above Examples, the compounds in the following Table 19 to Table 26 were obtained by the same procedure as in Example 67, Example 68, Example 69, Example 70 and/or Example 76. Each compound was isolated as a trifluoroacetate, and was identified by its retention time in high performance liquid chromatography analysis (column: Waters Puresil C18, eluent: 10mM-AcONH₄/H₂O(pH 4):CH₃CN=80:20 - 20:80 (30min)) and its LC/MS spectrum.

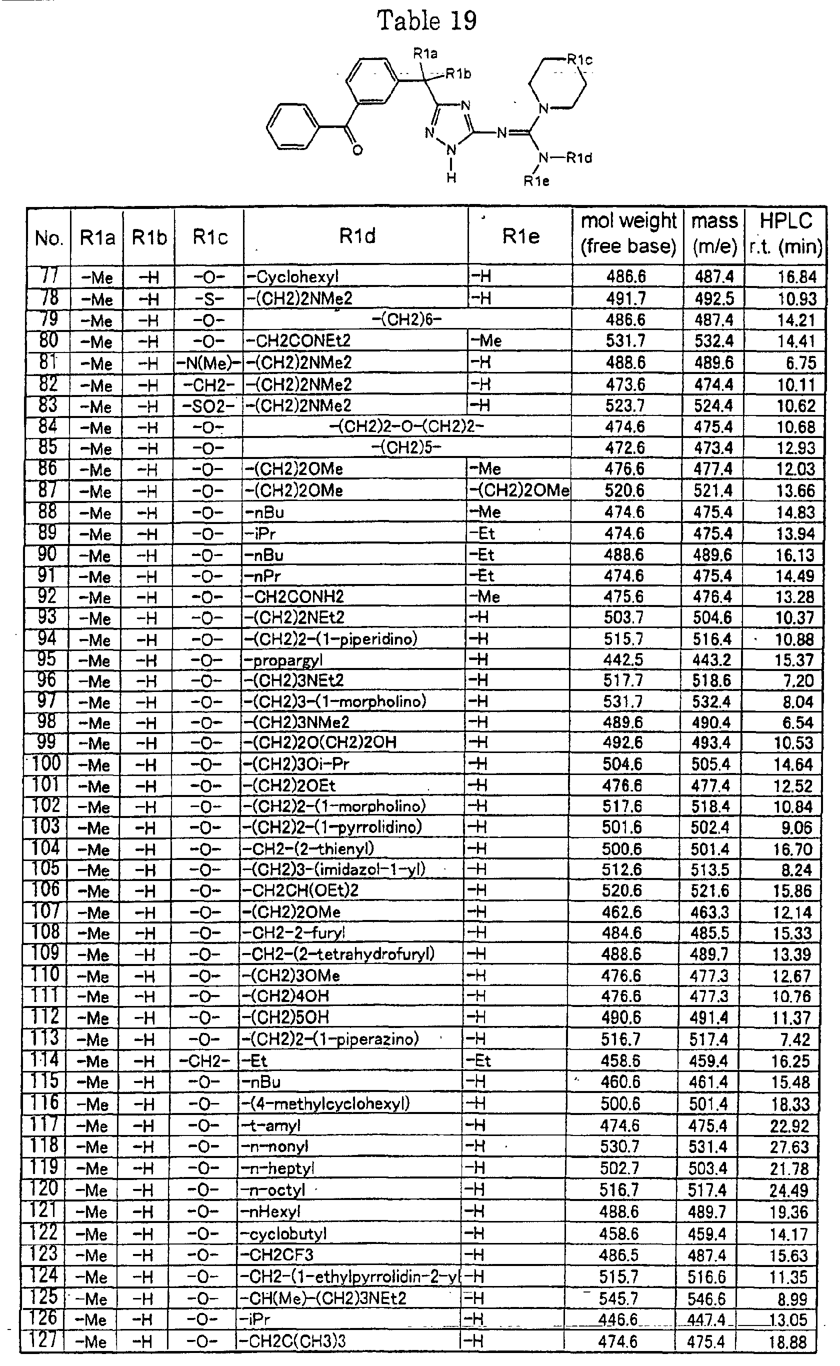

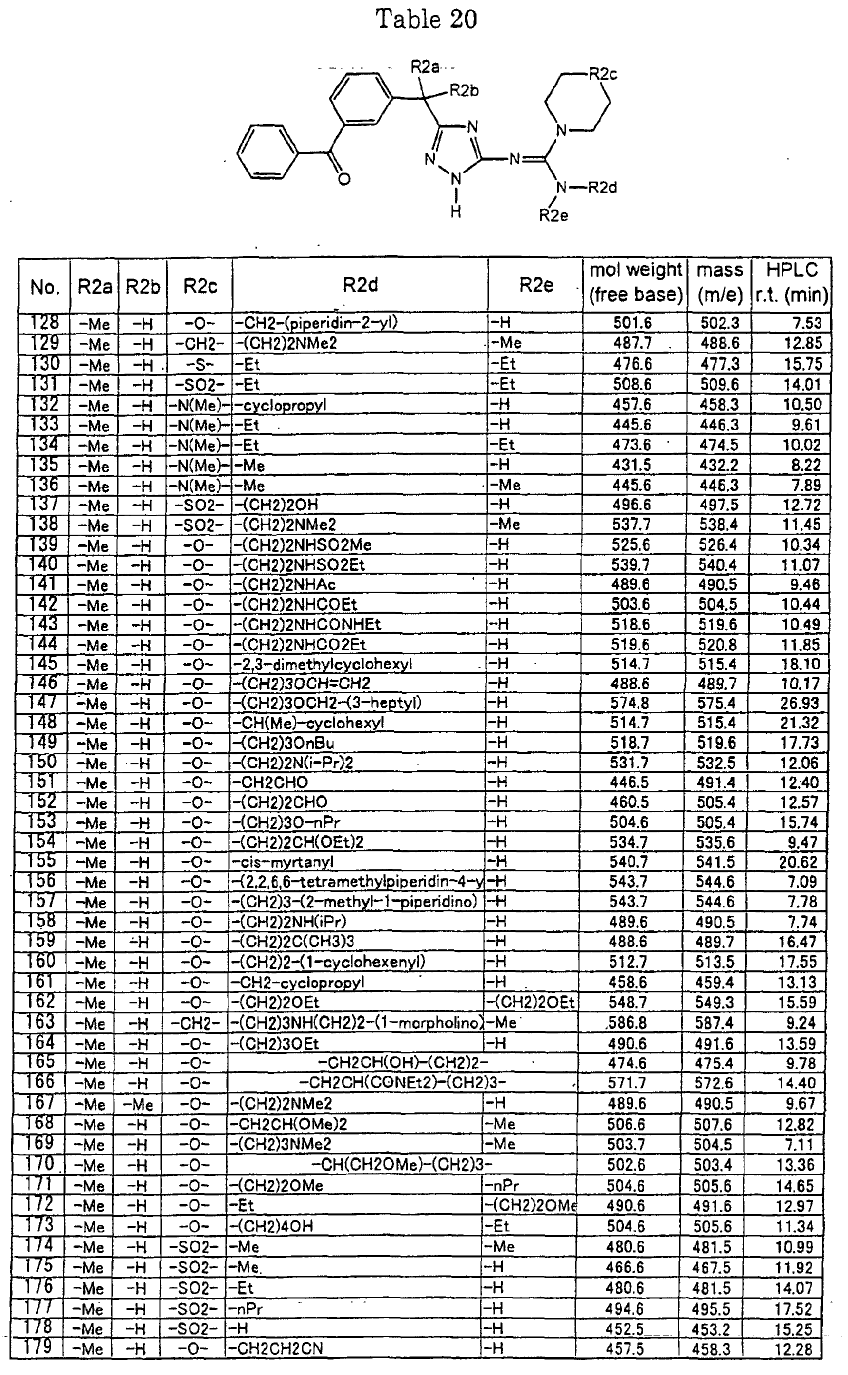

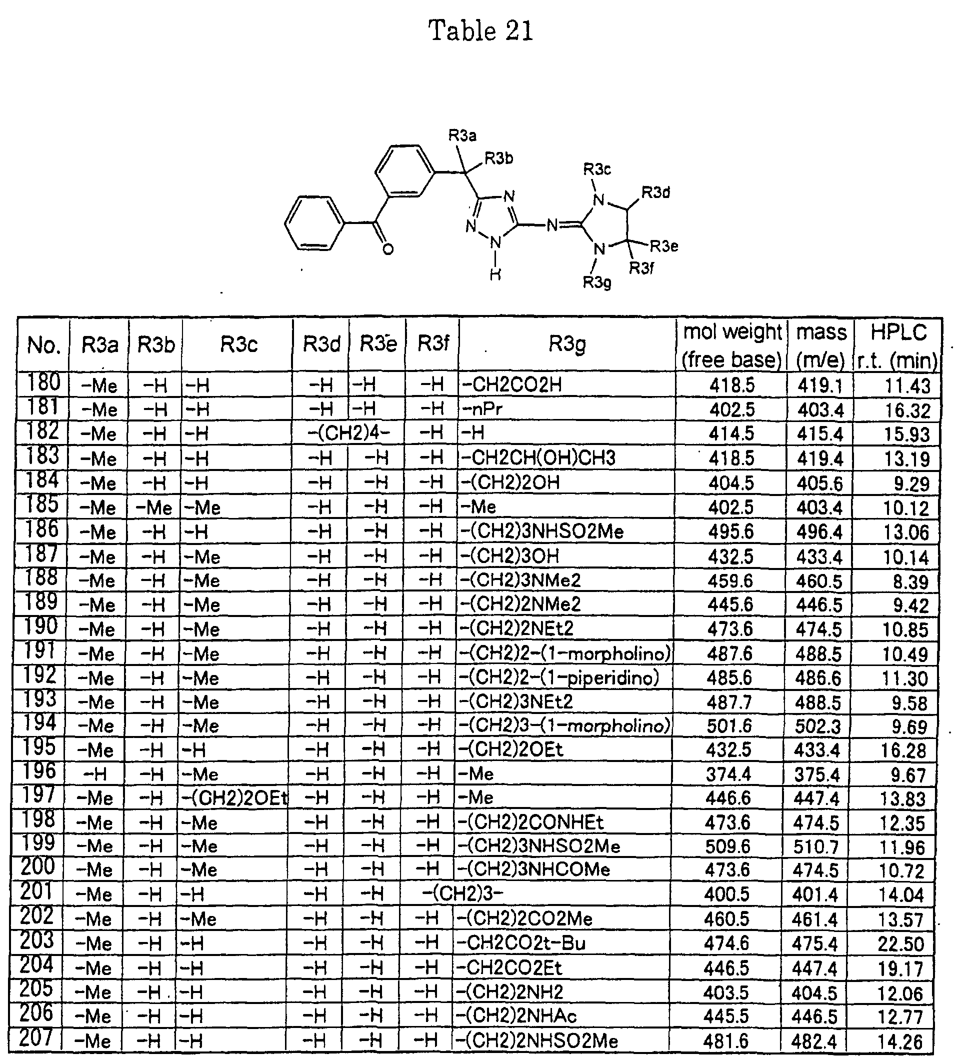

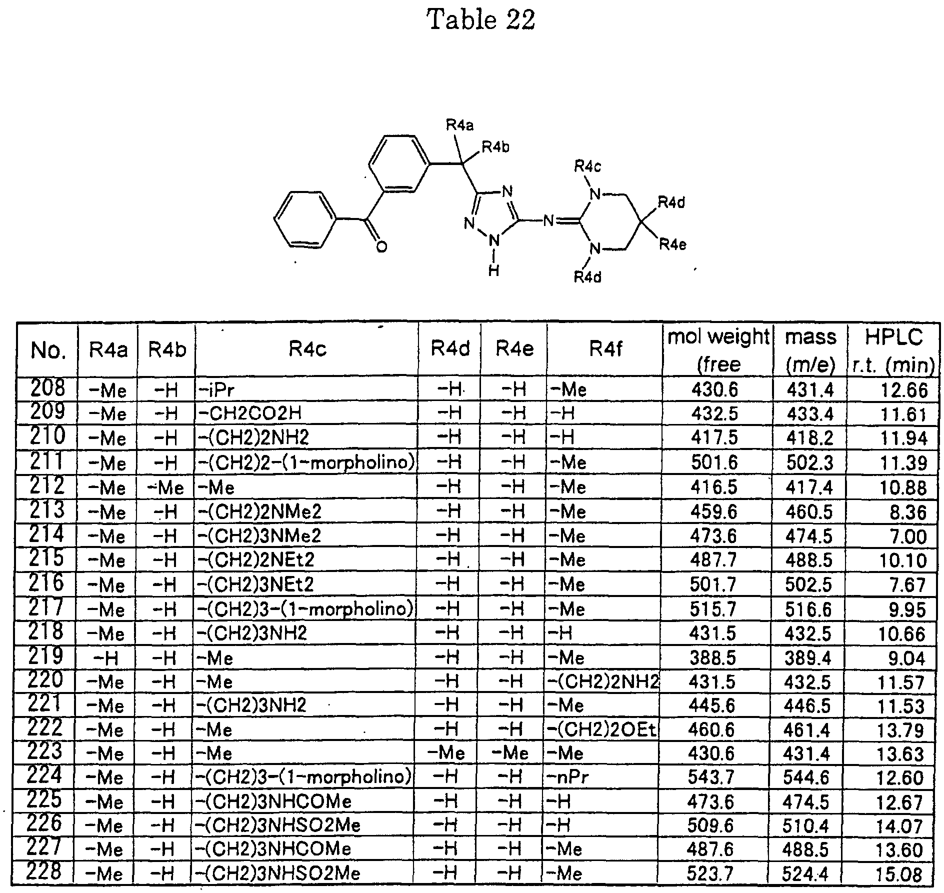

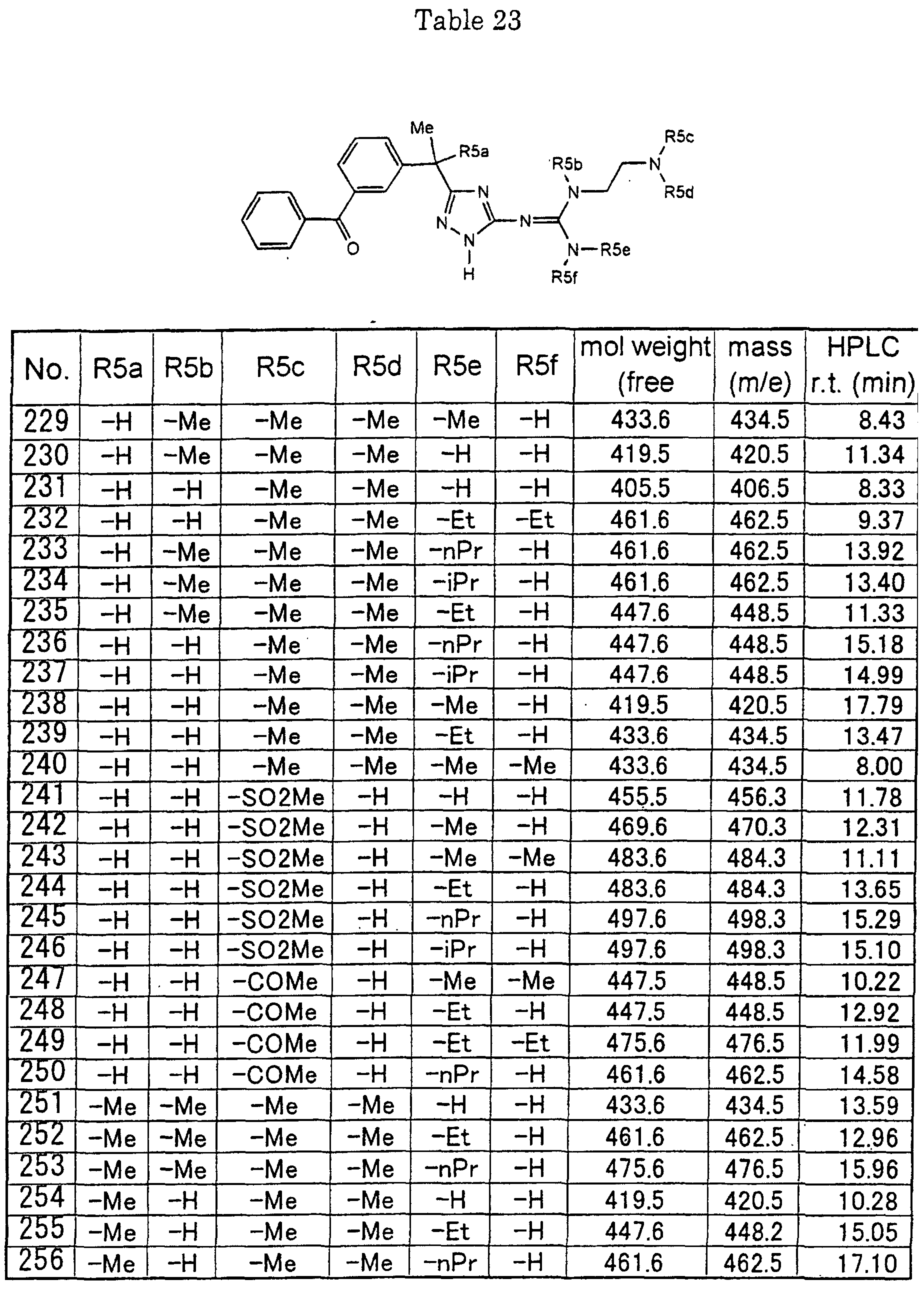

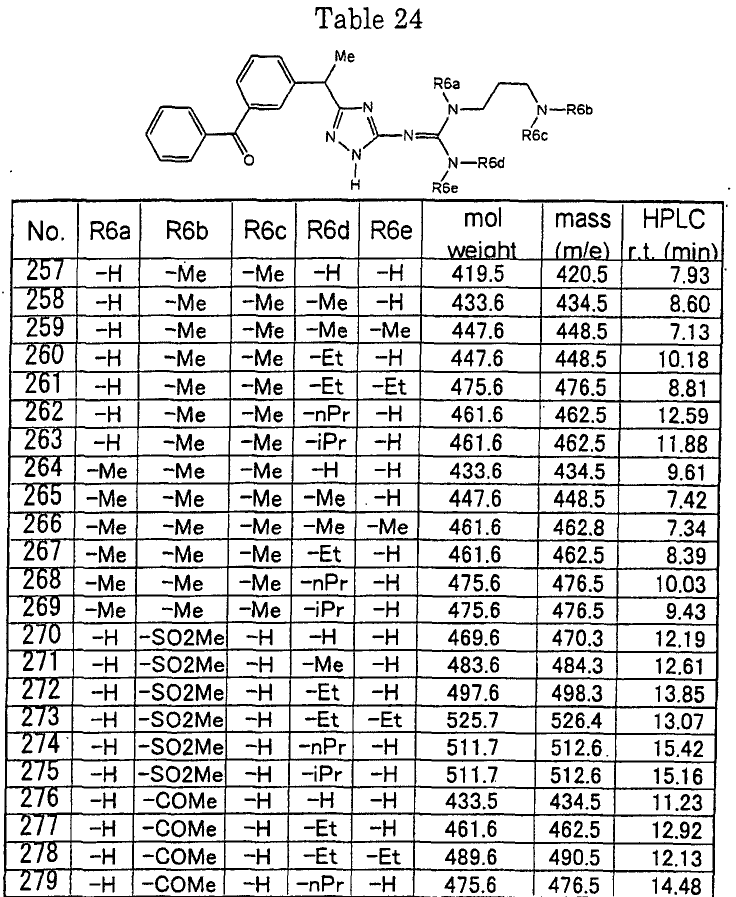

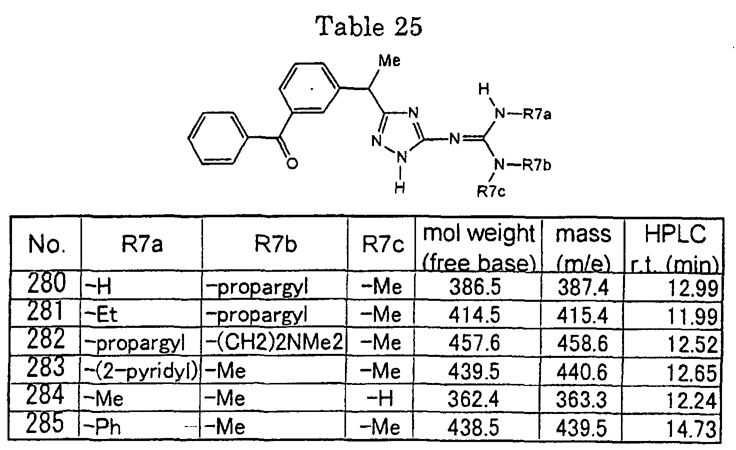

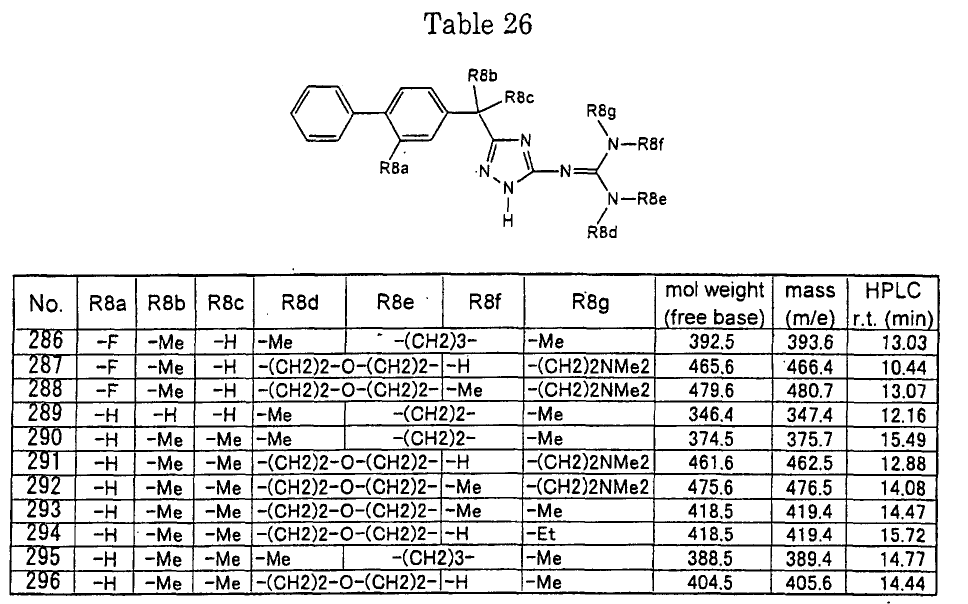

### Example 297 - Example 317

Further to the compounds of the above Examples, the compounds in the following Table 27 to Table 29 were obtained by the same procedure as in Example 67, Example 68, Example 69, Example 70 and/or Example 76.

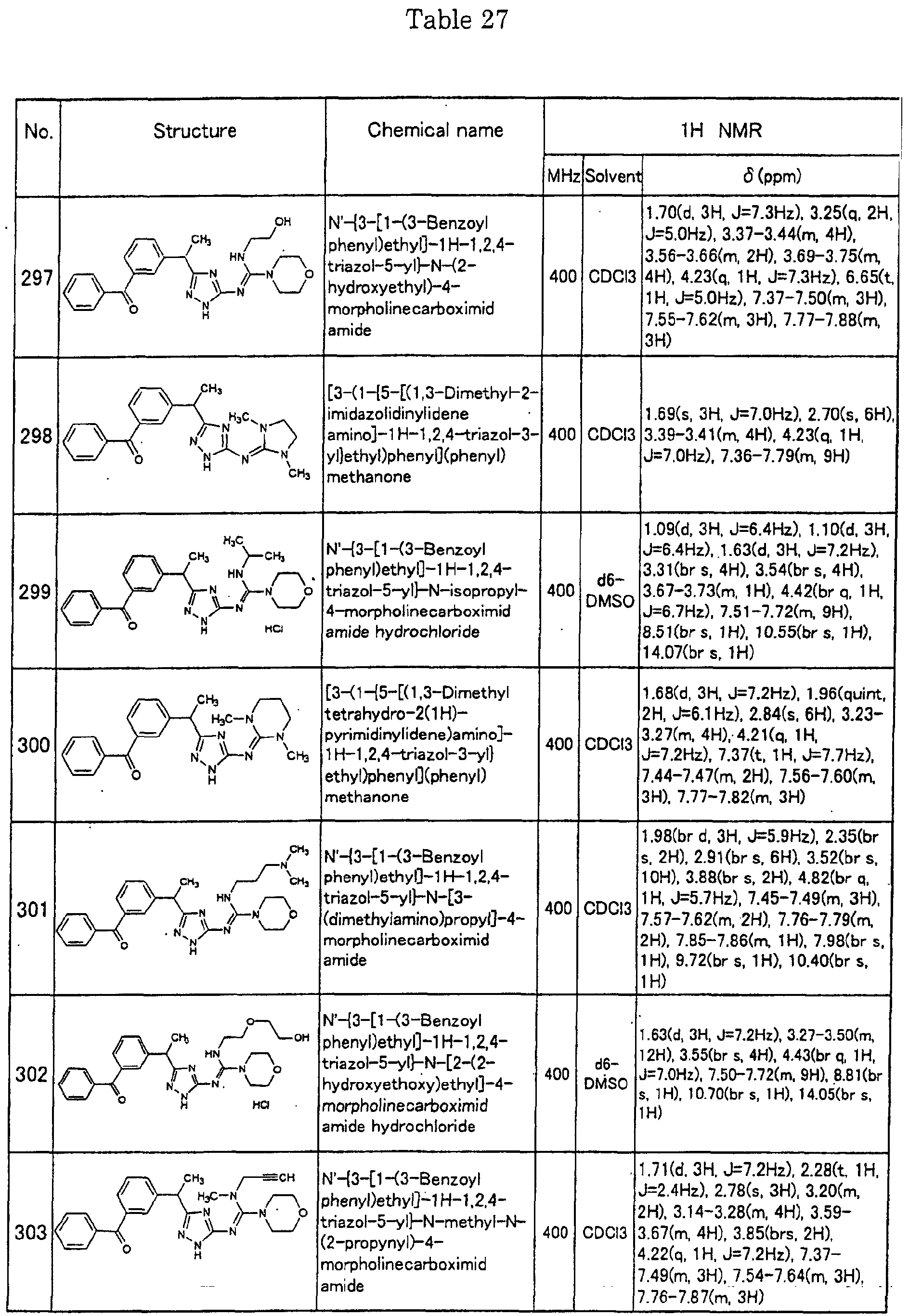

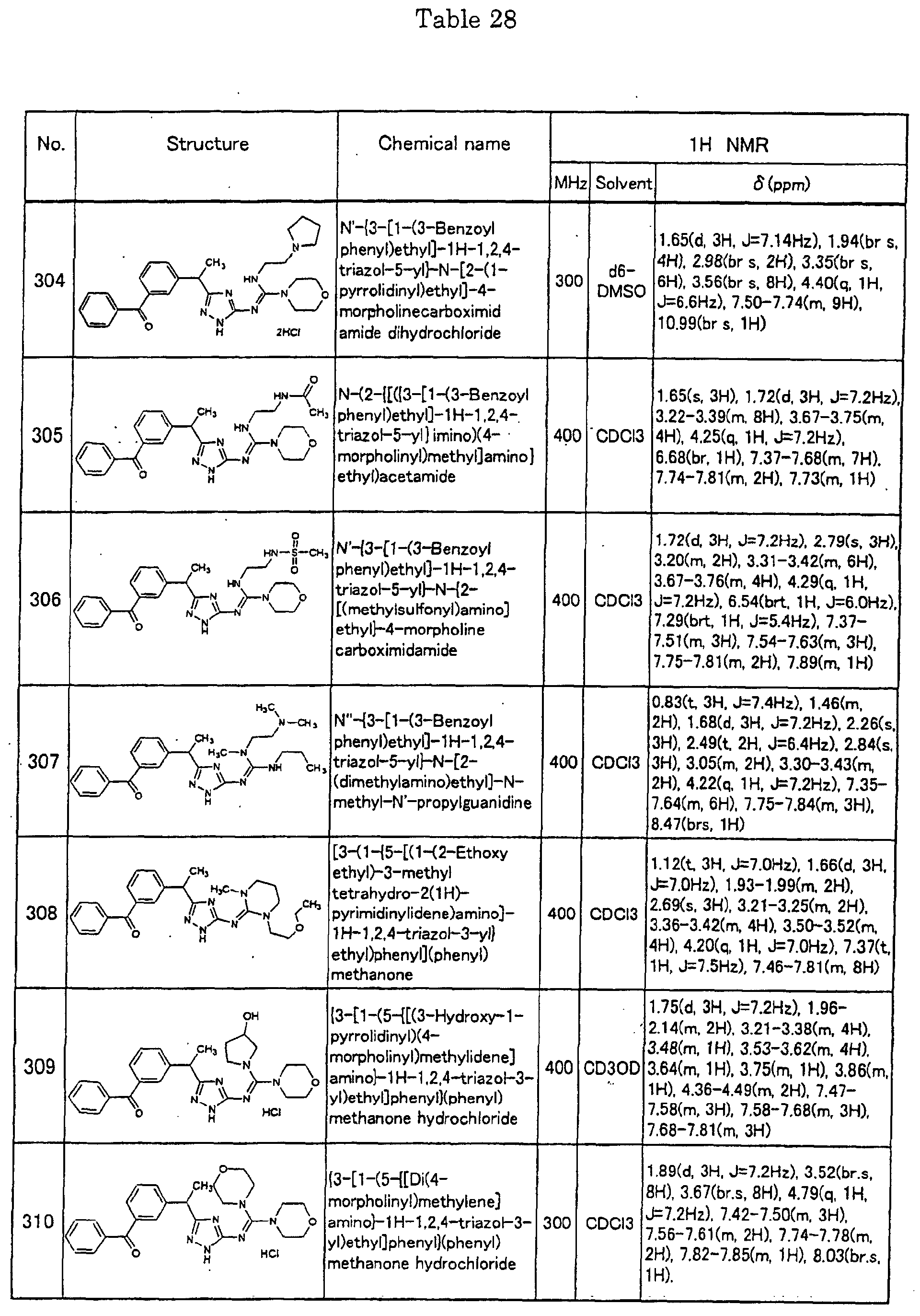

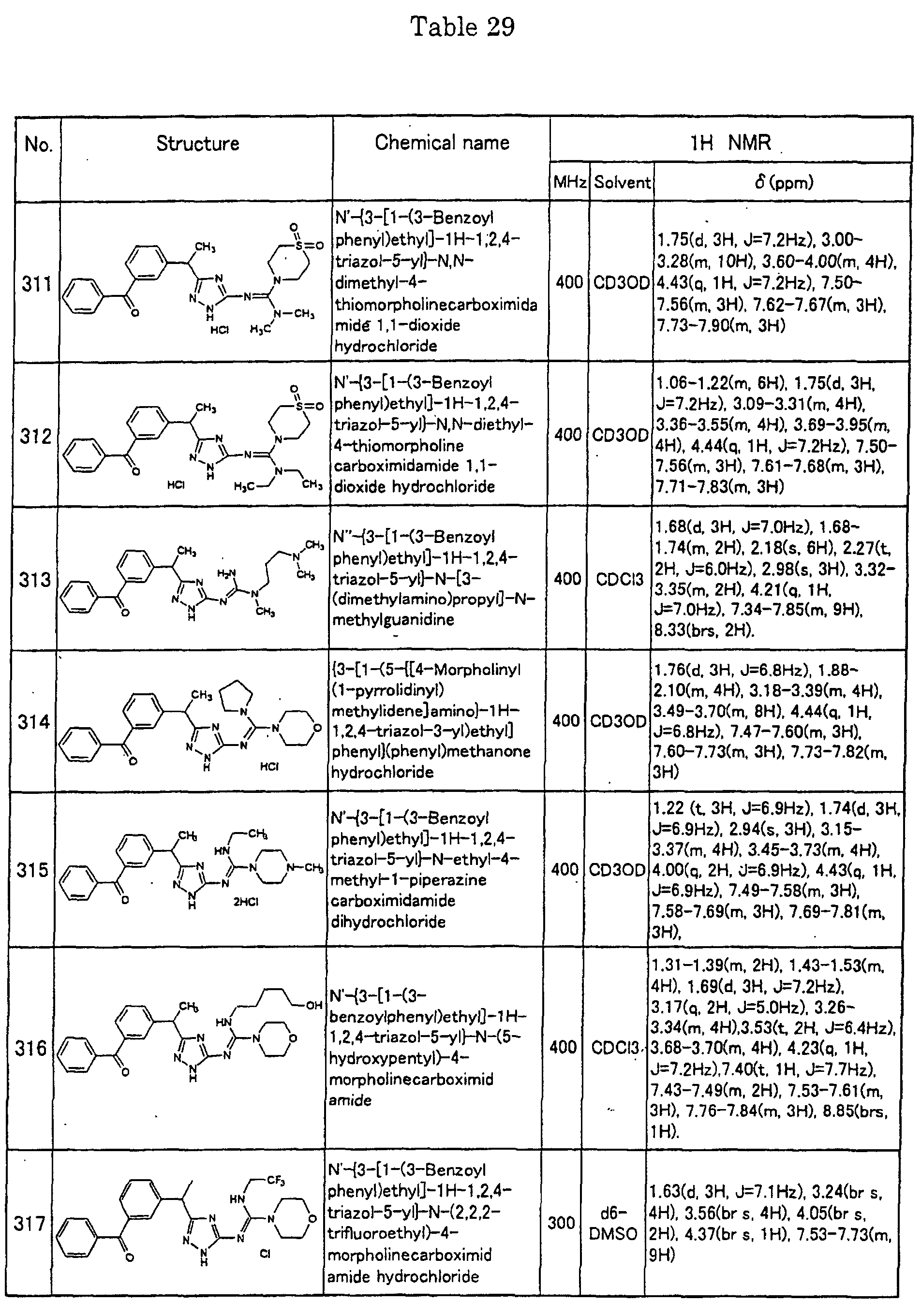

### Example 318

### (3-{1-[5-(2-(4-Morpholinyl)-5,6-dihydro-1(4H)-pyrimidinyl)-1H-1,2,4-triazol-3-yl]ethyl}phenyl)(phenyl)methanone

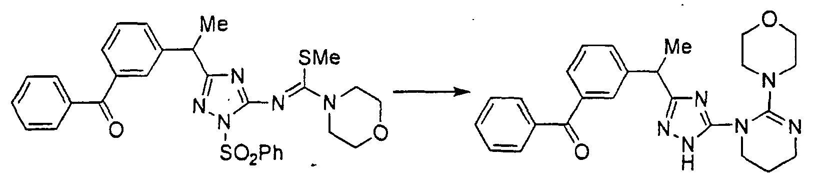

The compound (1.0 g) obtained in Reference Example 55 was dissolved in dioxane (10 mL), and 3-amino-1-propanol (1 mL) was added thereto and stirred at 70 °C for 1 hour. The mixture was cooled to room temperature, and ethyl acetate and water was added thereto, and the organic layer was separated. The organic layer was washed with water, dried, and concentrated. The residue was dissolved in dichloromethane (20 mL), and cooled to 0 °C. Triethylamine (0.41 mL) and methanesulfonyl chloride (0.24 mL) were added thereto and stirred for 2 hours. The reaction mixture was poured into a saturated aqueous ammonium chloride solution, and ethyl acetate and water were added thereto, and the organic layer was separated. The organic layer was washed with water, dried, and concentrated. The obtained residue was dissolved in dioxane (40 mL), and 2N aqueous sodium hydroxide solution (10 mL) was added thereto and stirred at 70 °C for 1 hour. The mixture was cooled to room temperature. Water and ethyl acetate were added thereto and the organic layer was separated and the aqueous layer was extracted five times with chloroform. The organic layers were dried over sodium sulfate and concentrated to obtain the desired compound (0.62 g).
¹H-NMR(300MHz, CDCl₃) δ ppm: 1.69(d, 3H, J=7.2Hz), 1.84(m, 2H), 3.01-3.09(m, 4H), 3.38-3.47(m, 2H), 3.58-3.72(m, 4H), 3.77-3.85(m, 2H), 4.24(q, 1H, J=7.2Hz), 7.38-7.51(m, 3H), 7.56-7.66(m, 3H), 7.77-7.83(m, 3H)

### Example 319

### [3-(1-{5-[2-(4-Morpholinyl)-4,5-dihydro-1H-imidazol-1-yl]-1H-1,2,4-triazol-3-yl}ethyl)phenyl](phenyl)methanone

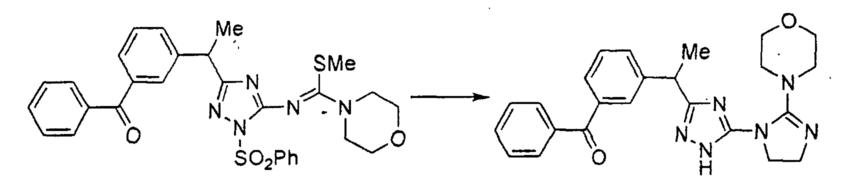

The desired compound was obtained from the compound obtained in Reference Example 55 and ethanolamine by the same procedure as in Example 318.
¹H-NMR(300MHz, CDCl₃) δ ppm: 1.77(d, 3H, J=7.2Hz), 3.30-3.42(m, 4H), 3.57-3.64(m, 4H), 3.71-3.77(m, 2H), 4.10(brt, J=8.3Hz), 4.52(q, 1H, J=7.2Hz), 7.40-7.50(m, 3H), 7.57-7.68(m, 3H), 7.75-7.80(m, 3H)

### Example 320

### {3-[1-(5-Amino-1H-1,2,4-triazol-3-yl)ethyl]phenyl}(phenyl)methanone hydrochloride

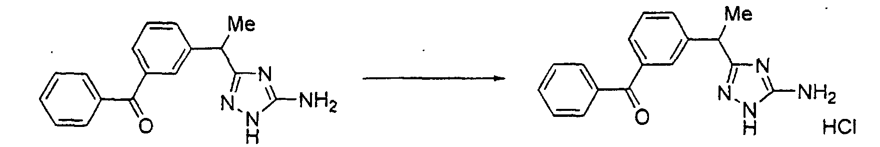

The desired compound was obtained by treating the compound obtained in Reference Example 50 with 1M HCl/ether.
¹H-NMR(400MHz, CD₃OD) δ ppm: 1.67(d, 3H, J=7.2Hz), 4.27(q, 1H, J=7.2Hz), 7.50-7.60(m, 4H), 7.62-7.70(m, 3H), 7.74-7.78(m, 2H)

### Reference Example 1

### 3-Amino-4-[3-(2-phenyl-1,3-dioxolan-2-yl)phenyl]-2-pentenonitrile

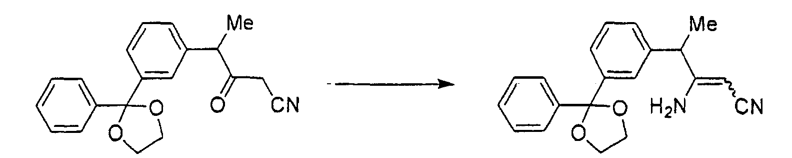

The compound (20 g) obtained in Reference Example 34 was dissolved in ethanol, and ammonia was added thereto. The mixture was kept at 170 °C in an autoclave for 10 hours. The solvent was evaporated. The residue was purifed by a silica gel column chromatography to obtain the desired compound (15.2 g).
¹H-NMR(300MHz, CDCl₃) δ ppm: 1.44(d, J=7.1Hz), 1.57(d, J=7.1Hz), (total 3H, 5:1), 3.54(q, J=7.1Hz), 4.34(q, J=7.1Hz), (total 1H, 5:1), 3.99-4.11(m, 5H), 4.53(br, 2H), 7.13-7.52(m, 9H)

### Reference Example 2

### 3-{1-[3-(2-Phenyl-1,3-dioxolan-2-yl)phenyl]ethyl}-5-isothiazolamine

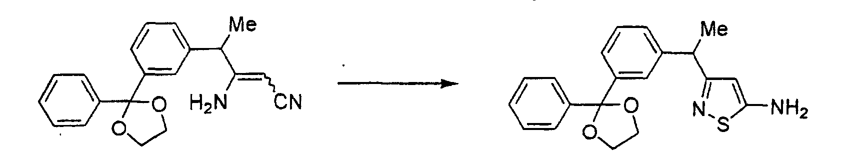

The compound (18.3 g) obtained in Reference Example 1 was treated with hydrogen sulfide in pyridine (100 mL) for 40 hours. Pyridine was evaporated under reduced pressure to give a crude thioamide intermediate (23.6 g). The thioamide was dissolved in acetonitrile (240 mL) and water (100 mL), and hydrogen peroxide (9.5 mL) was added dropwise thereto under ice-cooling and stirred for 1 hour. Acetonitrile was evaporated under reduced pressure. A saturated brine was added to the residue and the mixture was extracted with chloroform. The organic layer was dried and concentrated under reduced pressure. The crude compound was purified by a silica gel column chromatography (hexane/ethyl acetate = 4/1·1/1) to obtain the desired compound (7.5 g).
¹H-NMR(300MHz, CDCl₃) δ ppm: 1.61(d,3H,J=7.1Hz), 4.05(s,4H), 4.09(q,1H,J=7.1Hz), 4.30(br.s,2H), 5.97(s,1H), 7.13-7.18(m,1H), 7.21-7.36(m,5H), 7.44-7.53(m,3H).

### Reference Example 3

### Dimethyl 3-{1-[3-(2-phenyl-1,3-dioxolan-2-yl)phenyl]ethyl}-5-isothiazolyl dithioimidocarbonate

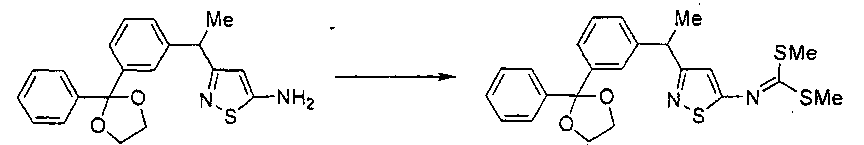

The desired compound was obtained from the compound obtained in Reference Example 2 by the same procedure as in Referernce Example 8.
¹H-NMR(300MHz, CDCl₃) δ ppm: 1.67(d,3H,J=7.1Hz), 2.56(br.s,6H), 4.05(s,4H), 4.23(q,1H,J=7.1Hz), 6.61(s,1H), 7.14-7.19(m,1H), 7.21-7.35(m,5H), 7.47-7.52(m,3H).

### Reference Example 4

### Methyl N-(3-{1-[3-(2-phenyl-1,3-dioxolan-2-yl)phenyl]ethyl}-5-isothiazolyl)-4-morpholinecarbimidothioate

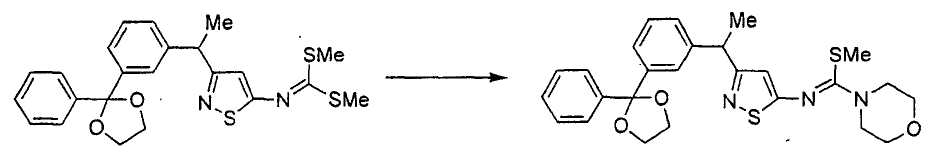

The desired compound was obtained from the compound obtained in Reference Example 3 by the same procedure as in Reference Example 9.
¹H-NMR(300MHz, CDCl₃) δ ppm: 1.65(d,3H,J=7.1Hz), 2.23(s,3H), 3.72(br.s,8H), 4.04(s,4H), 4.18(q,1H,J=7.1Hz), 6.45(s,1H), 7.15-7.35(m,6H), 7.47-7.51(m,3H).

### Reference Example 5

### N,N-Dimethyl-N'-(3-{1[3-(2-phenyl-1,3-dioxolan-2-yl)phenyl]ethyl} -5-isothiazolyl)carbamimidic chloride

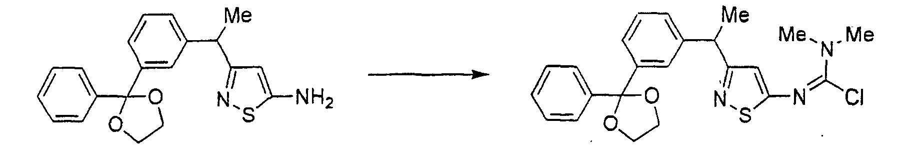

Phosgene iminium chloride (1.5 g) was added to a methylene chloride solution (50 mL) of the compound (3.00 g) obtained in Reference Example 2, and stirred for 1 hour. Chloroform (100 mL) was added to the mixture, and the mixture was washed with an aqueous saturated sodium bicarbonate solution, dried over sodium sulfate and concentrated to obtain the desired compound (4.00 g).
¹H-NMR(300MHz, CDCl₃) δ ppm: 1.65(d,3H,J=7.1Hz), 3.17(s,6H), 4.04(s,4H), 4.19(q,1H,J=7.1Hz), 6.49(s,1H), 7.14-7.35(m,6H), 7.48-7.52(m,3H).

### Reference Example 6

### 5-[1-(2-Fluoro[1,1'-biphenyl]-4-yl)ethyl]-1,3,4-oxadiazol-2-amine

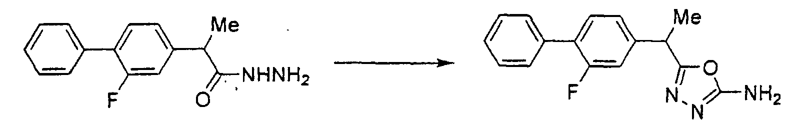

An aqueous solution (35 mL) of sodium bicarbonate (2.60 g) was added to a dioxane solution (100 mL) of 2-(2-fluoro[1,1'-biphenyl]-4-yl) propanohydrazine (7.26 g), which is known in Zagazig J. Pharm. Sci. (1996), 5(1), 29-35. Further 95% cyanogen bromide (3.45 g) was added thereto, and stirred at room temperature for 7 hours. The mixture was cooled with an ice-water bath. The precipitated crystals were collected by filtration, and dried to obtain the desired compound (5.18 g).
¹H-NMR(270MHz, d₆-DMSO) δ ppm: 1.59(d, 3H, J=7.2Hz), 4.34(q, 1H, J=7.2Hz), 6.92(br-s, 2H), 7.17-7.25(m, 2H), 7.3-7.7.55(m, 6H)

### Reference Example 7

### 5-{1-[3-(2-Phenyl-1,3-dioxolan-2-yl)phenyl]ethyl}-1,3,4-oxadiazol-2-amine

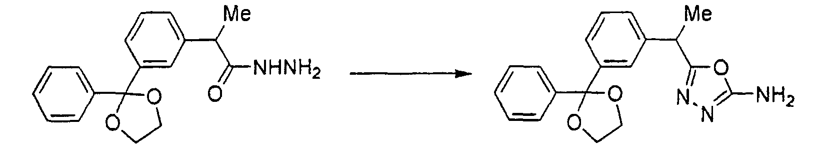

The compound (32.88 g) obtained in Reference Example 21 was dissolved in dioxane (500 mL), and an aqueous solution (150 mL) of sodium bicarbonate (9.73 g) was added thereto. Further 95% cyanogen bromide (12.92 g) was added thereto, and stirred at room temperature for 3 hours. Water (50 mL) was added thereto, and stirred for 1.5 hours. Then, the mixture was cooled with an ice-water bath. The precipitated crystals were collected by filtration, and dried to obtain the desired compound (23.10 g).
¹H-NMR(270MHz, CDCl₃) δ ppm: 11.67(d, 3H, J=7.2Hz), 4.03-4.08(m, 4H), 4.16(q, 1H, J=7.2Hz), 4.92(br-s, 2H), 7.17-7.52(m, 9H)

### Reference Example 8

### Dimethyl 5-{1-[3-(2-phenyl-1,3-dioxolan-2-yl)phenyl]ethyl}-1,3,4-oxadiazol-2-yldithioimidecarbonate

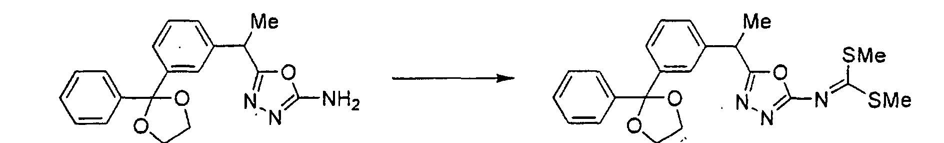

Under a nitrogen atmosphere, the compound (19.64 g) obtained in Reference Example 7 was dissolved in DMF (58 mL), and 20M aqueous sodium hydroxide solution (3.5 mL) was added dropwise thereto at 0 °C, which taking 10 min, and stirred for 30 min. Carbon disulfide (8.86 g) was added dropwise thereto, and stirred for 30 min. 20M Aqueous sodium hydroxide solution (3.5 mL) was added dropwise thereto, which taking 10 min, and warmed to room temperature and stirred for 30 min. Methyl iodide (19.83 g) was added thereto at 0 °C and stirred for 4 hours. The reaction mixture was poured into water (500 mL), extracted with ethyl acetate, dried, and concentrated. The residue was purified by a silica gel column chromatography (hexane/ethyl acetate = 5/1 - 5/2) to obtain the desired compound (20.18 g).
¹H-NMR(270MHz, CDCl₃) δ ppm: 1.73(d, 3H, J=7.3Hz), 2.60(s, 6H), 4.05(s, 4H), 4.30(q, 1H, J=7.3Hz), 7.22-7.39(m, 6H), 7.48-7.53(m, 3H)

### Reference Example 9

### Methyl N-(5-{1-[3-(2-phenyl-1,3-dioxolan-2-yl)phenyl]ethyl}-1,3,4-oxadiazol-2-yl)-4-morpholinecarboximidethioate

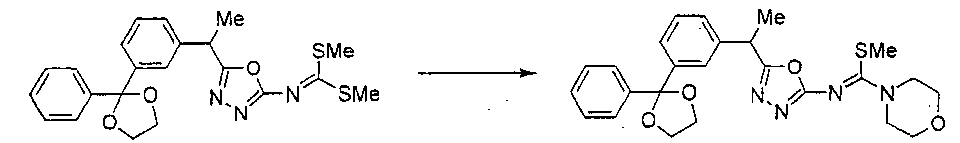

Under a nitrogen atmosphere, the compound (14.26 g) obtained in Reference Example 8 was dissolved in THF (280 mL), and morpholine (3.37 g) was added and stirred at room temperature for 3 hours. The mixture was concentrated under reduced pressure and purified by a silica gel column chromatography (chloroform) to obtain the desired compound (15.51 g).
¹H-NMR(270MHz, CDCl₃) δ ppm: 1.70(d, 3H, J=7.3Hz), 2.03(s, 3H), 3.68-3.72(m, 4H), 3.77-3.81(m, 4H), 4.04(s, 4H), 4.24(q, 1H, J=7.3Hz), 7.24-7.38(m, 6H), 7.46-7.50(m, 3H)

### Reference Example 10

### Methyl

### N-(5-{1-[3-(2-phenyl-1,3-dioxolan-2-yl)phenyl]ethyl}-1,3,4-oxadiazol-2-yl)-4 -thiomorpholinecarboximidethioate

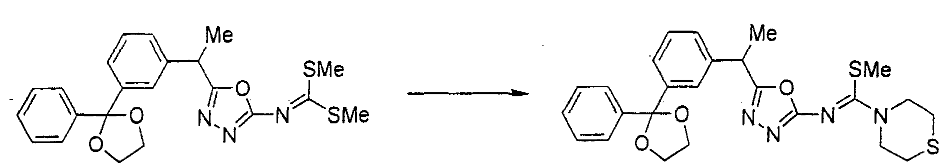

Under a nitrogen atmosphere, the compound (11.25 g) obtained in Reference Example 8 was dissolved in THF (250 mL), and thiomorpholine (2.76 g) was added and stirred at room temperature for 12 hours. The mixture was concentrated under reduced pressure and purified by a silica gel column chromatography (chloroform/methanol = 1/0 - 100/1 - 50/1) to obtain the desired compound (12.37 g, yellow oil).
¹H-NMR(300MHz, CDCl₃) δ ppm: 1.70(d, 3H, J=7.2Hz), 2.02(s, 3H), 2.66-2.70(m, 4H), 4.04-4.09(m, 4H), 4.24(q, 1H, J=7.2Hz), 7.22-7.39(m, 6H), 7.47-7.50(m, 3H)

### Reference Example 11

### (3-Bromo-4-methylphenyl)(phenyl)methanone

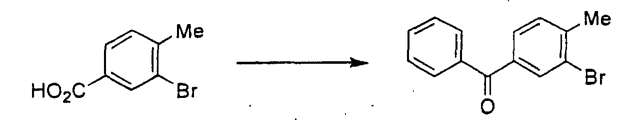

Thionyl chloride (50 mL) and DMF (0.1 mL) were added dropwise successively to 3-bromo-4-methylbenzoic acid (10 g), and stirred under reflux with a drying tube filled with calcium chloride for 4 hours. After cooled to room temperature, the mixture was concentrated under reduced pressure to obtain 3-bromo-4-methylbenzoyl chloride. Under a nitrogen atmosphere, aluminum chloride (7.4 g) was added to a benzene solution (100 mL) of the obtained residue and stirred under reflux for 6 hours. After cooled to room temperature, the reaction mixture was poured into 10% hydrochloric acid (300 mL) and extracted twice with toluene (200 mL). The obtained organic layers were washed with 10% aqueous sodium hydroxide solution (200 mL) and a saturated brine (200 mL), dried over sodium sulfate, and concentrated under reduced pressure. The residue was purified by a silica gel column chromatography (ethyl acetate/hexane = 1/10) to obtain the desired compound (8.8 g).
¹H-NMR(300MHz, CDCl₃) δ ppm: 2.49 (s, 3H), 7.35 (d, 1H, J=10.5 Hz), 7.50 (dd, 1H, J=11.0 Hz), 7.51 (d, 1H, J=10.5 Hz), 7.58-7.66 (m, 2H), 7.76-7.80 (m, 2H), 7.98 (s, 1H, J=2.0 Hz).

### Reference Example 12

### 5-Benzoyl-2-methylbenzonitrile

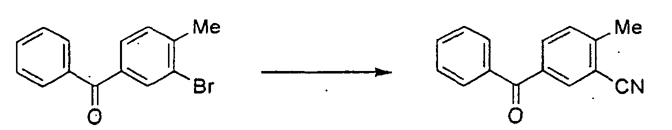

The compound (5.0 g) obtained in Reference Example 11 was dissolved in N-methyl-2-pyrrolidinone (10 mL) and copper cyanide (I) (2.0 g) was added thereto and stirred at 180 °C for 4 hours. After cooled to room temperature, the reaction mixture was poured into 10% aqueous ethylenediamine solution (200 mL) and extracted three times with ethyl acetate (100 mL). The obtained organic layers were washed with water (200 mL) and a saturated brine (200 mL), dried over sodium sulfate, and concentrated under reduced pressure. The residue was purified by a silica gel column chromatography (ethyl acetate/hexane = 1/6) to obtain the desired compound (3.4 g).
¹H-NMR(400MHz, CDCl₃) δ ppm: 2.66 (s, 3H), 7.47 (d, 1H, J=8.0 Hz), 7.52 (dd, 2H, J=7.5 Hz), 7.64 (dd, 1H, J=7.5 Hz), 7.77 (d, 2H, J=7.0 Hz), 7.95 (d, 1H, J=2.0, 8.0 Hz), 8.03 (s, 1H, J=2.0 Hz).

### Reference Example 13

### 5-Bromo-2-methylbenzoic acid

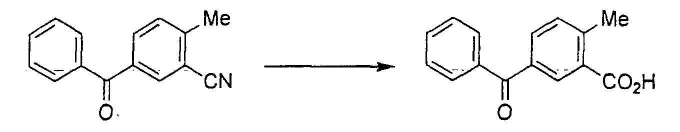

The compound (1.4 g) obtained in Reference Example 12 was dissolved in ethanol (20 mL) and 4N aqueous sodium hydroxide solution (5 mL) was added thereto at room temperature and stirred under reflux for 3 hours. After cooled to room temperature, the reaction mixture was poured into water (100 mL) and extracted with ethyl acetate (100 mL). The obtained organic layer was washed with a saturated brine (100 mL), dried over sodium sulfate, and concentrated under reduced pressure to obtain 5-benzoyl-2-methylbenzamide. An aqueous solution (10 mL) of sodium nitrite (1.7 g) was added slowly dropwise to a solution of the obtained residue in 75% aqueous sulfuric aicd solution (50 mL) under ice-cooling. After 30 min, the reaction mixture was warmed to room temperature and stirred for 6 hours. The reaction mixture was poured into ice-water (200 mL) and extracted with chloroform (100 mL). To the obtained organic layer was added 5% aqueous sodium bicarbonate solution (50 mL) and the aqueous layer was separated. To the aqueous layer was added chloroform (100 mL) and the mixture was acidified with 10% hydrochloric acid and extracted twice. The organic layers were washed with a saturated brine, dried over sodium sulfate and concentrated under reduced pressure to obtain the desired compound (1.3 g).

¹H-NMR(400MHz, CDCl₃) δ ppm: 2.75 (s, 3H), 7.43 (d, 1H, J=8.0 Hz), 7.51 (dd, 2H, J=7.5 Hz), 7.62 (dd, 1H, J=7.0 Hz), 7.80 (d, 2H, J=7.0 Hz), 7.94 (d, 1H, J=2.0, 8.0 Hz), 8.49 (s, 1H, J=2.0 Hz).

### Reference Example 14

### (5-Benzoyl-2-methylphenyl)acetic acid

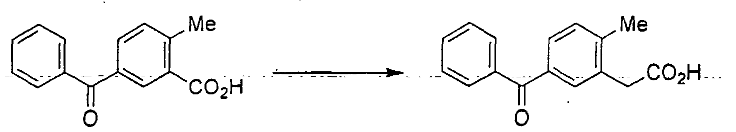

Under a nitrogen atmosphere, oxalyl chloride (3.3 mL) was added to a methylene chloride solution (30 mL) of the compound (3.0 g) obtained in Reference Example 13 at room temperature, and stirred for 12 hours. After concentrated under reduced pressure, the reaction mixture was subjected to azeotropical concentration with toluene twice. Under a nitrogen stream, into a solution of the obtained residue in THF (25 mL) and diethylether (25 mL) was blown diazomethane gas under stirring, which diazomethane gas was produced by adding slowly an aqueous solution (14 mL) of potassium hydroxide (4.2 g) to a mixture of 2-(2-ethoxy)ethoxyethanol (40 mL), diethylether (80 mL) and Diazald (13 g) at room temperature. After 1 hour, the mixture was kept under a nitrogen stream for 1 hour and concentrated under reduced pressure. Subsequently silver oxide (2.9 g) was added to an aqueous solution (50 mL) of sodium thiosulfate (5.3 g) at room temperature and stirred at 65 °C, and a 1,4-dioxane solution (25 mL) of the obtained residue was added dropwise thereto. After 2 hours, the precipitates were filtered off and washed with water (50 mL) and chloroform (50 mL). The filtrates were poured to water (100 mL) and extracted with chloroform (100 mL). The organic layer was washed with a saturated brine (100 mL), dried over sodium sulfate and concentrated under reduced pressure. Chloroform (100 mL) and 5% aqueous sodium hydroxide solution (100 mL) were added to the obtained residue and the aqueous layer was separated. To the aqueous layer was added chloroform (100 mL), acidified with 10% hydrochloric aicd and extracted twice. The organic layers were washed with a saturated brine (100 mL), dried over sodium sulfate and concentrated to obtain the desired compound (2.9 g).
¹H-NMR(400MHz, CDCl₃) δ ppm: 2.41 (s, 3H), 3.75 (s, 2H), 7.38 (d, 1H, J=8.0 Hz), 7.49 (dd, 2H, J=8.0 Hz), 7.58 (dd, 1H, J=8.0 Hz), 7.64 (d, 1H, J=1.5, 8.0 Hz), 7.70 (s, 1H, J=1.5 Hz), 7.78 (d, 2H, J=1.5, 8.0 Hz).

### Reference Example 15

### 2-(5-Benzoyl-2-methylphenyl)acetohydrazide

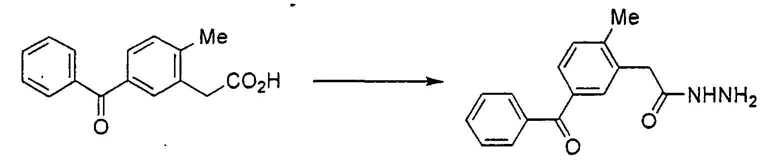

Oxalyl chloride (2 mL) was added to a methylene chloride solution (20 mL) of the compound (1.0 g) obtained in Reference Example 14 at room temperature, and stirred for 6 hours. After concentrated under reduced pressure, the reaction mixture was subjected to azeotropical concentration with toluene twice to obtain (5-benzoyl-2-methylphenyl) acetyl chloride. Under a nitrogen atmosphere, a THF solution (20 mL) of the (5-benzoyl-2-methylphenyl)acetyl chloride was added slowly to a THF solution (20 mL) of hydrazine monohydrate (3.8 mL) under ice-cooling. After 30 min, the reaction mixture was poured into water (50 mL) and extracted with ethyl acetate (50 mL). The organic layer was washed with a saturated brine, dried over sodium sulfate and concentrated under reduced pressure. The obtained residue was purified by a silica gel column chromatography (chloroform/methanol = 20/1) to obtain the desired compound (0.60 g).
¹H-NMR(400MHz, CDCl₃) δ ppm: 2.40 (s, 3H), 3.66 (s, 2H), 3.88 (brs, 2H), 6.63 (brs, 1H), 7.32 (d, 1H, J=8.0 Hz), 7.49 (dd, 2H, J=8.0 Hz), 7.60 (dd, 1H, J=1.5, 8.0 Hz), 7.64 (d, 1H, J=1.5, 8.0 Hz), 7.68 (s, 1H, J=1.5 Hz), 7.78 (d, 2H, J=1.5, 8.0 Hz).

### Reference Example 16

### {3-[(5-Amino-1,3,4-oxadiazol-2-yl)methyl]-4-methylphenyl}(phenyl) methanone

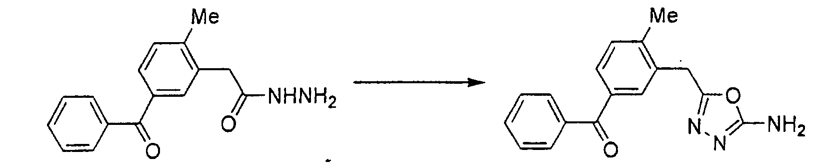

The compound (0.60 g) obtained in Reference Example 15 was dissolved in dioxane (20 mL) and sodium bicarbonate (0.21 g) and cyanogen bromide (0.26 g) were added thereto successively at room temperature, and stirred for 3 hours. The reaction mixture was poured into a saturated aqueous sodium bicarbonate solution (100 mL) and extracted with ethyl acetate (100 mL). The organic layer was washed with a saturated brine (100 mL), dried over sodium sulfate and concentrated under reduced pressure. The obtained residue was purified by a silica gel column chromatography (chloroform/methanol = 20/1) to obtain the desired compound (0.61 g).
¹H-NMR(400MHz, CDCl₃) δ ppm: 2.46 (s, 3H), 4.10 (s, 2H), 4.95 (brs, 2H), 7.30 (d, 1H, J=8.0 Hz), 7.48 (dd, 2H, J=8.0 Hz), 7.57-7.64 (m, 2H), 7.74 (s, 1H, J=1.5 Hz), 7.78 (d, 2H, J=1.5, 8.0 Hz).

### Reference Example 17

### 5-[1-(2-Fluoro[1,1'-biphenyl]-4-yl)ethyl]-1,3,4-thiadiazol-2-amine

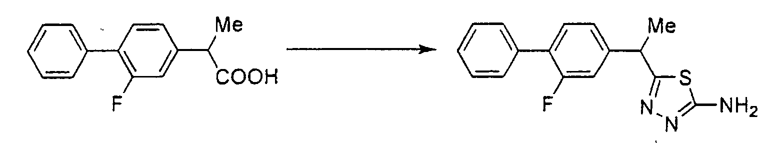

Flurbiprofen (10.0 g) was dissolved in dioxane (100mL) and thiosemicarbazide (3.73 g) was added thereto and stirred at 90 °C for 30 min. Phosphorus oxychloride (3.75 mL) was added thereto and stirred under reflux for 6 hours. The mixture was cooled to room temerature and concentrated under reduced pressure. Chloroform and 1N a queous sodium hydroxide solution were added thereto and stirred. The organic layer was separated and the aueous layer was extracted. The organic layers were dried and concentrated under reduced pressure. The desired compound (8.0 g) was recrystallized from the obtained crude crystals in ethanol.
¹H-NMR(300MHz, CDCl₃) δ ppm: 1.78(d, 3H, J=7.3Hz), 4.44(q, 1H, J=7.3Hz), 5.16(br-s, 2H), 7.08-7.19(m, 2H), 7.33-7.56(m, 6H)

### Reference Example 18

### Dimethyl 5-[1-(2-fluoro[1,1'-biphenyl]-4-yl)ethyl]-1,3,4-thiadiazol-2-yl dithioimidecarbonate

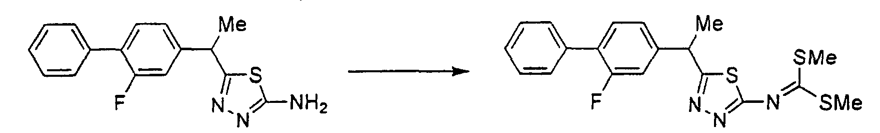

The desired compound was obtained from the compound obtained in Reference Example 17 by the same procedure as in Reference Example 26.
¹H-NMR(300MHz, CDCl₃) δ ppm: 1.84(d, 3H, J=7.2Hz), 2.59(s, 6H), 4.56(q, 1H, J=7.2Hz), 7.11-7.22(m, 2H), 7.32-7.56(m, 6H)

### Reference Example 19

### Methyl N-{5-[1-(2-fluoro[1,1'-biphenyl]-4-yl)ethyl]-1,3,4-thiadiazol-2-yl}-4-morpholinecarbimidothioate

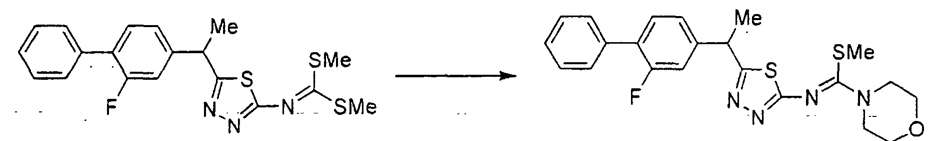

The desired compound was obtained from the compound obtained in Reference Example 18 by the same procedure as in Reference Example 27.
¹H-NMR(300MHz, CDCl₃) δ ppm: 1.81(d, 3H, J=7.2Hz), 2.24(s, 3H), 3.70-3.85(m, 8H), 4.52(q, 1H, J=7.2Hz), 7.10-7.22(m, 2H), 7.32-7.56(m, 6H)

### Reference Example 20

### Methyl 2-[3-(2-Phenyl-1,3-dioxolan-2-yl)phenyl]propanoate

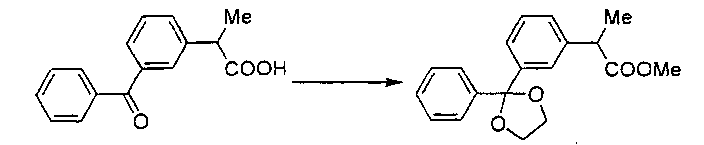

The desired compound was obtained from ketoprofen by the same procedure as described in JP-A-63-152368.
¹H-NMR(300MHz, CDCl₃) δ ppm: 1.48(d, 3H, J=7.1Hz), 3.63(s, 3H), 3.71(q, 1H, J=7.1Hz), 4.05-4.07(m, 4H), 7.23-7.52(m, 9H)

### Reference Example 21

### 2-[3-(2-Phenyl-1,3-dioxolan-2-yl)phenyl]propanohydrazide

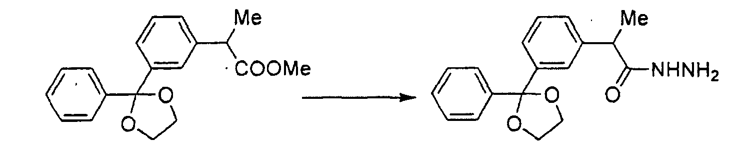

Ethanol (10 mL) and hydrazine monohydrate (6 mL) were added to the compound (10.0 g) obtained in Reference Example 20 and stirred under reflux for 2 hours. After cooled to room temperature, the mixture was poured into water and extracted with chloroform. The organic layer was dried and concentrated under reduced pressure to obtain the desired compound (9.8 g).
¹H-NMR(300MHz, CDCl₃) δ ppm: 1.52(d, 3H, J=7.2Hz), 3.52(q, 1H, J=7.2Hz), 3.80(br-s, 2H), 4.01-4.11(m, 4H), 6.55(br-s, 1H), 7.19-7.54(m, 9H)

### Reference Example 22

### {3-[1-(5-Amino-1,3,4-thiadiazol-2-yl)ethyl]phenyl}(pheny)methanone

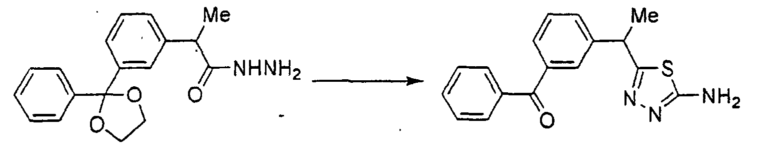

Benzoyl isothiocyanate (4.8 mL) was added dropwise to a chloroform solution (100 mL) of the compound (12.4 g) obtained in Reference Example 21 at room temperature and stirred for 30 min. The reaction mixture was concentrated under reduced pressure, dissolved in a concentrated sulfuric acid (50 mL) and stirred at room temperature for 30 min and at 70 °C for 4 hours. The reaction mixture was poured into ice-water, neutralized with 5N aqueous sodium hydroxide solution and extracted with ethyl acetate. The organic layer was washed with wter, dried and concentrated under reduced pressure. The residue was purified by a silica gel column chromatography to obtain the desired compound (4.2 g).
¹H-NMR(300MHz, CDCl₃) δ ppm: 1.78(d, 3H, J=7.2Hz), 4.48(q, 1H, J=7.2Hz), 5.05(br-s, 2H), 7.41-7.82(m, 9H)

### Reference Example 23

### 2-(2-Fluoro[1,1'-biphenyl]-4-yl)propanamide

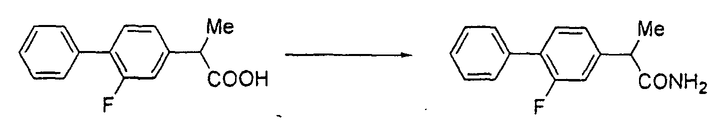

A mixture of flurbiprofen (30.0 g), toluene (300 mL), thionyl chloride (9.4 mL) and 2 drops of DMF was stirred under reflux for 2 hours. After cooled to room temperature, the solvent was evaporated and the mixture was subjected to azeotropical concentration using toluene (100 mL). The reaction mixture was concentrated under reduced pressure and toluene (300 mL) was added to the residue. Ammonia gas was blown into the mixture keeping it below 20 °C. After 3 hours, water and ethyl acetate were added thereto and the organic layer was separated. The organic layer was washed with water, dried and concentrated to obtain the desired compound (28.6 g).
¹H-NMR(300MHz, CDCl₃) δ ppm: 1.56(d, 3H, J=7.2Hz), 3.64(q, 1H, J=7.2Hz), 5.42(br-s, 1H), 5.58(br-s, 1H), 7.11-7.19(m, 2H), 7.34-7.56(m, 6H)

### Reference Example 24

### N'-Chloro-2-(2-fluoro[1,1'-biphenyl]-4-yl)propanimidamide

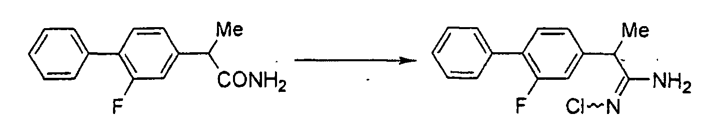

Dimethyl sulfate (8.1 mL) was added to the compound (20.0 g) obtained in Reference Example 23 and stirred at 100 °C for 6 hours. After cooled to room temperature, the mixture was stirred for 2 days. Ethyl acetate was added to the obtained oil and neutralized with a saturated sodium bicarbonate solution. The organic layer was separated and the aqueous layer was extracted. The organic layers were washed with water, dried and concentrated under reduced pressure. The residue was dissolved in methanol (200 mL) and ammonium chloride (4.4 g) was added thereto and stirred under reflux for 3.5 hours. The reaction mixture was concentrated under reduced pressure. Water (100 mL) and ethyl acetate (100 mL) were added thereto, and the aqueous layer was separated. To the aqueous layer was added diethyl ether (100 mL), cooled to 0 °C and an aqueous sodium hypochlorite solution was added dropwise until the reaction finished. The organic layer was separated, dried, concentrated under reduced pressure and the residue was purified by a silica gel chromatography to obtain the desired compound (7.0 g).
¹H-NMR(300MHz, CDCl₃) δ ppm: 1.59(d, 3H, J=7.2Hz), 3.87(q, 1H, J=7.2Hz), 5.10(br-s, 2H), 7.09-7.20(m, 2H), 7.35-7.59(m, 6H)

### Reference Example 25

### 3-[1-(2-Fluoro[1,1'-biphenyl]-4-yl)ethyl]-1,2,4-thiadiazol-5-amine

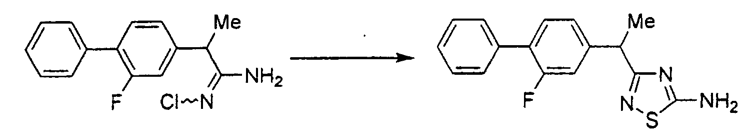

Potassium thiocyanate (3.7 g) was added to a methanol solution (140 mL) of the compound (7.0 g) obtained in Reference Example 24 at 0 °C and stirred at the same temperature for 30 min and at room temperature for 3 days. After concentrated under reduced pressure, water and ethyl acetate were added thereto and the organic layer was separated and the aqueous layer was extracted. The organic layers were washed with water, dried, concentrated under reduced pressure to obtain the desired compound (6.9 g).
¹H-NMR(300MHz, CDCl₃) δ ppm: 1.70(d, 3H, J=7.2Hz), 4.26(q, 1H, J=7.2Hz), 5.50(br-s, 2H), 7.09-7.18(m, 2H), 7.31-7.55(m, 6H)

### Reference Example 26

### Dimethyl 3-[1-(2-fluoro[1,1'-biphenyl]-4-yl)ethyl]-1,2,4-thiadiazol-5-yl dithioimidecarbonate

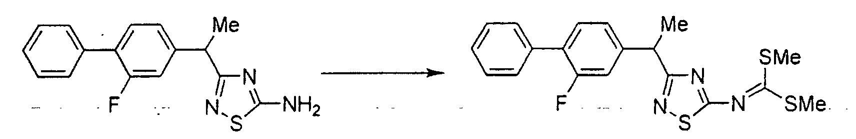

The compound (2.70 g) obtained in Reference Example 25 was dissolved in DMF (9 mL) and 20M aqueous sodium hydroxide solution (0.54 mL) was added thereto at 0 °C, and stirred at 0°C for 30 min. Carbon disulfide (1.08 mL) was added dropwise thereto and stirred at 0 °C for further 30 min. Then, 20M aqueous sodium hydroxide solution (0.54 mL) was added thereto again and stirred at 0 °C for 30 min, and methyl iodide (1.12 mL) was added dropwise and stirred at room temperature for 2 hours. The raction mixture was poured into a saturated brine and extracted with ethyl acetate. The organic layer was dried over magnesium sulfate and concentrated by an evapolater. The residue was purified by a silica gel column chromatography (hexane/ethyl acetate = 9/1 - 4/1) to obtain the desired compound (2.61 g, light yellow oil).
¹H-NMR(300MHz, CDCl₃) δ ppm: 1.78(d, 3H, J=7.1Hz), 2.64(s, 6H), 4.47(q, 1H, J=7.20Hz), 6.91-7.23(m, 2H), 7.31-7.45(m, 4H), 7.49-7.54(m, 2H)

### Reference Example 27

### Methyl N-{3-[1-(2-fluoro[1,1'-biphenyl]-4-yl)ethyl]-1,2,4-thiadiazol-5-yl}-4-morpholinecarboximidethioate

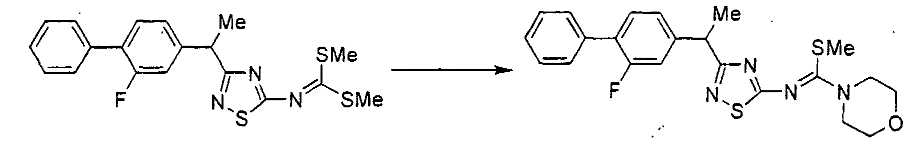

The compound (2.61 g) obtained in Reference Example 26 was dissolved in ethanol (6.5 mL) and morpholine (1.13 mL) was added thereto at room temperature and stirred for 1 hour. The raction mixture was poured into a saturated brine and extracted with ethyl acetate. The organic layer was dried over magnesium sulfate and concentrated by an evapolater. The residue was purified by a silica gel column chromatography (hexane/ethyl acetate = 1/1) to obtain the desired compound (2.35 g, yellow oil).
¹H-NMR(300MHz, CDCl₃) δ ppm:1.77(d, 3H, J=7.1Hz), 2.25(s, 3H), 3.72-3.75(m, 4H), 3.87-3.90(m, 4H), 4.40(q, 1H, J=7.2Hz), 7.15-7.23(m, 2H), 7.31-7.45(m, 4H), 7.51-7.53(m, 2H)

### Reference Example 28

### 2-(3-Benzoylphenyl)propanamide

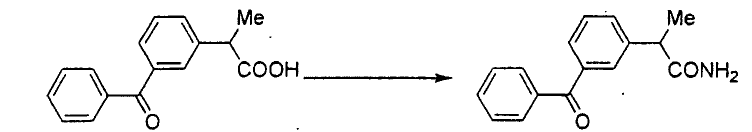

Toluene (500 mL), thionyl chloride (15 mL) and a drop of DMF were added to ketoprofen (50.0 g) and stirred at 90 °C for 1 hour. After cooled to room temperature, the reaction mixture was concentrated under reduced pressure. 25.5 g of the obtained residue was taken and dissolved in toluene (250 mL). Ammonia gas was blown into the mixture. After the reaction finished, water was added to the mixture and the mixture was extracted with ethyl acetate. The organic layer was washed with water, dried and concentrated under reduced pressure to obtain the desired compound (22.5 g).
¹H-NMR(300MHz, CDCl₃) δ ppm: 1.56(d, 3H, J=7.2Hz), 3.69(q, 1H, J=7.2Hz), 5.52(br-s, 2H), 7.44-7.84(m, 9H)

### Reference Example 29

### {3-[1-(5-Amino-1,2,4-thiadiazol-3-yl)ethyl]phenyl}(phenyl)methanone

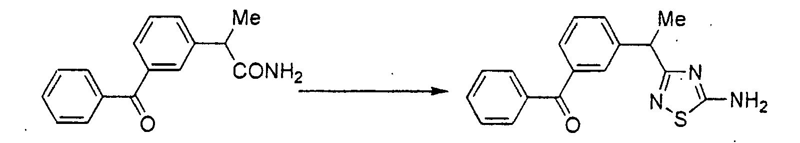

The desired compound was obtained from the compound obtained in Reference Example 28 by the same procedure as described in Reference Example 24 and Reference Example 25.
¹H-NMR(300MHz, CDCl₃) δ ppm: 1.69(d, 3H, J=7.2Hz), 4.26(q, 1H, J=7.2Hz), 6.09(br-s, 2H), 7.35-7.64(m, 6H), 7.74-7.84(m, 3H)

### Reference Example 30

### Dimethyl 3-[1(3-benzoylphenyl)ethyl]-1,2,4-thiadiazol-5-yl dithioimidecarbonate

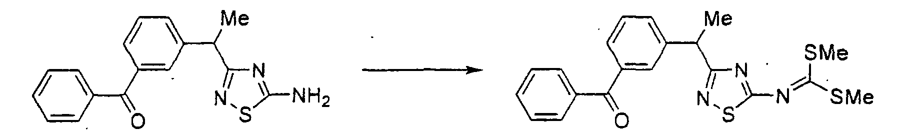

The desired compound was obtained from the compound obtained in Reference Example 29 by the same procedure as described in Reference Example 26.
¹H-NMR(300MHz, CDCl₃) δ ppm: 1.77(d, 3H, J=7.1Hz), 2.62(s, 6H), 4.50(q, 1H, J=7.1Hz), 7.36-7.86(m, 9H)

### Reference Example 31

### Methyl N-{3-[1-(3-benzoylphenyl)ethyl]-1,2,4-thiadiazol-5-yl}-4-morpholinecarbimidothioate

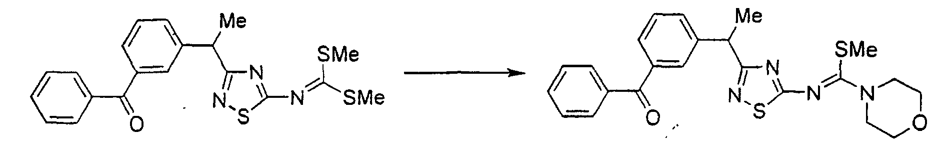

The compound (2.14 g) obtained in Reference Example 30 was dissolved in ethanol (50 mL) and morpholine (0.68 mL) was added thereto and stirred for 4 hours. The reaction mixture was concentrated by an evaporater. The residue was poured into a saturated brine and extracted with ethyl acetate. The organic layer was dried over magnesium sulfate and concentrated under reduced pressure. The residue was purified by a silica gel flash chromatography (hexane/ethyl acetate = 1/1) to obtain the desired compound (1.79 g, colorless oil).
¹H-NMR(300MHz, CDCl₃) δ ppm: 1.75(d, 3H, J=7.1Hz), 2.22(s, 3H), 3.70-3.73(m, 4H), 3.85-3.88(m, 4H), 4.44(q, 1H, J=7.1Hz), 7.37-7.68(m, 6H), 7.76-7.80(m, 3H)

### Reference Example 32

### 3-[1-(2-Fluoro[1,1'-biphenyl]-4-yl)ethyl]-1H-pyrazole-5-amine

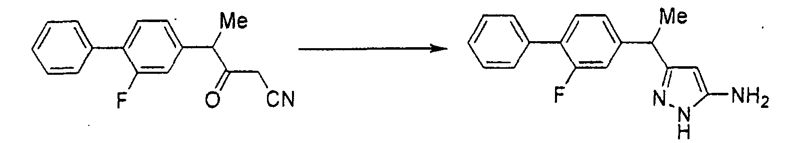

The known cyanide: 4-(2-fluoro[1,1'-biphenyl]-4-yl)-3-oxopentanonitrile (2.00 g) (JP-A-63-152368) was dissolved in ethanol (30 mL) and acetic acid (20 mL), and hydrazine monohydrate (0.73 mL) was added thereto and stirred at room temperature for 10 hours and at 50 °C for 5 hours. The reaction mixture was subjected to azeotropical concentration using toluene, and diluted with ethyl acetate. The organic layer was washed with a saturated brine, dried and concentrated under reduced pressure. The residue was purified by a silica gel column chromatography (hexane/ethyl acetate = 3/1) to obtain the desired compound (1.95 g).
¹H-NMR(270MHz, CDCl₃) δ ppm: 1.41(d, 3H, J=7.3Hz), 4.07(q, 1H, J=7.3Hz), 5.15(s, 1H), 6.87-7.55(m, 8H)

### Reference Example 33

### 3-[1-(2-Fluoro[1,1'-biphenyl]-4-yl)ethyl]-1-methyl-1H-pyrazole-5-amine

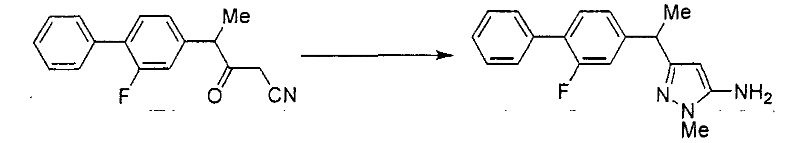

Methylhydrazine (1.2 mL) was added to an ethanol solution (30 mL) of the known cyanide: 4-(2-fluoro[1,1'-biphenyl]-4-yl)-3-oxopentanonitrile (3.0 g) (JP-A-63-152368) and stirred at 60°C for 3 hours. The reaction mixture was concentrated under reduced pressure. The residue was purified by a silica gel column chromatography (ethyl acetate) to obtain the desired compound (2.6 g).
¹H-NMR(300MHz, CDCl₃) δ ppm: 1.58(d, 3H, J=7.2Hz), 3.42(br-s, 2H), 3.62(s, 3H), 4.05(q, 1H, J=7.2Hz), 5.36(s, 1H), 7.02-7.14(m, 2H), 7.28-7.53(m, 6H)

### Reference Example 34

### 3-Oxo-4-[3-(2-phenyl-1,3-dioxolan-2-yl)phenyl]pentanonitrile

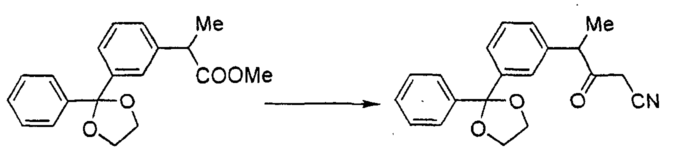

A THF suspension (200 mL) of 60% sodium hydride (8.44 g) was stirred under reflux and a solution of the compound (30.0 g) obtained in Reference Example 20 in acetonitrile (8.67 g) and THF (100 mL) was added dropwise thereinto, which taking 4 hours. After the addition, the mixture was stirred under reflux for futher 2 hours. The mixture was cooled to room temperature, neutralized with an aqueous saturated ammonium chloride solution and extracted with ethyl acetate. The organic layer was washed with a saturated brine, dried and concentrated under reduced pressure. The residue was purified by a silica gel column chromatography to obtain the desired compound (28.2 g).
¹H-NMR(300MHz, CDCl₃) δ ppm: 1.42(d, 3H, J=7.0Hz), 3.33(s, 2H), 3.88(q, 1H, J=7.0Hz), 4.03-4.08(m, 4H), 7.25-7.51(m, 9H)

### Reference Example 35

### {3-[1-(5-Amino-1-methyl-1H-pyrazol-3-yl)ethyl]phenyl}(phenyl)methanone

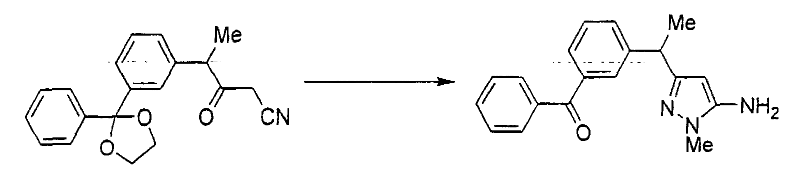

Methylhydrazine (1.3 mL) was added to an ethanol solution (40 mL) of the compound (4.0 g) obtained in Reference Example 34, and stirred at 60 °C for 3 hours. The reaction mixture was cooled to room temperature and concentrated under reduced pressure. The residue was dissolved in THF (20 mL). 1N Hydrochloric acid (20 mL) was added thereto, and stirred at 60 °C for 2.5 hours. The mixture was neutralized with 1N aqueous sodium hydroxide solution, extracted with ethyl acetate, washed with water, dried and concentrated under reduced pressure. The residue was purified by a silica gel column chromatography (ethyl acetate) to obtain the desired compound (2.3 g).
¹H-NMR(300MHz, CDCl₃) δ ppm: 1.60(d, 3H, J=7.2Hz), 3.42(br-s, 2H), 3.62(s, 3H), 4.11(q, 1H, J=7.2Hz), 5.33(s, 1H), 7.34-7.82(m, 9H)

### Reference Example 36

### 3-[1-(2-Fluoro[1,1'-biphenyl]-4-yl)ethyl]-1H-1,2,4-triazole-5-amine

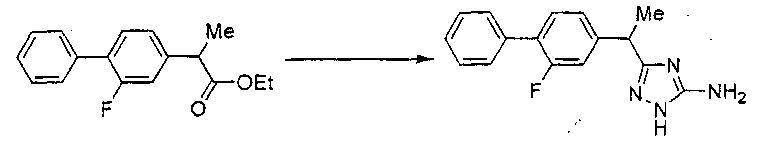

A sodium ethoxide solution was prepared by adding sodium (6.8 g) to ethanol (200 mL). To this solution was added dropwise aminoguanidine hydrochloride (32 g) and an ethanol solution (200 mL) of flurbiprofen ethyl ester (20 g) at 0 °C successively, and stirred under reflux for 13 hours. After cooled to room temperature, ethanol was evaporated. Water was added to the residue, and the mixture was neutralized with 1N hydrochloric acid and extracted with ethyl acetate. The organic layer was washed with water, dried, and concentrated under reduced pressure. The obtained residue was purified by a silica gel column chromatography (chloroform/methanol = 10/1) to obtain the desired compound (4.0 g).
¹H-NMR(300MHz, CDCl₃) δ ppm: 1.69(d, 3H, J=7.2Hz), 4.15(q, 1H, J=7.2Hz), 7.07-7.17(m, 2H), 7.32-7.56(m, 6H)

### Reference Example 37

### N-Benzoyl-N'-{3-[1-(2-fluoro[1,1'-biphenyl]-4-yl)ethyl]-1H-1,2,4-triazol-5-yl}thiourea

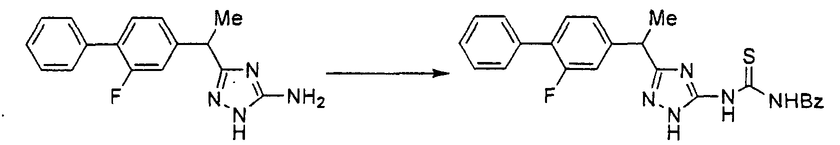

The compound (1.0 g) obtained in Reference Example 36 was dissolved in 1,2-dichloroethane (15 mL), and benzoylisothiocyanate (0.53 mL) was added thereto and stirred under reflux for 5 hours. The reaction mixture was concentrated under reduced pressure and purified by a silica gel column chromatography (ethyl acetate/hexane = 1/2) to obtain the desired compound (0.8 g).
¹H-NMR(300MHz, CDCl₃) δ ppm: 1.72(d, 3H, J=7.2Hz), 4.26(q, 1H, J=7.2Hz), 7.08-7.19(m, 2H), 7.30-7.68(m, 9H), 7.85-7.92(m, 2H)

### Reference Example 38

### N-{3-[1-(2-Fluoro[1,1'-biphenyl]-4-yl)ethyl]-1H-1,2,4-triazol-5-yl}thiourea

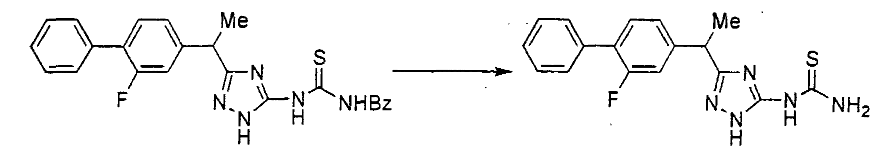

The compound (0.7 g) obtained in Reference Example 37 was dissolved in THF (5 mL) and methanol (5 mL), and potassium carbonate (0.33 g) was added thereto and stirred at 70 °C for 2 hours. The reaction mixture was cooled to room temperature and water was added thereto. The mixture was extracted with ethyl acetate, and the organic layer was washed with water, dried and concentrated under reduced pressure. The residue was purified by a silica gel column chromatography (ethyl acetate/hexane = 1/1 - 3/1) to obtain the desired compound (0.3 g).
¹H-NMR(300MHz, CDCl₃) δ ppm: 1.61(d, 3H, J=7.2Hz), 4.34(q, 1H, J=7.2Hz), 7.16-7.28(m, 2H), 7.35-7.54(m, 6H), 8.69(br-s, 1H), 9.06(br-s, 1H)

### Reference Example 39

### Methyl N'-{5-[1-(2-fluoro[1,1'-biphenyl]-4-yl)ethyl]-1H-1,2,4-triazol-3-yl} imidethiocarbamate

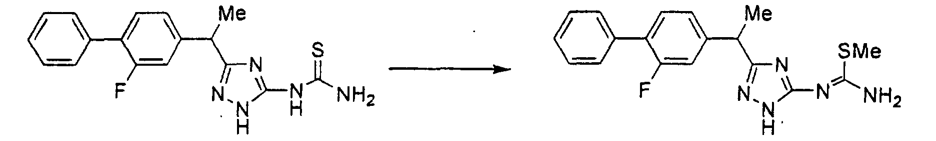

The compound (4.2 g) obtained in Reference Example 38 was dissolved in acetone (100 mL), and potassium carbonate (1.86 g) and methyl iodide (0.84 mL) were added thereto at room temperature and stirred for 1.5 hours. Water was added to the reaction mixture, and acetone was evaporated. The mixture was extracted with ethyl acetate. The organic layer was washed with water, dried, and concentrated, and the residue was purified by a silica gel column chromatography (ethyl acetate/hexane = 1/2) to obtain the desired compound (4.0 g).
¹H-NMR(300MHz, CDCl₃) δ ppm: 1.70(d, 3H, J=7.3Hz), 2.48(s, 3H), 4.23(q, 1H, J=7.3Hz), 7.11-7.20(m, 2H), 7.30-7.55(m, 6H)

### Reference Example 40

### Methyl N'-{3-[1-(2-fluoro[1,1'-biphenyl]-4-yl)ethyl]-1-methyl-1H-1,2,4-triazol-5-yl}imidethiocarbamate

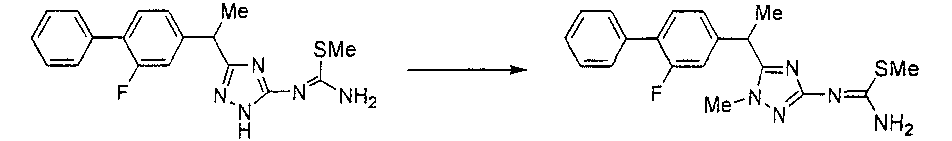

A THF solution (15 mL) of the compound (2.0 g) obtained in Reference Example 39 was added dropwise to a THF suspension (30 mL) of 60% sodium hydride (0.25 g) at 0 °C, and stirred for 30 min. Methyl iodide (0.39 mL) was added thereto and stirred for 1 hour. The mixture was warmed to room temperature and kept overnight. Water and ethyl acetate were added thereto, and the organic layer was separated, washed with water, dried, and concentrated. The residue was purified by a silica gel column chromatography (ethyl acetate/hexane = 1/1 - 3/1) to obtain the desired compound (1.6 g).
¹H-NMR(300MHz, CDCl₃) δ ppm: 1.75(d, 3H, J=7.2Hz), 2.56(s, 3H), 3.60(s, 3H), 4.18(q, 1H, J=7.2Hz), 6.99-7.08(m, 2H), 7.33-7.54(m, 6H)

### Reference Example 41

### 2-(3-Benzoylphenyl)propanonitrile

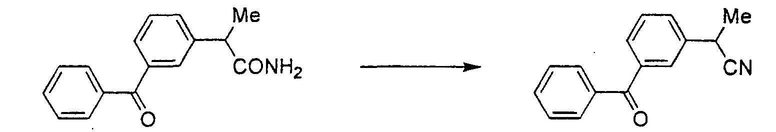

The compound (86 g) obtained in Reference Example 28 was dissolved in toluene (1000 mL), and phosphorus oxychloride (35 mL) was added thereto, and stirred under reflux for 1 hour. The reaction mixture was poured into a saturated sodium bicarbonate solution and stirred for a while. The toluene layer was separated, washed with water, dried, and the solvent was evaporated to obtain the desired compound (80 g).
¹H-NMR(300MHz, CDCl₃) δ ppm: 1.69(d, 3H, J=7.2Hz), 3.99(q, 1H, J=7.2Hz), 7.47-7.67(m, 5H), 7.72-7.83(m, 4H)

### Reference Example 42

### Methyl 2-(3-benzoylphenyl)propanimidoate hydrochloride

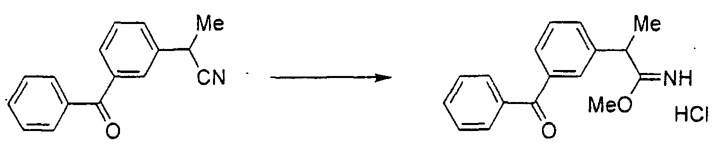

The compound (80 g) obtained in Reference Example 41 was dissolved in toluene (600 mL), and methanol (13.8 mL) was added thereto. The mixture was cooled to 0 °C, and hydrogen chloride gas was blown thereinto. The mixture was kept at 0 °C for 2 days, and concentrated. The precipitated crystals were collected and dried under reduced pressure to obtain the desired compound (100 g).
¹H-NMR(300MHz, CDCl₃) δ ppm: 1.70(d, 3H, J=7.3Hz), 4.30(s, 3H), 4.65(q, 1H, J=7.3Hz), 7.45-7.54(m, 3H), 7.57-7.73(m, 2H), 7.77-7.90(m, 4H)

### Reference Example 43

### Methyl (1Z)-2-(3-benzoylphenyl)-N-cyanopropanimidoate

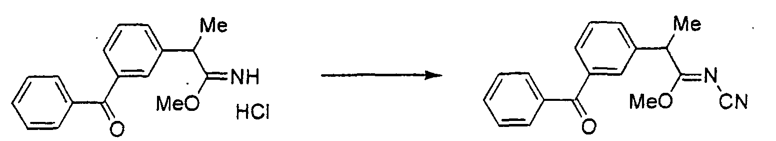

The compound (30 g) obtained in Reference Example 42 was dissolved in methanol (300 mL), and cyanamide (13 g) was added thereto, and stirred at 50 °C for 2 hours. Methanol was evaporated, and chloroform and water were added to the residue. The organic layer was separated and the aqueous layer was extracted. The organic layers were dried and concentrated to obtain the desired compound (36 g).
¹H-NMR(300MHz, CDCl₃) δ ppm: 1.60(d, 3H, J=7.2Hz), 3.88(s, 3H), 4.49(q, 1H, J=7.2Hz), 7.43-7.52(m, 3H), 7.56-7.73(m, 2H), 7.71(m, 1H), 7.76-7.82(m, 3H)

### Reference Example 44

### {3-[1-(5-Amino-1-methyl-1H-1,2,4-triazole-3-yl)ethyl]phenyl}(phenyl) methanone

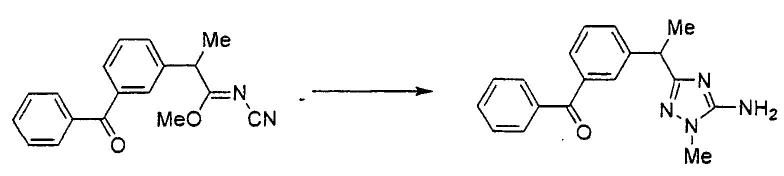

The compound (31.5 g) obtained in Reference Example 43 was dissolved in ethanol (300 mL), and methylhydrazine (4.8 mL) was added thereto and stirred at 0 °C for 10 min and at room temperature for 30 min. Water was added thereto and ethanl was evaporated. The mixture was extracted with chloroform. The organic layer was dried and concentrated, and the residue was purified by a silica gel column chromatography (ethyl acetate - chloroform/methanol = 5/1) to obtain the desired compound (18.8 g).
¹H-NMR(300MHz, CDCl₃) δ ppm: 1.66(d, 3H, J=7.2Hz), 3.60(s, 3H), 4.12(q, 1H, J=7.2Hz), 5.38(br, 2H), 7.34-7.62(m, 6H), 7.77-7.82(m, 3H)

### Reference Example 45

### N-Benzoyl-N'-{3-[1-(3-benzoylphenyl)ethyl]-1-methyl-1H-1,2,4-triazol-5-yl} thiourea

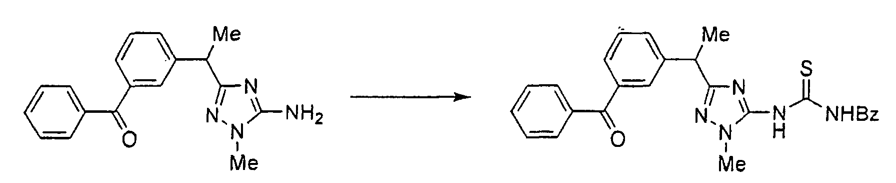

The compound (4.8 g) obtained in Reference Example 44 was dissolved in dichloroethane (50 mL), and benzoylisothiocyanate (2.8 mL) was added thereto and stirred under reflux for 4 hours. The reaction mixture was concentrated, and the residue was purified by a silica gel column chromatography (ethyl acetate/hexane = 2/3 - 1/1) to obtain the desired compound (5.6 g).
¹H-NMR(300MHz, CDCl₃) δ ppm: 1.73(d, 3H, J=7.3Hz), 3.81(s, 3H), 4.31(q, 1H, J=7.3Hz), 7.40-7.72(m, 9H), 7.77-7.92(m, 5H), 9.32(br, 1H), 12.12(br, 1H)

### Reference Example 46

### Methyl N'-{3-[1-(3-benzoylpheyl)ethyl]-1-methyl-1H-1,2,4-triazol-5-yl} imidethiocarbamate

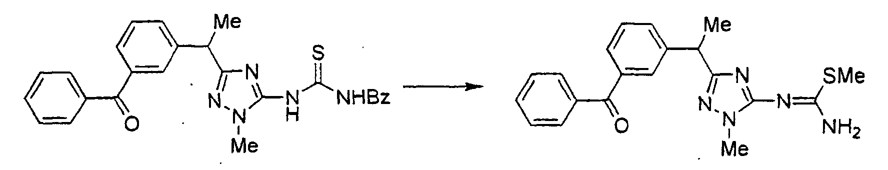

The compound (5.6 g) obtained in Reference Example 45 was dissolved in THF (50 mL) and methanol (50 mL), and potassium carbonate (2.47 g) was added thereto and stirred under reflux for 4 hours. Water was added thereto and the solvent was evaporated. The mixture was extracted with chloroform. The organic layer was dried, concentrated, and dissolved in acetone (70 mL). Potassium carbonate (1.81 g) and methyl iodide (0.82 mL) were added thereto at room temperature and stirred for 2 hours. Acetone was evaporated, and water and chloroform were added to the reaction mixture. The organic layer was separated and the aqueous layer was extracted. The organic layers were dried, and concentrated, and the residue was purified by a silica gel column chromatography (ethyl acetate/hexane = 1/2) to obtain the desired compound (3.4 g).
¹H-NMR(300MHz, CDCl₃) δ ppm: 1.67(d, 3H, J=7.3Hz), 2.49(s, 3H), 3.74(s, 3H), 4.21(q, 1H, J=7.3Hz), 7.35-7.50(m, 3H), 7.53-7.64(m, 3H), 7.76-7.86(m, 3H)

### Reference Example 47

### 3-[1-(3-Benzoylphenyl)ethyl]-5-{[(Z)-[(tert-butoxycarbonyl)amino] (methylsulfanyl)methylidene]amino}-1-methyl-1H-1,2,4-triazole

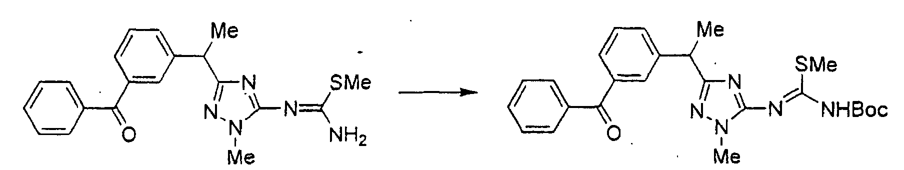

A THF solution (20 mL) of the compound (1.9 g) obtained in Reference Example 46 was added dropwise to a THF suspension (20 mL) of 60% sodium hydride (0.50 g) at 0 °C, and stirred for 5 min. Boc₂O (2.19 g) was added dropwise thereto and stirred for 45 min. The mixture was warmed to room temperature and stirred for further 3 hours. Water and ethyl acetate were added thereto. The organic layer was separated and the aqueous layer was extracted. The organic layers were washed with water, dried, and concentrated, and the residue was purified by a silica gel column chromatography (ethyl acetate/hexane = 1/2) to obtain the desired compound (2.1 g).
¹H-NMR(300MHz, CDCl₃) δ ppm: 1.49(s, 3H), 1.70(d, 3H, J=7.3Hz), 2.43(s, 3H), 3.77(s, 3H), 4.24(q, 1H, J=7.3Hz), 7.38-7.50(m, 3H), 7.54-7.65(m, 3H), 7.76-7.81(m, 3H), 12.35(br, 1H)

### Reference Example 48

### Dimethyl 3-[1-(3-benzoylphenyl)ethyl]-1-methyl-1H-1,2,4-triazol-5-yl dithioimidecarbonate

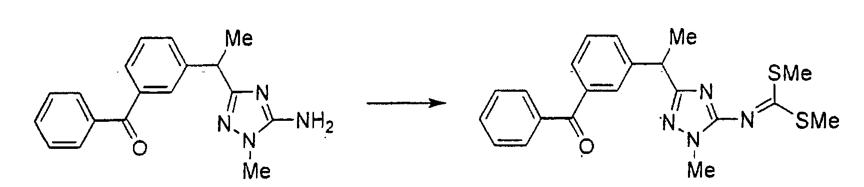

The desired compound was obtained from the compound obtained in Reference Example 44 by the same procedure as described in Reference Example 18.
¹H-NMR(300MHz, CDCl₃) δ ppm: 1.70(d, 3H, J=7.2Hz), 2.55(brs, 6H), 3.75(s, 3H), 4.27(q, 1H, J=7.2Hz), 7.36-7.52(m, 3H), 7.54-7.67(m, 3H), 7.77-7.90(m, 3H)

### Reference Example 49

### Methyl N-{3-[1-(3-benzoylphenyl)ethyl]-1-methyl-1H-1,2,4-triazol-5-yl}-4-morpholinecarbimidothioate

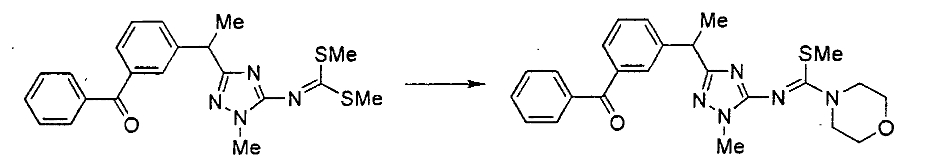

The desired compound was obtained from the compound obtained in Reference Example 48 by the same procedure as described in Reference Example 19.
¹H-NMR(300MHz, CDCl₃) δ ppm: 1.69(d, 3H, J=7.3Hz), 1.97(s, 3H), 3.60(s, 3H), 3.67-3.75(m, 8H), 4.23(q, 1H, J=7.3Hz), 7.35-7.50(m, 3H), 7.54-7.64(m, 3H), 7.76-7.83(m, 3H)

### Reference Example 50

### {3-[1-(5-Amino-1H-1,2,4-triazol-3-yl)ethyl]phenyl}(phenyl)methanone

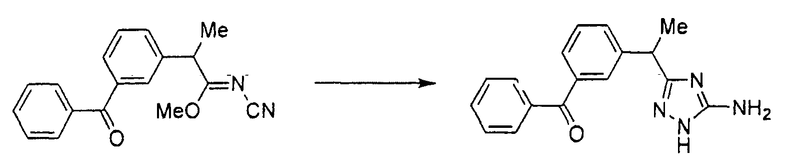

The compound (36 g) obtained in Reference Example 43 was dissolved in ethanol (300 mL), and hydrazine monohydrate (4.8 mL) was added thereto and stirred at 0 °C for 10 min and at room temperature for 20 min. Water was added to the reaction mixture, and ethanol was evaporated, and the mixture was extracted with chloroform. The organic layer was dried and concentrated, and the residue was purified by a silica gel column chromatography (ethyl acetate/hexane = 3/1 - chloroform/methanol = 10/1) to obtain the desired compound (20.9 g).
¹H-NMR(400MHz, d₆-DMSO) δ ppm: 1.58(d, 3H, J=7.0Hz), 4.11(q, 1H, J=7.0Hz), 5.94(br, 2H), 7.51-7.83(m, 10H)

### Reference Example 51

### Methyl N'-{3-[1-(3-benzoylphenyl)ethyl]-1H-1,2,4-triazol-5-yl}imide thiocarbamate

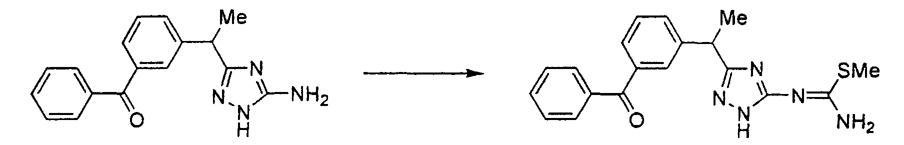

The desired compound was obtained from the compound obtained in Reference Example 50 by the same procedure as described in Reference Example 46 and Reference Example 47.
¹H-NMR(300MHz, CDCl₃) δ ppm: 1.70(d, 3H, J=7.2Hz), 2.45(s, 3H), 4.20(q, 1H, J=7.2Hz), 7.31-7.63(m, 6H), 7.70-7.83(m, 3H)

### Reference Example 52

### Methyl N'-{5-[1-(3-benzoylphenyl)ethyl]-1-methyl-1H-1,2,4-triazol-3-yl} imidethiocarbamate

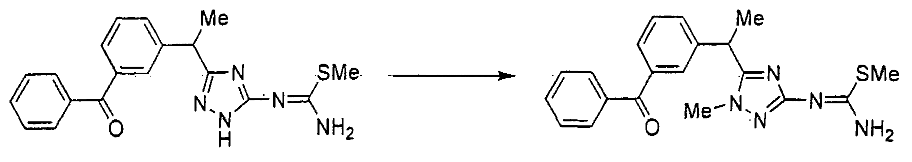

The desired compound was obtained from the compound obtained in Reference Example 51 by the same procedure as described in Reference Example 40.
¹H-NMR(300MHz, CDCl₃) δ ppm: 1.73(d, 3H, J=7.2Hz), 2.52(s, 3H), 3.57(s, 3H), 4.21(q, 1H, J=7.2Hz), 7.34-7.50(m, 4H), 7.54-7.64(m, 2H), 7.72-7.78(m, 3H)

### Reference Example 53

### (3-{1-[5-Amino-1-(phenylsulfony)-1H-1,2,4-triazol-3-yl]ethyl}phenyl) (phenyl)methanone

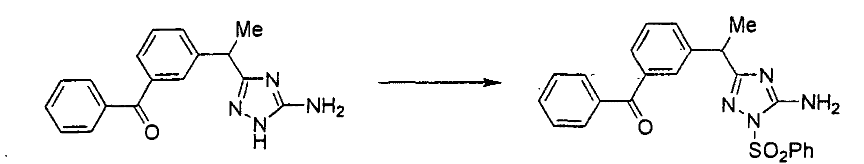

A solution of the compound (5.0 g) obtained in Reference Example 50 in THF (20 mL) and DMF (10 mL) was added dropwise to a THF suspension (30 mL) of 60% sodium hydride (0.75 g) at 0 °C, and stirred for 30 min. Benzenesulfonyl chloride (2.4 mL) was added thereto and stirred for 30 min. Water and ethyl acetate were added thereto. The organic layer was separated and the aqueous layer was extracted. The organic layers were washed with water, dried, and concentrated, and the residue was purified by a silica gel column chromatography (ethyl acetate/hexane = 1/3) to obtain the desired compound (6.6 g).
¹H-NMR(300MHz, CDCl₃) δ ppm: 1.50(d, 3H, J=7.2Hz), 3.98(q, 1H, J=7.2Hz), 5.83(br, 2H), 7.25-7.93(m, 14H)

### Reference Example 54

### Dimethyl 3-[1-(3-benzoylphenyl)ethyl]-1-(phenylsulfonyl)-1H-1,2,4-triazol-5-yldithioimidecarbonate

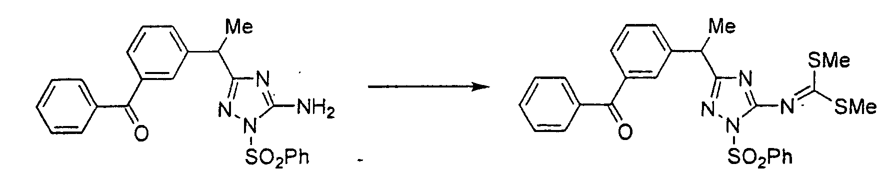

The compound (6.6 g) obtained in Reference Example 53 was dissolved in THF (100 mL), and 60% sodium hydride (0.67 g) was added thereto at 0 °C, and stirred for 30 min. Carbon disulfide (1.85 mL) was added thereto and stirred for 50 min. 60% Sodium hydride (0.67 g) was added thereto and stirred for 1.5 hours. Methyl iodide (3.8 mL) was added dropwise thereto and stirred at 0 °C for 1 hour. Water and ethyl acetate were added thereto and the organic layer was separated and the aqueous layer was extracted. The organic layers were washed with water, dried, and concentrated. The residue was purified by a silica gel column chromatography (ethyl acetate/hexane = 1/2) to obtain the desired compound (6.2 g).
¹H-NMR(300MHz, CDCl₃) δ ppm: 1.64(d, 3H, J=7.3Hz), 2.58(s, 6H), 4.24(q, 1H, J=7.3Hz), 7.31-7.64(m, 9H), 7.73-7.84(m, 3H), 7.96-8.02(m, 2H)

### Reference Example 55

### Methyl N-[3-[1-(3-benzoylphenyl)ethyl]-1-(phenylsulfonyl)-1H-1,2,4-triazol-5-yl]-4-morpholinecarbothioate

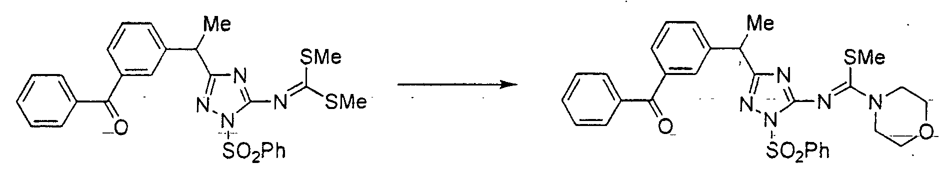

The desired compound was obtained from the compound obtained in Reference Example 54 by the same procedure as described in Reference Example 27.
¹H-NMR(300MHz, CDCl₃) δ ppm: 1.63(d, 3H, J=7.3Hz), 1.97(s, 3H), 3.67(s, 8H), 4.19(q, 1H, J=7.3Hz), 7.31-7.64(m, 9H), 7.73-7.78(m, 3H), 7.95-8.01(m, 2H)

### Reference Example 56

### Methyl N-cyano-4-morpholinecarbimidothioate

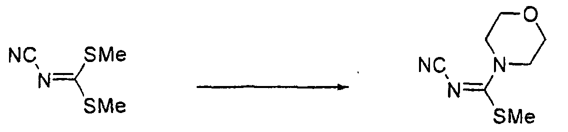

Dimethylcyanodithioiminocarbonate (50 g) was dissolved in ethanol (500 mL), and morpholine (45 mL) was added thereto at room temperature, and stirred for 2 hours. The precipitated crystals were collected and washed with ethanol. The obtained crystals were dried to obtain the desired compound (51 g).
¹H-NMR(300MHz, CDCl₃) δ ppm: 2.80(s, 3H), 3.70-3.75(m, 4H), 3.83-3.88(m, 4H)

### Reference Example 57

### O-Ethyl 2-(2-fluoro[1,1'-biphenyl]-4-yl)propanethioate

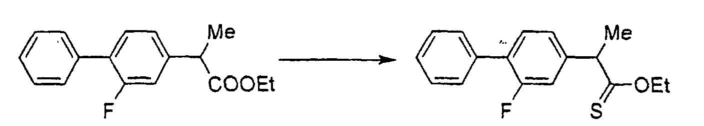

Lawesson's Reagent (17.8 g) was added to a xylene solution of flurbiprofen ethyl ester (20.0 g), and stirred under reflux for 39 hours. The reaction mixture was cooled to room temperature, concentrated and the residue was purified by a silica gel column chromatography to obtain the desired compound (14.2 g).
¹H-NMR(300MHz, CDCl₃) δ ppm: 1.37(t, 3H, J=7.1Hz), 1.62(d, 3H, J=7.1Hz), 4.17(q, 1H, J=7.1Hz), 4.45-4.56(m, 2H), 7.15-7.21(m, 2H), 7.34-7.46(m, 4H), 7.52-7.55(m, 2H)

### Reference Example 58

### 5-[1-(2-Fluoro[1,1'-biphenyl]-4-yl)ethyl]-1,2,4-thiadiazol-3-amine

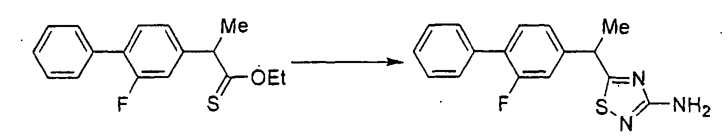

55% Sodium hydride (1.94 g) was divided to several portions, which were added succesively to a THF suspension (20.0 mL) of acetylguanidine (4.10 g) under ice-cooling, and stirred for 30 min. The reaction mixture was warmed to room temperature, and the compound (14.0 g) obtained in Rreference Example 57 was added thereto. After the starting material disappeared, hexane (200 mL) was added thereto and the supernatant was removed. Ethanol (50 mL) and acetic acid (2.6 mL) were added to the residue, and bromine (2.71 mL) dissolved in chloroform (20 mL) was added dropwise thereto under ice-cooling. The reaction mixture was warmed to room temperature and stirred for 30 min. The mixture was neutralized with a saturated sodium bicarbonate solution, and extracted with ethyl acetate. The organic layer was washed with a saturated brine, dried, and concentrated under reduced pressure, and the residue was purified by a silica gel column chromatography to obtain the desired compound (2.65 g).
¹H-NMR(300MHz, CDCl₃) δ ppm: 1.68(d, 3H, J=7.1Hz), 4.62(q, 1H, J=7.1Hz), 6.69(s, 2H), 7.29-7.56(m, 8H)

### Reference Example 59

### O-Methyl 2-(3-benzoylphenyl)propanethiate

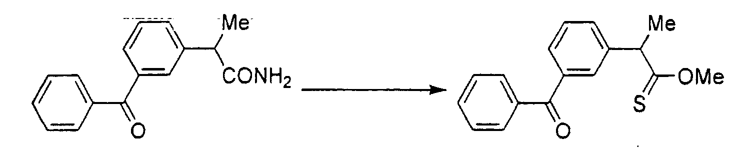

Dimethyl sulfate (29.7 g) was added to the compound (59.8 g) obtained in Reference Example 28 and stirred at 100 °C for 1 hour. After cooled to room temperature, the mixture was diluted with 1,4-dioxane (200 mL). Pyridine (70 mL) was added thereto under ice-cooling, and hydrogen sulfide gas was blown thereinto for 1.5 hours. The reaction mixture was poured to a saturated brine, and extracted with ethyl acetate. The organic layer was washed with 1N hydrochloric acid, a saturated sodium bicarbonate solution and a saturated brine, and dried, concentrated under reduced pressure and the residue was purified by a silica gel chromatography to obtain the desired compound (19.8 g).
¹H-NMR(300MHz, CDCl₃) δ ppm: 1.64(d, 3H, J=7.1Hz), 4.05(s, 3H), 4.23(q, 1H, J=7.1Hz), 7.40-7.69(m, 6H), 7.79-7.82(m, 3H)

### Reference Example 60

### {3-[1-(3-Amino-1,2,4-thiadiazol-5-yl)ethyl]phenyl}(phenyl}methanone

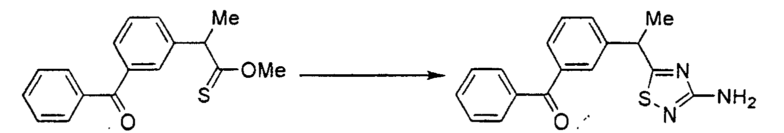

The desired compound was obtained from the compound obtained in Reference Example 59 by the same procedure as described in Reference Example 58.
¹H-NMR(300MHz, CDCl₃) δppm: 1.78(d, 3H, J=7.1Hz), 4.46(q, 1H, J=7.1Hz), 4.96(s, 2H), 7.45-7.51(m, 3H), 7.55-7.63(m, 2H), 7.72(ddd, 1H, J=7.5, 1.5, 1.5Hz), 7.78-7.81(m, 3H)

### Reference Example 61

### N-{5-[1-(3-Benzoylphenyl)ethyl]-1,2,4-thiadiazol-3-yl}-4-morpholine carbothioamide

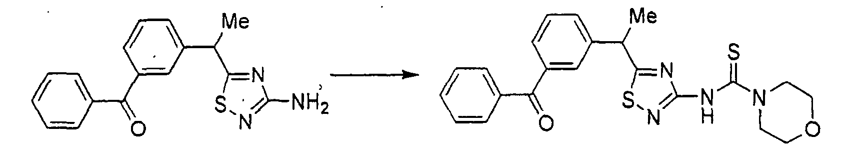

A mixture of the compound (1.57 g, 5 mmol) obtained in Reference Example 60, chloroform (100 mL) and sodium bicarbonate (2.31 g, 27.5 mmol) was stirred under ice-cooling. Thiophosgen (0.95 mL, 12.5 mmol) was added dropwise thereto, and stirred under ice-cooling for 5 hours. The organic layer was separated, washed with a saturated brine, dried over sodium sulfate, and the solvent was evaporated. The residue was dissolved in acetonitrile (10 mL), and morpholine (1.7 mL) was added thereto and stirred at room temperature for 30 min. Water and ethyl acetate were added. The organic layer was separated, washed with a saturated brine, dried over sodium sulfate, and the solvent was evaporated. The residue was purified by a silica gel chromatography to obtain the desired compound (1.415 g, yellow oil).
¹H-NMR(300MHz, CDCl₃) δppm: 8.18(s,1H), 7.71-7.81(m,4H), 7.46-7.64(5H,m), 4.52(q,J=7.1Hz,1H), 3.83-3.91(m,4H), 3.72-3.81(m,4H), 1.80(d,J=7.1Hz,3H)

### Reference Example 62

### Methyl N-{5-[1-(3-benzoylphenyl)ethyl]-1,2,4-thiadiazol-3-yl}-4-morpholinecarbimidothioate

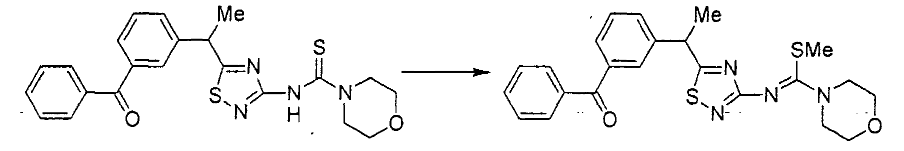

The desired compound was obtained from the compound obtained in Reference Example 61 by the same procedure as described in Reference Example 39.
¹H-NMR(300MHz, CDCl₃) δppm: 7.76-7.82(m,3H), 7.67-7.73(m,1H) 7.55-7.63(m,2H), 7.42-7.51(m,3H), 4.55(q,J=7.1Hz,1H), 3.77-3.81(m,4H), 3.70-3.75(m,4H), 1.99(s,3H), 1.83(d,J=7.1Hz,3H)

### Reference Example 63

### 2-(2-Fluoro[1,1'-biphenyl]-4-yl)-2-methylpropanoic acid

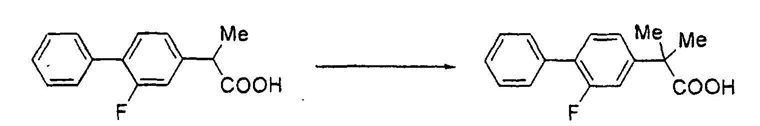

A DMF solution (300 mL) of flurbiprofen (30 g) was added dropwise to a DMF suspension (300 mL) of 60% sodium hydride (11.3 g) at 0 °C, which taking 30 min. The mixture was warmed to room temperatue, and stirred for 1 hour. Methyl iodide (18.4 mL) was added thereto and stirred overnight. The reaction mixture was poured into ice-water, extracted with ethyl acetate. The organic layer was washed with water, dried, concentrated under reduced pressure. The residue was dissolved in ethanol (300 mL) and 6N aqueous potassium hydroxide solution was added thereto. The reaction mixture was stirred at 50 °C for 30 min. Water was added thereto and ethanol was evaporated under reduced pressure. The obtained aqueous solution was washed with ethyl acetate/hexane (1/1 (v/v)). The aqueous layer was acidified with 1N hydrochloric acid, and extracted with ethyl acetate. The organic layers were washed with water, dried, and concentrated under reduced pressure to obtain the desired compound (24 g).
¹H-NMR(300MHz, CDCl₃) δ ppm: 1.63(s, 6H), 7.19-7.56(m, 8H)

### Reference Example 64

### 2-(3-Benzoylphenyl)-2-methylpropanoic acid

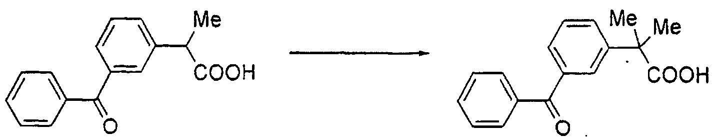

The desired compound was obtained from ketoprofen by the same procedure as described in Reference Example 63.
¹H-NMR(300MHz, CDCl₃) δ ppm: 1.68(s, 6H), 7.40-7.72(m, 6H), 7.77-7.89(m, 3H)

### Reference Example 65

### 2-(3-Benzoylphenyl)-2-methylpropanohydrazide

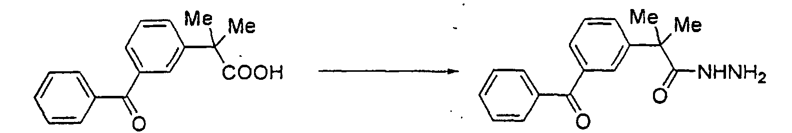

The desired compound was obtained from the compound obtained in Reference Example 64 by the same procedure as described in Reference Example 15.
¹H-NMR(400MHz, CDCl₃) δ ppm: 1.63 (s, 6H), 3.83 (brs, 2H), 6.51 (brs, 1H), 7.33-7.85 (m, 9H)

### Reference Example 66

### {3-[1-(5-Amino-1H-1,2,4-triazol-3-yl)-1-methylethyl]phenyl}(phenyl) methanone

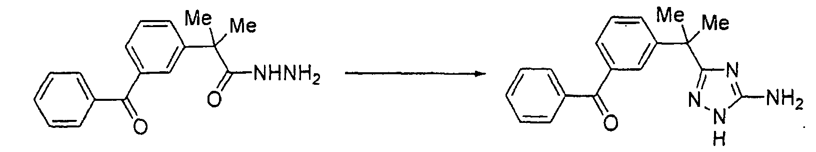

Water (20 mL) and methylthiourea hemisulfate (1.07 g) were added to a DMF solution (20 mL) of the compound (2.95 g) obtained in Reference Example 65 at room temperature and stirrd under reflux for 48 hours. The reaction mixture was poured to an aqueous saturated sodium bicarbonate solution (100 mL), and extracted three times with ethyl acetate (100 mL). The organic layers were washed with a saturated brine, dried over sodium sulfate and concentrated under reduced pressure. The resulting residue was purified by a silica gel column chromatography (chloroform/methanol = 20/1) to obtain the desired compound (860 mg).
¹H-NMR(400MHz, CDCl₃) δ ppm: 1.77 (s, 6H), 4.27 (brs, 2H), 7.41-7.83 (m, 9H)

### Reference Example 67

### (3-{1-[5-Amino-1-(phenylsulfonyl)-1H-1,2,4-triazol-3-yl]-1-methylethyl}. phenyl)(phenyl)methanone

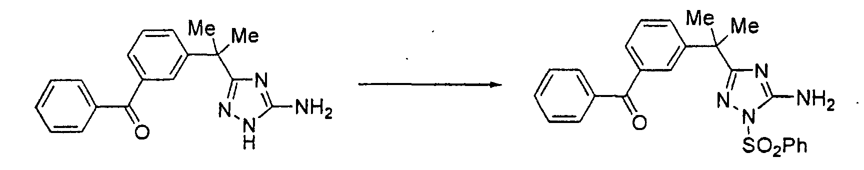

The desired compound was obtained from the compound obtained in Reference Example 66 by the same procedure as described in Reference Example 53.
¹H-NMR(400MHz, CDCl₃) δ ppm: 1.62 (s, 6H), 6.05 (brs, 2H), 7.32-7.95 (m, 14H)

### Reference Example 68

### Dimethyl 3-[1-(3-benzoylphenyl)-1-methylethyl]-1-(phenylsulfonyl)-1H-1,2,4-triazol-5-yldithioimidecarbonate

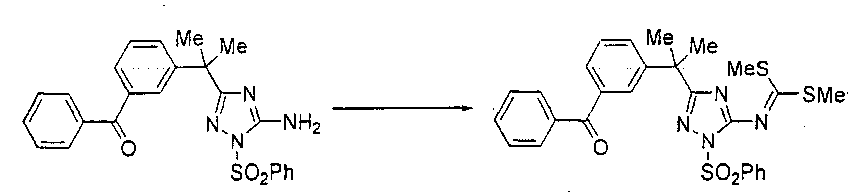

The desired compound was obtained from the compound obtained in Reference Example 67 by the same procedure as described in Reference Example 26.
¹H-NMR(400MHz, CDCl₃) δ ppm: 1.76 (s, 6H), 2.58 (s, 6H), 7.31-8.01 (m, 14H)

### Reference Example 69

### Methyl N-[3-[1-(3-benzoylphenyl)-1-methylethyl]-1-(phenylsulfonyl)-1H-1,2,4-triazol-5-yl]-4-morpholinecarbimidothiate

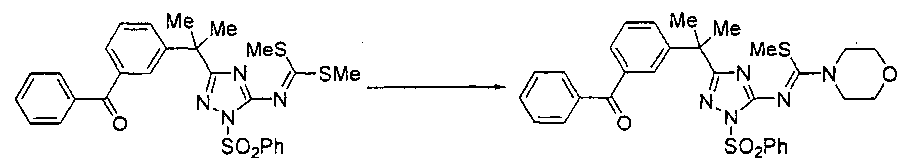

The desired compound was obtained from the compound obtained in Reference Example 68 by the same procedure as described in Reference Example 27.
¹H-NMR(400MHz, CDCl₃) δ ppm: 1.73 (s, 6H), 2.00 (s, 3H), 3.69 (s, 8H), 7.30-8.01 (m, 14H)

### Reference Example 70

### (3-{[5-Amino-1H-1,2,4-triazol-3-yl]methyl}phenyl)(phenyl)methanone

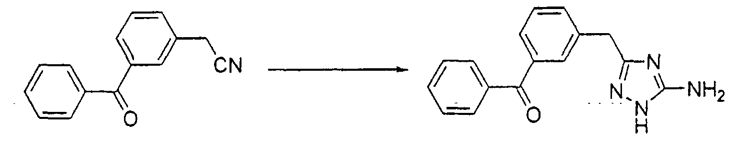

The desired compound was obtained from 3-benzoylbenzylcyanide by the same procedure as described in Reference Example 42, Reference Example 43 and Reference Example 50.

### Reference Example 71

### (3-{[5-Amino-1-(phenylsulfonyl)-1H-1,2,4-triazol-3-yl]methyl}phenyl) (phenyl)methanone

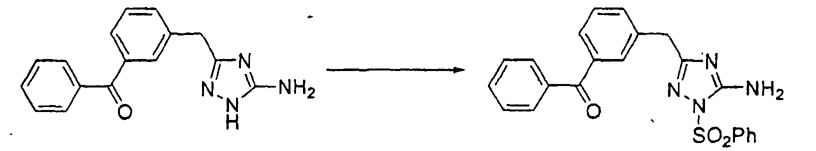

The desired compound was obtained from the compound obtained in Reference Example 70 by the same procedure as described in Reference Example 53.
¹H-NMR(400MHz, CDCl₃) δ ppm: 3.87(s, 2H), 5.97(brs, 2H), 7.35-7.69(m, 8H), 7.75-7.80(m, 2H), 7.96-8.01(m, 2H)

### Reference Example 72

### Dimethyl 3-(3-benzoylbenzyl)-1-(phenylsulfonyl)-1H-1,2,4-triazol-5-yl dithioimidecarbonate

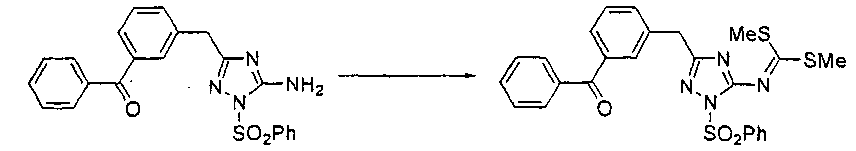

The desired compound was obtained from the compound obtained in Reference Example 71 by the same procedure as described in Reference Example 27.

### Reference Example 73

### 2-[1,1'-Biphenyl]-4-yl-2-methylpropionic acid

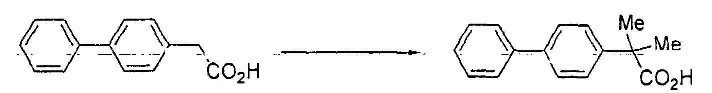

The desired compound was obtained from biphenylacetic acid by the same procedure as described in Reference Example 63.
¹H-NMR(300MHz, CDCl₃) δ ppm: 1.55(s, 6H), 7.24-7.57(m, 9H)

### Reference Example 74

### 2-[1,1'-Biphenyl]-4-yl-N-cyano-2-methylpropanamide

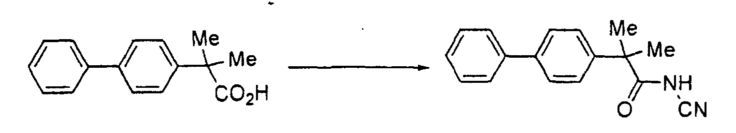

The compound (2.40 g) obtained in Reference Example 73 was suspended in toluene (50 mL), and 5 drops of DMF and thionyl chloride (1.6 mL) were added thereto and stirred at room temperture for 4 hours. Excess thionyl chloride and the solvent were removed by an evaporater and the resulting mixture was dissolved in acetone (25 mL). To this mixture was addeddropwise a solution of cyanamide monohydrate (631 mg) in 2M aqueous sodium hydroxide solution (8 mL), and stirred at room temperature for 3 hours. The mixture was diluted in water, extracted with ethyl acetate and dried over magnesium sulfate. The mixture was concentrated by an evaporater to obtain the desired compound quantitatively.
¹H-NMR(300MHz, CDCl₃) δ ppm: 1.65(s, 6H), 7.34-7.48(m, 5H), 7.56-7.64(m, 4H)

### Reference Example 75

### 3-(1-[1,1'-Biphenyl]-4-yl-1-methylethyl)-1H-1,2,4-triazole-5-amine

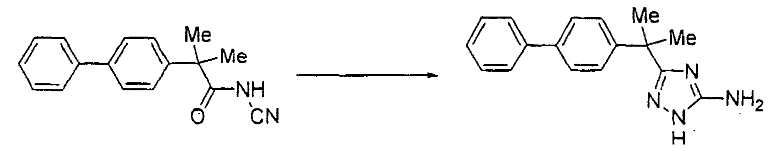

The desired compound was obtained from the compound obtained in Reference Example 74 by the same procedure as described in Reference Example 53.
¹H-NMR(400MHz, CDCl₃) δ ppm: 1.71(s, 6H), 4.45(br s, 2H), 7.32-7.43(m, 5H), 7.50-7.55(m, 4H)

### Reference Example 76

### Dimethyl 3-(1-[1,1'-biphenyl]-4-yl-1-methylethyl)-1-(phenylsulfonyl)-1H-1,2,4-triazol-5-yldithioimidecarbonate

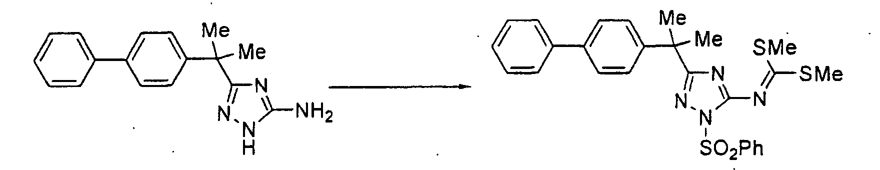

The desired compound was obtained from the compound obtained in Reference Example 75 by the same procedure as described in Reference Example 53 and Reference Example 26.
¹H-NMR(300MHz, CDCl₃) δ ppm: 1.76(s, 6H), 2.59(s, 6H), 7.27-7.34(m, 3H), 7.38-7.46(m, 4H), 7.51-7.57(m, 4H), 7.63-7.68(m, 1H), 8.02-8.06(m, 2H)

### Reference Example 77

### Methyl N-[3-(1-[1,1'-biphenyl]-4-yl-1-methylethyl)-1-(phenylsulfonyl)-1H-1,2,4-triazol-5-yl]-4-morpholinecarbimidothioate

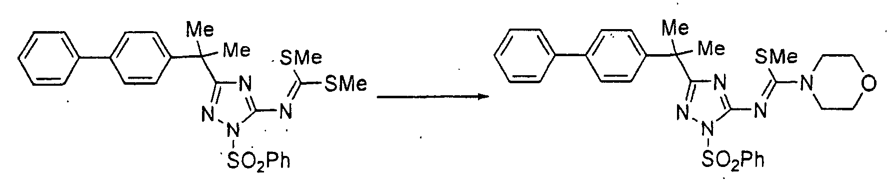

The desired compound was obtained from the compound obtained in Reference Example 76 by the same procedure as described in Reference Example 27.
¹H-NMR(300MHz, CDCl₃) δ ppm: 1.74(s, 6H), 2.01(s, 3H), 3.68(s, 8H), 7.29-7.34(m, 3H), 7.40-7.45(m, 4H), 7.51-7.56(m, 4H), 7.63-7.69(m, 1H), 8.01-8.04(m, 2H)

### Reference Example 78

### Methyl 2-[1,1'-biphenyl]-4-ylethanimidoate hydrochloride

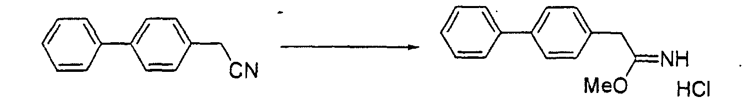

The desired compound was obtained from 4-biphenylacetonitrile by the same procedure as described in Reference Example 42.
¹H-NMR(300MHz, d₆-DMSO) δ ppm: 4.09(s, 5H), 7.35-7.40 (m,1H), 7.44-7.49(m,4H), 7.62-7.69(m,4H)

### Reference Example 79

### Methyl 2-[1,1'-biphenyl]-4-yl-N-cyanoethanimidoate

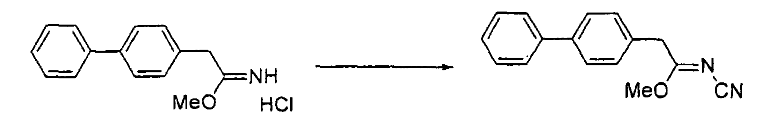

The desired compound was obtained from the compound obtained in Reference Example 78 by the same procedure as described in Reference Example 43.
¹H-NMR(300MHz, CDCl₃) δ ppm: 3.88 (s, 3H), 4.02 (s, 2H), 7.32-7.47 (m, 5 H), 7.54-7.59 (m, 4H)

### Reference Example 80

### 3'-([1,1'-Biphenyl]-4-ylmethyl)-1H-1,2,4-triazol-5-amine

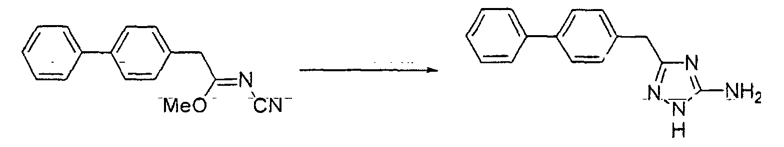

The desired compound was obtained from the compound obtained in Reference Example 79 by the same procedure as described in Reference Example 50.
¹H-NMR(300MHz, d₆-DMSO) δ ppm: 3.83 (s, 2H), 5.64 (br.s, 2H) 7.28-7.37(m, 3H), 7.38-7.45(m, 2H), 7.48-7.54(m, 2H), 7.54-7.59 (m, 2H), 11.57(br.s, 1H)

### Reference Example 81

### 3-([1,1'-Biphenyl]-4-ylmethyl)-1-(phenylsulfonyl)-1H-1,2,4-triazol-5-amine

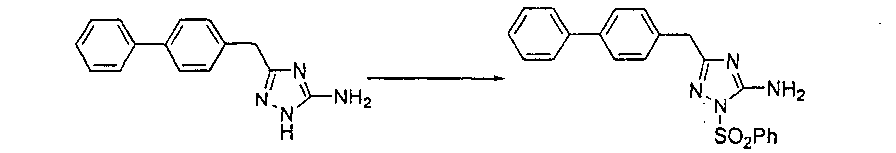

The desired compound was obtained from the compound obtained in Reference Example 80 by the same procedure as described in Reference Example 53.
¹H-NMR(300MHz, CDCl₃) δ ppm: 3.83 (s, 2 H), 6.04 (br.s, 2 H), 7.20-7.25 (m, 2 H), 7.28-7.35 (m, 1 H), 7.38-7.45 (m, 2 H), 7.47-7.59 (m, 6 H), 7.65-7.72 (m, 1 H), 7.94-7.98 (m, 2 H)

### Reference Example 82

### Dimethyl 3-(1-[1,1'-biphenyl]-4-ylmethyl)-1-(phenylsulfonyl)-1H-1,2,4-triazol-5-yldithioimidecarbonate

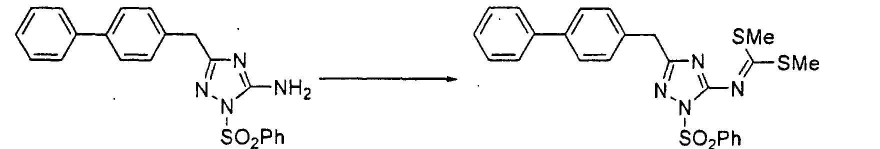

The desired compound was obtained from the compound obtained in Reference Example 81 by the same procedure as described in Reference Example 26.
¹H-NMR(300MHz, CDCl₃) δ ppm: 2.58 (s, 6 H), 4.04 (s, 2 H), 7.25-7.33 (m, 1 H), 7.33-7.42 (m, 4H), 7.45-7.56 (m, 6H), 7.57-7.64 (m, 1 H), 8.00-8.05 (m, 2 H)

### Reference Example 83

### Methyl N-[3-([1,1'-biphenyl]-4-ylmethyl)-1-(phenylsulfonyl)-1H-1,2,4-triazol-5-yl]-4-morpholinecarbimidothioate

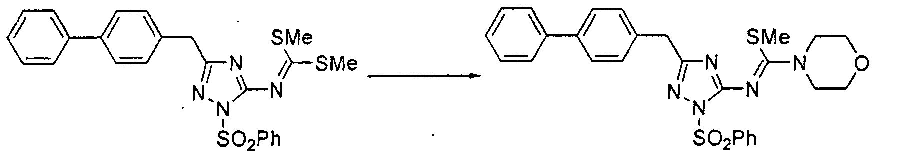

The desired compound was obtained from the compound obtained in Reference Example 82 by the same procedure as described in Reference Example 27.
¹H-NMR(300MHz, CDCl₃) δ ppm: 2.03 (s, 3 H), 3.69 (s, 8 H), 3.97 (s, 2 H), 7.30-7.37 (m, 3 H), 7.39-7.44 (m, 2 H), 7.47-7.52 (m, 2 H), 7.52-7.57 (m, 4 H), 7.63-7.68 (m, 1 H), 8.03-8.07 (m, 2 H)

### Reference Example 84

### 2-[1,1'-Biphenyl]-4-ylpropionitrile

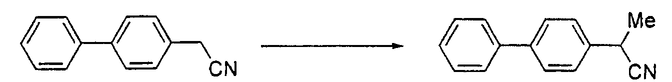

A chloroform solution of biphenylacetonitrile (19.3 g) and methyl iodide (6.2 mL) was added dropwise under stirring to a mixture of 40% aqueous tetrabutylammmonium hydroxide solution (64.8 g) and water (45 mL). The reaction was monitored by HPLC. A chloroform solution (10 mL) of methyl iodide (6.2 mL) was added dropwise thereto and stirred for 14 hours. The organic layer was separated and concentrated. The residue was purifed with a silica gel column chromatography to obtain the desired compound (13 g).
¹H-NMR(300MHz, CDCl₃) δ ppm: 1.69 (d, 3 H, J = 7.2 Hz), 3.95 (q, 1 H, J = 7.2 Hz), 7.33-7.39 (m, 1 H), 7.40-7.47 (m, 4H), 7.55-7.63 (m, 4H)

### Reference Example 85

### Methyl 2-[1,1'-biphenyl]-4-ylpropammidoate hydrochloride

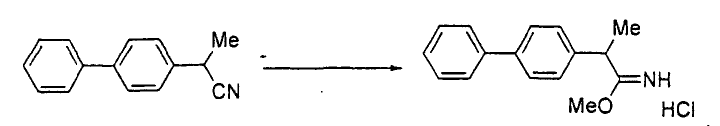

The desired compound was obtained from the compound obtained in Reference Example 84 by the same procedure as described in Reference Example 42.
¹H-NMR(300MHz, CDCl₃) δ ppm: 1.69 (m, d, 3 H, J = 7.2 Hz), 4.30 (s, 3 H), 4.59 (q, 1 H, J = 7.2 Hz), 7.32-7.39 (m, 1 H), 7.40-7.47 (m, 2 H), 7.53-7.62 (m, 6 H), 11.75 (br.s, 1 H), 12.80 (br.s, 1 H)

### Reference Example 86

### Methyl 2-[1,1'-biphenyl]-4-yl-N-cyanopropanimidoate

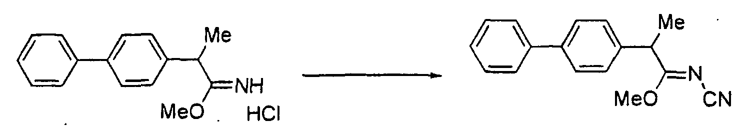

The desired compound was obtained from the compound obtained in Reference Example 85 by the same procedure as described in Reference Example 43.
¹H-NMR(300MHz, CDCl₃) δ ppm: 1.62 (d, 3 H, J = 7.1 Hz), 3.90 (s, 3 H), 4.49 (q, 1 H, J = 7.1 Hz), 7.32-7.38 (m, 1 H), 7.40-7.47 (m, 4 H), 7.53-7.60 (m, 4 H)

### Reference Example 87

### 3-(1-[1,1'-Biphenyl]-4-ylethyl)-1H-1,2,4-triazol-5-amine

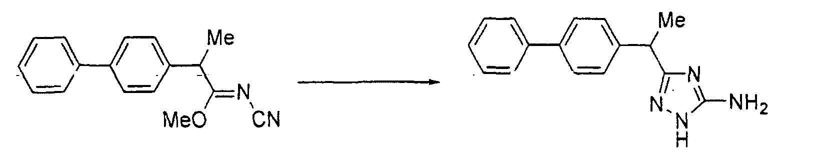

The desired compound was obtained from the compound obtained in Reference Example 86 by the same procedure as described in Reference Example 50.
¹H-NMR(300MHz, d₆-DMSO) δ ppm: 1.64 (d, 3 H, J = 7.1 Hz), 4.11 (q, 1 H, J = 7.1 Hz), 7.28-7.35 (m, 1 H), 7.36-7.45 (m, 4 H), 7.48-7.58 (m, 4 H)

### Reference Example 88

### 3-(1-[1,1'-Biphenyl]-4-ylethyl)-1-(phenylsulfonyl)-1H-1,2,4-triazol -5-amine

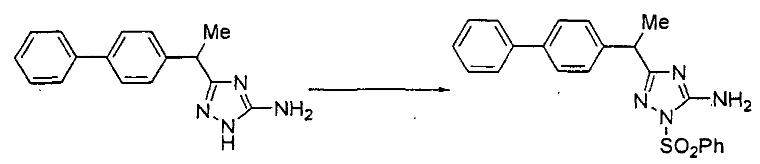

The desired compound was obtained from the compound obtained in Reference Example 85 by the same procedure as described in Reference Example 53.
¹H-NMR(300MHz, CDCl₃) δ ppm: 1.57 (d, 3 H, J = 7.1 Hz), 4.00 (q, 1 H, J = 7.1 Hz), 6.17 (br.s, 2 H), 7.18-7.25 (m, 2 H), 7.28-7.35 (m, 1 H), 7.38-7.45 (m, 2 H), 7.45-7.60 (m, 6 H), 6.62-6.69 (m, 1 H), 7.89-7.96 (m, 2 H)

### Reference Example 89

### Dimethyl 3-(1-[1,1'-biphenyl]-4-ylethyl)-1-(phenylsulfonyl)-1H-1,2,4-triazol-5-yldithioimidecarbonate

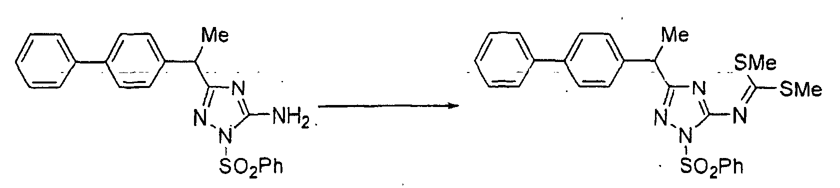

The desired compound was obtained from the compound obtained in Reference Example 88 by the same procedure as described in Reference Example 26.
¹H-NMR(400MHz, CDCl₃) δ ppm: 1.67 (d, 3 H, J = 7.2 Hz), 2 62 (s, 6 H), 4.24 (q, 1 H, J = 7.2 Hz), 7.28-7.34 (m, 1 H), 7.38-7.43 (m, 4 H), 7.46-7.50 (m, 2 H), 7.50-7.57 (m, 4 H), 7.62-7.67 (m, 1 H), 8.01-8.05 (m, 2 H)

### Reference Example 90

### Methyl N-[3-(1-[1,1'-biphenyl]-4-ylethyl)-1-(phenylsulfonyl)-1H-1,2,4-triazol-5-yl]-4-morpholinecarbimidothioate

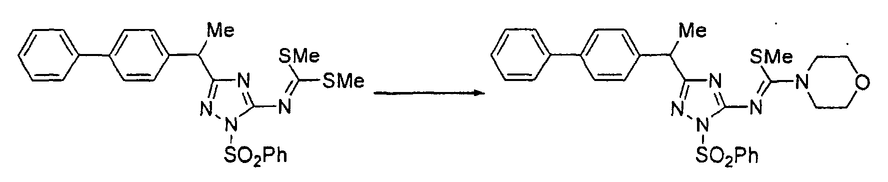

The desired compound was obtained from the compound obtained in Reference Example 89 by the same procedure as described in Reference Example 27.
¹H-NMR(400MHz, CDCl₃) δ ppm: 1.67 (d, 3 H, J = 7.2 Hz), 1.99 (s, 3 H), 3.69 (s, 8 H), 4 18 (q, 1 H, J = 7.2 Hz), 7.29-7.34 (m, 1 H), 7.36-7.44 (m, 4 H), 7.46-7.51 (m, 2 H), 7.51-7.56 (m, 4 H), 7.62-7.67 (m, 1 H), 8.01-8.05 (m, 2 H)

### Reference Example 91

### [4-(Bromomethyl)phenyl](phenyl)methanone

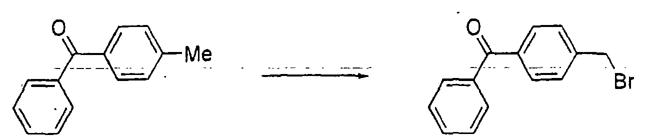

4-Methylbenzophenone (25 g) was dissolved in carbon tetrachloride (250 mL), and AIBN (0.2 g) and NBS (22.7 g) were added thereto. The mixture was stirred under reflux for 2 hours. The precipitated succinimide was filtrated off and the filtrate was concentrated. The desired compound (19.6 g) was recrystallized from the filtrate.
¹H-NMR(300MHz, CDCl₃) δ ppm: 1.58(s, 3H), 4.54(s, 2H), 7.46-7.54(m, 4H), 7.60(m, 1H), 7.76-7.83(m, 4H)

### Reference Example 92

### (4-Benzoylphenyl)acetonitrile

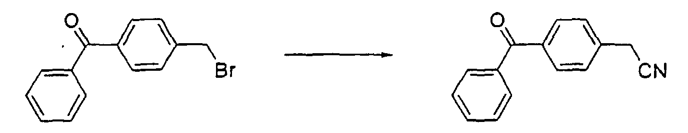

The compound (16.3 g) obtained in Reference Example 91 was dissolved in DMF (200 mL), and sodium cyanide (3.2 g) was added thereto and stirred at room temperature for 1 hour. The reaction mixture was poured in water, and extracted with ethyl acetate. The organic layer was washed with water, dried, and concentrated. The obtained residue was puried by a silica gel column chromatography to obtain the desired compound (6.1 g).
¹H-NMR(300MHz, CDCl₃) δ ppm: 1.57(s, 3H), 3.86(s, 2H), 7.45-7.54(m, 4H), 7.61(m, 1H), 7.77-7.86(m, 4H)

### Reference Example 93

### Methyl (4-benzoylphenyl)acetate

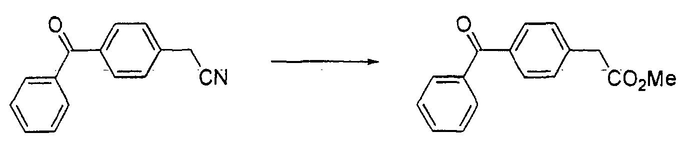

The compound (7.2 g) obtained in Reference Example 92 was dissolved in sulfuric acid (30 mL), acetic acid (30 mL) and water (30 mL), and stirred at 80 °C for 5 hours. The reaction mixture was poured in ice-water, stirred for a while and extracted with ethyl acetate. The organic layer was washed with water, dried, and concentrated to give a carboxyl acid. The carboxyl acid was dissolved in toluene (100 mL), and thionyl chloride (2.4 mL) and 2 drops of DMF were added thereto and stirred under reflux for 1 hour. Methanol (15 mL) was added thereto and stirred for 1 hour. The reaction mixture was concentrated and the residue was puried by a silica gel column chromatography to obtain the desired compound (8.5 g).
¹H-NMR(300MHz, CDCl₃) δ ppm: 3.72(s, 2H), 3.73(s, 3H), 7.38-7.53(m, 4H), 7.59(m, 1H), 7.76-7.82(m, 4H)

### Reference Example 94

### Methyl 2-(p-benzoylphenyl)-2-methylpropionate

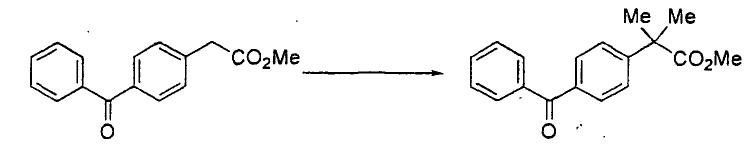

The compound (8.11 g, 31.9 mmol) obtained in Reference Example 93 was dissolved in DMF (80 mL), and methyl iodide (5.6 mL, 90 mmol) was added thereto. The reaction mixture was cooled with an ice-cooled bath, and sodium hydride (60% in oil, 3.0 g, 75 mmol) was added portionwise thereto, which taking 30 min. The mixture was stirred under ice-cooling for 1 hour and at room temperature for 5 hours. Execessive amount of 1N hydrochloric acid was added to stop the reaction. The mixture was extracted with toluene, followed by conventional after-treatments. The desired compound was obtained quantitatively.
¹H-NMR(300MHz, CDCl₃) δ ppm: 1.63(s, 3H+3H), 3.69(s, 3H), 7.42-7.82(m, 9H)

### Reference Example 95

### 2-(p-Benzoylphenyl)-2-methylpropionic acid

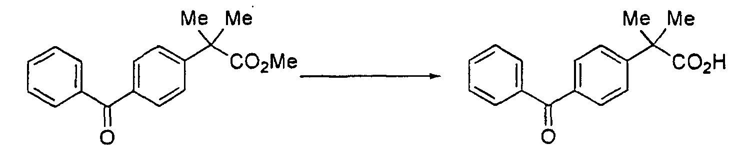

All of the compound obtained in Reference Example 94 was dissolved in ethanol (80 mL), and 10N sodium hydroxide solution (20 mL) was added thereto and stirred at 70 °C for 2.5 hours. The mixture was cooled to room temperature, and the reaction was quenched with acetic acid (10 mL). The mixture was extracted with ethyl acetate. The crude compound was puried by a silica gel column chromatography (choroform/methanol = 20/1) to obtain the desired compound (4.13 g, 95%, light orange crystals).
¹H-NMR(300MHz, CDCl₃) δ ppm: 1.66(s, 6H), 7.45-7.62(m, 5H), 7.78-7.82(m, 4H)

### Reference Example 96

### {4-[1-(5-Amino-1H-1,2,4-triazol-3-yl)-1-methylethyl]phenyl}(phenyl) methanone

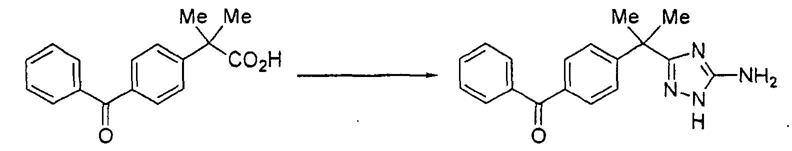

The desired compound was obtained from the compound obtained in Reference Example 95 by the same procedure as described in Reference Example 15 and Reference Example 66.
¹H-NMR (400MHz, CDCl₃) δ ppm: 1.76 (s, 6H), 4.36(brs, 2H), 7.26-7.50(m, 4H), 7.54-7.60(m, 1H), 7.73-7.81(m, 4H)

### Test Example 1

### Inhibition of adjuvant-induced arthritis

Male SD rats were used as test subjects. Heat-kllled Mycobacterium butyricum suspended in liquid paraffin in a concentration of 0.5% was subcutaneously injected into the right hind paw of each rat. After 17 days, animals showing the clear onset of secondary inflammation also in the left hind paw were selected, and each compound of the present invention suspended in a 0.5% methyl cellulose solution was orally administered to the animals for 5 consecutive days. The volume of each hind paw at the completion of the administration was compared with that at the beginning of the administration, and the swelling-inhibitory effect was evaluated by the difference between them. The results are shown in Table 30.

**Table 30**

| No. | Compound administered | Oral dose (mg/kg) | Number of animals | Increase of edema volume (ml) | |
|---|---|---|---|---|---|
| | | | | Injected paw | Non-injected paw |
| 1 | Control | - | 10 | 0.23 | 0.06 |
| | Compound of Experiment 49 | 50 | 10 | -0.24 | -0.17 |
| | Indomethacin | 0.5 | 10 | -0.70 | -0.57 |
| 2 | Control | - | 8 | 0.42 | 0.67 |
| | Compound of 50 Experiment 1 | | 8 | 0.12 | 0.02 |
| | Compound of Experiment 73 | 50 | 8 | -0.95 | -0.78 |
| | Indomethacin | 0.5 | 8 | -0.88 | -0.59 |
| 3 | Control | - | 8 | 0.21 | 0.36 |
| | Compound of Experiment 75 | 50 | 8 | -1.08 | -0.53 |
| | Indomethacin | 0.5 | 8 | -1.34 | -0.57 |
| 4 | Control | - | 8 | 0.69 | 0.22 |
| | Compound of Experiment 320 | 25 | 8 | -1.20 | -0.53 |
| | Indomethacin | 0.5 | 8 | -0.45 | -0.39 |

### Industrial Applicability

The compounds of claim 1 of the present invention have excellent curative effects to immuno-imbalance and choronic inflammation, and are useful as drugs for the treatment or prophylaxis of autoimmune diseases or inflammatory diseases, etc. The triazole compounds of claim 11 are not only useful for synthetic intermediates for the compounds of claim 1, but also themselves have curative effects to immuno-imbalance and anti-inflammatory effect, and are useful as drugs for the treatment or prophylaxis of autoimmune diseases or inflammatory diseases, etc. Especially, these compounds have good water-solubility, and thus have excellent bio-availability.

## Claims

1. A heteroaromatic ring compound represented by the following formula, or a pharmaceutically acceptable salt thereof: 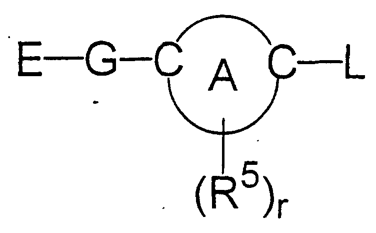 wherein E is a group of the formula: 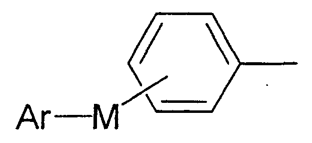 wherein Ar is benzene, furan, thiophene or pyridine; and M is single bond, -O-, -S-, -SO-, -SO₂-, -CQ-, -CH(OR¹¹)-, -C(OR¹¹)₂-, -C(=NOR¹¹)-, -C(=NR¹²)-, -C(=NNR¹³R¹⁴)-, -CO- or -CS-,
wherein -CQ- is 1,3-dioxane ring or 1,3-dioxolane ring; R¹¹ is hydrogen atom or a lower alkyl group; R¹² is hydrogen atom or a lower alkyl group; R¹³ and R¹⁴ are independently hydrogen atom, a lower alkyl group, or a substituted or unsubstituted aryl group;
or E is a group of the formula: 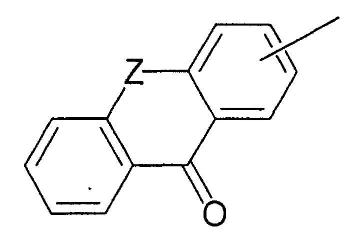 wherein Z is single bond, -O-, -S-, -SO- or -SO₂-;
or E is a group of the formula: 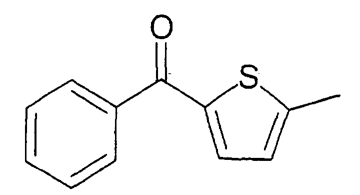 wherein said E may be substituted by one to four members optionally selected from the group consisting of halogen atoms, lower alkyl groups, nitro group, formyl group, acetyl group, cyano group, -OR¹¹, -CO₂R²⁹ and -CONR³⁰R³¹,
wherein R¹¹ is as defined above; R²⁹ is a lower alkyl group; R³⁰ and R³¹ are independently hydrogen atom or a lower alkyl group;
G is -C(R⁶R⁷)- or -C(=CR⁶R⁷)- and is connected with the carbon atom of the ring A,
wherein R⁶ and R⁷ are independently hydrogen atom, a lower alkyl group or a lower alkoxy group; or R⁶ and R⁷ may be taken together with the carbon atom attached thereto to form a substituted or unsubstituted hydrocarbon ring, a substituted or unsubstituted 1,3-dioxane ring, or a substituted or unsubstituted 1,3-dioxolane ring;
A is pyrrole, furan, thiophene, isothiazole, 1,3,4-oxadiazole, 1,3,4-thiadiazole, 1,2,4-thiadiazole, pyrazole, 1,2,4-triazole, pyridine, pyrazine, pyrimidine, pyridazine or 1,3,5-triazine;
R⁵ is a substituent connected with a carbon atom or a nitrogen atom of the ring A, and r is an integer of 0 to 3;
when R⁵ is a substitutent connected with a carbon atom of pyrrole, furan, thiophene, pyrazole, isothiazole, pyridine, pyrazine, pyrimidine, pyridazine or 1,3,5-triazine, R⁵ is a halogen atom, hydroxy group, nitro group, cyano group, carboxy group, a substituted or unsubstituted amino group, a substituted or unsubstituted hydroxyamino group, a substituted or unsubstituted carbamoyl group, a substituted or unsubstituted sulfamoyl group, -R⁸, -OR⁸, -CO₂R⁹, -SR¹⁰, -SO-R¹⁰, -SO₂-R¹⁰, -C(O)SR¹⁰, -C(S)OR¹⁰ or -CS₂R¹⁰,
wherein R⁸ is a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, a substituted or unsubstituted aryl group, a substituted or unsubstituted aralkyl group, a substituted or unsubstituted heterocyclic group or an acyl group;
R⁹ is a substituted or unsubstituted alkyl group, a substituted or unsubstituted alkenyl group, a substituted or unsubstituted alkynyl group, a substituted or unsubstituted aryl group, a substituted or unsubstituted aralkyl group, or a substituted or unsubstituted heterocyclic group; and
R¹⁰ is a substituted or unsubstituted alkyl group, a substituted or unsubstituted aryl group, or a substituted or unsubstituted aralkyl group;
when R⁵ is a substitutent connected with a nitrogen atom of pyrrole, pyrazole or 1,2,4-triazole, R⁵ is nitro group, cyano group, a substituted or unsubstituted carbamoyl group, a substituted or unsubstituted sulfamoyl group, a protective group for NH group, -R⁸, -OR⁸ or -CO₂R⁹, wherein R⁸ and R⁹ are as defined above;
L is a group of the following formula, which is connected with a carbon atom of the ring A: 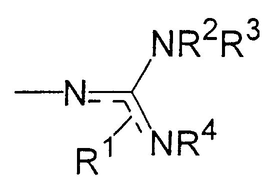 wherein one of the two broken lines is a double bond together with the solid line, while the other is a single bond together with the other solid line;
R¹ is bonded to the nitrogen atom bonded through the single bond represented by the broken line and the solid line; and
R¹, R², R³ and R⁴ are independently hydrogen atom, hydroxy group, nitro group, cyano group, a substituted or unsubstituted amino group, a substituted or unsubstituted hydroxyamino group, a substituted or unsubstituted carbamoyl group, a substituted or unsubstituted sulfamoyl group, a protecting group for NH group, -R⁸, -OR⁸, -CO₂R⁹, -SR¹⁰, -SO-R¹⁰, -SO₂-R¹⁰, -C(O)SR¹⁰, -C(S)OR¹⁰ or -CS₂R¹⁰, wherein R⁸, R⁹ and R¹⁰ are as defined above; or
any two of R¹, R², R³ and R⁴ may be taken together with one nitrogen atom or with two nitrogen atoms and one carbon atom to form a substituted or unsubstituted nitrogen-containing aliphatic heterocyclic ring ; or
any three of R¹, R², R³ and R⁴ may be taken together with two nitrogen atoms and one carbon atom to form a substituted or unsubstituted bicyclic nitrogen-containing aliphatic heterocyclic ring; or
the formula: -NR²R³ may be a group of the formula:
-N=C(NR⁴³R⁴⁴)NH₂ or -NH-C(NR⁴³R⁴⁴)=NH,
wherein R⁴³ and R⁴⁴ are as defined in (1) or (2),
(1) each is independently hydrogen atom, an acyl group, a substituted or unsubstituted alkyl group, or a protecting group for NH group,
(2) when taken together, they form with the nitrogen atom a substituted or unsbustituted 5- to 7-membered nitrogen-containing aliphatic heterocyclic group.

2. A heteroaromatic ring compound or a pharmaceutically acceptable salt thereof according to claim 1, wherein E is a group of the formula: 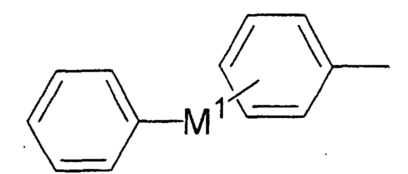 wherein M¹ is single bond, -CQ-, -CH(OR¹¹)-, -C(OR¹¹)₂-, ·C(=NOR¹¹)-, -C(=NR¹²)-, -C(=NNR¹³R¹⁴)-, -CO- or -CS-, wherein Q, R¹¹, R¹², R¹³ and R¹⁴ are as defined above;
wherein said E may be substituted by one to four members optionally selected from the group consisting of halogen atoms, lower alkyl groups, nitro group, formyl group, acetyl group, cyano group, -OR¹¹, -CO₂R²⁹ and -CONR³⁰R³¹, wherein R¹¹, R²⁹, R³⁰ and R³¹ are as defined above.

3. A heteroaromatic ring compound or a pharmaceutically acceptable salt thereof according to claim 1 or 2, wherein E is a group of the formula: 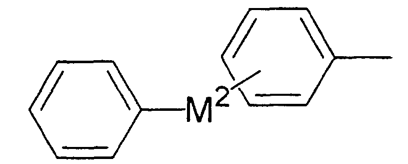 wherein M² is single bond or - CO-;
wherein said E may be substituted by one or two members optionally selected from the group consisting of halogen atoms, lower alkyl groups, acetyl group, cyano group and -OR¹¹, wherein R¹¹ is as defined above.

4. A heteroaromatic ring compound or a pharmaceutically acceptable salt thereof according to any one of claims 1 to 3, wherein E is biphenyl-4-yl, 2-fluorobiphenyl-4-yl, 2'-fluorobiphenyl-4-yl, 3-benzoylphenyl or 4-benzoylphenyl.

5. A heteroaromatic ring compound or a pharmaceutically acceptable salt thereof according to any one of claims 1 to 4, wherein G is -C(R²³R²⁴)- wherein R²³ and R²⁴ are independently hydrogen atom, a lower alkyl group or a lower alkoxy group; or R²³ and R²⁴ may be taken together with the carbon atom attached thereto to form a hydrocarbon ring of 3 to 6 carbon atoms, 1,3-dioxane ring, or 1,3-dioxolane ring.

6. A heteroaromatic ring compound or a pharmaceutically acceptable salt thereof according to any one of claims 1 to 5, wherein G is -C(R⁴⁹R⁵⁰)- wherein R⁴⁹ and R⁵⁰ are independently hydrogen atom or methyl group; or R⁴⁹ and R⁵⁰ may be taken together with the carbon atom to form cyclopropane.

7. A heteroaromatic ring compound or a pharmaceutically acceptable salt thereof according to any one of claims 1 to 6, wherein A is pyrrole, furan, thiophene, isothiazole, 1,3,4-oxadiazole, 1,3,4-thiadiazole, 1,2,4-thiadiazole, pyrazole, 1,2,4-triazole, pyridine, pyrazine, pyrimidine, pyridazine or 1,3,5-triazine.

8. A heteroaromatic ring compound or a pharmaceutically acceptable salt thereof according to any one of claims 1 to 7, wherein A is a group of the formulae: 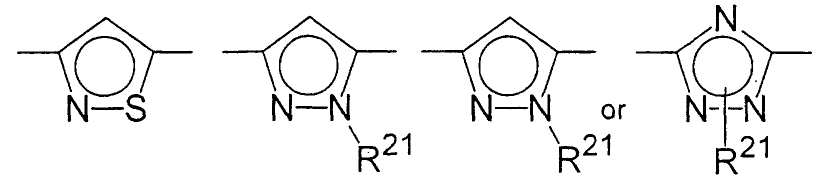 wherein R²¹ is a substituent connected with a nitrogen atom and is hydrogen atom, a lower alkyl group or acetyl group.

9. A heteroaromatic ring compound or a pharmaceutically acceptable salt thereof according to any one of claims 1 to 8, wherein L is a group selected from the groups of the formulae [1] to [6]: 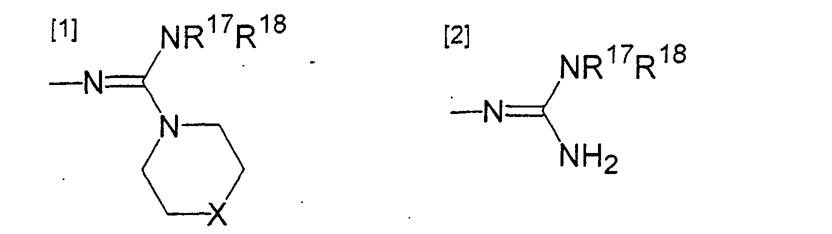 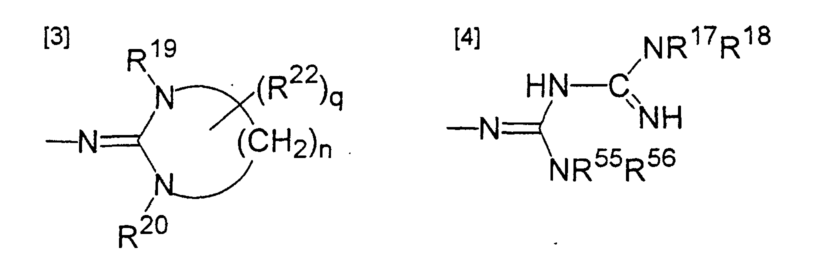 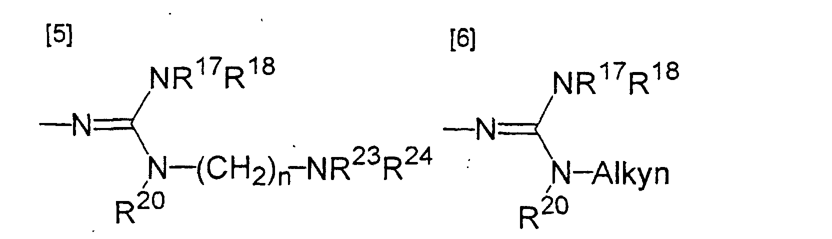 wherein,
in the formulae [1] and [2], X is -CH₂-, -O-, -S- or -SO₂-; R¹⁷ and R¹⁸ are independently hydrogen atom, a substituted or unsubstituted alkyl group or cyano group; or R¹⁷ and R¹⁸ may be taken together with the nitrogen atom to form a substituted or unsubstituted 5- to 7-membered nitrogen-containing aliphatic heterocyclic ring;
in the formula [3], R¹⁹ and R²⁰ are independently hydrogen atom or a substituted or unsubstituted alkyl group; R²² is a substituted or unsubstituted alkyl group, a substituted or unsubstituted alkoxy group, a substituted or unsubstituted amino group or hydroxy group; q is an integer of 0 to 4; n is an integer of 2 to 4; or R²² and R¹⁹ may be taken together with two nitrogen atoms and two carbon atoms to form a substituted or unsubstituted 8- to 11-membered bicyclic nitrogen-containing aliphatic heterocyclic ring;
in the formula [4], R¹⁷ and R¹⁸ are as defined above; R⁵⁵ and R⁵⁶ are independently hydrogen atom or a substituted or unsubstituted alkyl group; or R⁵⁵ and R⁵⁶ may be taken together with the nitrogen atom to form a substituted or unsubstituted 5- to 7-membered nitrogen-containing aliphatic heterocyclic ring;
in the formula [5], R¹⁷, R¹⁸, R²⁰ and n are as defined above; R²³ and R²⁴ are independently hydrogen atom or a substituted, unsubstituted alkyl group, an acyl group, a substituted or unsubstituted carbamoyl group or -SO₂-R¹⁰ wherein R¹⁰ is as defined above; or R²³ and R²⁴ may be taken together with the nitrogen atom to form a substituted or unsubstituted 5- to 7-membered nitrogen-containing aliphatic heterocyclic ring;
in the formula [6] R¹⁷, R¹⁸ and R²⁰ are as defined above; Alkyn is an alkynyl group.

10. A heteroaromatic ring compound or a pharmaceutically acceptable salt thereof according to any one of claims 1 to 9, wherein L is a group selected from the groups of the formulae [1] or [3]: 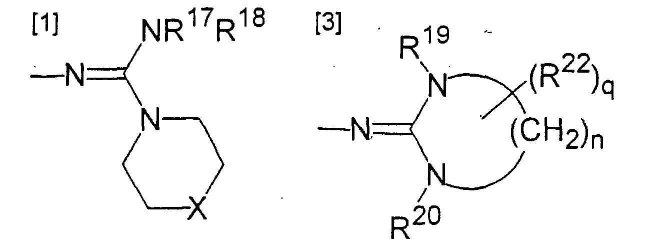 wherein, in the formulae [1] and [3], X, R¹⁷, R¹⁸, R¹⁹, R²⁰, R²², q and n are as defined above.

11. A heteroaromatic ring compound represented by the following formula or a pharmaceutically acceptable salt thereof: 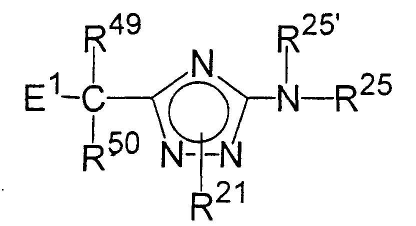 wherein E¹ is a group of the formula: 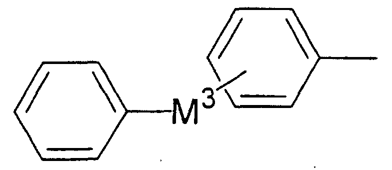 wherein M³ is single bond, -CQ or -CO-, wherein Q is as defined above;
wherein said E¹ may be substituted by one or two halogen atom(s) or lower alkyl group(s); R²¹, R⁴⁹ and R⁵⁰ are as defined above; R²⁵ and R²⁵' are independently hydrogen atom or a lower alkyl group.

12. A pharmaceutical composition comprising as an active ingredient a heteroaromatic ring compound or a pharmaceutically acceptable salt thereof according to any one of claims 1 to 11.

13. A pharmaceutical composition according to claim 12, which is a drug for the treatment or prophylaxis of autoimmune diseases or inflammatory diseases.

14. A pharmaceutical composition according to claim 12, which is an anti-rheumatic drug or an anti-inflammatory drug.
